# EUROPEAN PATENT SPECIFICATION

(11) **EP 2 683 419 B1**
(45) Date of publication and mention of the grant of the patent: **09.05.2018**
(21) Application number: 12707813.7
(22) Date of filing: 12.03.2012
(51) Int. Cl.: A61L 27/00, C07K 14/00, A61L 31/08, A61L 31/16, C07K 14/40

(54) **MOLECULES AND METHODS FOR INHIBITION AND DETECTION OF PROTEINS**
MOLEKÜLE UND VERFAHREN FÜR HEMMUNG UND NACHWEIS VON PROTEINEN
MOLÉCULES ET PROCÉDÉS D'INHIBITION ET DE DÉTECTION DE PROTÉINES

(30) Priority: 11.03.2011 EP 11157842; 11.03.2011 US 201161451855 P; 18.05.2011 US 201161487595 P; 05.08.2011 EP 11176725
(43) Date of publication of application: 15.01.2014
(73) Proprietor: VIB VZW, 9052 Gent (BE); Vrije Universiteit Brussel, 1050 Brussel (BE); Katholieke Universiteit Leuven K.U. Leuven R&D, 3000 Leuven (BE); Universiteit Gent, 9000 Gent (BE)
(72) Inventor: SCHYMKOWITZ, Joost, B-3391 Meensel-Kiezegem (BE); ROUSSEAU, Frederic, B-1702 Groot-Bijgaarden (BE)
(74) Representative: Michalík, Andrej
(86) International application number: PCT/EP2012/054285
(87) International publication number: WO 2012/123419

(56) References cited:
- WO-A1-2007/071789
- WO-A1-2010/076642
- US-A1- 2010 184 681
- RAMESH RATHINAKUMAR ET AL: "Broad-Spectrum Antimicrobial Peptides by Rational Combinatorial Design and High-Throughput Screening: The Importance of Interfacial Activity", JOURNAL OF THE AMERICAN CHEMICAL SOCIETY, vol. 131, no. 22, 10 June 2009 (2009-06-10), pages 7609-7617, XP055012553, ISSN: 0002-7863, DOI: 10.1021/ja8093247
- KUHN DUNCAN M ET AL: "Candida biofilms: antifungal resistance and emerging therapeutic options.", CURRENT OPINION IN INVESTIGATIONAL DRUGS (LONDON, ENGLAND : 2000) FEB 2004 LNKD- PUBMED:15043393, vol. 5, no. 2, February 2004 (2004-02), pages 186-197, XP009154257, ISSN: 1472-4472
- A. T. FRANK ET AL: "Structure and Function of Glycosylated Tandem Repeats from Candida albicans Als Adhesins", EUKARYOTIC CELL, vol. 9, no. 3, 1 March 2010 (2010-03-01), pages 405-414, XP055009166, ISSN: 1535-9778, DOI: 10.1128/EC.00235-09
- STEFAN AUER ET AL: "Self-Templated Nucleation in Peptide and Protein Aggregation", PHYSICAL REVIEW LETTERS, vol. 101, no. 25, 1 December 2008 (2008-12-01), pages 258101-258101, XP055008913, ISSN: 0031-9007, DOI: 10.1103/PhysRevLett.101.258101
- H. N. OTOO ET AL: "Candida albicans Als Adhesins Have Conserved Amyloid-Forming Sequences", EUKARYOTIC CELL, vol. 7, no. 5, 1 May 2008 (2008-05-01), pages 776-782, XP055009164, ISSN: 1535-9778, DOI: 10.1128/EC.00309-07
- KLOTZ STEPHEN A ET AL: "Inhibition of adherence and killing of Candida albicans with a 23-mer peptide (Fn/23) with dual antifungal properties", ANTIMICROBIAL AGENTS AND CHEMOTHERAPY, AMERICAN SOCIETY FOR MICROBIOLOGY, WASHINGTON, DC, US, vol. 48, no. 11, 1 November 2004 (2004-11-01), pages 4337-4341, XP002542304, ISSN: 0066-4804, DOI: 10.1128/AAC.48.11.4337-4341.2004
- BISHWAJIT KUNDU ET AL: "Manipulation of Unfolding-Induced Protein Aggregation by Peptides Selected for Aggregate-Binding Ability through Phage Display Library Screening", BIOCHEMICAL AND BIOPHYSICAL RESEARCH COMMUNICATIONS, vol. 291, no. 4, 1 March 2002 (2002-03-01) , pages 903-907, XP055008914, ISSN: 0006-291X, DOI: 10.1006/bbrc.2002.6549
- LOIS L. HOYER ET AL: "Discovering the secrets of the Candida albicans agglutinin-like sequence (ALS) gene family - a sticky pursuit", MEDICAL MYCOLOGY, vol. 46, no. 1, 1 January 2008 (2008-01-01), pages 1-15, XP055008957, ISSN: 1369-3786, DOI: 10.1080/13693780701435317
- Y. LIU ET AL: "Candida albicans Als3, a Multifunctional Adhesin and Invasin", EUKARYOTIC CELL, vol. 10, no. 2, 29 November 2010 (2010-11-29), pages 168-173, XP055009182, ISSN: 1535-9778, DOI: 10.1128/EC.00279-10
- N. V. ARTEMOVA ET AL: "Acceleration of protein aggregation by amphiphilic peptides: Transformation of supramolecular structure of the aggregates", BIOTECHNOLOGY PROGRESS, vol. 27, no. 3, 1 March 2011 (2011-03-01), - 1 May 2010 (2010-05-01), pages 846-854, XP055008915, ISSN: 8756-7938, DOI: 10.1002/btpr.574
- ANA-MARIA FERNANDEZ-ESCAMILLA ET AL: "Prediction of sequence-dependent and mutational effects on the aggregation of peptides and proteins", NATURE BIOTECHNOLOGY, vol. 22, no. 10, 1 October 2004 (2004-10-01), pages 1302-1306, XP055012264, ISSN: 1087-0156, DOI: 10.1038/nbt1012
- AMY J. KARLSSON ET AL: "Polyelectrolyte Multilayers Fabricated from Antifungal [beta]-Peptides: Design of Surfaces that Exhibit Antifungal Activity Against Candida albicans", BIOMACROMOLECULES, vol. 11, no. 9, 13 September 2010 (2010-09-13), pages 2321-2328, XP055012518, ISSN: 1525-7797, DOI: 10.1021/bm100424s
- AMY J. KARLSSON ET AL: "Effect of Sequence and Structural Properties on 14-Helical [beta]-Peptide Activity against Candida albicans Planktonic Cells and Biofilms", ACS CHEMICAL BIOLOGY, vol. 4, no. 7, 17 July 2009 (2009-07-17), pages 567-579, XP055012521, ISSN: 1554-8929, DOI: 10.1021/cb900093r
- CHITI F ET AL: "RATIONALIZATION OF THE EFFECTS OF MUTATIONS ON PEPTIDE AND PROTEIN AGGREGATION RATES", NATURE, NATURE PUBLISHING GROUP, LONDON, GB, vol. 424, 14 August 2003 (2003-08-14), pages 805-808, XP001174166, ISSN: 0028-0836, DOI: 10.1038/NATURE01891
- LILIAN S. AMER ET AL: "Antimicrobial and antibiofilm activity of cathelicidins and short, synthetic peptides against Francisella", BIOCHEMICAL AND BIOPHYSICAL RESEARCH COMMUNICATIONS, vol. 396, no. 2, 1 May 2010 (2010-05-01), pages 246-251, XP55030359, ISSN: 0006-291X, DOI: 10.1016/j.bbrc.2010.04.073
- GIOVANNA BATONI ET AL: "Use of Antimicrobial Peptides Against Microbial Biofilms: Advantages and Limits", CURRENT MEDICINAL CHEMISTRY, vol. 18, no. 2, 1 January 2011 (2011-01-01), pages 256-279, XP55030366, ISSN: 0929-8673, DOI: 10.2174/092986711794088399
- C. B. RAMSOOK ET AL: "Yeast Cell Adhesion Molecules Have Functional Amyloid-Forming Sequences", EUKARYOTIC CELL, vol. 9, no. 3, 1 March 2010 (2010-03-01), pages 393-404, XP55031107, ISSN: 1535-9778, DOI: 10.1128/EC.00068-09
- Joke Reumers ET AL: "Protein sequences encode safeguards against aggregation", Human Mutation, vol. 30, no. 3, 1 March 2009 (2009-03-01), pages 431-437, XP055064584, ISSN: 1059-7794, DOI: 10.1002/humu.20905
- ROUSSEAU F ET AL: "How Evolutionary Pressure Against Protein Aggregation Shaped Chaperone Specificity", JOURNAL OF MOLECULAR BIOLOGY, ACADEMIC PRESS, UNITED KINGDOM, vol. 355, no. 5, 3 February 2006 (2006-02-03), pages 1037-1047, XP024950630, ISSN: 0022-2836, DOI: 10.1016/J.JMB.2005.11.035 [retrieved on 2006-02-03]

## Description

### Field of the invention

The present application belongs to the field of functional peptides and more particularly to the field of controlled protein aggregation. The application discloses molecules of a peptide structure as defined in the claims and specification and methods of using such molecules for therapeutic applications and for diagnostic uses, as well as in other applications such as in the agbio field and in industrial biotechnology. The molecules can be used for curing and/or stabilizing infections such as bacterial, fungal and viral diseases, but are also useful in non-infectious human and veterinary diseases. The molecules can also be used for the detection of protein biomarkers and for the prognosis and diagnosis of a variety of diseases.

### Background

Protein aggregation is caused by the misfolding and subsequent agglutination of proteins in insoluble agglomerates. Protein aggregation is essentially a self-association process in which many identical protein molecules form higher order conglomerates of low solubility that eventually precipitate. On the basis of their macroscopic morphology, they are generally classified as either ordered or disordered aggregates. Under physiological conditions, almost any protein can be induced at high concentration to form amorphous aggregates; under the same conditions, a much smaller set of proteins form highly ordered β-rich amyloid fibers. However, on a microscopic level, the differentiation between these two types of aggregates is more subtle. Amorphous aggregates are not just clusters of misfolded proteins that stick to each other through non-specific hydrophobic contacts. Rather, they are also often enriched in cross-β structure and their formation propensity correlates not only with hydrophobicity, but also with secondary structure propensity and charge, suggesting a specific mechanism of formation (Chiti et al., PNAS 99:16419-16426 (2002); Chiti et al., Nature 424:805-808 (2003); Chiti et al., Nat Struct Biol 9:137-143 (2002)). On the other hand, not all reported aggregates and fibers are enriched in β-structure, as both amorphous aggregates and fibers have been reported that retain native-like spectral properties and even enzymatic activity. In these cases, aggregation is proposed to occur by other mechanisms of oligomerization, such as three-dimensional domain swapping (Rousseau et al., PNAS 98: 5596-5601, 2001; Liu and Eisenberg, Protein Sci 11: 1285-1299, 2002) as is often seen in protein dimers. The focus on protein aggregation is, for a large part, inspired by the observation that a range of human diseases are characterized by protein deposits composed of one or a very limited number of proteins.
Examples of such diseases where conversion of normally soluble proteins into conformationally altered insoluble proteins is known to be of causal relevance are for example the occurrence of amyloid beta peptide in Alzheimer's disease and cerebral amyloid angiopathy, α-synuclein deposits in Lewy bodies of Parkinson's disease, prions in Creutzfeldt-Jacob disease, superoxide dismutase in amyotrophic lateral sclerosis and tau in neurofibrillary tangles in frontal temporal dementia and Pick's disease. Thus far, protein aggregation has mainly been studied as an unwanted, disease-causing phenomenon and it is now widely accepted that cross-beta mediated aggregation is the most frequently occurring and biologically relevant mechanism of aggregation. Although protein aggregation has long been considered to be a disordered process mediated by non-specific hydrophobic interactions, it is now clear that particularly amyloid aggregation is in many instances essentially a specific self-association process. Aggregates formed both *in vitro* and *in vivo* are generally enriched in one particular protein and although aggregation is a spontaneous process *in vitro,* in the cellular environment this process is actively controlled by chaperones. The most common mechanism by which misfolded proteins aggregate consists in the self-association of specific polypeptide segments from identical proteins into a growing intermolecular beta sheet (e.g. Makin et al., PNAS 102(2): 315-20 (2005); Sawaya et al., Nature 447(7143):453-7 (2007)). These aggregation-nucleating segments are generally short, consisting of 5-15 residues, and can be accurately predicted using available biophysical algorithms. There is now abundant data to show that the individual strands interact to form an intermolecular beta sheet and that this structure forms the backbone of the aggregate. Aggregating sequences are very common in globular proteins, and occur with about the same frequency in α, β, α+β and α/β proteins (SCOP classification: Lo Conte et al., Nucleic Acids Res 28:257-259 (2000)) (Linding et al., J Mol Biol 342: 345-353 (2004)). These short aggregation-prone stretches are sufficient to induce aggregation of a protein, as shown by grafting experiments which demonstrated that transplanting an aggregation-nucleating segment from an aggregating protein on a non-aggregating one transfers both aggregation propensity and aggregate structure from the former to the latter (Esteras-Chopo et al., PNAS 102: 16672-16677, 2005).
It can be considered that aggregation-sensitive protein sequences are the price to be paid for the existence of globular protein structures: as tertiary sidechain interactions mainly occur in the hydrophobic core, protein stretches spanning this region generally have a propensity to aggregate. However, for native globular proteins, aggregation is generally not an issue, as aggregation-prone protein stretches are generally sequestered by the protein structure and thereby protected from self-association. On the other hand, during protein translation and folding, or in the case of cellular stress or destabilizing mutations, partially unfolded states are much more likely to self-associate and induce aggregation and amyloidosis.

Since most proteins harbor aggregation-prone peptide sequences within their primary structure, and since aggregation is sequence specific, it was previously successfully shown that it was possible to develop a general strategy for the specific induction of aggregation of a chosen target protein (see WO2007071789). In the latter method a target protein was exposed to a carrier displaying a short target-specific, aggregation-prone peptide (i.e. a beta-aggregating region derived from a chosen target protein; it was surprisingly demonstrated that exposure to a short aggregating nucleating region taken from the protein is a sufficient condition for aggregation). This carrier (designated as a solubilizing moiety see e.g. Figure 1 and its legend in WO2007071789) was essential for preventing the aggregation - and hence also the stability - of the β-aggregating region before this region was exposed to the target.

It would be advantageous to provide further, improved, interferor molecules that do not require a carrier or solubilizing moiety to remain in solution and still succeed in inhibiting the function of proteins by co-aggregation. Such molecules would be easier to synthesize or produce. Moreover, it would be advantageous to accurately define the structural determinants that allow molecules to on the one hand remain soluble as such, while on the other hand being capable of inducing aggregation of a target protein. Also, molecules that are capable of inducing stable intermolecular beta-aggregate formation with favorable kinetics could be very useful for diagnostic and therapeutic applications ('red' biotechnology), as well as in agro-biotech applications ('green' biotechnology), applications for marine and freshwater organisms ('blue' biotechnology), industrial ('white') biotechnology or for research use.

### Summary

The present invention provides improved molecules (herein further designated as interferor molecules) for causing aggregation of selected proteins upon contact. These improved interferor molecules do not require the presence of a solubilizing moiety anymore while retaining the properties of stability (i.e. to prevent premature aggregation) and while still being able to cause co-aggregation with a target protein. With premature aggregation, it is meant that the molecules aggregate with themselves in such a way that they cannot achieve aggregation or inhibition of a target protein. Surprisingly, it was found that aggregation-inducing sequences which are flanked by sequences or residues with a low beta-sheet forming potential (i.e., aggregation-breaking residues) are not only more soluble but retain at the same time extremely efficient and also specific aggregation-inducing properties. The molecules described herein may have more than one aggregation-inducing region, which are then each flanked by aggregation-breaking residues - most particularly, the aggregation-inducing regions are separated by a linker. If the targeted proteins are biologically active or functional, inducing aggregation will typically result in functional inhibition of the protein. As will be apparent from the appended examples the improved interferors of the invention have important therapeutic and diagnostic applications, as well as applications in agbio, white biotech and as a research tool.

Accordingly, the present invention provides subject-matter as set forth in any one and all of (1) to (32) below:
(1) A method for inducing aggregation of a protein comprising contacting said protein with a molecule of the following structure: (X₂ᵢ₋₁-Yᵢ-X₂ᵢ-Zᵢ)ₙ, wherein:
   - n is an integer from 1 to 5 and i increases from 1 to n with each repeat;
   - each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids occurring in said protein; and
   - each Zᵢ is a linker and Zₙ is independently selected from a linker or nothing;
   wherein the molecule is capable of inducing aggregation of said protein;
   and wherein the method is not practiced on the human or animal body.
(2) A method for down-regulating the biological function of a protein comprising contacting said protein with a molecule of the following structure: (X₂ᵢ₋₁-Yᵢ-X₂ᵢ-Zᵢ)ₙ, wherein:
   - n is an integer from 1 to 5 and i increases from 1 to n with each repeat;
   - each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in said protein; and
   - each Zᵢ is a linker and Zₙ is independently selected from a linker or nothing;
   wherein the molecule is capable of down-regulating the biological function of said protein;
   and wherein the method is not practiced on the human or animal body.
(3) A molecule of the following structure: (X₂ᵢ₋₁-Yᵢ-X₂ᵢ-Zᵢ)ₙ, wherein:
   - n is 1 to 5 and i increases from 1 to n with each repeat;
   - each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein; and
   - each Zᵢ is a linker and Zₙ is independently selected from a linker or nothing;
   wherein the molecule is capable of binding to, inducing aggregation of or down-regulating the biological function of said protein and wherein binding of the molecule to said protein, aggregation of said protein or downregulation of the biological function of said protein in a subject is therapeutically useful;
   for use as a medicament.
(4) The molecule of (3), wherein the expression or overexpression of said protein is associated with or causative of a disease, for use in treatment or prevention of said disease.
(5) The molecule of (3), wherein binding of the molecule to said protein, aggregation of said protein or downregulation of the biological function of said protein in a subject is therapeutically useful in cancer, such as wherein the expression or overexpression of said protein is associated with or causative of cancer, for use in treatment of cancer.
(6) The molecule of (3), wherein binding of the molecule to said protein, aggregation of said protein or downregulation of the biological function of said protein in a subject is therapeutically useful in AMD, such as wherein the expression or overexpression of said protein is associated with or causative of AMD, for use in treatment of AMD.
(7) The molecule of (3), wherein binding of the molecule to said protein, aggregation of said protein or downregulation of the biological function of said protein in a subject is therapeutically useful in inflammatory disease, such as wherein the expression or overexpression of said protein is associated with or causative of inflammatory disease, for use in treatment of inflammatory disease.
(8) A molecule of the following structure: (X₂ᵢ₋₁-Yᵢ-X₂ᵢ-Zᵢ)ₙ, wherein:
   - n is 1 to 5 and i increases from 1 to n with each repeat;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of a pathogenic organism; and
   - each Zᵢ is a linker and Zₙ is independently selected from a linker or nothing;
   wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism;
   for use as an antipathogenic compound in treatment of an infectious disease caused by the pathogenic organism.
(9) A method for killing a pathogenic organism or inhibiting the growth and/or reproduction of a pathogenic organism comprising contacting the pathogenic organism with a molecule of the following structure: (X₂ᵢ₋₁-Yᵢ-X₂ᵢ-Zᵢ)ₙ, wherein:
   - n is 1 to 5 and i increases from 1 to n with each repeat;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of the pathogenic organism; and
   - each Zᵢ is a linker and Zₙ is independently selected from a linker or nothing;
   wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism;
   and wherein the method is not practiced on the human or animal body.
(10) The molecule for use of (8) or the method of (9), wherein the pathogen is a viral organism.
(11) The molecule for use of (8) or the method of (9), wherein the pathogen is a microbial organism selected from Gram-positive bacteria, Gram-negative bacteria, mycobacteria, fungi, yeasts and moulds.
(12) The molecule for use of any one of (3)-(8) or the method of (9), wherein the molecule is provided on an implantable device at least partly coated with the molecule.
(13) An implantable device at least partly coated with molecules of the structure (X₂ᵢ₋₁-Yᵢ-X₂ᵢ-Zᵢ)ₙ, wherein:
   - n is an integer from 1 to 5 and i increases from 1 to n with each repeat;
   - each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of a pathogenic organism; and
   - each Zᵢ is a linker and Zₙ is independently selected from a linker or nothing;
   wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism.
(14) A method to screen for new inhibitory and/or detection compounds, comprising the steps of:
   a) identifying in at least one protein at least one region of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
   b) synthesizing a molecule of the following structure: (X₂ᵢ₋₁-Yᵢ-X₂ᵢ-Zᵢ)ₙ, wherein:
      - n is 1 to 5 and i increases from 1 to n with each repeat;
      - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
      - each Yᵢ is a beta-aggregating sequence independently selected from 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein identified in step a); and
      - each Zᵢ is a linker and Zₙ is independently selected from a linker or nothing;
   c) bringing the molecule made in step b) in contact with the protein of step a); and
   d) assessing the function and/or aggregation of the protein.
(15) A method to identify new targets for inhibitory compounds, comprising the method of (14), wherein the protein in step a) is not a known target for inhibitory compounds.
(16) A method to detect a protein in a sample, comprising the steps of:
   a) contacting a sample suspected of containing the protein with a molecule of the following structure: (X₂ᵢ₋₁-Yᵢ-X₂ᵢ₋Zᵢ)ₙ, wherein:
      - n is 1 to 5 and i increases from 1 to n with each repeat;
      - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
      - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein to be detected; and
      - each Zᵢ is a linker and Zₙ is independently selected from a linker or nothing;
      wherein the molecule is capable of binding to, inducing aggregation of or down-regulating the biological function of said protein;
   b) detecting the presence of the molecules reacted with the protein.
(17) The method of (16), wherein the at least one Yᵢ which is identical to or differs with no more than one amino acid substitution from the contiguous stretch of amino acids naturally occurring in the protein to be detected is unique to said protein in said sample.
(18) The method of (16) or (17), wherein the molecule comprises a detectable label, and the detecting in step b) is through detection of the detectable label.
(19) The method of any one of (16) to (18), wherein the molecule is provided on a solid support.
(20) The method of (19), wherein at least two different molecules capable of binding to, inducing aggregation of or down-regulating the biological function of two different proteins to be detected are provided on the solid support.
(21) An *in vitro* method for diagnosis of a disease status comprising detecting a protein which is indicative of the disease status in a sample from a subject, wherein the protein is detected by a method comprising the steps of:
   a) contacting a sample suspected of containing the protein with a molecule of the following structure: (X₂ᵢ₋₁-Yᵢ-X₂ᵢ₋Zᵢ)ₙ, wherein:
      - n is 1 to 5 and i increases from 1 to n with each repeat;
      - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
      - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein to be detected; and
      - each Zᵢ is a linker and Zₙ is independently selected from a linker or nothing;
      wherein the molecule is capable of binding to, inducing aggregation of or down-regulating the biological function of the protein to be detected;
   b) detecting the presence of the molecules reacted with the protein.
(22) A method for down-regulating the biological function of a protein in a plant or plant cell or plant seed, comprising contacting said protein with a molecule of the following structure: (X₂ᵢ₋₁-Yᵢ-X₂ᵢ-Zᵢ)ₙ, wherein:
   - n is an integer from 1 to 5 and i increases from 1 to n with each repeat;
   - each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
   - each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in said protein in said plant, plant cell or plant seed; and
   - each Zᵢ is a linker and Zₙ is independently selected from a linker or nothing;
   wherein the molecule is capable of down-regulating the biological function of said protein.
(23) The method according to (22), wherein the molecule is a polypeptide encoded by a nucleotide sequence present on a recombinant vector and which, upon introduction into the plant cell, plant seed or plant, produces said polypeptide in said plant cell, plant seed or plant.
(24) The method of any one of (1)-(2), (9)-(12), (14)-(20), or (21)-(23), the molecule for use of any one of (3)-(8), or (10)-(12), or the implantable device of (13), wherein each X₂ᵢ₋₁ and X₂ᵢ are 1 or 2 amino acids.
(25) The method of any one of (1)-(2), (9)-(12), (14)-(20), (21)-(24), the molecule for use of any one of (3)-(8), (10)-(12), or (24), or the implantable device of (13) or (24), wherein at least one, and most particularly all, Yᵢ is a stretch of 6 to 13 amino acids, particularly of 6 to 9 amino acids, more particularly 6 or 7 amino acids.
(26) The method of any one of (1)-(2), (9)-(12), (14)-(20), (21)-(25), the molecule for use of any one of (3)-(8), (10)-(12), or (24)-(25), or the implantable device of (13) or (24)-(25), wherein at least two, and particularly every, Yᵢ are each independently identical to or differ with no more than one amino acid substitution from a stretch of 6 to 16 contiguous amino acids naturally occurring in a protein.
(27) The method of any one of (1)-(2), (9)-(12), (14)-(20), (21)-(26), the molecule for use of any one of (3)-(8), (10)-(12), or (24)-(26), or the implantable device of (13) or (24)-(26), wherein each Zᵢ is independently selected from stretch of between 0 and 20 units, wherein a unit is an amino acid, a monosaccharide, a nucleotide, trioxatridecan-succinic acid (Ttds) or PEG.
(28) The method of any one of (1)-(2), (9)-(12), (14)-(20), (21)-(26), the molecule for use of any one of (3)-(8), (10)-(12), or (24)-(26), or the implantable device of (13) or (24)-(26), wherein each Zᵢ is independently selected from stretch of between 0 and 20 units, wherein a unit is an amino acid.
(29) The method of any one of (1)-(2), (9)-(12), (14)-(20), (21)-(26), the molecule for use of any one of (3)-(8), (10)-(12), or (24)-(26), or the implantable device of (13) or (24)-(26), wherein each Zᵢ is independently selected from stretch of between 0 and 10 units, wherein a unit is an amino acid or PEG.
(30) The method of any one of (1)-(2), (9)-(12), (14)-(20), (21)-(29), the molecule for use of any one of (3)-(8), (10)-(12), or (24)-(29), or the implantable device of (13) or (24)-(29), wherein n is 1, X₁ and X₂ are in total no more than 5 amino acids, Y₁ is a stretch of between 6 and 10 amino acids and Z₁ is a stretch of 0 units.
(31) The method of any one of (1)-(2), (9)-(12), (14)-(20), (21)-(29), the molecule for use of any one of (3)-(8), (10)-(12), or (24)-(29), or the implantable device of (13) or (24)-(29), wherein n is 2, Z₁ is a linker and Z₂ is nothing.
(32) The method of any one of (1)-(2), (9)-(12), (14)-(20), (21)-(31), the molecule for use of any one of (3)-(8), (10)-(12), or (24)-(31), or the implantable device of (13) or (24)-(31), further comprising a moiety that increases solubility of the molecule.

The subject-matter provided by the invention specifically belongs to the disclosure, description and teaching of the present specification.

The specification describes molecules (which include molecules in accordance with the aspects and embodiments of the invention) that have the following structure:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing.

Particularly for molecules where n=1, additional limitations may apply. For instance, a set of limitations that is envisaged for molecules where n is 1, is as follows:
- X₁ and X₂ are 1 or 2 amino acids selected from R, K, E, D and P; and
- Y₁ is a stretch of 6 to 11 contiguous amino acids,
   - at least 75% of which are hydrophobic amino acids,
   - in which at least 50% of the amino acids are aliphatic or F residues,
   - in which no P, R, K, D, E or H residue is present,
   - in which no more than one C, M, N, Q, W, G, S, A or T residue is present,
   - in which no more than 3 Y or F residues are present,
   - in which no two contiguous identical non-aliphatic residues are present
   - in which no more than 2 contiguous identical aliphatic residues are present,
   - in which no two consecutive non-aromatic polar residues are present,
   - wherein no more than 50% identical residues are present,
   - wherein the 1^{st} and/or last residue is an aliphatic or F residue,
   - wherein the sum of A and G residues is no more than 2,
   - wherein the total percentage of A, G and S residues is no more than 25%,
   - wherein the total percentage of C, M, N, Q and W residues is no more than 25%,
   - and wherein the total percentage of small residues other than V (i.e. selected from A, C, G, S, N, T) is no more than 25%.

It will be understood by the skilled person that, since Zᵢ is a linker between the individual X₂ᵢ₋₁-Yᵢ-X₂ᵢ units and Zₙ is an optional linker at one end of the molecule, the formula (X₂ᵢ₋₁-Yᵢ-X₂ᵢ-Zᵢ)ₙ is equivalent to the formula (Zᵢ-X₂ᵢ₋₁-Yᵢ-X₂ᵢ)ₙ, wherein each Z₂ to Zₙ is a linker, and Z₁ is independently selected from a linker or nothing. Instead of stating that Zₙ (or the equivalent N-terminal Z₁) is nothing, it can also be said that this moiety is absent - i.e., Zₙ is either a linker or absent (or in full: each Zi is an independently selected linker, and Zₙ (or the equivalent N-terminal Z₁) is a linker or absent.

An alternative formula could thus also be: (X₂ᵢ₋₁-Yᵢ-X₂ᵢ)ₙ - with X₂ᵢ₋₁ and X₂ᵢ, Yᵢ, i and n as defined above - wherein the n units are fused together with independently selected linkers. The molecules may further N- and/or C-terminally comprise an additional linker. According to this formula, the linkers are present between the X₂ᵢ moiety of the i-th unit and the X₂ᵢ₋₁ moiety of the subsequent (i+1)-th unit, and there is a total of (n-1) linkers in the molecule (i.e., Zᵢ to Zₙ₋₁ or Z₂ to Zₙ); the last linker (Zₙ or Z₁ respectively) is either a linker or absent. Yet another way of rephrasing the formula is Z₀-(X₂ᵢ₋₁-Yᵢ-X₂ᵢ-Zᵢ)ₙ, wherein X₂ᵢ₋₁ and X₂ᵢ, Yᵢ, i and n are as defined above, each Zᵢ is a linker and both Z₀ and Zₙ are independently selected from a linker or nothing (i.e., the molecules have either a C- or N-terminal linker, none or both).

Although the molecules typically consist of the structure described above, it is also envisaged that molecules consist essentially of that structure. By this, it is meant that, according to particular embodiments, the molecules can N- or C-terminally contain further amino acids (i.e. are N- or C-terminally fused to further amino acids), particularly 1 to 10 amino acids, more particularly 1 to 5 amino acids. Such additional amino acids are particularly envisaged for embodiments where n is at least two. The additional amino acids particularly have no specific profile, i.e. they are not a hydrophobic stretch such as the Yᵢ moiet(y)(ies) - in other words, they contain less than 50% hydrophobic residues - or they are not just selected from the residues that make up a numbered X moiety. However, in some instances, particularly where an X moiety does not contain charged amino acids, it is envisaged that this is additionally flanked with one or two hydrophobic amino acids (on the side of the X moiety that does not flank the Y moiety). This is particularly the case where the (non-charged) X moiety and the hydrophobic amino acids are identical to the protein sequence flanking the sequence corresponding to the Yᵢ moiety in the protein; in other words, where the part in the molecule corresponding in sequence to the protein sequence comprises at least one X moiety in addition to the Yᵢ moiety. Most particularly however, the molecules end both N- and C-terminally with an X moiety.

As mentioned, in the above formula n is an integer from 1 to 5 and i increases from 1 to n with each repeat. In other words, i starts at 1 and is increased with 1 with each repeat until n is reached; or i is the number of the repeat (and is an integer from 1 to n).

The formula thus encompasses the following structures:
X₁-Y₁-X₂-Z₁ (i.e., n=1),
X₁-Y₁-X₂-Z₁-X₃-Y₂-X₄-Z₂ (i.e., n=2),
X₁-Y₁-X₂-Z₁-X₃-Y₂-X₄-Z₂-X₅-Y₃-X₆-Z₃ (i.e., n=3),
X₁-Y₁-X₂-Z₁-X₃-Y₂-X₄-Z₂-X₅-Y₃-X₆-Z₃-X₇-Y₄-X₈-Z₄ (i.e., n=4), and
X₁-Y₁-X₂-Z₁-X₃-Y₂-X₄-Z₂-X₅-Y₃-X₆-Z₃-X₇-Y₄-X₈-Z₄-X₉-Y₅-X₁₀-Z₅ (i.e., n=5), wherein each numbered X, Y and Z are as defined above.
It should be noted that the molecules are linear, to make sure the Yᵢ moieties can be brought into contact with proteins to be targeted. Branched linear molecules (where at least two repeating X₂ᵢ₋₁-Yᵢ-X₂ᵢ units are independently attached through a linker to another X₂ᵢ₋₁-Yᵢ-X₂ᵢ unit) and cyclic molecules are only envisaged insofar the Yi moieties are accessible to other molecules.

The Yᵢ in the above formula are aggregation-inducing sequences, by which beta-aggregation inducing sequences are meant. Most particularly, the sequences are non-amyloid beta-aggregation sequences (sometimes referred to as amorphous beta-aggregation sequences). Amyloid and non-amyloid beta-aggregation differs in higher-order structure, in aggregation kinetics and in the protein sequences suitable for aggregation (Rousseau et al., Current Opinion in Structural Biology 16:118-126, 2006). Indeed, amino acid preferences will be much more position specific in an amyloid fiber than in amorphous cross-β aggregates. For example, in amyloid hexapeptides, positions 3 and 4 are extremely selective, as only some amino acid types are compatible with a highly ordered amyloid structure. On the other hand, positions 1, 2 and 6 are much more tolerant, as almost any residue type allows amyloid formation (Lopez de la Paz et al., PNAS 101:87-92, 2004). In contrast, almost any residue can be accommodated at any position of a hexapeptide for β-aggregation to occur, as long as the sequence as a whole has a good propensity to be in a β-extended conformation, and is sufficiently hydrophobic and/or neutral in charge (Fernandez-Escamilla et al., Nat Biotechnol 22:1302-1306, 2004). Amylogenic sequences are therefore more position specific, but also more tolerant to polar and charged residues than β-aggregating sequences. This will also have consequences on the kinetics of both processes. Due to its less stringent conformational requirements, β-aggregation is generally much faster than amyloidosis. Note however that there is only a thin line dividing sequences compatible with highly ordered cross-β amyloid structures and sequences that form amorphous cross-β aggregates (Lopez de la Paz et al., PNAS 101:87-92, 2004; Rousseau et al., Current Opinion in Structural Biology 16:118-126, 2006). Although amorphous aggregation is primarily envisaged herein, as in some settings amyloid aggregation is not desirable (and should thus be excluded), for many applications the nature of the aggregates does not matter. Thus, amyloid aggregates are envisaged as well.
The sequences as defined above were found to have a high beta-aggregation tendency (this can be determined using e.g. algorithms such as TANGO, Zyggregator, ...), particularly a non-amyloid beta-aggregation tendency, in view of the restrictions on polar and charged residues.

Specific to the molecules described herein is that the aggregation-inducing sequences (with high beta-sheet forming potential) are flanked by residues that have low beta-sheet forming potential or even 'break' beta-sheets, so-called gatekeeper residues. These are the numbered X moieties in the formula. Surprisingly, it was found that aggregation-inducing sequences which are demarcated by such specific residues, are not only more soluble than sequences which aren't flanked by such gatekeepers, but retain at the same time very good and specific aggregation-inducing properties. Especially the latter is surprising, since it is generally assumed that in proteins, hydrophobic sequences are interspersed with polar or charged residues in order to prevent aggregation. In the molecules described herein, the flanking gatekeepers as it were ensure that the aggregation-inducing sequence is properly presented to the protein of interest. Without being bound to a particular mechanism, this may be helped by stabilizing interactions (e.g. H-bonds or charge complementarity) between the gatekeeper residues and the protein of interest. This may also lead to increased specificity of interaction. Note that aggregation tendency is in part determined by the environment, the properties listed above are particularly envisaged in physiological conditions, e.g. at physiological pH ranges. This does not imply that the methods are limited to physiological conditions, as e.g. detection of proteins can occur in non-physiological conditions.

The X₂ᵢ₋₁ and X₂ᵢ are contiguous stretches of 1 to 4 independently selected specific amino acids: R, K, E, D, P, N, S, A, H, G and Q. Although these amino acids can all be used, best results are obtained when using residues that are rarely, or even not, present in the hydrophobic Yi stretches, particularly charged and/or non-hydrophobic residues, particularly R, K, E, D, P, N, S, H, Q and G (note that although G is typically considered as hydrophobic residue, the absence of side chains and its tiny size means it does not particularly favor beta sheet aggregation), more particularly R, K, E, D, P, and H (i.e. charged residues including H, or proline), even more particularly R, K, E, D, and P (i.e. charged residues or proline, which due to its particular side chain induces a kink and is a good breaker peptide), most particularly R, K and P (positive residues or proline), or R and P (non-hydrophobic positive residue or proline). Alternatively, R, D and P are envisaged as gatekeepers: R is a bulkier and non-hydrophobic residue than the (hydrophobic) K and thus more disruptive for beta-sheet formation. While D is smaller, its charge is closer to the backbone of the peptide or protein and more difficult to negate. According to very particular embodiments, most particularly when n is 1, K is not envisaged as a gatekeeper - thus, the amino acids of the X moiety are selected from R, E, D, P, N, S, A, H, G, and Q or a subset thereof.

As will be explained further, for embodiments where n=1, additional limitations may apply to the molecules. This is not because these molecules are non-functional, but rather because the prior art may have described molecules of peptidic nature (for a different purpose) that have the same general structure as described here. Limitations are particularly on length, and nature of residues envisaged in specific moieties, as described in the detailed description. Although these stricter limitations typically will only be needed for molecules where n=1, they are also envisaged for n=2 (or even higher n).

According to particular embodiments, each X₂ᵢ₋₁ and X₂ᵢ is 1 or 2 amino acids. Residues further from the aggregating sequence play less of a role in keeping the molecule in solution. According to alternative, but not exclusive, embodiments, each X₂ᵢ₋₁ and X₂ᵢ has a total charge of no more than 2. Alternatively, the total number of amino acids in both X moieties is 5 or less, particularly 4 or less. Alternative embodiments provide that the total charge of both X moieties surrounding the hydrophobic Y region is less than 5, particularly 4 or less. The embodiments in this paragraph are most particularly envisaged for molecules where n is 1, or molecules where n is two.

The Yᵢ as described in the formula herein is a beta-aggregating sequence. More particularly, it is an independently selected stretch of 4 to 17, particularly 4 to 16, or 4 to 15 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T. Particularly, no more than 1, and preferably none, P, R, K, D or E residue is present in the sequence. However, according to very particular embodiments, two residues selected from R, K, D and E may be present, as long as the net charge is zero (i.e., if their charges are opposite). As K is more envisaged than R in a hydrophobic stretch, two residues selected from K, D and E may be present in the Yi moiety. Since the charge needs to be zero in these embodiments, this is equivalent as saying that two charged residues are present, one of which is a K residue and the other is selected from a D and E residue. According to further specific embodiments, either no P, R, K, D or E residue is present in the Yi moiety, or two charged residues are present which have a complementary charge (so that the net charge is zero).

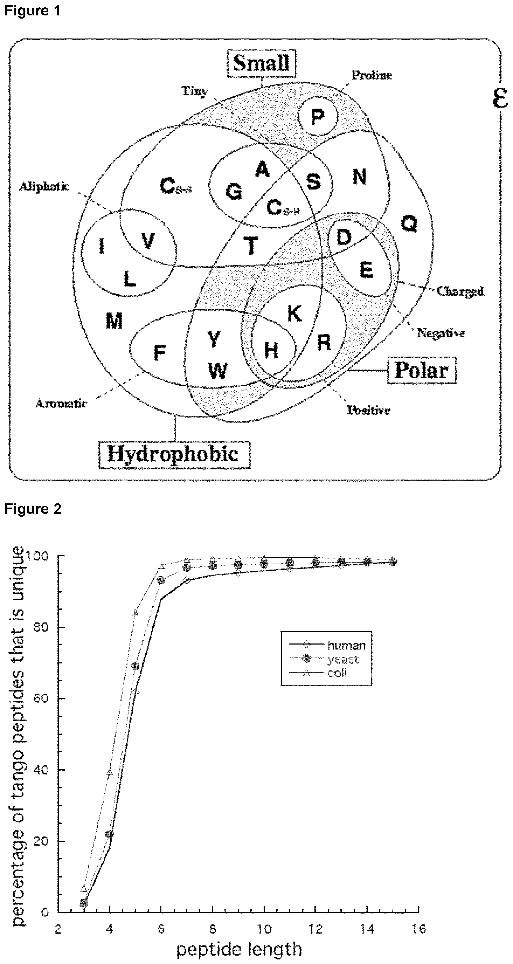

The length of the aggregating sequence will typically be influenced by the desired specificity, the ease of synthesis and the sequence of the protein of interest. The present specification describes that at least one, and particularly all, Yᵢ may be a stretch of 4 to 17 amino acids, 4 to 16 amino acids, 4 to 15 amino acids, 4 to 14 amino acids, 4 to 13 amino acids, particularly of 4 to 11 amino acids, of 4 to 10 amino acids, of 4 to 9 amino acids, or of 4 to 8 amino acids. The present specification describes that the length of the Yi stretch may be at least 5 amino acids. Accordingly, at least one, and particularly all, Yᵢ may be a stretch of 5 to 13 amino acids, particularly of 5 to 11 amino acids, of 5 to 10 amino acids, of 5 to 9 amino acids, or of 5 to 8 amino acids. According to further specific embodiments, the length of the Yi stretch is at least 6 amino acids. Accordingly, at least one, and particularly all, Yᵢ are a stretch of 6 to 13 amino acids, particularly of 6 to 11 amino acids, of 6 to 10 amino acids, of 6 to 9 amino acids, or of 6 to 8 amino acids. Most particularly, at least one, and particularly all, Yᵢ are stretches of 6 or 7 amino acids. Such stretches shorter than 16 amino acids are particularly envisaged in embodiments where n is 1.
Note that longer stretches than 16 amino acids (e.g. up to 20 amino acids) are possible, but barely add specificity towards a protein of interest (see Figure 2) while they do increase the difficulty and cost of synthesis, as well as decrease the solubility of the molecules. The ease of synthesis and handling is also why molecules where n is 1 or 2 are particularly envisaged herein. However, for transgenic approaches or when the molecules are produced recombinantly, length is much less of a (cost-)limiting factor, and particularly in these approaches it is also envisaged to work with longer molecules.

It is an object to provide molecules that are capable of specifically downregulating proteins, particularly in a sequence-dependent manner. The present specification describes that at least one of the Yᵢ in the molecule is a stretch of 4 to 16 amino acids, or in accordance with aspects or embodiments of the invention a stretch of 6 to 16 amino acids, that is identical to a contiguous stretch naturally occurring in a protein. According to further specific aspects, this is the case for more than one Yᵢ in the molecule, particularly for two Yᵢ or at least two Yᵢ in the molecule, most particularly for all Yᵢ in the molecule. The different lengths envisaged apply to this embodiment as well.

This relies on the surprising observation that non-amyloid beta-aggregation is sequence-specific. Indeed, it was generally accepted that since, contrary to amyloid aggregation, almost any residue can be accommodated at any position for β-aggregation to occur, as long as the sequence as a whole has a good propensity to be in a β-extended conformation, and is sufficiently hydrophobic and/or neutral in charge, amorphous beta-aggregation was not sequence-specific but depended on hydrophobic and H-bond interactions. Surprisingly, aggregation tendency for a given sequence is much higher with an identical sequence stretch than with another hydrophobic sequence, to the extent that it can be used for specific aggregation (inhibition and/or detection) of proteins in complex mixtures.

The present specification describes that the at least one stretch of 4 to 16 contiguous amino acids, or in accordance with aspects or embodiments of the invention the at least one stretch of 6 to 16 contiguous amino acids, naturally occurring in a protein is unique to said protein in the organism (or species) in the genome of which the protein is encoded. In other words, the sequence corresponds or is identical to only one other protein sequence in the proteome of said organism/species. The result is that, if such molecule is administered to an organism of said species, only that protein will be downregulated.

According to alternative embodiments, the sequence is not necessarily unique to the protein, but is unique to the organism or species. This may be envisaged when the molecules described herein are administered to more than one different species simultaneously (e.g. a mixture of microorganisms), while only in one species protein(s) need to be downregulated (e.g. to target a pathogenic species, while not interfering with beneficial organisms). According to further particular embodiments, the sequence is unique to the protein and unique to the organism/species.

Instead of species, the above considerations can also apply to a genus, a family, an order or a class of organisms, although the likelihood of sequence conservation, and finding a unique sequence, decreases with increase in taxonomic rank.

The present specification describes that the at least one stretch of 4 to 16 contiguous amino acids, or in accordance with aspects or embodiments of the invention the at least one stretch of 6 to 16 contiguous amino acids, naturally occurring in a protein is present in more than one protein of the organism or species in the genome of which said protein is encoded. I.e., the sequence corresponds or is identical to more than one other protein sequence in the proteome of said organism/species. This allows more than one protein to be downregulated. Yet another (non-exclusive) alternative is that the sequence stretch is present in a protein of more than one organism/species. Thus, the sequence corresponds or is identical to at least one other protein sequence in the proteome of at least two different organisms/species. This allows downregulation of a protein in more than one organism. This can be useful for cross-reactivity (e.g. to allow the use of one molecule in subjects of different species). However, it is also particularly envisaged for simultaneous downregulation of proteins in (particularly infectious) organisms while administering only one molecule to a subject. Combinations of both, i.e. the stretch is present in more than one protein and more than one organism/species, are of course also envisaged. Here also, species can be replaced with genus, family, order or class of organisms.

It should be noted that targeting of more than one protein or more than one organism/species can also be achieved using molecules with at least two Yi regions, wherein at least two of the Yi regions correspond to a stretch in at least two different proteins, and/or to a stretch in at least two different organisms/species/etc. The at least two Yi regions can (each independently) be unique to a protein or organism, or can occur in more than one protein or organism.

Typically, the Yi stretch that is identical to a stretch naturally occurring in a protein will be completely identical to that of the protein. However, in some instances, it is envisaged that non-identical, but closely related, sequences can be used, i.e. sequences which have one or two substitutions. In order to maintain specificity, it is envisaged that for non-conservative substitutions, for Yi stretches less than 6 amino acids, only one amino acid difference is tolerated. For sequences of at least 6 amino acids (particularly at least 7 or at least 8 amino acids), one or two amino acids can be substituted. Thus, the present specification describes that at least one Yᵢ differs with no more than one amino acid substitution from a stretch in a naturally occurring protein if the length of that Yᵢ is less than 6 amino acids, or at least one Yᵢ differs with one or two amino acid substitutions from a stretch in a naturally occurring protein if the length of that Yᵢ is at least 6 amino acids. In accordance with aspects or embodiments of the invention, at least one Yi is a stretch identical to or differing with no more than one amino acid substitution from a contiguous stretch of amino acids occurring in a protein of interest. In such instances, the substitutions are typically as compared to the stretch present in the protein of interest (i.e., the Yi stretch is identical to the stretch present in the protein of interest except for the one (or two) amino acid substitutions). As the skilled person will realize, making a (particularly non-conservative) substitution may result in altered specificity (i.e. making the stretch identical to one of another protein in the organism), so it should be checked whether this happens if the altered targeting is undesired. (In some instances, targeting more than one protein may be desired, see also below). To ensure substitution does not result in too much loss of specificity, preferably substitution is only done in one or two of the Yi regions. According to specific embodiments, if a Yi region with a substituted amino acid is present, at least one other Yi region is present wherein no substitution occurred.

According to particular embodiments, the substitution is with a gatekeeper residue, particularly with one selected from R, K, E, D, P, N, S, A, H, G, Q, more particularly selected from R, K, E, D and P, most particularly selected from R, K and P residues.

According to alternative embodiments, substitution is conservative substitution. This is envisaged when downregulating a family of proteins is envisaged, and the proteins share a conserved, but not identical, sequence motif. In such instances, aggregation of these closely related proteins can be achieved using a consensus sequence motif (i.e., a similar, but not identical sequence, where 'similar' is used in the context of sequence alignment). However, it is possible that aggregation is less efficient when the sequence match is not 100%.

According to yet further alternative embodiments, substitution is substitution of a residue with relatively low beta-sheet propensity with a residue with higher beta-sheet propensity; in order to enhance aggregation. Typically, a residue with a Chou-Fasman P(b-sheet) score lower than 100 can be replaced with a residue with a P(b-sheet) score> 100.

Of note, substitution is always with the same number of residues, deletions or insertions will abolish the specific beta-aggregation.

According to specific embodiments, n is higher than one (i.e., two to five), meaning that more than one Yi region is present in the molecule (at least two, and up to five). To target a protein, only one Yᵢ region identical to a stretch in that protein needs to be present in the molecule. Further Yᵢ regions may e.g. be synthetic aggregation-inducing sequences. Typically however, the present specification describes that for detecting or downregulating proteins, when n is higher than one, at least two, and particularly every, Yᵢ are a stretch of 4 to 16, or in accordance with aspects or embodiments of the invention a stretch of 6 to 16, contiguous amino acids naturally occurring in a protein. (For each stretch independently, the above considerations apply). According to further particular embodiments, the at least two Yᵢ occur in the same protein. This increases the likelihood of causing the protein to aggregate. According to further particular embodiments, said at least two Yᵢ are partly overlapping. This may be the case when the protein of interest has a long stretch that fulfills the criteria for the Yᵢ region of the molecule. In this case, one Yi of the molecule may correspond to one part of the stretch in the protein, while another Yi corresponds to another, partly overlapping part of the same stretch. According to yet further particular embodiments, the at least two Yᵢ are identical. Thus, when two Yi regions correspond to stretches in the same protein, they may be identical or different.

In alternative embodiments, however, it is envisaged that the at least two Yᵢ can be used to achieve targeting of more than one protein. Thus, in these embodiments, the at least two Yᵢ are derived from (at least two) different proteins, i.e. over its length, the sequence of the at least two Yᵢ corresponds to or is identical to at least two different protein sequences. The number of proteins can be as high as the number of Yᵢ regions, but of course, it is also possible that e.g. 4 Yᵢ regions are used to target two different proteins.

Similarly, according to alternative embodiments, the at least two Yᵢ can be used to achieve targeting of more than one organism/species. Thus, in these embodiments, the at least two Yᵢ are derived from (at least two) different organisms/species, i.e. the sequence of the at least two Yᵢ corresponds to or is identical to at least one other protein sequence in the proteome of at least two different organisms/species. Here also, species can be replaced with genus, family, order or class of organisms.

As mentioned before, β-aggregating sequences are less tolerant to the presence of polar and particularly charged residues than amylogenic sequences. Accordingly, in specific embodiments, the total charge of at least one Yᵢ, particularly of at least two Yᵢ, more particularly of each Yᵢ is not higher than 1. According to further specific embodiments, the number of charged residues in at least one Yᵢ, particularly of at least two Yᵢ, more particularly of each Yᵢ is not higher than 1. Most particularly however, the number of charged residues or prolines in a Yᵢ moiety is zero.

As outlined in the formula above, the molecules described herein also contain linker moieties, Zᵢ. According to particular embodiments, the molecules only contain internal linkers and no N- or C-terminal linkers. Thus, in specific embodiments, Zₙ is nothing (or is a linker of zero linking units).

The nature of the linker moieties is not vital to the invention, although long flexible linkers are preferably not used. According to particular embodiments, each Zᵢ is independently selected from stretch of between 0 and 20 identical or non-identical units, wherein a unit is an amino acid, a monosaccharide, a nucleotide or a monomer. Non-identical units can be non-identical units of the same nature (e.g. different amino acids, or some copolymers). They can also be non-identical units of a different nature, e.g. a linker with amino acid and nucleotide units, or a heteropolymer (copolymer) comprising two or more different monomeric species. According to particular embodiments, the length of at least one, and particularly each Zᵢ other than Zₙ, is at least 1 unit. According to other particular embodiments, Zₙ is 0 units. According to particular embodiments, all Zᵢ linkers other than Zₙ are identical. According to further embodiments, all Zᵢ moieties are identical.

According to specific embodiments, at least one, and particularly all, Zi are of between 0 and 10 units of the same nature, particularly between 0 and 5 units of the same nature. According to particular embodiments, at least one Zi moiety, and particularly all Zᵢ moieties except Zₙ, is a peptide or polypeptide linker. Particularly envisaged sequences of such linkers include, but are not limited to, PPP, PP or GS. The linker can also be of a chemical nature. Particularly envisaged chemical linkers include PEG and Ttds (aka 4,7,10-trioxatridecan-13-succinamic acid).

Typically, long linkers are not used. However, according to the particular embodiments where the Yi moieties correspond to aggregation-inducing regions of more than one protein, it is envisaged that long linkers may be used. Indeed, to ensure that the molecule can (e.g. simultaneously) interact with more than one protein, it may be beneficial to increase the distance between the different targeting Yi moieties, so that the interaction is not prevented due to steric hindrance. In these instances, the Zi linker may be a stretch of between 0 and 100 identical or non-identical units, wherein a unit is an amino acid, a monosaccharide, a nucleotide or a monomer; or of between 0 and 90, 0 and 80, 0 and 70, 0 and 60, 0 and 50, 0 and 40, 0 and 30 or 0 and 20. Particularly, the minimal length of the Zi linker is at least 1 unit, at least 2 units, at least 3 units, at least 4 units, or at least 5 units.

In case the Yi moieties are identical to aggregating regions of two different proteins, the molecules are called bispecific (for three proteins, trispecific, and so on).

According to specific embodiments, the total length of the molecules described herein does not exceed 60, 55 or 50 amino acids. More particularly, the length does not exceed 40 amino acids, 30 amino acids, 25 amino acids or even 20 amino acids.

Particularly envisaged molecules are those where n=1 or n=2, e.g. those with the following structure: X₁-Y₁-X₂-Z₁ (i.e. n is 1), wherein X₁ and X₂ are in total no more than 5 amino acids; Y₁ is a stretch of between 4 and 10 amino acids and Z₁ is a stretch of 0 units; and X₁-Y₁-X₂-Z₁-X₃-Y₂-X₄-Z₂ (i.e., n is 2), wherein Z₁ is a linker and Z₂ is nothing.

The molecule can further comprise (or can be further fused to) other moieties. It is particularly envisaged that the molecule further comprises a detectable label. The detectable label can be N- or C-terminally or even internally fused to the molecule (e.g. through the linker, or the linker can be used as the detectable label). Alternatively, the detectable label can refer to the use of one or more labeled amino acids in one or more of the X-Y-Z moieties of the molecule (e.g. fluorescently or radioactively labeled amino acids).

Since the Yi moieties identical to a region in a protein can target the protein specifically (on condition that the sequence is unique to the protein in the organism in the genome of which said protein is encoded), another moiety that can be attached to the molecules is a small molecule or a drug, so that this small molecule or drug can be targeted to the right protein, cellular compartment, cell type, ... where it needs to be delivered. In these instances, at least one of the Yi regions will be identical to a sequence of a protein that is present where the drug or small molecule needs to be delivered.

Another moiety which can be attached to the molecule is a moiety that increases solubility of the molecule. Such moieties are well known in the art, and examples include, but are not limited to PEG (polyethylene glycol) or PEG derivatives, a peptide, a protein or a protein domain. The nature of the moiety will depend on the application, as can be determined by the skilled person.

Note that, for embodiments where Zₙ is present, the detectable label (or other moiety, like a solubilisation tag) can be fused to the Zₙ linker moiety. (Although this notation would entail that the tag is added at the C-terminus, N-terminal tags are envisaged as well - this corresponds to the equivalent notation of (Zᵢ-X₂ᵢ₋₁-Yᵢ-X₂ᵢ)ₙ, wherein each Z₂ to Zₙ is a linker, and Z₁ is the linker to which the tag is fused).

In those instances where other moieties are fused to the molecules, it is envisaged in particular embodiments that these moieties can be removed from the molecule. Typically, this will be done through incorporating a specific protease cleavage site or an equivalent approach. The cleavage site may be incorporated separately or may be an integral part of the external Zₙ linker (or external Z₁ linker if the moiety is N-terminal). According to very specific embodiments, the moiety may be part of an internal Zi linker, or may even be the whole Zi linker.

In many typical applications, the interferor molecules described herein can be used or added as such. However, according to a very particular aspect, it is envisaged that these molecules are provided as nucleic acids encoding the molecules. It goes without saying that the interferor molecules according to these embodiments are entirely of polypeptide nature, since they need to be able to be encoded. I.e., all numbered X, Y and Z moieties present in the interferor molecules are of polypeptide nature.

Thus, the present specification also describes a nucleic acid molecule that encodes (or whose sequence encodes) a molecule having the structure as described above, particularly the following structure:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing.

It is particularly envisaged that the nucleic acid sequences encode the molecules with all the limitations and variations described herein, *mutatis mutandis.* Thus, the encoded polypeptide is in essence as described herein, that is to say, the variations mentioned for the interferor molecules that are compatible with this aspect are also envisaged as variations for the polypeptides encoded by the nucleic acid sequences. By way of example, embodiments specifying the sequence or the length of the X or Y moieties are compatible with being encoded in nucleic acids, embodiments wherein the Z moiety is of a non-amino acid nature are not.

According to specific embodiments, the encoded polypeptide sequence is a non-naturally occurring polypeptide. According to further particular embodiments, the nucleic acid molecule is an artificial gene. Since the nucleic acid aspect is most particularly suitable in applications making use of transgenic expression, particularly envisaged embodiments are those where the nucleic acid molecule (or the artificial gene) is fused to another moiety, particularly to further nucleic acids encoding a moiety that increases solubility and/or stability of the gene product. Indeed, transgenic expression of peptides sometimes may be difficult due to rapid degradation of the product.

The present specification also describes recombinant vectors comprising such a nucleic acid molecule encoding (or with a sequence encoding) a molecule as herein described. These recombinant vectors are ideally suited as a vehicle to carry the nucleic acid sequence of interest inside a cell where the protein to be downregulated is expressed, and drive expression of the nucleic acid in said cell. The recombinant vector may persist as a separate entity in the cell (e.g. as a plasmid or a viral or non-viral carrier), or may be integrated into the genome of the cell.

Accordingly, the present specification also describes cells comprising a nucleic acid molecule encoding (or with a sequence encoding) a molecule as herein described, or comprising a recombinant vector that contains a nucleic acid molecule encoding such interferor molecule. The cell may be a prokaryotic or eukaryotic cell. In the latter case, it may be a yeast, algae, plant or animal cell (e.g. insect, mammal or human cell). The cell may be provided as a cell line.

However, the cell may also be part of an organism (or e.g. a stem cell). Accordingly, the present specification also describes non-human transgenic organisms comprising a a nucleic acid molecule encoding (or with a sequence encoding) a molecule as herein described, or comprising a recombinant vector that contains a nucleic acid molecule encoding such interferor molecule. The organism may be any organism (including micro-organism). It is particularly envisaged that the transgenic organism is a non-human mammalian organism. According to yet other alternative embodiments, the transgenic approach is used in non-human animals, e.g. for target validation in animal models of disease. This may be in mammals (e.g. transgenic mice) or other vertebrates, or even in non-vertebrate animals, e.g. nematodes (such as C. elegans) or fruit flies (such as D. melanogaster).

According to alternative particular embodiments, the transgenic approach (i.e. the provision of interferor molecules encoded in nucleic acid instead of directly as polypeptides) is particularly suited for use in plants. Accordingly, the present specification also describes plants, or plant cells, or plant seeds that contain a nucleic acid molecule, artificial gene or a recombinant vector as described herein. Or, the cells or transgenic organisms provided herein are plants, plant cells or plant seeds.

According to a further aspect, the molecules described herein can be used for downregulating or inhibiting the function of a protein. Typically, this is achieved by inducing aggregation of that protein. The present specification describes methods for downregulating the function of a protein comprising contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing.

Particularly, for molecules where n is 1, additional limitations might apply. For instance, X1 and X2 can each be 1 or 2 amino acids instead of 1 to 4. X1 and X2 may also be selected from P, R, K, D, E. The length of the Y1 moiety may be adapted.
None of these limitations is necessary to result in downregulation of molecules, but they describe particularly well-working embodiments. A particular limitation that is also envisaged for methods to downregulate protein where n is 1, is that Y₁ is a stretch of 4 to 11 contiguous amino acids. Another particular limitation (which, like most limitations herein, can be combined) is that Z₁ is not an amino acid linker. According to even further particular embodiments, Z1 is nothing (i.e. a linker of 0 units).

For the molecules, the same considerations and limitations as above apply. Particularly, it should be noted that, as long as downregulation of the function is achieved, one or two substitutions in the Yi occurring in the protein can be tolerated, as described earlier.

According to further particular embodiments, these methods are provided for downregulating a protein in a disease setting, or for making a diagnosis. The specification describes a molecule of the following structure: (X₂ᵢ₋₁-Yᵢ-X₂ᵢ-Zᵢ)ₙ, wherein:
- n is 1 to 5 and i runs from 1 to n (or i increases from 1 to n with each repeat);
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 15 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing;
for use as a medicament or diagnostic.

Here also, it is particularly envisaged that if n is 1, Y₁ is a stretch of 4 to 11 contiguous amino acids. Also, if n is 1, it is particularly envisaged that Z₁ is not an amino acid linker. According to further embodiments, Z₁ is nothing.

As used herein, methods and uses are often interchangeable, i.e. a molecule for use as a medicament, or more particularly a molecule for use in treatment of a specific disease is equivalent to a method for treating the disease comprising the use of (e.g. contacting with) the molecule, is equivalent to use of the molecules for the manufacture of a medicament for the treatment of the disease. Thus, 'second medical use' claims, 'method of treatment' claims and so-called 'Swiss-style' claims are used interchangeably herein, and when one of them is listed, the others are implied as well.

Since the molecules are provided for use as a medicament or diagnostic, or in methods of treatment or diagnosis, it is also envisaged that they can be provided as pharmaceutical. Accordingly, the present specification describes pharmaceutical compositions comprising the molecules as described herein. Particularly, the pharmaceutical compositions comprise at least one molecule having the following structure:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 15 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing;
and a pharmaceutically acceptable carrier.

More particularly, pharmaceutical compositions are described comprising at least one molecule having the following structure: (X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 15 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; wherein at least one Yᵢ is a stretch of 4 to 15 contiguous amino acids naturally occurring in a protein; and
- each Zᵢ is a linker and Zₙ is independently selected from a linker or nothing;
and a pharmaceutically acceptable carrier.

Most particularly, in the pharmaceutical compositions, at least one Yᵢ of the molecules is present in a protein to be downregulated in the subject to which the composition will be administered. Note that this does not imply that this protein is encoded in the genome of that subject. Indeed, for subjects suffering from infection, it is envisaged that molecules are administered that target proteins of the infectious organism and not of the subject itself.

According to particular embodiments, the molecule can be provided in lyophilized form with a physiological buffer. According to particular embodiments, the pharmaceutical compositions, if a liquid, are provided at physiological pH, particular between pH 5 and 9, more particular between pH 6 and pH 8.

In specific embodiments, the molecules can be used to downregulate a (one or more) protein that needs to be downregulated in a disease setting.

Accordingly, the present specification describes molecules of the structure (X₂ᵢ₋₁-Yᵢ-X₂ᵢ-Zᵢ)ₙ for use in treatment or prevention of a disease, wherein:
- n is 1 to 5 and i runs from 1 to n (or i increases from 1 to n with each repeat);
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with the disease; and
- each Zᵢ is a linker and Zₙ is independently selected from a linker or nothing.

As mentioned before, the molecules described for use in treatment or prevention of a disease is equivalent as saying that methods are described to treat or prevent disease in a subject in need thereof, comprising: administering to the subject a molecule having the structure (X₂ᵢ₋₁-Yᵢ-X₂ᵢ-Zᵢ₎ₙ, wherein:
- n is 1 to 5 and i runs from 1 to n (or i increases from 1 to n with each repeat);
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with the disease; and
- each Zᵢ is a linker and Zₙ is independently selected from a linker or nothing.

Another equivalent way of phrasing this is that uses of molecules having this structure are provided for the manufacture of a medicament for treatment of a disease.

The subject particularly is an animal, more particularly a mammal (e.g. cat, dog, rabbit, horse, cow, pig, sheep, goat, llama, mouse, rat, monkey, other primate...), most particularly a human.

Particularly envisaged diseases include, but are not limited to, cancer, age-related macular degeneration (AMD) and inflammation. Thus, the molecules can be provided for treatment of those diseases (or methods are provided for treating these diseases; or uses of molecules for the manufacture of a medicament for treatment of these diseases), wherein at least one Yᵢ is a stretch naturally occurring in a protein whose expression or overexpression is associated with cancer, AMD, or inflammation/inflammatory disease respectively.

Most particularly, the protein to be downregulated in cancer is VEGFR-2 or EGFR. Also most particularly, the protein to be downregulated in AMD is VEGFR-2. Most particularly envisaged proteins for downregulation in inflammatory disease include TNF-α and IL-1β.

According to a further aspect, the molecules are used as a medicament in infectious disease. I.e., they are used to downregulate the function of proteins in organisms causing infections in a subject, such organisms as viruses, bacteria, fungi, or macroparasites such as ticks, mites, nematodes, flatworms etc. The present specification describes molecules of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pathogenic organism; and
- each Zᵢ is a linker and Zₙ is independently selected from a linker or nothing;
for use as an antipathogenic compound.

This is equivalent as saying that methods are described to prevent or treat pathogenic infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pathogenic organism; and
- each Zᵢ is a linker and Zₙ is independently selected from a linker or nothing.

Another equivalent phrasing is that uses of molecules having this structure are provided for the manufacture of a medicament for treatment of infection with a pathogenic organism.

According to very specific embodiments, the Yi region may contain exactly 2 charged residues (selected from R, K, D, E), as long as the net charge of the Yi region is zero. More particularly however, the Yi region does not contain any charged residue (or proline for that matter).

The pathogens may be viral organisms. Accordingly, the specification describes molecules of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a viral organism; and
- each Zᵢ is a linker and Zₙ is independently selected from a linker or nothing;
for use as an antiviral.

This is equivalent as saying that methods are described to prevent or treat viral infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a viral organism; and
- each Zᵢ is a linker and Zₙ is independently selected from a linker or nothing.

Another equivalent phrasing is that uses of molecules having this structure are provided for the manufacture of a medicament for treatment of infection with a viral organism.

Particularly, the molecules are used as antimicrobial agents. Thus, the specification describes molecules of the following structure: (X₂ᵢ₋₁-Yᵢ-X₂ᵢ-Zᵢ)ₙ, wherein:
- n is 1 to 5 and i runs from 1 to n (or i increases from 1 to n with each repeat);
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism; and
- each Zᵢ is a linker and Zₙ is independently selected from a linker or nothing;
for use as an antimicrobial.

This is equivalent as saying that methods are described to prevent or treat microbial infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism; and
- each Zᵢ is a linker and Zₙ is independently selected from a linker or nothing.

Another equivalent phrasing is that uses of molecules having this structure are provided for the manufacture of a medicament for treatment of infection with a microbial organism.

Further steps of these methods may include the evaluation of (or the measuring of) the presence of the pathogenic, viral or microbial organism.

According to particular embodiments where the pathogen is a microbial organism, the protein of a microbial organism is a protein of a microbial organism selected from Gram-positive bacteria, Gram-negative bacteria, mycobacteria, protozoa, Archaea, fungi (such as yeasts and moulds). More particularly, it is a protein of Gram-positive bacteria, Gram-negative bacteria, mycobacteria, and fungi.

According to particular embodiments, the pathogenic organism is a drug-resistant organism, strain or variant. Thus, the protein of the pathogenic organism is a protein from a drug-resistant organism, strain or variant. Administering molecules directed to these organisms will still have an effect on these organisms, as is shown in the Examples section.

A particular example of drug resistance is antibiotic resistance. According to particular embodiments, the protein of a microbial organism is a protein from an antibiotic-resistant organism, strain or variant. Administering molecules directed to these organisms will still have an effect on these organisms, as is shown in the Examples section. More particularly, methods for administration are provided wherein the molecules as described herein are administered at least once a day for at least ten days. Particularly, this treatment regimen is not accompanied by the development of antibiotic resistance. Thus, these embodiments foresee that the MIC values over this range in time do not increase more than fourfold, or do not double. Most particularly, it is envisaged that, during prolonged administration, the MIC value of the interferor molecule for the specific microbial organism remains below the clinical breakpoint of the interferor molecule for the specific microbial organism.

According to specific embodiments, the molecules can be used to kill the pathogenic (e.g. microbial) organism, or the methods result in killing of the pathogenic (e.g. microbial) organism. According to yet further specific embodiments, the molecules succeed in quickly killing the pathogenic (e.g. microbial) organism, particularly within an hour or less, or within 30 minutes or less. According to alternative embodiments, the methods using the molecules inhibit growth and/or reproduction of the pathogenic (e.g. microbial) organism without killing it. Viruses in this context are also considered as 'living' organisms, for instance a reduction in viral titer is indicative of killing the viral organism, while e.g. stabilization of the viral titer is indicative of inhibition of reproduction.

According to particular embodiments, the protein of the pathogenic/viral/microbial organism is an essential protein, i.e. the organism cannot survive if it is depleted of the protein. According to other (non-exclusive) particular embodiments, the protein of the pathogenic (e.g. microbial) organism is involved in biofilm formation. According to further embodiments, methods are provided to treat or prevent biofilm formation, wherein the protein of the pathogenic (e.g. microbial) organism is involved in biofilm formation. According to yet further embodiments, the biofilm formation is on an object, particularly an implantable device, such as a catheter or stent.

Thus, the present specification also describes methods to prevent, inhibit or reverse microbial biofilm growth on a surface, comprising contacting the surface with a molecule of the structure (X₂ᵢ₋₁-Yᵢ-X₂ᵢ-Zᵢ₎ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; wherein at least one Yi is a stretch present in a protein of a microbial organism; and
- each Zᵢ is a linker and Zₙ is independently selected from a linker or nothing.

Most particularly, the protein of the microbial organism is a protein involved in biofilm formation. The surface on which biofilm formation is prevented, inhibited or reversed can be a biotic or abiotic surface. Particularly envisaged surfaces are those of implantable devices, such as those of catheters or stents.

Regarding abiotic surfaces, devices coated with the molecules described herein are also envisaged within the scope of the invention. Coating of the devices can be done directly by applying the molecules to the device. Alternatively, they can be coated on the device using (cross-)linkers. The devices can be fully coated (e.g. by submersion of the device in a solution of the molecules), or only parts of the device can be coated. It is particularly envisaged that the devices are coated with molecules against a protein present in a microbial organism, particularly a protein involved in biofilm formation.

Thus, the present specification describes implantable devices at least partly coated with molecules of the structure (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing

According to a further aspect, methods to screen for new compounds are provided. These compounds are the molecules described herein and can be used as inhibitory compounds or compounds in detection. The present specification describes screening methods comprising the steps of:
a) identifying in at least one protein at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing a molecule of the following structure: (X₂ᵢ₋₁-Yᵢ-X₂ᵢ-Zᵢ)ₙ, wherein:
   - n is 1 to 5 and i increases from 1 to n with each repeat;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is independently selected from 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is is a stretch naturally occurring in a protein identified in step a); and
   - each Zᵢ is a linker and Zₙ is independently selected from a linker or nothing;
c) bringing the molecule made in step b) in contact with the protein of step a); and
d) assessing the function and/or aggregation of the protein.

These methods have particular advantages: they can be used for unbiased screening, as any protein of the proteome of an organism may be used. Alternatively, they can be used for selective screening, e.g. to find new targets (i.e. proteins which are not targeted by inhibitory compounds yet). According to these embodiments, the protein in step a) is not a known target for inhibitory compounds.

The bringing in contact of the molecule and the protein in step c) may be entirely in vitro, e.g. with purified protein in a test tube or a plate. However, the methods can also be used in cellular systems: the molecules made in step b) can e.g. be contacted with the protein, by adding the molecules to a cell line. The way the molecules will be contacted with the protein will e.g. depend on the organism or cell in which the protein is present, as this will determine the availability of the protein in natural or in vitro conditions.

Function can e.g. be assessed using suitable reporter read-outs. If screening is for detection compounds rather than inhibitory compounds, detecting the presence or aggregation of the compounds will typically be the read-out rather than a functional one. However, it is envisaged that the same compound can both be used for inhibition and detection (see e.g. Example 4).

These methods can also be used for screening for improved compounds (instead of just screening for new ones). For instance, when an inhibitory compound is known, variations of this compound can be screened, e.g. by varying the residues used for the X moieties, by trying different linkers, by shortening or lengthening the Yi moiety, and the like.

For a screening for antipathogenic compounds (i.e. wherein the protein in step a) is a protein of a pathogenic organism), it is particularly envisaged that the contacting can be done by adding or administering the molecule to the pathogenic organism, especially for pathogenic micro-organisms. Also particularly envisaged is that, if the at least one protein is a protein of a pathogenic organism, assessing function and/or aggregation of the protein in step d) may be done by assessing survival, reproduction and/or growth of the pathogenic organism.

Here also, the at least one region of 4 to 16 contiguous amino acids, or in accordance with aspects or embodiments of the invention the at least one region of 6 to 16 contiguous amino acids, identified in step a) may occur in more than one protein of a pathogenic organism. This will likely increase the chance of success, as more than one protein is targeted this way.
Likewise, the at least one region of 4 to 16 contiguous amino acids, or in accordance with aspects or embodiments of the invention the at least one region of 6 to 16 contiguous amino acids, identified in step a) may occur in at least one protein of more than one pathogenic organism. This allows the targeting of more than one pathogenic organism with the same compound, similar to e.g. broad-spectrum antibiotics.

The specification describes methods to screen for new antimicrobial compounds. These methods comprise the steps of:
a) identifying in at least one microbial protein at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n (or i increases from 1 to n with each repeat);
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism identified in step a); and
   - each Zᵢ is a linker and Zₙ is independently selected from a linker or nothing;
c) adding the at least one molecule made in step b) to the microbial organism in the genome of which the protein of step a) is encoded; and
d) assessing survival and/or growth or reproduction of the microbial organism.

In assessing the survival, the MIC values can be determined. In order to identify a compound with antimicrobial activity, i.e. to classify a molecule as a hit, the MIC should particularly be lower than 100 µg/ml.

In embodiments covering methods to identify new targets for antimicrobial compounds, it is particularly envisaged that the microbial protein in step a) is not a known target for antibiotics. This would make the identified compounds particularly useful in combination therapy, as different targets are tackled.

Another particular advantage is that the screening methods can be performed without selection bias for a particular protein as an interesting target. Indeed, the entire proteome of the microbial organism can be analyzed, and the suitable sequences can be used into molecules described herein, and tested for effectiveness. This has a high chance of yielding new targets. Moreover, when such analysis is done, it is envisaged that the regions identified in step a) are regions that occur more than once in the proteome. More particularly, the at least one region of 4 to 16 contiguous amino acids, or in accordance with aspects or embodiments of the invention the at least one region of 6 to 16 contiguous amino acids, identified in step a) occurs in more than one protein of the microbial organism.

Since in many instances, it is desirable to target more than one microbial organism (e.g. broad spectrum antimicrobials), it can also be ensured that the at least one region of 4 to 15 contiguous amino acids, or in accordance with aspects or embodiments of the invention the at least one region of 6 to 16 contiguous amino acids, identified in step a) occurs in a protein, or at least one protein, of more than one microbial organism.

Most particularly, the microbial organisms targeted herein are pathogenic microbial organisms. In order to make sure that no beneficial microbial organisms are killed (e.g. beneficial microorganisms in the gut flora of a subject), it can be checked whether the stretch identified in the microbial organism is specific to pathogenic organism(s) and does not occur in beneficial microorganisms.

According to a further aspect, the molecule is used in detection or as diagnostic. Accordingly, methods for detection and diagnosis are provided. The present specification describes methods to detect a protein in a sample, comprising the steps of:
a) contacting a sample suspected of containing the protein with a (i.e. at least one) molecule of the following structure: (X₂ᵢ₋₁-Yᵢ-X₂ᵢ-Zᵢ)ₙ, wherein:
   - n is 1 to 5 and i runs from 1 to n (or i increases from 1 to n with each repeat);
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in the protein to be detected; and
   - each Zᵢ is a linker and Zₙ is independently selected from a linker or nothing;
b) detecting the presence of molecules reacted with the protein.

The sample can be provided as such or can be pre-processed. Detection can be direct or indirect (i.e., either the reacted or non-reacted fraction is detected) and can be done through detection of the molecules (e.g. labeled molecules) or the protein (e.g. detection of the (non-)reacted fraction). The nature of the detection method is not vital to the invention, any suitable method known to one skill in the art may be used (e.g. antibody-based, mass-based, adsorption-based, ...).

According to particular embodiments, the at least one Yᵢ naturally occurring in the protein to be detected is unique to said protein in said sample. Thus, for instance, in a sample of human origin, the sequence also occurring in the protein to be detected is encoded only once in the human genome (or occurs only once in the human proteome), namely in the (sequence encoding the) protein to be detected. Although this uniqueness is not always necessary - e.g. when different proteins have an identical aggregating sequence and are detected together, they can still be further discriminated, e.g. based on size - it is particularly envisaged to facilitate detection. The 'in said sample' part is important in determining the uniqueness of the protein stretch: e.g. if the sample is pre-processed, it is likely to contain less different proteins than detection in complex mixtures or in samples from different (micro-)organisms.

According to very specific embodiments, the Yi region may contain exactly 2 charged residues (selected from R, K, D, E), as long as the net charge of the Yi region is zero. More particularly however, the Yi region does not contain any charged residue (or proline for that matter).

It should be noted that the present detection methods are highly similar to established methods, typically antibody-based detection methods. The significant difference is the use of the particular molecules. In fact, it should be noted that in the detection methods described herein, the molecules as defined herein fulfill a similar role as antibodies. Thus, in detection methods which are normally antibody-based, the molecules described herein can replace at least one antibody. For instance, molecules where at least one Yᵢ is a stretch naturally occurring in the protein to be detected can replace a primary antibody in detection assays (and can be labeled 'primary interferors'), molecules where Yi is a stretch naturally occurring in a primary antibody used for detection can replace a secondary antibody. Alternatively, the secondary interferor molecule can be directed to a tag or label fused to a primary antibody, or can be directed to a 'primary interferor' or moiety fused thereto. As with antibodies, a 'primary interferor' can also serve as a capture agent, where the rest of the detection occurs with antibodies (or e.g. with another primary interferor and antibody). This set-up is usually referred to as a sandwich assay.

According to specific embodiments, the molecule used for detection comprises a detectable label. According to further specific embodiments, the detecting in step b) is through detection of the detectable label.

According to particular embodiments, the methods additionally comprise a separation of molecules reacted with the protein and molecules not reacted with the protein prior to detection in step b). Alternatively, the methods may comprise a separation of protein reacted with the molecules and protein not reacted with the molecules prior to detection in step b).

The detection in step b) can be direct or indirect, e.g. through detection of the non-reacted fraction of molecules. The detection can be qualitative, semi-quantitative or quantitative.

According to particular embodiments, the sample is from a subject, particularly from an animal or plant subject, more particularly from a mammalian subject, most particularly from a human subject.

However, any sample that can contain proteins may be used, and according to particular embodiments, the sample is not from an organism. This is particularly the case for applications in white biotech, where for instance analysis is done for purity of products, or for detection of proteins in food or feed, or for detecting polluting proteins in water, and the like.

Although detecting the protein can be the last step of the method, often a next step can follow of correlating the presence (or absence, or amount) of the detected protein with a particular status. For instance, in the white biotech applications mentioned, the presence of the protein may give an indication of pollution or purity. E.g. in samples from plant material, the amount of a protein detected may indicate which line produces most of a particular product, information which e.g. may be used to select plants for further breeding.

According to specific embodiments, the presence, absence or amount of protein detected in the sample is indicative of a disease status. Such a disease status can e.g. be presence of disease, absence of disease, progression of disease (e.g. malignancy, metastasis, response to drug therapy, relapse). Examples of proteins associated with disease status, e.g. biomarkers, are well documented in the art. Non-limiting examples of such proteins include CRP (often used to monitor inflammation) and PSA (used in the early detection of prostate cancer), as well as IL-1β and TNF-α, which are also inflammation markers. According to further specific embodiments, these methods additionally comprise a step c) correlating the presence, absence or amount of protein detected in the sample with a disease status in the subject. As also seen from the markers, particularly envisaged diseases for diagnosis are cancer and inflammatory disease.

The present specification also describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein which is indicative of a disease status; and
- each Zᵢ is a linker and Zₙ is independently selected from a linker or nothing;
for use in diagnosis of said disease status.

Here also, cancer and inflammatory disease are two types of disease that are particularly envisaged (or similar, use of the molecules for the manufacture of a diagnostic for disease (e.g. cancer, inflammatory disease) is provided).

As e.g. in vitro diagnostics are envisaged herein, also provided are kits comprising at least one molecule as described herein (including a nucleic acid molecule encoding such molecule or a recombinant vector as described herein) and at least a suitable buffer.

The present specification also describes, as a special form of kit, solid supports that contain at least two molecules of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing.

According to particular embodiments, the molecules are linked to the solid support through a linker. The at least two molecules typically will be at least two different molecules. Particular examples of solid supports include, but are not limited to, micro-arrays, precoated plates, nanoparticles and lab-on-a-chip devices. As many of these devices are used to detect more than one protein (often even up to tens or hundreds of proteins at the same time), it is envisaged that solid supports are provided comprising at least 5 molecules, at least 10 molecules, at least 20 molecules, at least 50 molecules, at least 100 molecules, at least 200 molecules, at least 500 molecules or at least 1000 molecules.

According to a further specific aspect, the molecules are used to downregulate the functions of proteins in plants, plant cells or plant seeds. Accordingly, the present specification describes methods for down-regulating the biological function of a protein in a plant or plant cell or plant seed, comprising contacting said protein with a molecule of the following structure: (X₂ᵢ₋₁-Yᵢ-X₂ᵢ-Zᵢ₎ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein in said plant, plant cell or plant seed; and
- each Zᵢ is a linker and Zₙ is independently selected from a linker or nothing.

Most particularly, the molecule is a polypeptide encoded by a nucleotide sequence present on a recombinant vector and which, upon introduction into the plant cell, plant seed or plant, produces said polypeptide in said plant cell, plant seed or plant.

According to very specific embodiments, the Yi region may contain exactly 2 charged residues (selected from R, K, D, E), as long as the net charge of the Yi region is zero. More particularly however, the Yi region does not contain any charged residue (or proline for that matter).

The specification also describes agrochemical compositions. These compositions comprise at least one molecule with the following structure(X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing;
and an agronomically acceptable carrier.

For agrochemical compositions, the at least one Yᵢ particularly will be a stretch naturally occurring in a protein in or on a plant, plant cell or plant seed. This may be proteins of a plant (typically the plant whereon or near which the agrochemical composition is applied), or proteins of plant pests that may occur on plants.

### Brief description of the Figures

**Figure 1****:** Venn diagram grouping amino acids according to their properties. Adapted from Livingstone & Barton, CABIOS, 9, 745-756, 1993.
**Figure 2****:** Percentage of unique sequences for a given peptide length in the proteome of different organisms. To generate the figure, the genome sequence of an organism was taken, all peptides of length X encoded in the genome were generated, then it was counted how often each peptide occurs, and the fraction that is unique (i.e. unique sequences vs all sequence of this length) is plotted.

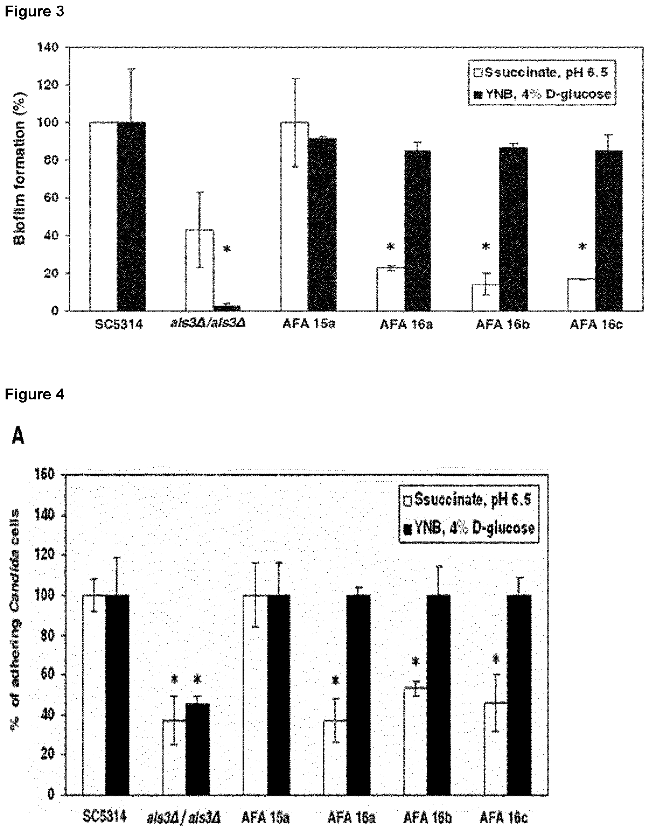

**Figure 3****:** Quantification of mature biofilms of strains expressing the Als3 aggregator construct under inducing and repressing conditions. Mature biofilms were grown on silicone square discs for 48 h in succinate (white bars) or in YNB, 4% D-glucose (black bars). *C*. *albicans* SC5314 was used as a control. Recombinant strains expressing Als3p interferors showed significantly reduced biofilm forming capacity when induced in comparison to control (**p* < 0.001). Quantification was performed by CFU counting and the data are presented as the percentage of the viable cells compared to the control (100%). Standard deviations were calculated from two independent experiments.

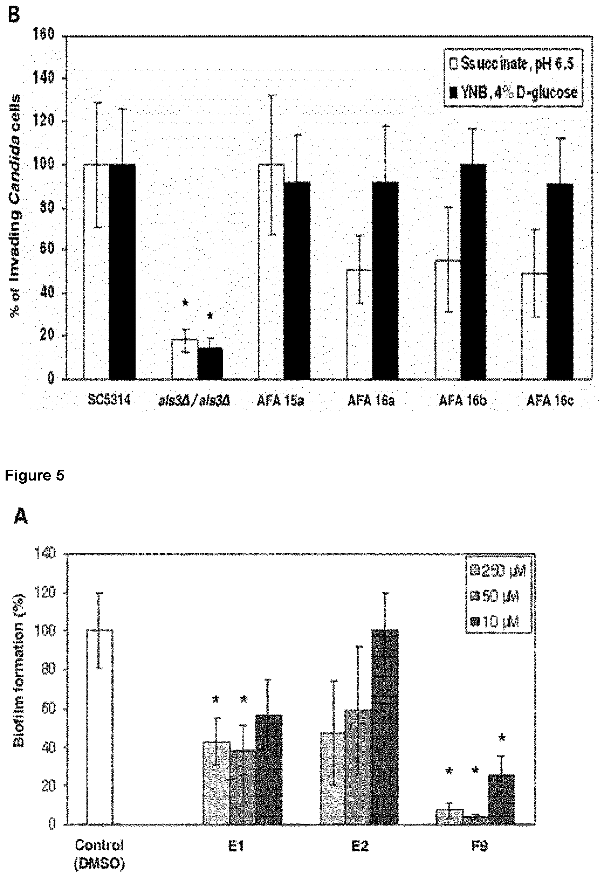

**Figure 4****:** Induced *Candida albicans* interferor constructs demonstrated reduced adhesion (panel A) and invasion (panel B) to epithelial cell line TR-146.

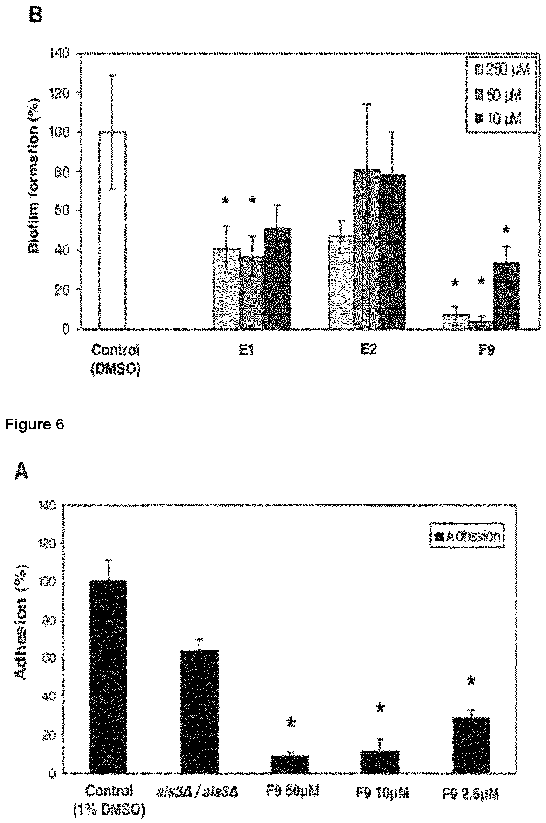

**Figure 5****:** Candidate peptide F9 results in strong inhibition of biofilm formation. *Candida albicans* SC5314 biofilm formation on 96-well polystyrene well plate in the presence of different concentrations (250 µM, 50 µM and 10 µM) of peptide E1 (RNGIVIVATTR (SEQ ID NO: 7)), peptide E2 (RLQQYTLLLIYLSVATAK (SEQ ID NO: 8)) and peptide F9 (RKLLFNLGSRNGIVIVATTR (SEQ ID NO: (9)). (A) Peptides were added only during *Candida* adhesion phase and mature biofilm development continued in fresh RPMI1640-MOPS, whereas on (B), peptides were present during whole biofilm development, including adhesion period. Biofilms were quantified by XTT reduction assay. Control (100%) contained only fresh medium with the final concentration of 1% DMSO. The presence of peptide E1 and F9, resulted in significant reduction in biofilm formation (**p* < 0.001). The data represent the percentage of the metabolic activity of the peptide-treated *Candida* cells compared to the control (100%). Standard deviations were calculated from three independent experiments. Each concentration of the peptide was tested in triplicate.

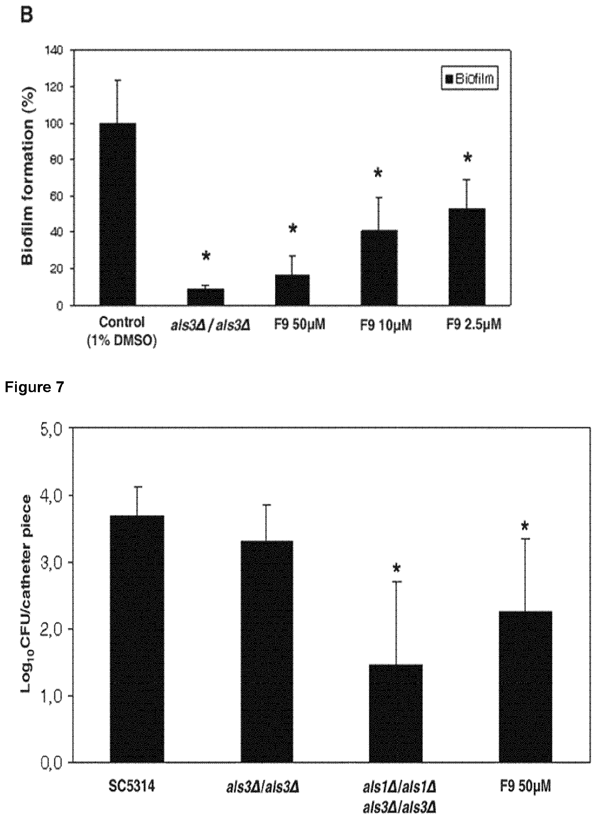

**Figure 6****:** Peptide F9 diminished *Candida albicans* SC5314 adhesion and biofilm formation on polystyrene. (A) C. *albicans* SC5314 adhesion on 96 well polystyrene plates in the presence of peptide F9 (50 µM, 10 µM and 2.5 µM). The XTT reduction assay was performed after 90 min of adhesion at 37 °C. (B) *In vitro* mature biofilm formation of *Candida* cells pretreated with different concentrations of peptide during period of adhesion. Further biofilm development (48 h) continued in RPMI1640-MOPS. Control sample was treated with 1% DMSO (100%). *C*. *albicans als3Δ*/*als3Δ* was used as a control. Peptide F9 significantly decreased adhesion and further mature biofilm development (**p* < 0.001). The data represent the percentage of the metabolic activity of the peptide-treated *Candida* cells, after adhesion (90 min) or 48 h biofilm formation, compared to the control (100%). Standard deviations were calculated from five independent experiments. Each concentration of the peptide was tested in triplicate.
**Figure 7****:** Peptide-treated *Candida albicans* SC5314 cells decreased the ability to form biofilm on polyurethane catheters *in vivo. C. albicans* SC5314 cells were incubated in the presence of peptide F9 (50 µM) during period of adhesion (90 min) at 37 °C. Afterwards, catheters were washed and implanted subcutaneously. Biofilms were formed for 6 days. *C*. *albicans als1Δ*/*als1Δ als3Δ*/*als3Δ* was used as a control. *C*. *albicans als3Δ*/*als3Δ* formed biofilms similarly to the wild type. Treatment of *Candida* cells during adhesion period with peptide F9 resulted in less *in vivo* mature biofilm formation (**p* < 0.001). The data are presented as the mean of Log₁₀ CFU obtained per device. In total, 20 devices were studied. In each experiment, two animals were used per strain or condition.

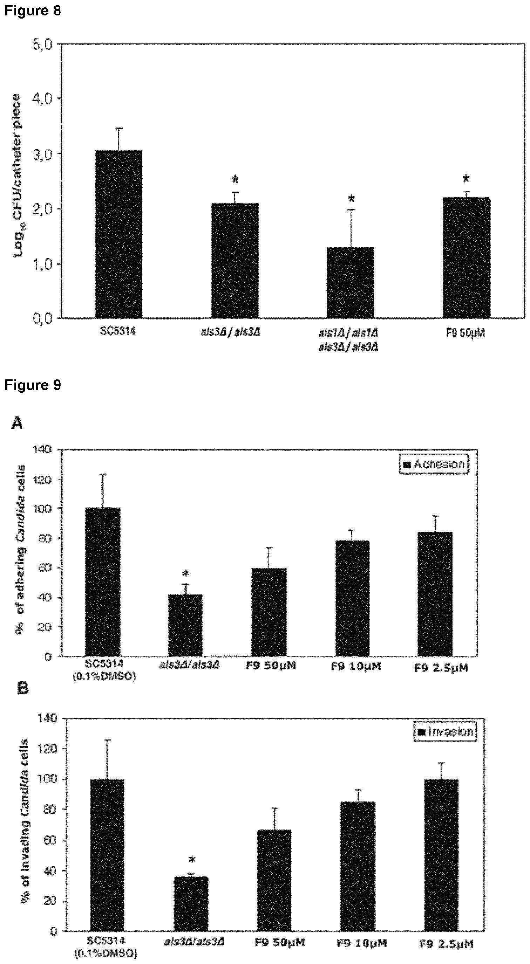

**Figure 8****:** Peptide F9 decreased the ability of *Candida albicans* SC5314 to adhere to polyurethane catheters. C. *albicans* SC5314 cells were treated with peptide F9 (50 µM) during the period of adhesion (90 min at 37 °C). Devices were washed and the quantification of attached *Candida* cells was performed by CFU counting. *C*. *albicans als3Δ*/*als3Δ* and C. *albicans als1Δ*/*als1Δ als3Δ*/*als3Δ* adhered significantly less compared to the control (**p* < 0.001). Wild type *Candida* cells treated with peptide F9 demonstrated reduced adhesion properties compared to non-treated cells (**p* < 0.001). The data are presented as the mean of Log₁₀ CFU obtained per device after period of adhesion. In total, 9 catheters were used per strain or condition tested.
**Figure 9****:** Peptide F9 decreases the ability of *Candida albicans* SC5314 to adhere and to invade epithelial cells. Three different concentrations (50 µM, 10 µM and 2.5 µM) of peptide F9 were administrated during *C*. *albicans* SC5314 adhesion (1 h) (A) and invasion (3 h) (B) to epithelial cell line TR-146. Adherent and invading parts of fungal cells within epithelial cells were stained with calcofluor white and counterstained with Alexa Fluor 488 secondary antibody. The coverslips were observed by epifluorescence. The percentage of adhered cells was calculated as an average of attached *Candida* cells on one hundred areas spread over the entire surface of the coverslip. Adherence and invading abilities of *C*. *albicans als3Δ*/*als3Δ* were significantly decreased (**p* < 0.001). The percentage of invading *C*. *albicans* cells was determined by dividing the number of internalized cells by the total number of adherent cells. At least 100 fungal cells were counted on each coverslip. Standard deviations were calculated from three independent experiments and each concentration of the peptide was tested in duplicate.

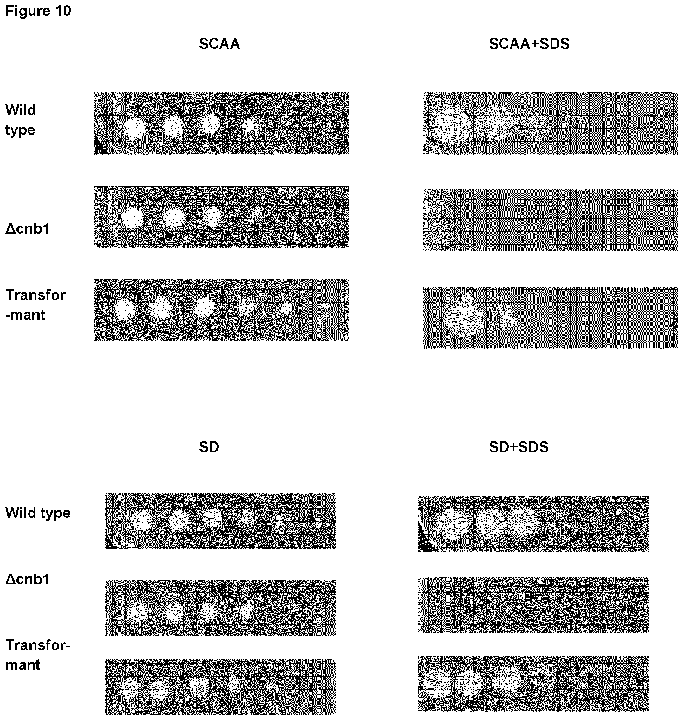

**Figure 10****:** The wild type, the homozygous deletion mutant and the Cnb1 transformant have a similar phenotype in normal promoter inducing/repressing conditions. When stress is induced by adding SDS (0,01%) to the medium, the transformant shows a phenotype that is similar to the wild type in promoter repressing conditions (SD medium), while the phenotype in promoter inducing conditions (SCAA medium) shows a bigger resemblance with the homozygous deletion mutant. Data shown are representative for the 4 constructs.

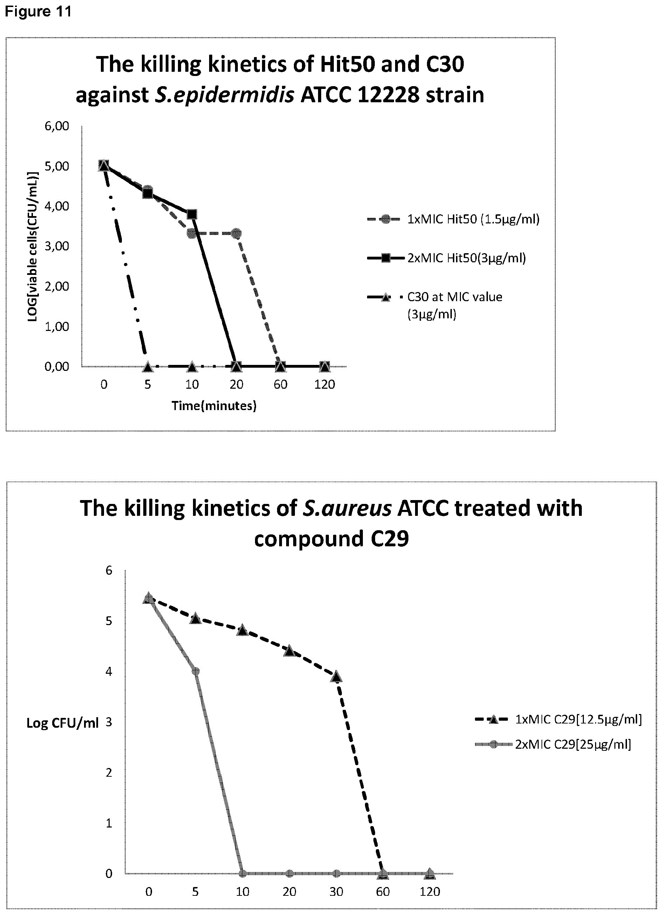

**Figure 11****:** The killing kinetics for *S.epidermidis* and *S.aureus,* treated with different interferor molecules, at their minimum inhibitory concentration (MIC) and 2x MIC.

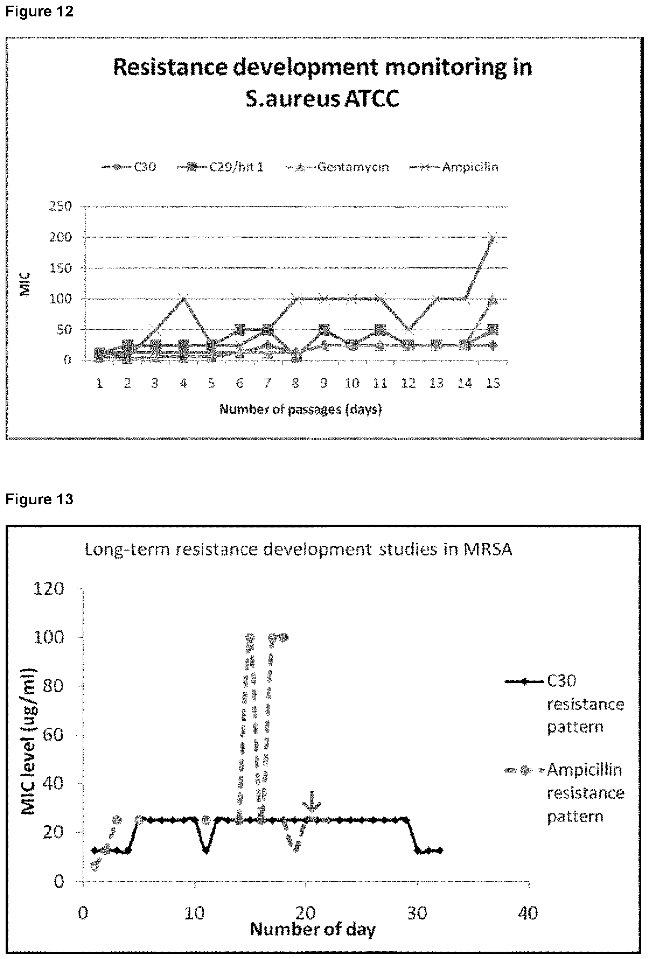

**Figure 12****:** Resistance development monitoring in S. aureus ATCC
**Figure 13****:** Long term resistance development studies in MRSA. Red line and arrow depict factor 2 drop in resistance for C30 due to withdrawal of peptide from the medium for 1 day.

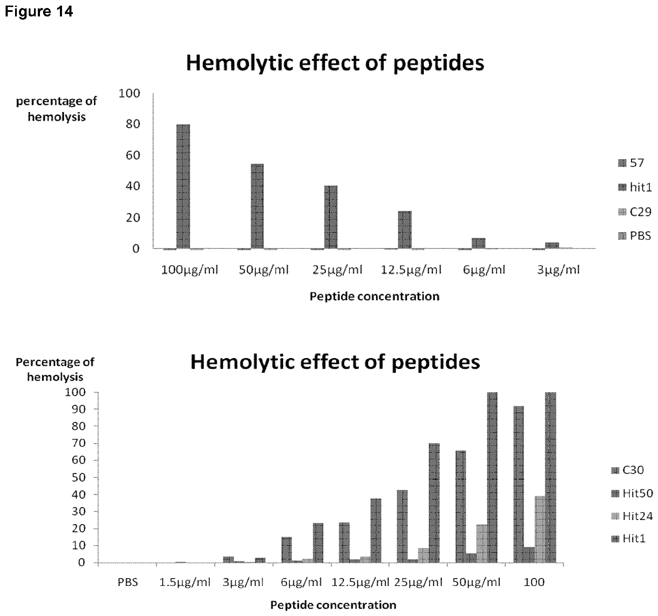

**Figure 14****:** Hemolytic properties of interferor peptides

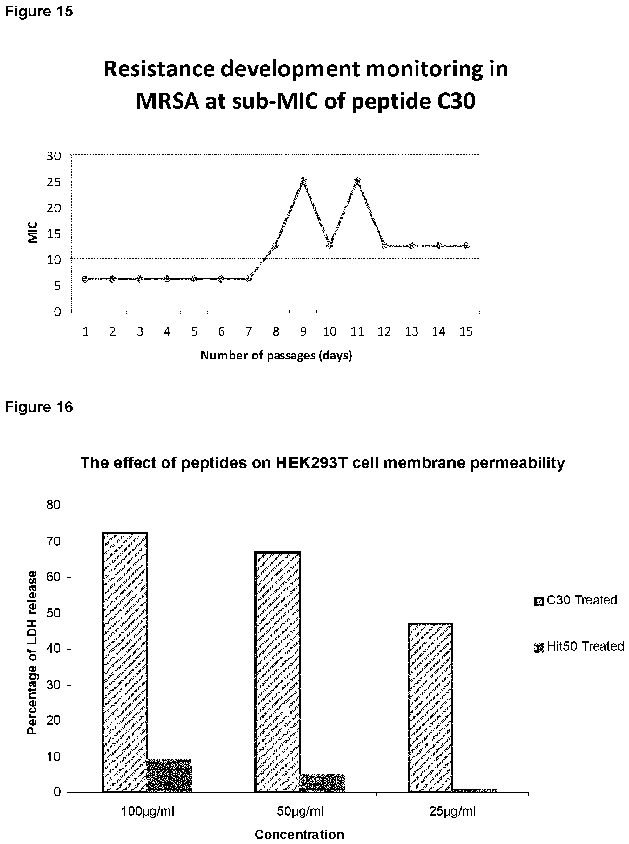

**Figure 15****:** Resistance curve for *S.aureus* cultured at sub-MIC over 15 days.
**Figure 16****:** Lactate dehydrogenase-release from Human embryonic kidney cells at different concentrations of peptide interferors C30 and Hit50

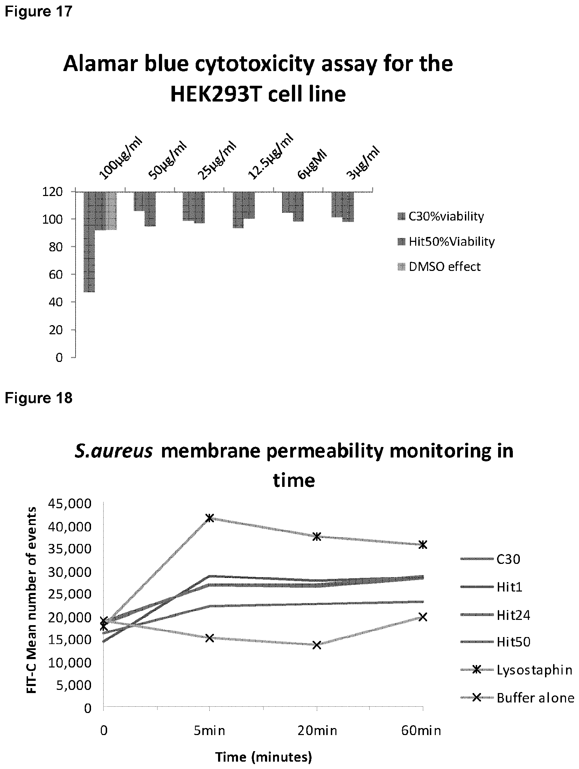

**Figure 17****:** Human epithelial kidney cell line recovery percentage after 24 hours incubation period in the presence of two different antibacterial peptide interferors (compound30 and Hit50).The figure shows a 90 percent recovery of HEK cells treated with 100µg/ml of Hit50. The growth inhibition is due to the presence of DMSO as buffer rather than it is due to the interferor peptide (the buffer used for interferor peptide solubilisation was 50% DMSO in ultrapure water).
**Figure 18****:** Green fluorescence intensity measurement of cells treated with various interferor peptides in time. Lysostaphin (upper line in the figure) represents here the lytic control, since it is known to disrupt the Staphylococcal cell wall very rapidly and leads to rapid cell content release. The lower line in the figure represents the buffer.

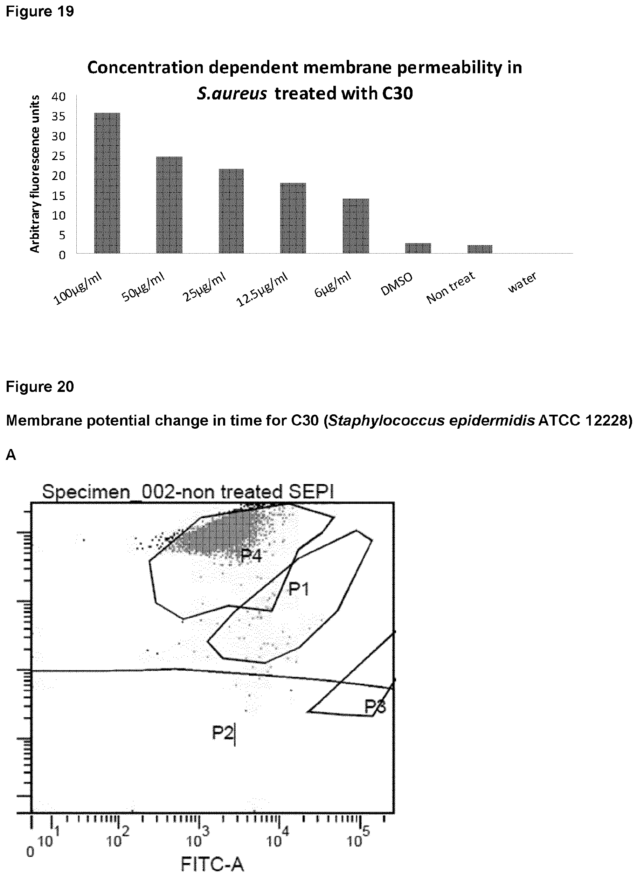

**Figure 19****:** Green fluorescence intensity (Sytox Green) measurement of cells treated with various concentration of interferor peptide C30.

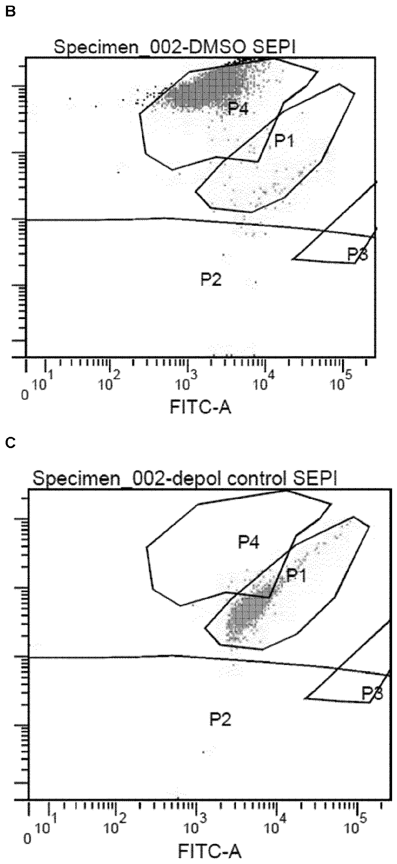

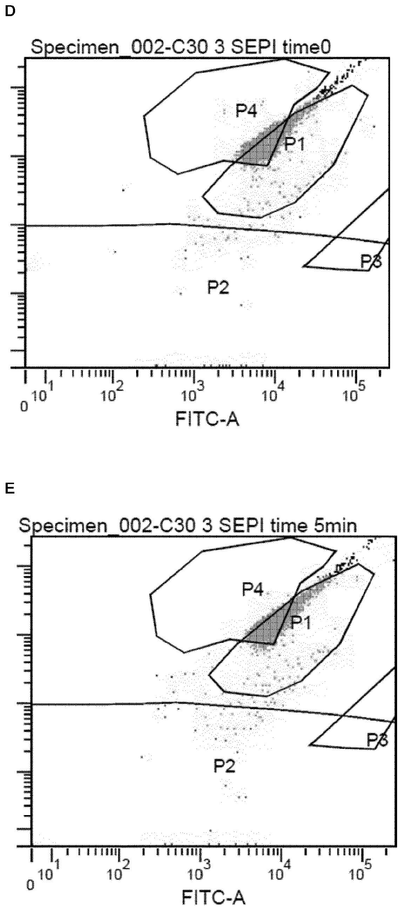

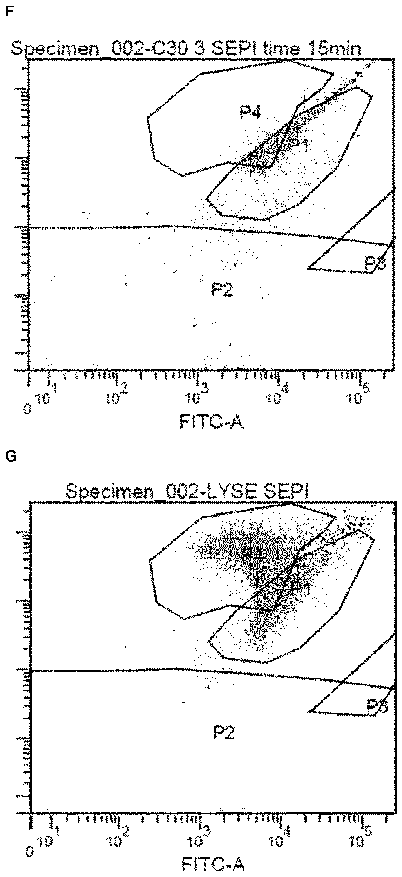

**Figure 20****:** Membrane potential (DiOC₂ red/green fluorescence dot plot) of non treated bacteria (A), DMSO-treated (B), depolarized by CCCP control(C), treated with lysostaphin (G), treated with C30 (25µg/ml) measured at 0 (D), 5 (E) and 15(F) minutes time points.

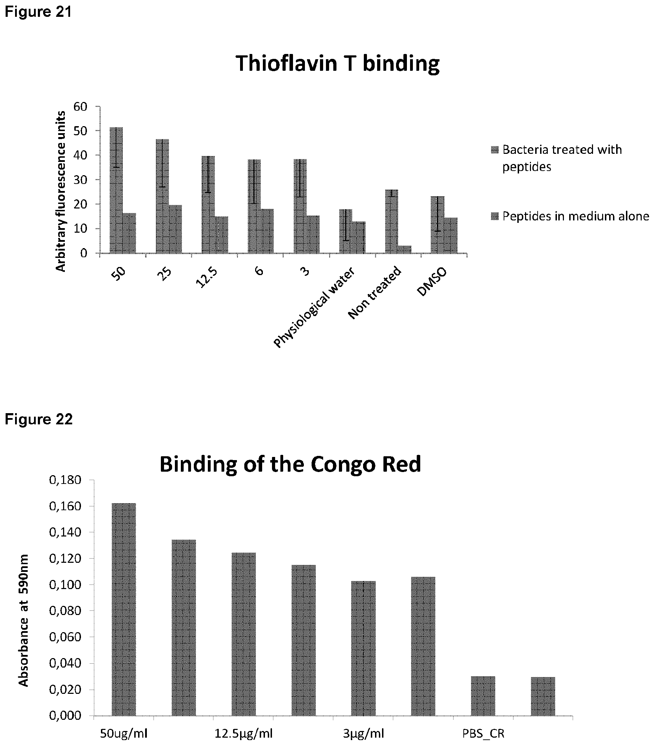

**Figure 21****:** Binding of thioflavin T amyloid diagnostic dye to *S.aureus* treated with different concentration of compound C30. Note that peptide interferor C30 solubilized in medium also give a certain background fluorescence.
**Figure 22****:** Binding of Congo red amyloid diagnostic dye to bacterial cells treated with increasing concentrations of Compound C30

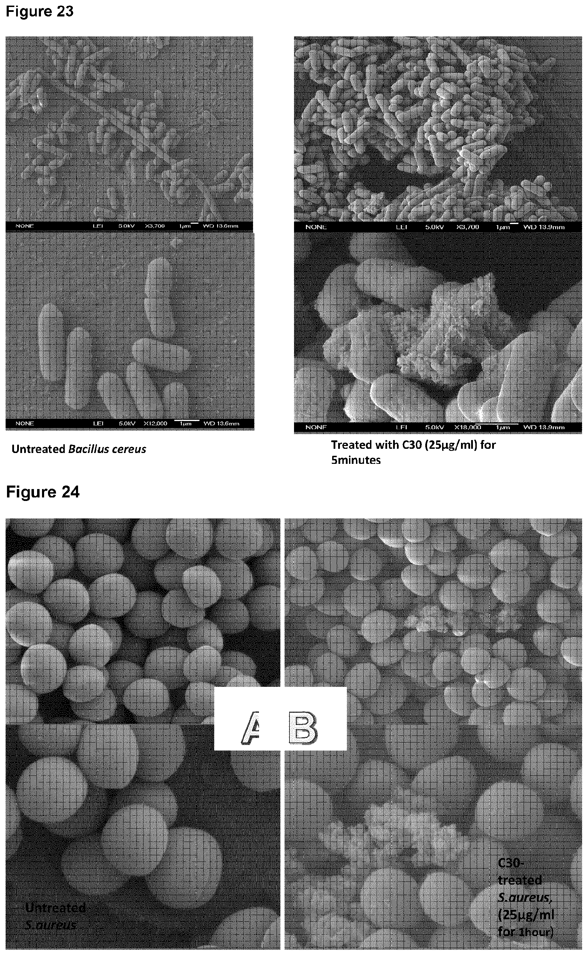

**Figure 23****:** SEM micrographs of untreated *Bacillus cereus* (left panel) and treated with compound C30 at 3xMIC for 5 minutes (right panel, top) or for 20 minutes (right panel, bottom). There is an obvious cell wall shrink of treated cells, with apparent content release. Additionally cells appear shorter, indicating hampered cell division. There are no visible pores, nor craters present on the cell surface.
**Figure 24****:** SEM micrographs of untreated (A) and C30-treated (B) *Staphylococcus aureus* ATCC. There is an obvious difference in a number of cells containing division septa, suggesting hindered division in cells treated with C30. After one hour of treatment some bacterial cells begin to lose their content.

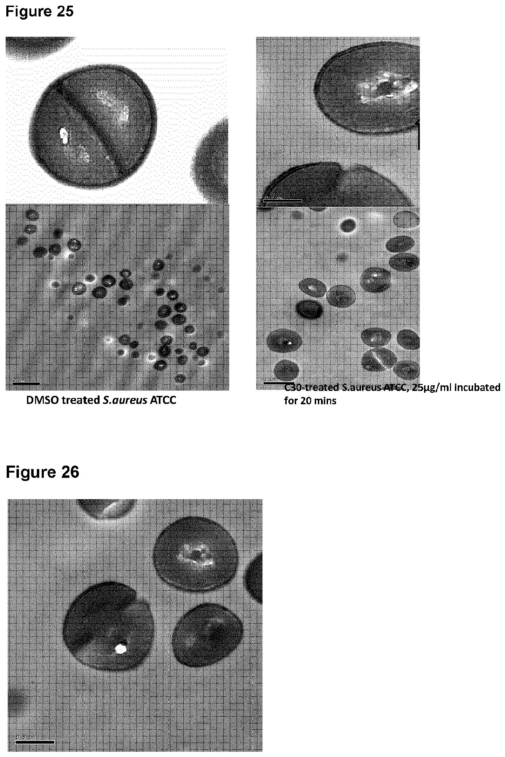

**Figure 25****:** Transmission electron micrographs of C30 treated (right panel) and DMSO-treated (left panel) *Staphylococcus aureus,* fixed after 20 minutes of treatment. The cells are round and intact, with a well-defined cell membrane. Some mesosome-like structures can be seen in the treated cell population (pointing arrow).
**Figure 26****:** TEM micrograph of staphylococci treated with C30 for 20 minutes at 4xMIC.The cytoplasmic content starts to shrink, however no obvious cell lysis can be observed. The DNA-region has very high electron density suggesting DNA condensation, which is a late stage sign of apoptosis.

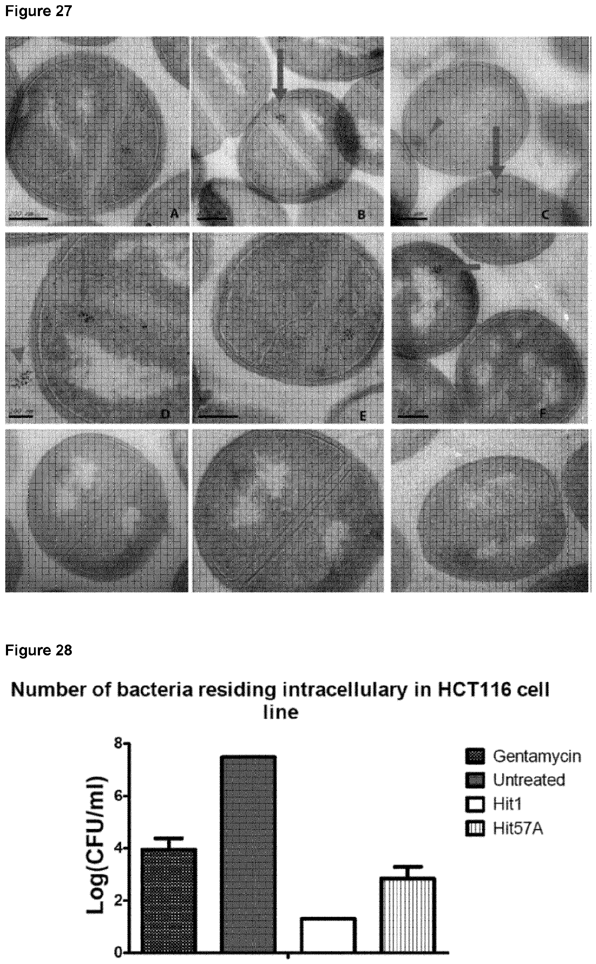

**Figure 27****:** Transmission electron micrographs of immuno-labeled ultrathin sections of *Staphylococcus aureus* exposed to FITC-tagged C30 (two top rows, images A to F) and immuno-labelled, unexposed to peptide sections (bottom row, unlabelled images). Red arrows shows peptide aggregates. Septa of peptide- treated cells show irregular shape and cell division is asymmetric. Peptides are taken up by the cell and aggregates reside in the cytoplasm.
**Figure 28****:** Peptide Hit1 (sequence: RWVSMLLRRGSRWVSMLLRR (SEQ ID NO: 31)) and Hit57A (sequence: RFFIGLSRRGSRIQAYLYRR (SEQ ID NO: 78)) effectively reduced the numbers of bacteria in infected cell monolayers. All compounds were used at 100 µg/ml.

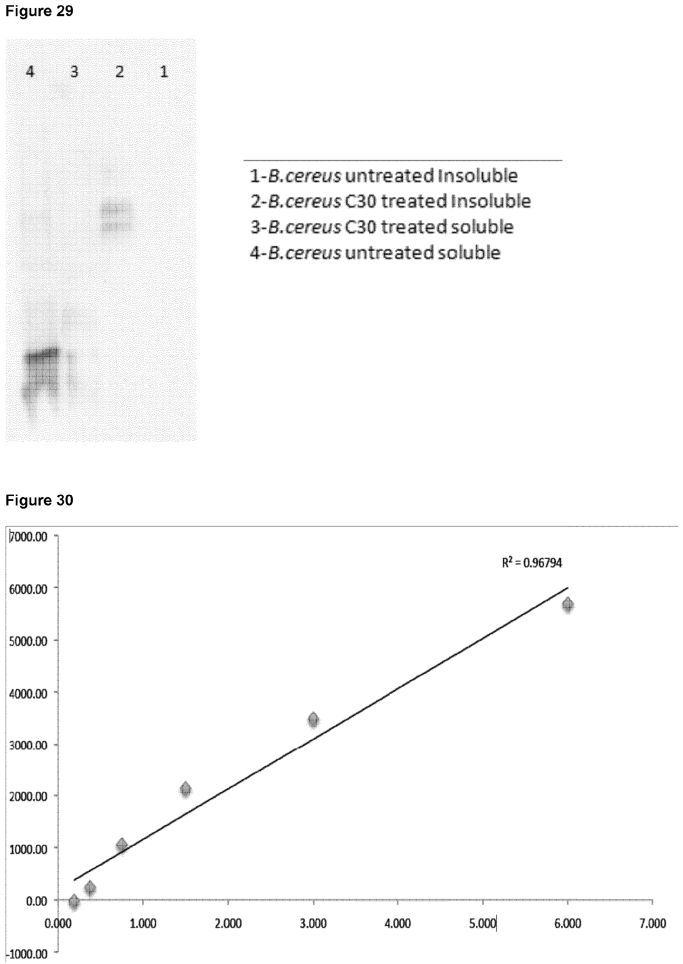

**Figure 29****:** Blots probed with anti-ClpC peptide
**Figure 30****:** Streptavidin-HRP signal intensity of spots containing different concentration of peptides

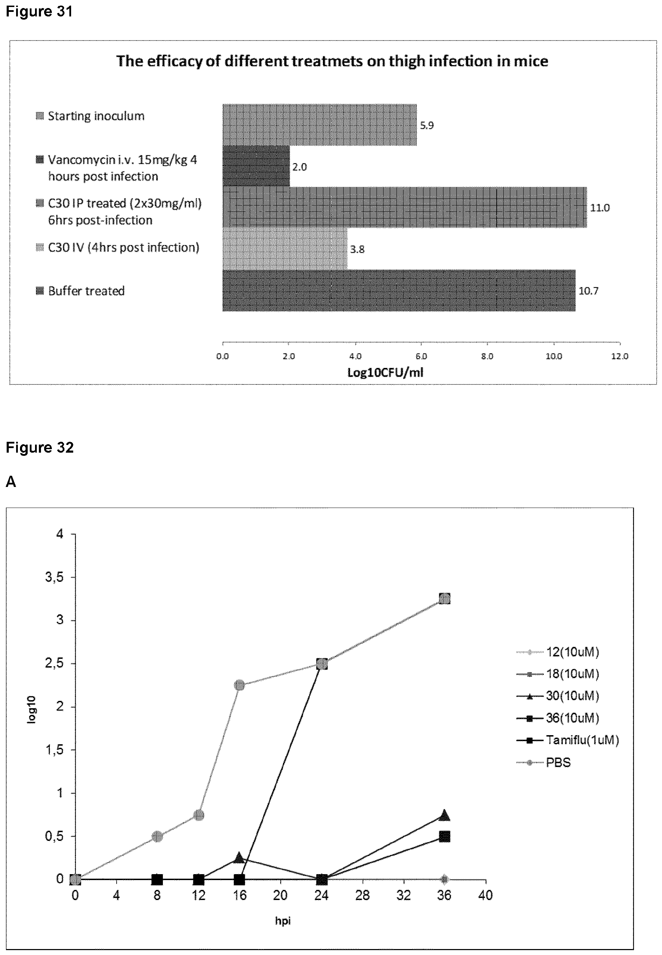

**Figure 31****:** Efficacies of vancomycin versus compound C30 delivered intravenously and intraperitoneally against Staphylococcus aureus MRSA 326 in the neutropenic-mouse thigh model

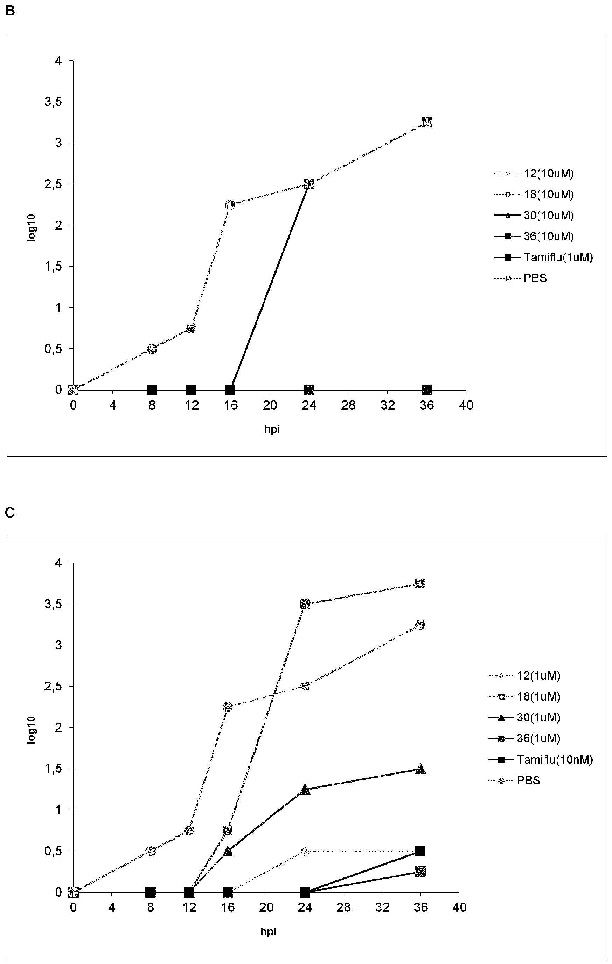

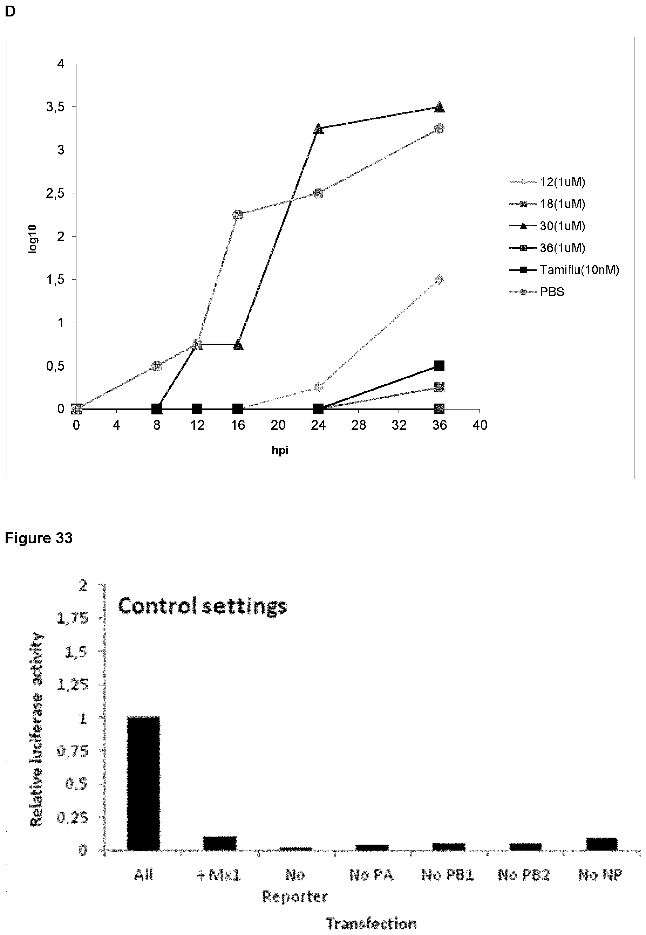

**Figure 32****:** Multistep virus growth curves on cells treated with 10µM (panels A, B) or 1µM (panels C, D) antiviral interferors, Tamiflu or PBS. Hpi, hours post infection.
**Figure 33****:** Relative luciferase activity following transfection of all or a subset of the minireplicon components. "Mx1": in addition to all minireplicon components an expression vector for Mx1 was also transfected.

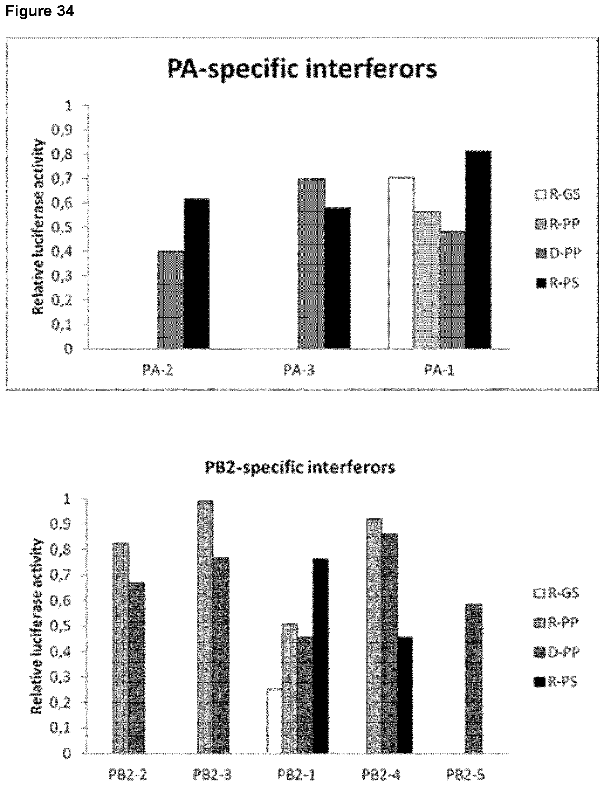

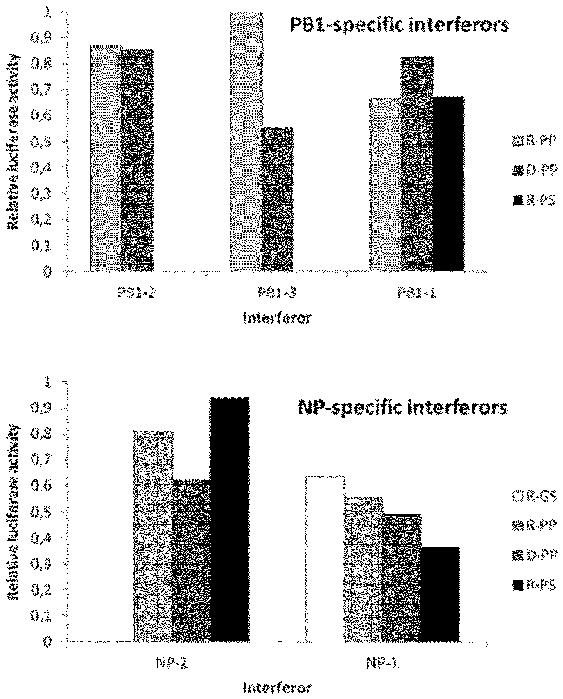

**Figure 34****:** Relative luciferase activity in the presence of interferors directed against different viral proteins. R-GS, R-PP, D-PP, R-PS indicate nature of gatekeeper (before hyphen) and internal linker (after hyphen) used.

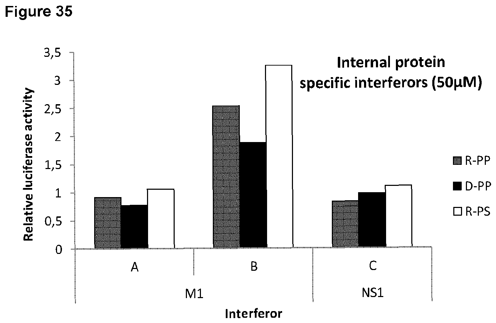

**Figure 35****:** Relative luciferase activity in the presence of interferors directed against different internal viral proteins (M1 and NS1) that are not involved in the minireplicon assay. Value of control = 1. R-PP, D-PP, R-PS indicate nature of gatekeeper (before hyphen) and internal linker (after hyphen) used.

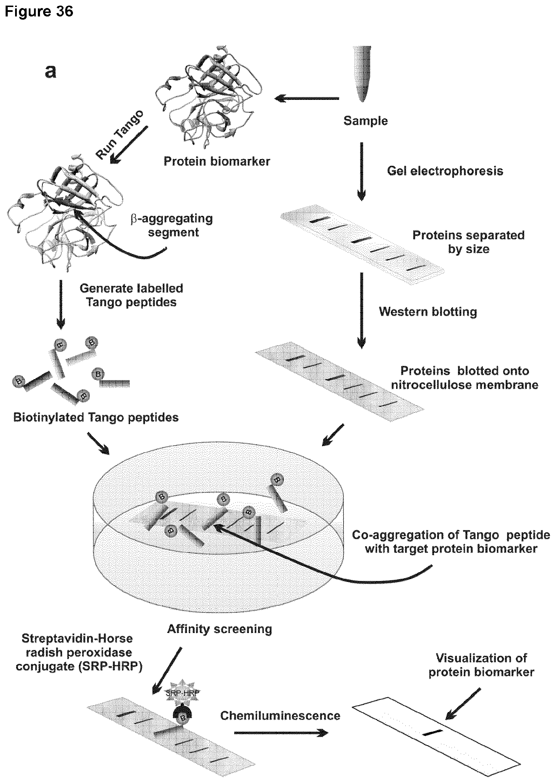

**Figure 36****:** schematic overview of the detection technology by means of specific interferors

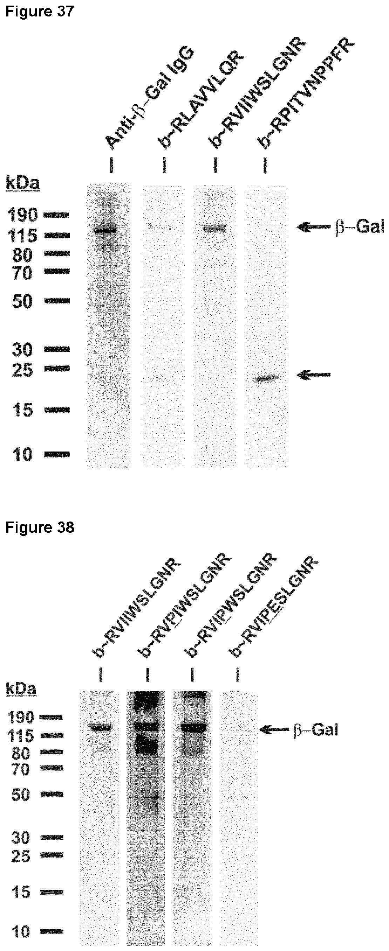

**Figure 37****:** Western blot and PepBlot analysis of β-Gal from complete bacterial BL21 cell lysates. Lane 1 shows the detection with rabbit polyclonal anti-β-Gal antibody; Lane 2 shows the detection with the Tango 1 peptide interferor (*b*^{∼}RLAVVLQR (SEQ ID NO: 79)); Lane 3 shows the detection with the Tango 2 peptide interferor (*b*^{∼}RVIIWSLGNR (SEQ ID NO: 80)); and Lane 4 shows the interaction with the off-target peptide interferor (*b*^{∼}RPITVNPPFR (SEQ ID NO: 81)). The membranes were exposed with chemiluminescence HRP substrate for 100 s. The upper arrow depicts the predicted position of the β-Gal protein on the blot. The lower arrow depicts the aspecific binding product, which is presumably free SRP.
**Figure 38****:** PepBlot analysis of wt and mutant Tango 2 peptide binding specificity to β-Gal from whole BL21 cell lysates: Lane 1 - wt peptide (*b*^{∼}RVIIWSLGNR (SEQ ID NO: 80)); Lane 2 - mut_1 peptide (*b*^{∼}RVPIWSLGNR (SEQ ID NO: 82)); Lane 3 - mut_2 peptide (*b*^{∼}RVIPWSLGNR (SEQ ID NO: 83)); and Lane 4 - mut_3 peptide (*b*^{∼}RVIPESLGNR (SEQ ID NO: 84)). The membranes were exposed with chemiluminescence HRP substrate for 100 s. The arrow indicates the position of β-Gal on the membrane strips.

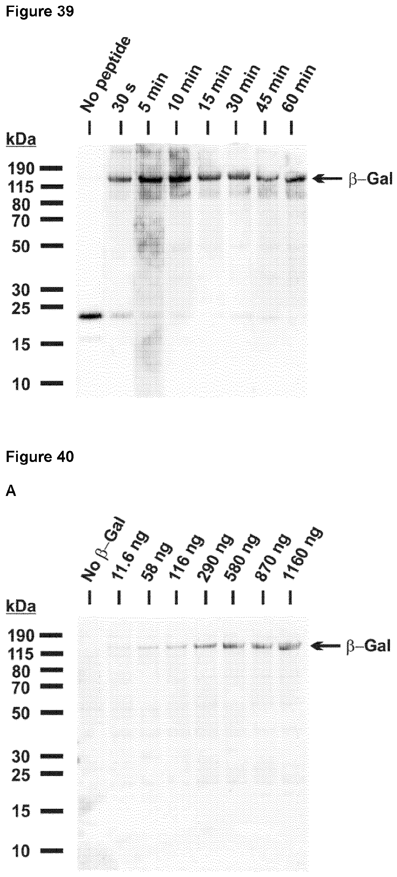

**Figure 39****:** PepBlot analysis of Tango 2 peptide (*b*^{∼}RVIIWSLGNR (SEQ ID NO: 80)) binding kinetics to β-Gal from whole BL21 cell lysates. The proteins separated in the membrane were exposed to the Tango 2 interferor peptide in the binding buffer for different time points: Lane 1 - No exposure to peptide; Lane 2 - 30 s; Lane 3 - 5 min; Lane 4 - 10 min; Lane 5 - 15 min; Lane 6 - 30 min; Lane 7 - 45 min; and Lane 8 - 60 min incubation. The membranes were exposed with chemiluminescence HRP substrate for 100 s. The arrow indicates the position of β-Gal on the membrane strips.

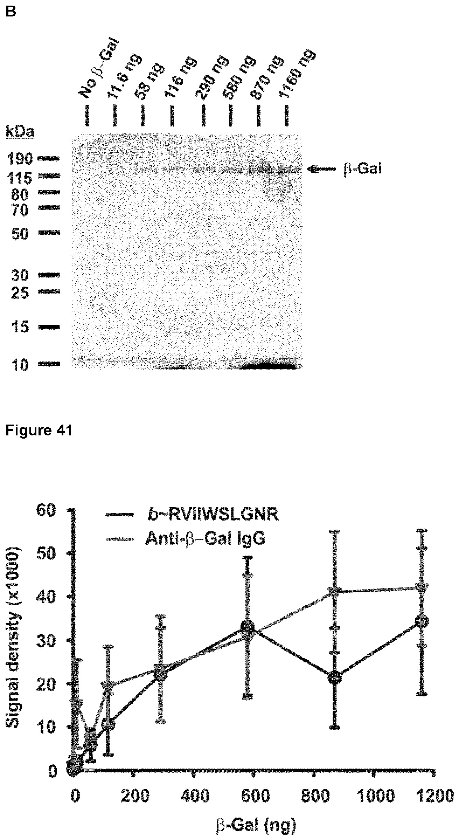

**Figure 40****:** Analysis of different β-gal protein levels spiked in non-induced complete BL21 cell lysates. Detection was accomplished by using (A) PepBlot with Tango 2 peptide interferor (*b*^{∼}RVIIWSLGNR (SEQ ID NO: 80)) and (B) WB with rabbit polyclonal anti-β-Gal antibody. Lane 1 - No β-Gal; Lane 2 - 11.6 ng β-Gal; Lane 3 - 58 ng β-Gal; Lane 4 - 116 ng β-Gal; Lane 5 - 290 ng β-Gal; Lane 6 - 580 ng β-Gal; Lane 7 - 870 ng β-Gal; and Lane 8 - 1160 ng β-Gal. The membranes were exposed with chemiluminescence HRP substrate for 100 s. The arrow shows the position of β-gal on the membrane.
**Figure 41****:** Comparison of the PepBlot and WB detection signal density of Tango 2 peptide interferor (*b*^{∼}RVIIWSLGNR (SEQ ID NO: 80)) (black line with circles) and anti-β-Gal antibody (red line with triangles) of different β-gal protein levels spiked in non-induced complete BL21 cell lysates.

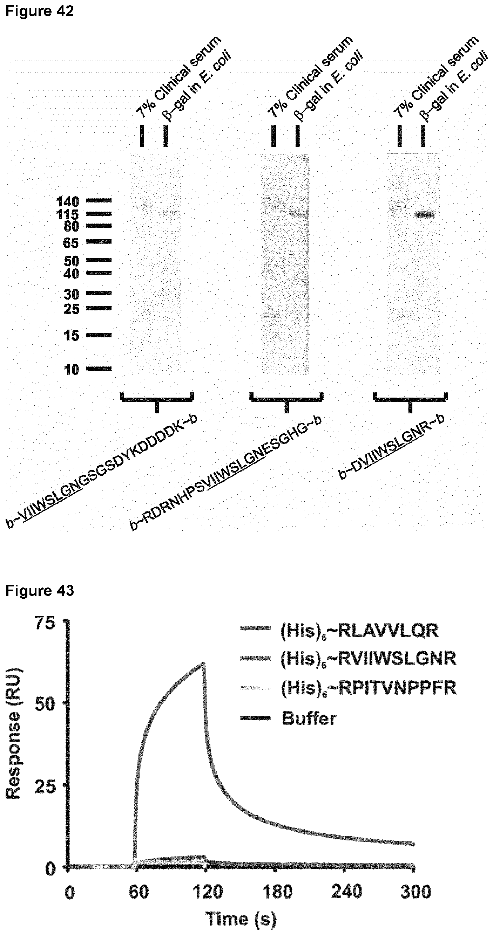

**Figure 42****:** Influence of gatekeeper residues on the specificity of interferor peptide based PepBlot detection of β-gal in complete *E. coli* lysate in the presence of serum proteins in adjacent lane. The detection was performed in a competitive platform with lane 1 of each membrane containing 7% clinical serum and lane 2 is the β-gal in complete *E. coli* BL21 cell lysate. Peptides were labeled with biotin on both the terminus and linked by Ttds linker. Membranes containing separated serum and E. *coli* proteins were incubated with 25 nM interferor peptides for 15 min, followed by SRP-HRP conjugate and exposed to chemiluminescence HRP substrate for 120 s. Note the high MW band observed for serum is due to cross reaction of SRP-HRP conjugate.
**Figure 43****:** Experimental sensorgram comparing the affinity of various peptides to bind β-Gal: Tango 1 (red), Tango 2 (green, highest curve), off-target peptide (yellow) and the buffer reference (black). Note that the peptides (1 µM) flowed over the same chip with a surface regeneration step after each contact.

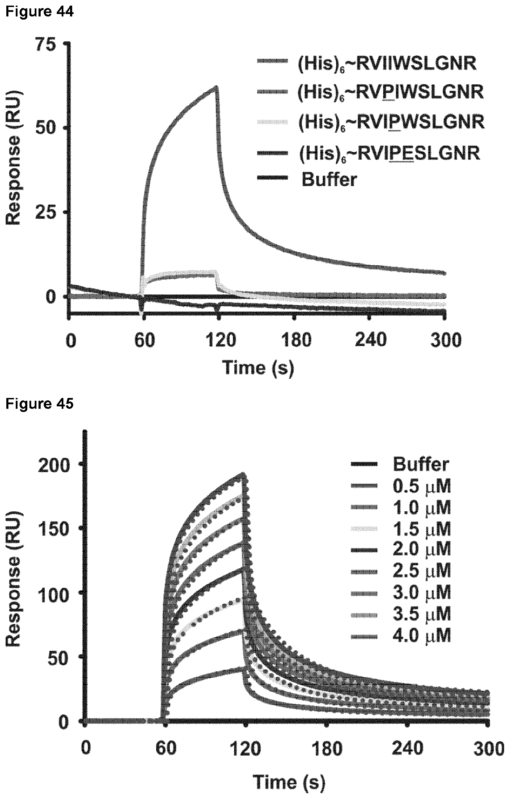

**Figure 44****:** Experimental sensorgram comparing the affinity of wt and mutant Tango 2 peptides to bind β-Gal: Tango 2 wt (red, highest curve), Tango 2 Mut_1 (green), Tango 2 Mut_2 (yellow), Tango 2 Mut_3 (blue) and the buffer reference (black). Note that the peptides (1 µM) flowed over the same chip with a surface regeneration step after each contact.
**Figure 45****:** Representative sensorgram of fitted data from the kinetic analysis of peptide, (His)₆^{∼}RVIIWSLGNR (SEQ ID NO: 80), binding to β-Gal immobilized on the sensor chip. The solid line is the experimental sensorgram and the dot line superimposed on the response is the fitted curve. The analyte concentrations from the bottom to top were, 0.5 µM, 1.0 µM, 1.5 µM, 2.0 µM, 2.5 µM, 3.0 µM, 3.5 µM and 4.0 µM, The middle curve (2 µM) was run in duplicate. Note the peptides flowed over the same chip with a surface regeneration step after each contact.

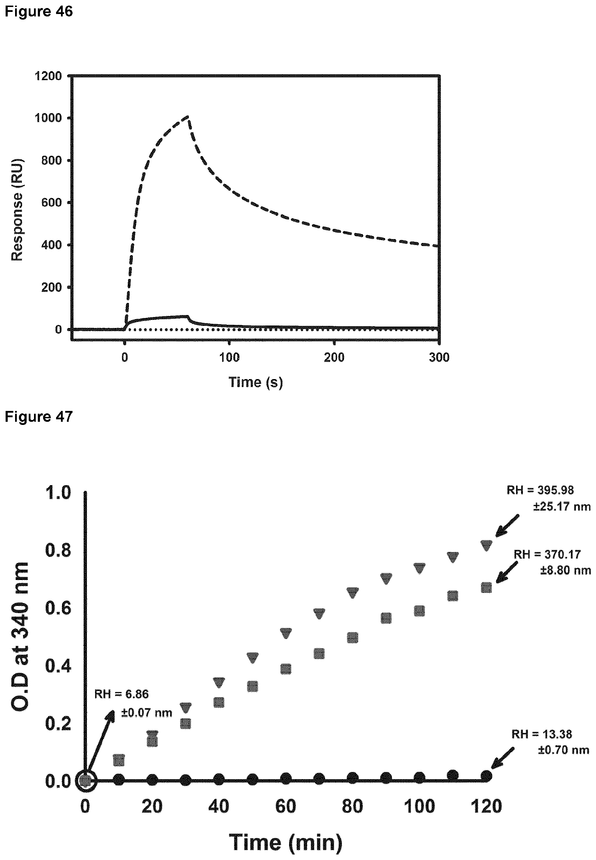

**Figure 46****:** Experimental sensorgram comparing the affinity of single Tango 2 (solid line) and tandem Tango 2 (dash line) peptides to bind β-Gal. The buffer reference is shown as dotted line. Note that the peptides (1 µM) flowed over the same chip with a surface regeneration step after each contact.
**Figure 47****:** Comparison of Tango 1 peptide (triangle) and Tango 2 peptide (square) co-aggregation kinetics with β-gal. The concentrations of interferor peptides and β-gal were 10 µM. The black circles represent the β-gal in the absence of the peptide. The arrows point to the hydrodynamic radius (RH) of the particles measured using DLS at the respective time point.

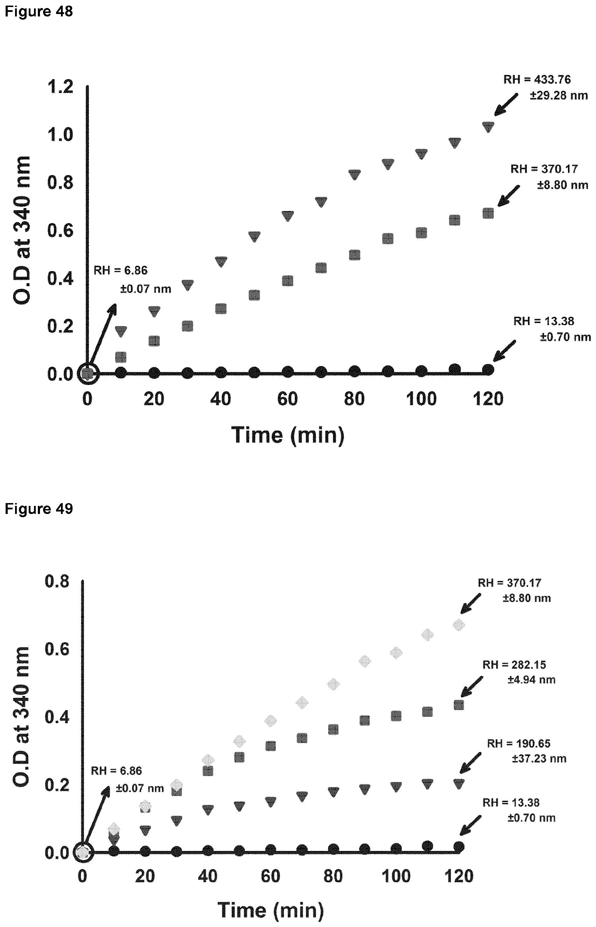

**Figure 48****:** Comparison of single Tango 2 peptide (square) and tandem repeat Tango 2 peptide (triangle) co-aggregation kinetics with β-gal. The amount of interferor peptides and β-gal were 10 µM. The black circles represent β-gal in the absence of the peptide. The arrows point to the hydrodynamic radius (RH) of the particles measured using DLS at the respective time point.
**Figure 49****:** Effect of peptide concentration on the kinetics of Tango 2 peptide co-aggregation with β-gal. The peptide to β-gal molar ratio was: 1:5 (triangle), 1:2 (square) and 1:1 (diamond). The black circles represent the β-gal in the absence of the peptide. The arrows point to the hydrodynamic radius (RH) of the particles measured using DLS at the respective time point.

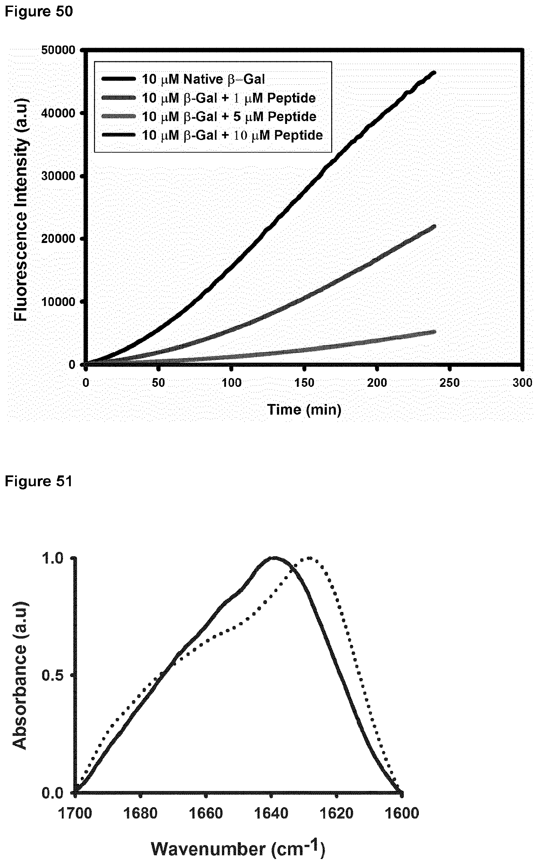

**Figure 50****:** Effect of the Tango 2 interferor peptide on the enzymatic activity of β-gal to cleave the non-fluorescent substrate FDG to fluorescing fluorescein. The highest fluorescent intensity is obtained with 10 µM native beta-Gal without interferor, followed by 10 µM β-Gal + 1 µM peptide interferor, followed by 10 µM beta-Gal + 5 µM peptide interferor. No fluorescence was observed in the condition 10 µM β-Gal + 10 µM peptide interferor.
**Figure 51****:** Attenuated total reflectance FT-IR spectra of native β-Gal (solid line) and β-Gal-Tango 2 peptide interferor co-aggregate suspension (dot line) in 20 mM PB, pH 6.8.

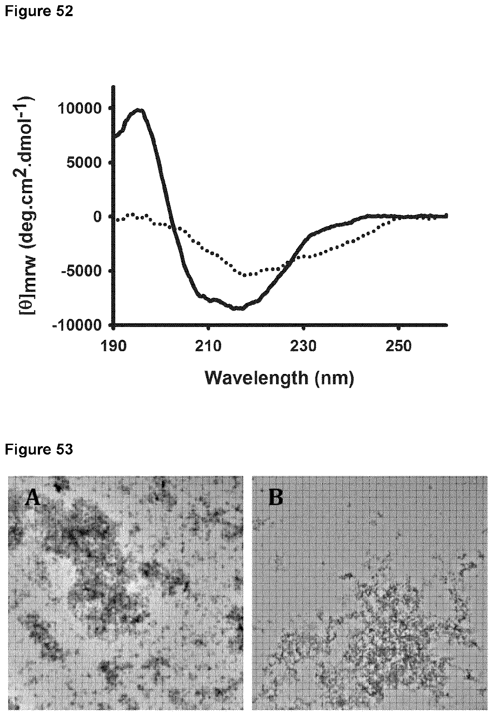

**Figure 52****:** CD spectra of native β-Gal (solid line) and β-Gal-Tango 2 peptide interferor co-aggregate suspension (dot line) in 20 mM PB, pH 6.8.
**Figure 53****:** EM of (A) native β-Gal and (B) β-Gal-Tango 2 peptide interferor co-aggregate

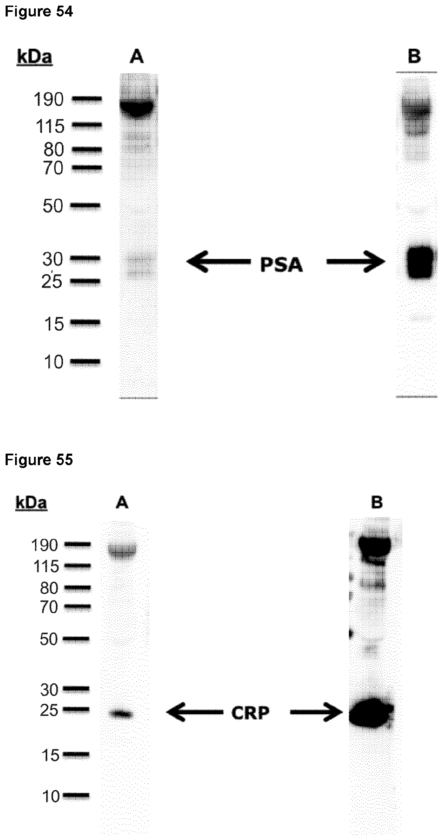

**Figure 54****:** PepBlot and WB analysis of PSA using peptide interferor, *b*^{∼}RQWVLTAAR (SEQ ID NO: 85) (panel A) and rabbit monoclonal anti-PSA antibody (panel B). The membrane contains 1.55 µg PSA spiked in 5% human serum. The arrow depicts the PSA position on the blots. The membranes with interferor peptide and anti-PSA antibody were exposed with chemiluminescence HRP substrate for 180 s and 10 s, respectively. The high molecular band noticeable in PepBlot and WB is due to non-specific binding of the SRP-HRP conjugate and the secondary antibody-HRP, respectively.
**Figure** 55: PepBlot and WB analysis of 1.25 µg CRP spiked in 5% human serum using interferor peptide, *b*^{∼}RILIFWSR (SEQ ID NO: 86) (panel A) and rabbit monoclonal anti-CRP antibody (panel B). The membranes with interferor peptide and anti-CRP antibody were exposed to chemiluminescence HRP substrate for 60 s and 10 s, respectively. The arrow depicts the CRP position in the blots. The high molecular band noticeable in interferor and antibody blot is due to non-specific binding of the SRP-HRP conjugate and the secondary antibody-HRP, respectively.

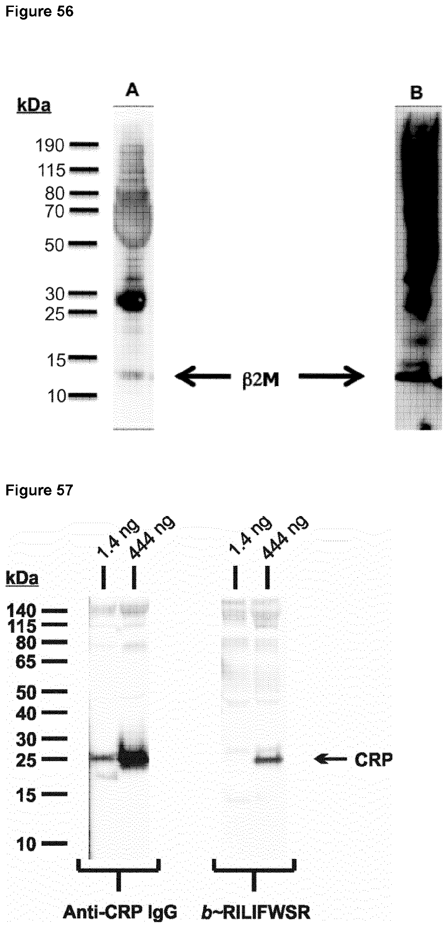

**Figure 56****:** PepBlot and WB analysis of 0.6 µg β-2M spiked in 5% human serum using peptide interferor, *b*^{∼}RWSFYLLYYTR (SEQ ID NO: 87) (panel A) and anti-β-2M antibody (panel B). The membranes with interferor peptide and anti-β-2M antibody were exposed to chemiluminescence HRP substrate for 180 s and 10 s, respectively. The arrow depicts the β-2M positions in the blots.
**Figure 57****:** CRP detection in clinical serum samples by WB using rabbit monoclonal anti-CRP antibody and PepBlot using interferor peptide *b*^{∼}RILIFWSR (SEQ ID NO: 86).

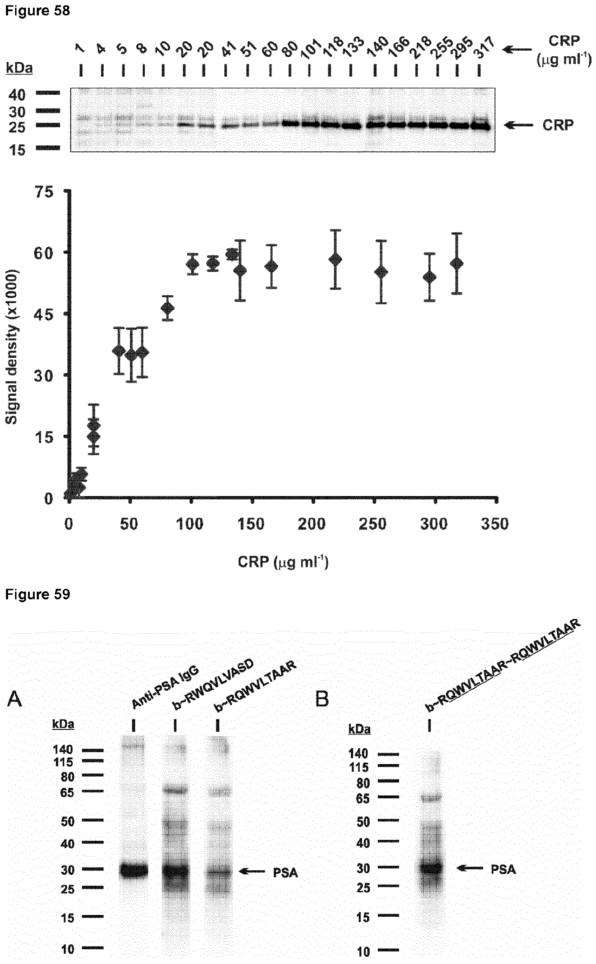

**Figure 58****:** CRP detection in clinical serum samples of 20 patients by PepBlot using the interferor peptide *b*^{∼}RILIFWSR (SEQ ID NO: 86) (top panel). The concentrations of CRP in clinical serum determined by immunoturbidimetric assay were in the range 1 µg ml⁻¹ to 317 µg ml⁻¹. Bottom panel shows the plot of CRP band signal density detected by the peptide *b*^{∼}RILIFWSR (SEQ ID NO: 86) versus concentration determined independently with an immunoturbidimetric assay at a clinical laboratory.
**Figure 59****:** (A) Detection of PSA secreted in human seminal plasma by PepBlot using the peptides *b*^{∼}RWQVLASD (SEQ ID NO: 88) (lane 2) and *b*^{∼}RQWVLTAAR (SEQ ID NO: 85) (lane 3). The WB of monoclonal antibody (EP1588Y) specific to the C-terminal sequence of PSA is shown in lane 1. (B) PSA detection using the tandem repeat interferor peptide *b*^{∼}RQWVLTAARGSGSAPAARQWVLTAAR (SEQ ID NO: 89). The biotin (b) is linked to the peptide by 'Ttds-APAA'(SEQ ID NO: 77) linker and the two aggregating sequences were linked by 'GSGSAPAA' (SEQ ID NO: 90) linker. Note the concentration of the single and tandem repeat peptide were 250 nM and 250 pM, respectively. The membranes were exposed to chemiluminescence HRP substrate for 36 s.

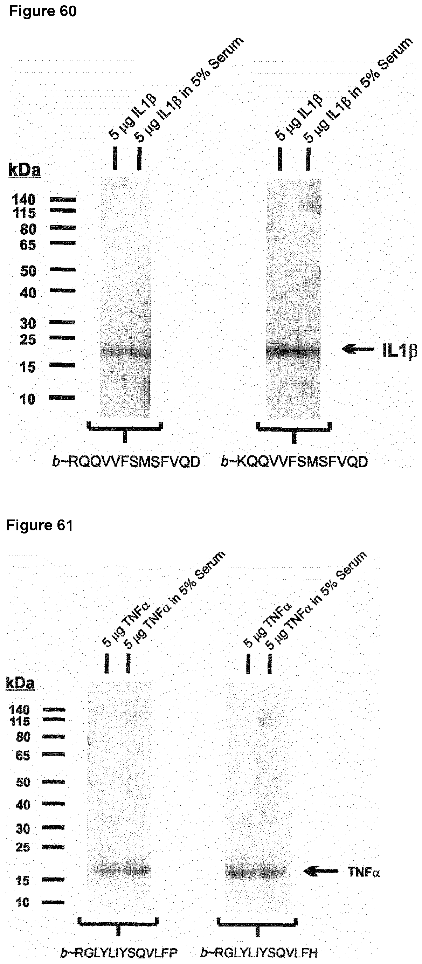

**Figure 60****:** PepBlot (i.e. interferor-based) detection of 5 µg IL1β spiked in 5% human serum
**Figure 61****:** PepBlot detection of 5 µg TNFα spiked in 5% human serum

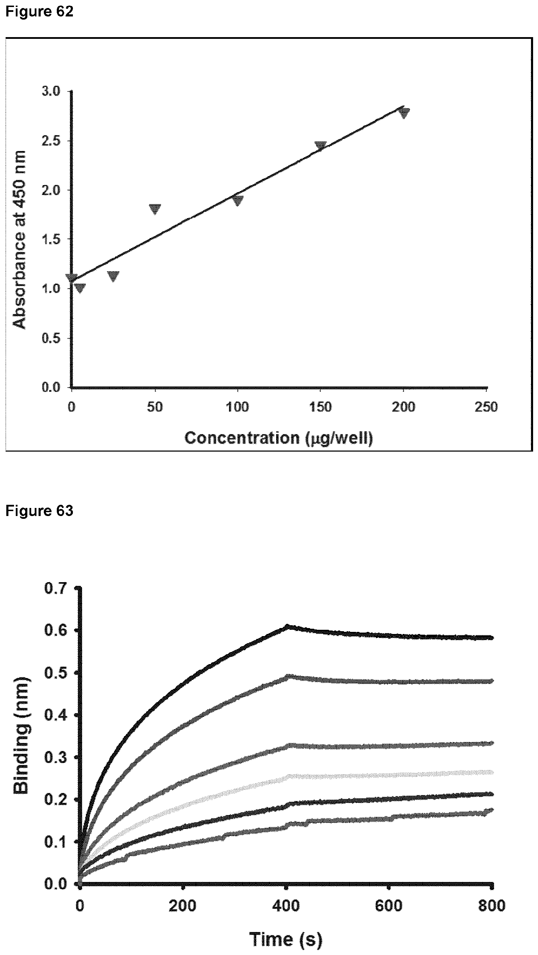

**Figure 62****:** Quantitative detection of different levels of β-gal spiked in complete *E. coli* cell lysates using the peptide *b*^{∼}RVIIWSLGNRGSGSAPAARVIIWSLGNR (SEQ ID NO: 91). The biotin (b) is linked to the peptide by 'Ttds-APAA' (SEQ ID NO: 77) linker and the two aggregating sequences were linked by 'GSGSAPAA'(SEQ ID NO: 90) linker.
**Figure 63****:** Kinetic characterization of the peptide *b*^{∼}RVIIWSLGNRGSGSAPAARVIIWSLGNR (SEQ ID NO: 91) interaction with β-Gal. The concentrations of the target protein β-Gal were in the range 31 pM to 1 µM.

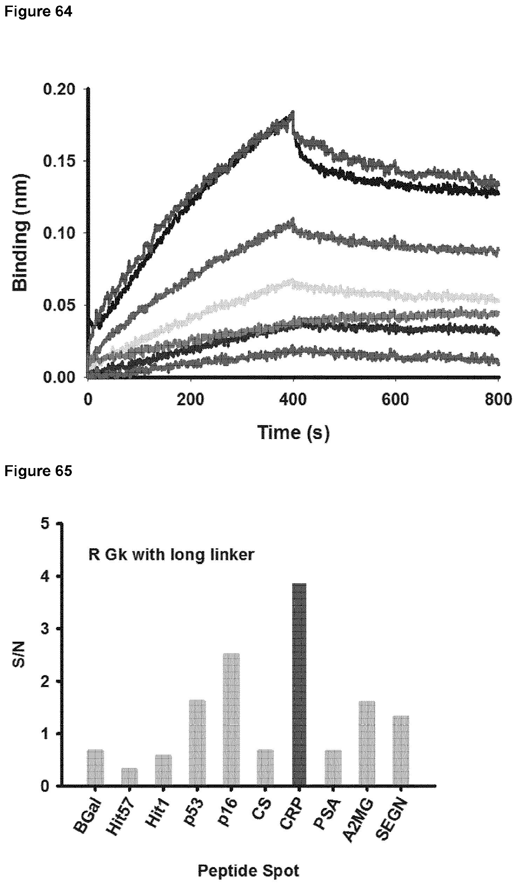

**Figure 64****:** Kinetic characterization of the peptide *b*^{∼}RILIFWSRGSGSAPAARILIFWSR (SEQ ID NO: 92) interaction with CRP. The concentrations of the target protein CRP were in the range 37 µM to 582 mM.

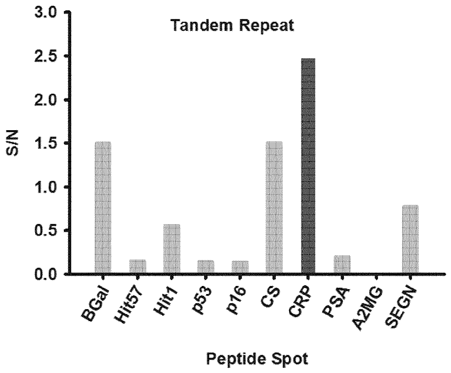

**Figure 65****:** CRP detection in clinical serum by using 'R' flanked single interferor peptide (top panel) or 'R' flanked tandem repeat interferor peptide (bottom panel) microarray. X axis shows peptides spotted, Y axis indicates signal to noise ratio. The concentration of CRP in serum was 317 µg ml⁻¹.

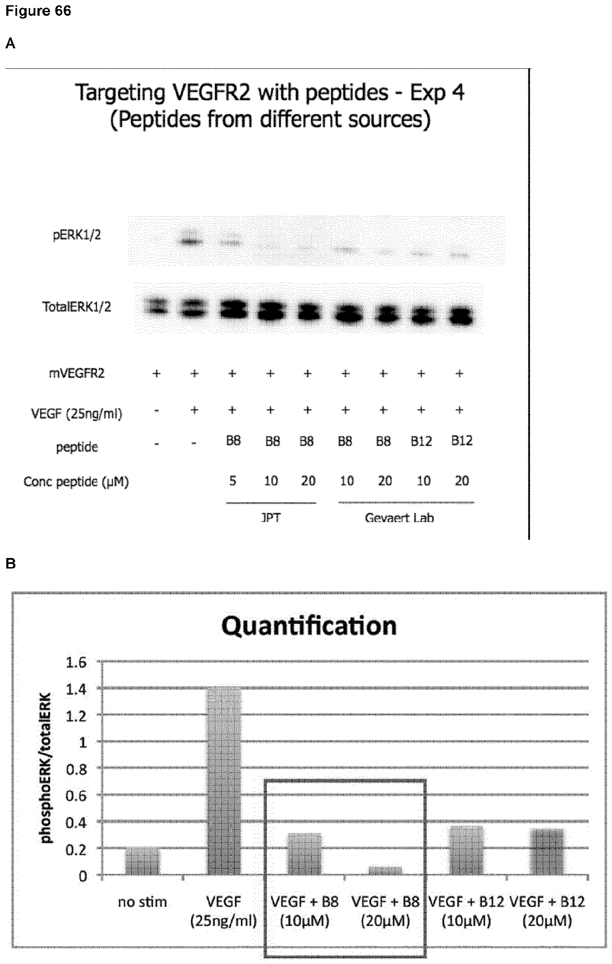

**Figure 66****:** A, Western blot analysis of phospho- and total ERK1/2 of Hek293 cells transfected with mVEGFR2, followed by an overnight starvation in presence or absence of peptides from different sources, and stimulated for 5 minutes with VEGF (25ng/ml). B, quantification of the ratio phosphorylated versus total ERK1/2 levels for some of the peptides shown in A.

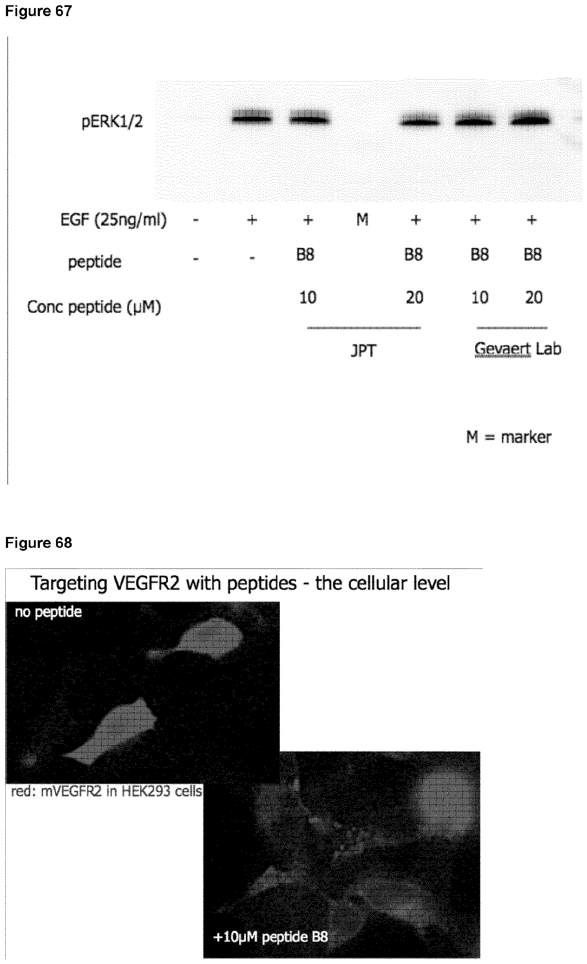

**Figure 67****:** Specificity of peptide B8 towards the VEGFR2 receptor. Western blot analysis for phosphorylated ERK1/2 in HeLa cells that were stimulated for 5 min with EGF (25ng/ml) after overnight starvation in the presence of DMSO or peptide B8 at the indicated concentration. No reduction in ERK1/2 phosphorylation could be observed induced by the peptides directed against VEGFR2.
**Figure 68****:** Immunohistochemical staining for mVEGFR2, expressed in HEK293 cells treated with either DMSO (top panel) or 10 µM peptide B8 (bottom panel).

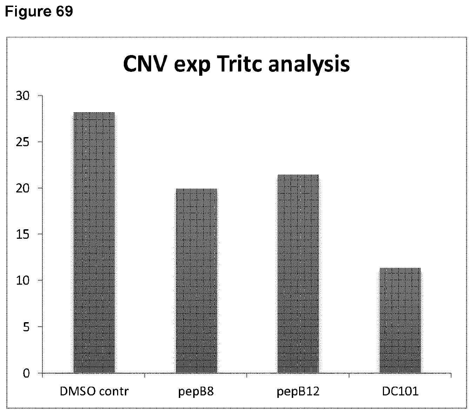

**Figure 69****:** vessel neovascularization in the eye of mice treated with control, anti-VEGFR2 interferor peptides, or anti-VEGFR2 mAb. The Y axis denotes the % of TRITC-positive area over the total border area of the lesion.

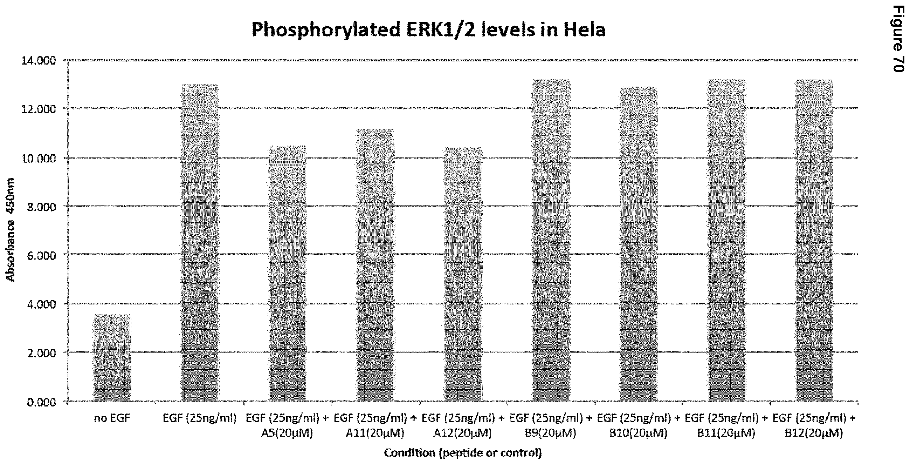

**Figure 70****:** Phosphorylated ERK1/2 levels in HeLa cells upon stimulation of EGF, in the presence or absence of peptides directed against EGFR. Y-axis shows phospho-ERK levels corrected for negative control. Sequence of A5, RWGLLLALRPPRWGLLLALR (SEQ ID NO: 93); A11, RTGYLYISRPPRTGYLYISR (SEQ ID NO: 94); A12, RIISAVVGRPPRIISAVVGR (SEQ ID NO: 95); B9, DVWSYGVTDPPDVWSYGVTD (SEQ ID NO: 96); B10, DITGYLYIDPPDITGYLYID (SEQ ID NO: 97); B11, DLLGISLTDPPDLLGISLTD (SEQ ID NO: 98); B12, DWGLLLALDPPDWGLLLALD (SEQ ID NO: 99)

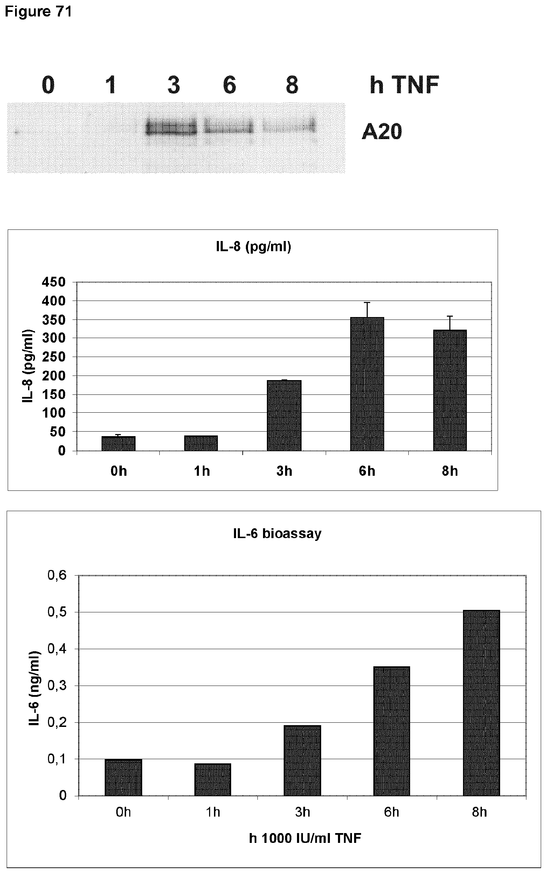

**Figure 71****:** A20 (Western blot in top panel) and IL-8 (ELISA in middle panel) or IL-6 (bio-assay in lower panel) induction at different time points after stimulation with hTNF.

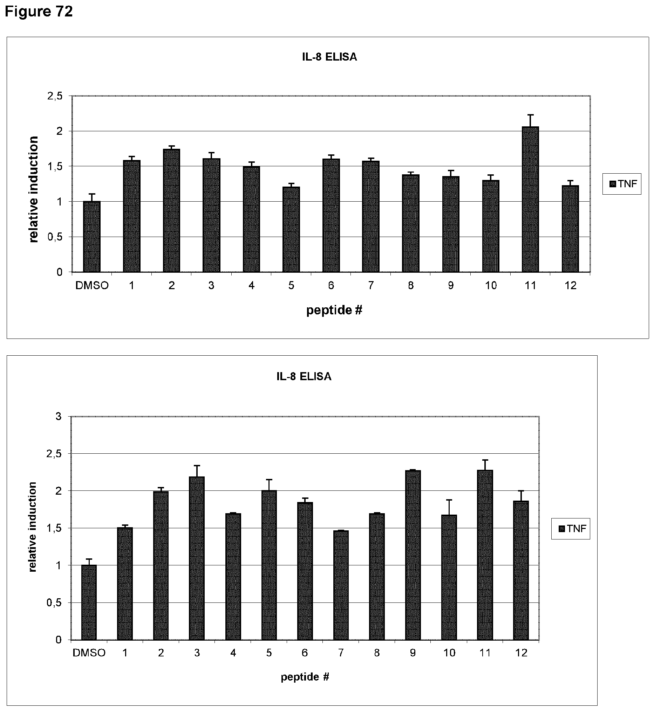

**Figure 72****:** Relative induction of IL-8 levels of A549 cells treated with different interferors after TNF stimulation, as compared to treatment with serum-free medium and 2% DMSO (DMSO in figure). A and B show results of 2 independent experiments.

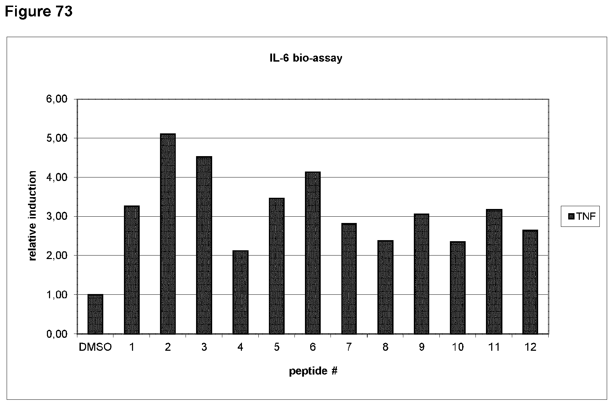

**Figure** 73: Relative induction of IL-6 levels of A549 cells treated with different interferors after TNF stimulation, as compared to treatment with serum-free medium and 2% DMSO (DMSO in figure).

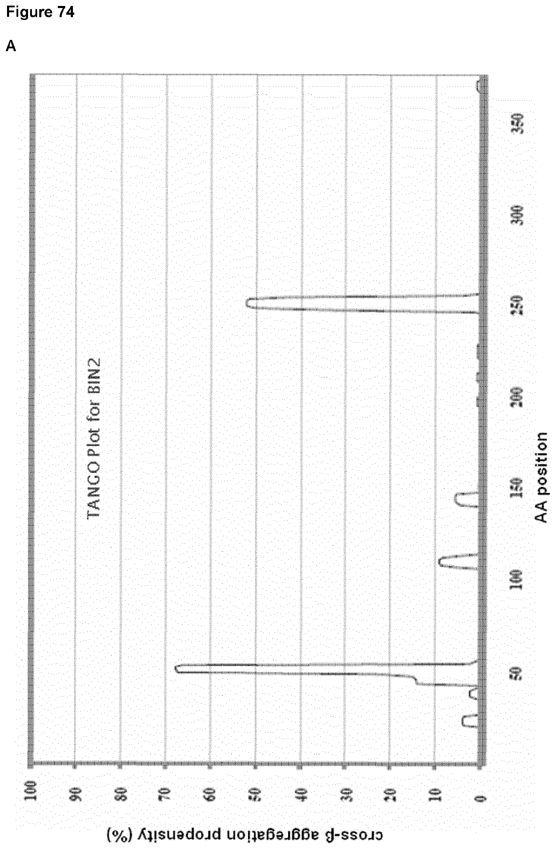

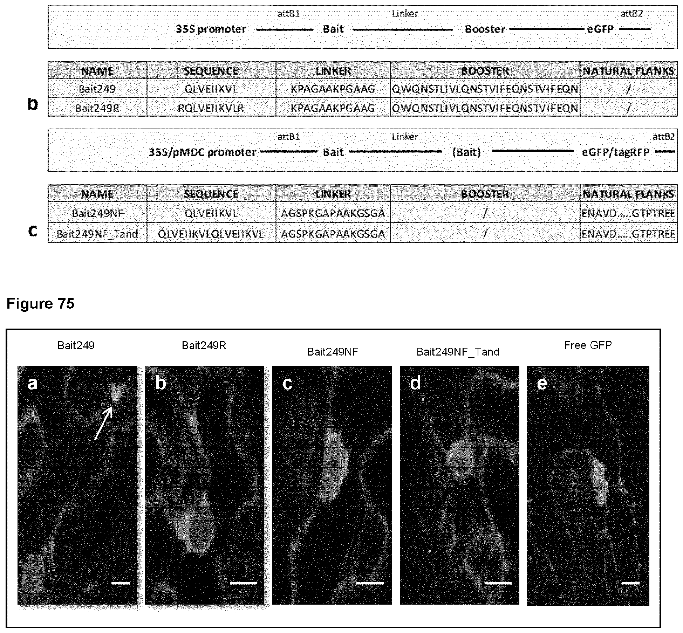

**Figure 74****.** (a) TANGO plot diagram for BIN2; the peaks represent the peptide sequences with the highest propensity to aggregate within the BIN2 protein. (b) Schematic representation of the bait249 expression vectors containing a booster of aggregation N-terminally fused to GFP. (c) Representation of bait249 expression vectors including different linker and flanking sequences (aa sequences are indicated) but not containing any booster of aggregation.
**Figure 75****.** (a-e) CLSM evaluation of aggregates formation in *N. benthamiana* agro-infiltrated leaves transiently transformed with the GFP expressing constructs indicated above each panel. Epidermal cells are GFP positive but show different localization patterns mainly in the perinuclear area. White arrow indicates an insoluble inclusion body. Size bars: 10 µm.

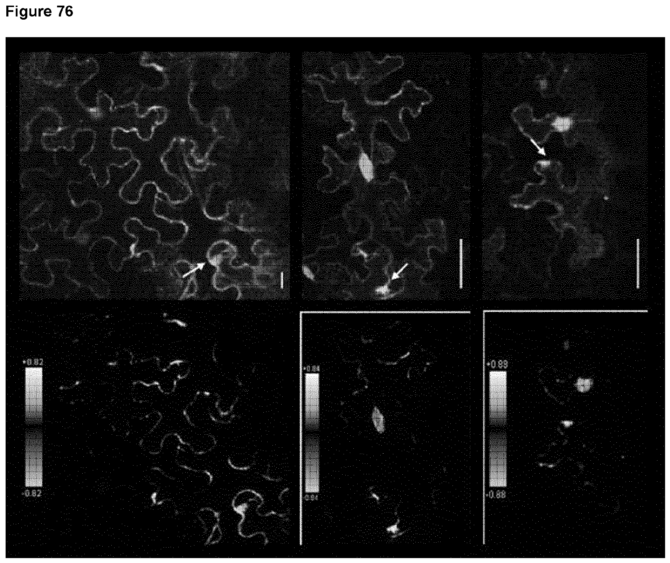

**Figure 76****.** Upper panel: CLSM images of *N. benthamiana* epidermal cells after 4,5 days from co-injection with 35SBIN2GFP and pMDCbait249NF_Tand expressing strains. In the bottom panel: corresponding images representing co-localization quantification performed by ImageJ MBF software. Mander's overlap coefficients (0<R<1) for each picture are indicated; size bars represent 50 µm.

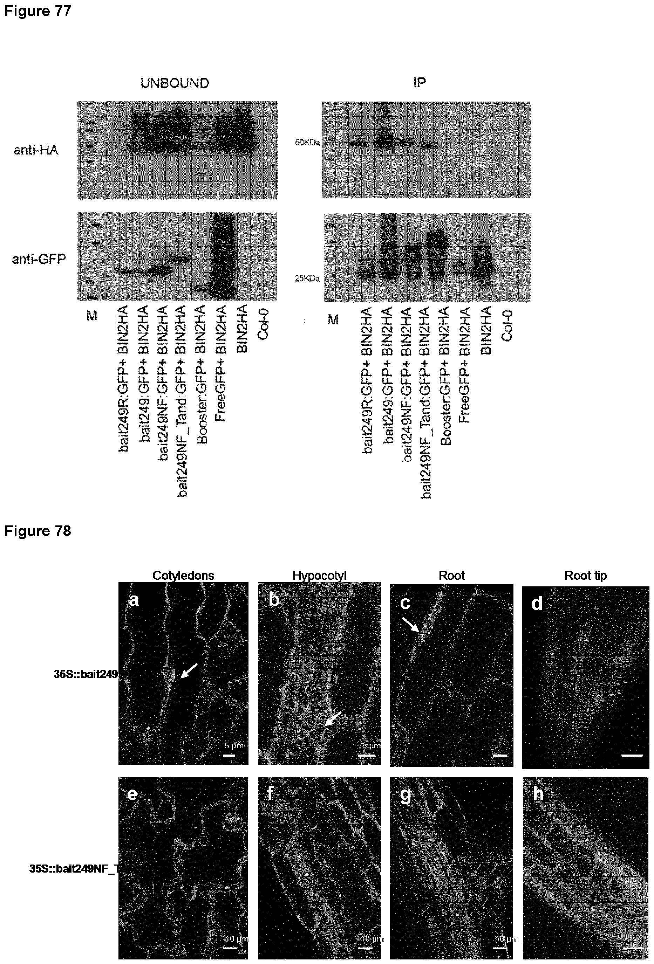

**Figure 77****.** Co-immunoprecipitation of 35S::bait249-GFPvariants co-expressed in *N. benthamiana* with 35S::BIN2:HA. In the left panel the Western blot detection of the unbound fractions of the plant proteins extracts after 4 hours of incubation with anti-GFP beads and detection with anti-HA antibody (left upper panel) or with anti-GFP antibodies (left low panel). In the right panel the detection of the Immuno Precipitated (IP) beads with anti-HA (right upper panel) or with anti-GFP antibody (right lower panel).
**Figure 78****.** (a-d) CLSM images of *Arabidopsis* 8 D.A.S. T3 seedlings expressing 35S::bait249R-GFP construct. Epidermal cells in cotyledons (a), hypocotyl (b) and root cells (c) show perinuclear aggregation (white arrows). The root tip shows no clear cytosolic aggregation and weak GFP signal (d). (e-h) CLSM images of *Arabidopsis* 8 D.A.S. T3 seedlings expressing 35S::bait249NF_Tand-GFP construct. Epidermal cells in cotyledons (e), hypocotyl (f) and root cells (g) show cytosolic aggregation. The root tip shows weaker GFP intensity (h). Size bars are indicated.

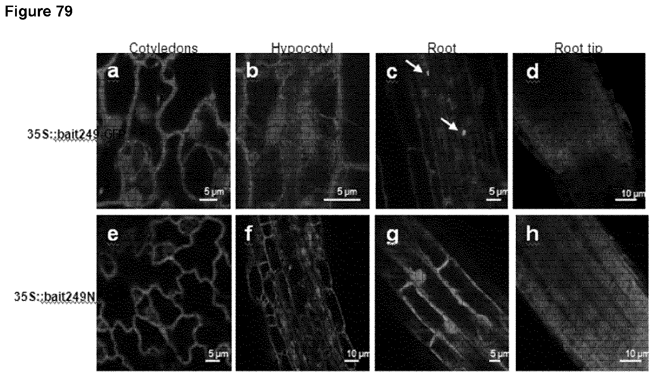

**Figure 79****.** (a-d) CLSM images of *Arabidopsis* 8 D.A.S. T3 seedlings expressing 35S::bait249-GFP construct. No clear aggregation is visible in plant tissues but only weak GFP expression is visible in cells in cotyledons (a), hypocotyls (b), and root tip (d). In root cells (c) the presence of insoluble aggregates in the form of round-shaped bodies is evidenced (white arrow). (e-h) CLSM images of *Arabidopsis* 8 D.A.S. T3 seedlings expressing 35S::bait249NF -GFP construct. The bait is expressed in any plant tissue showing perinuclear aggregation only in root cells (g). Size bars are indicated.

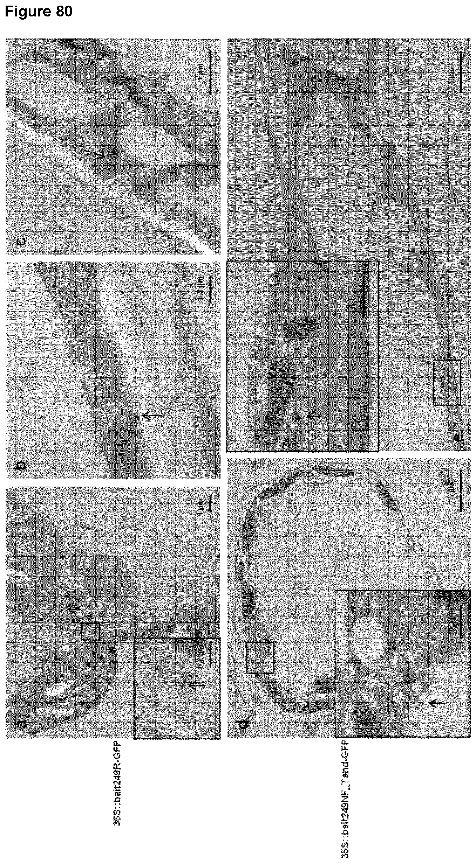

**Figure 80****.** TEM evaluation of immunogold labelled ultrathin section of 8 D.A.S. *Arabidopsis* seedlings incubated with an anti-GFP antibody. (a) Hypocotyl vascular parenchyma cell expressing 35S::bait249R-GFP showing labeled cytosolic fibrillar material (a) enlarged in the inset. (b-c) Details of root elongation area cells showing clustered labeling of bait249NF_Tand-GFP in the cytosol (b) and close to a Golgi stack (c). (d) Cotyledon palisade cell expressing bait249NF_Tand-GFP evidencing perinuclear labeling, enlarged in the inset. (e) Root elongation area cell showing cytoplasmic labeling of bait249NF_Tand in the cytosol. Size bars are indicated.

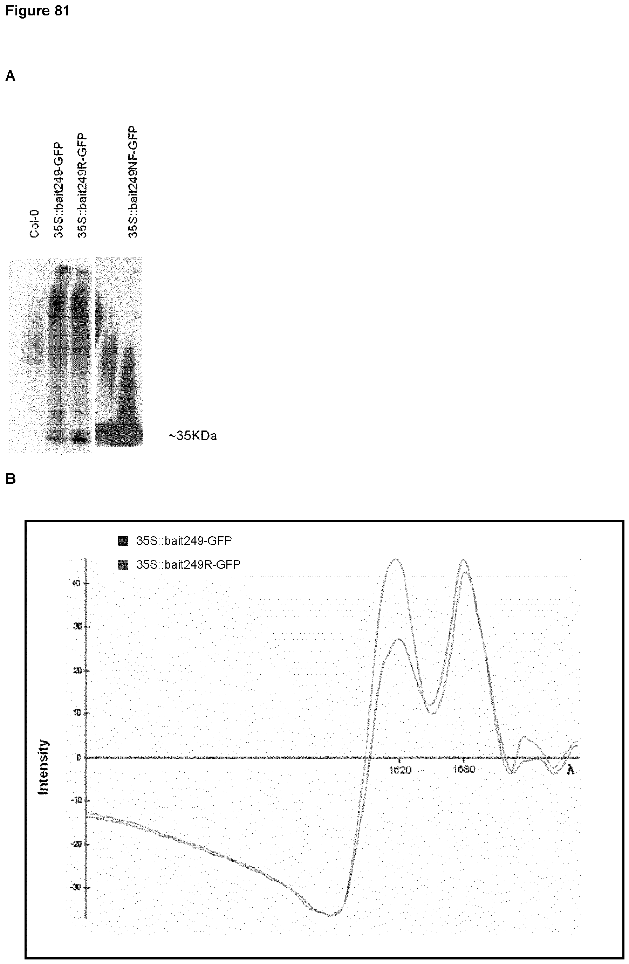

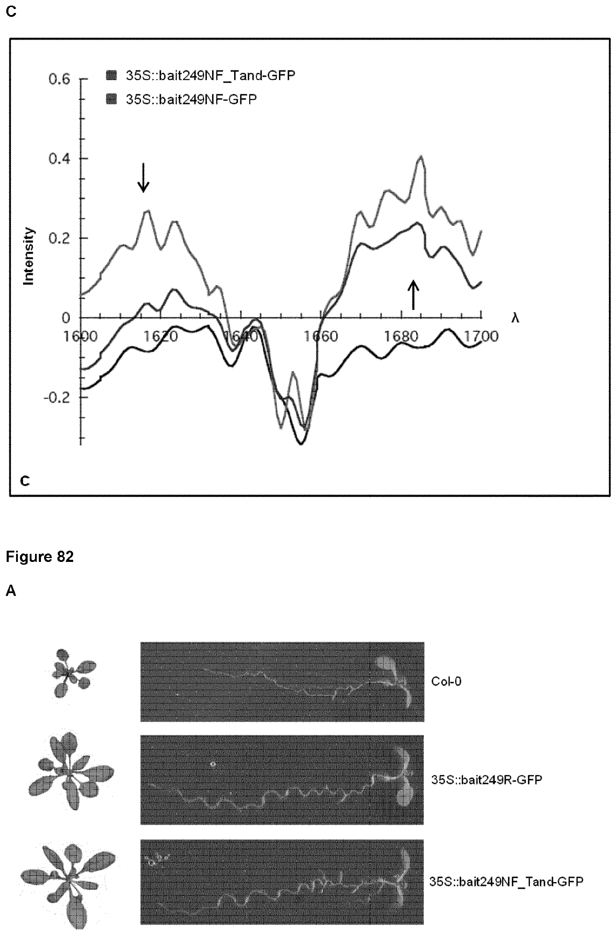

**Figure 81****.** (a) Native-PAGE and anti-GFP detection of high molecular weight complexes (framed) in protein extracts from transgenic *Arabidopsis* plants stably expressing the BIN2 bait249 lines respect to wild type (Col-0) plant extracts. (b,c) FT-IR spectroscopy on immuno-precipitated material from transgenic plants expressing 35S::bait249-GFP, 35S::bait249R-GFP, 35S::bait249NF_Tand-GFP and 35S::bait249NF-GFP. The increased absorbance at 1616 and at 1680 (black arrows) values indicate the presence of β-sheet aggregates.

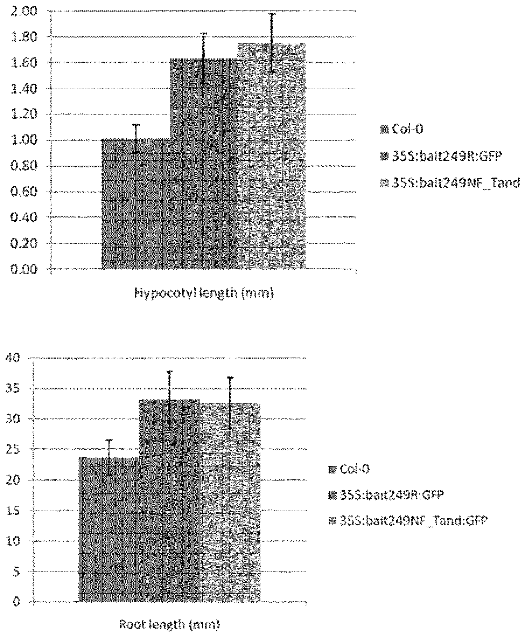

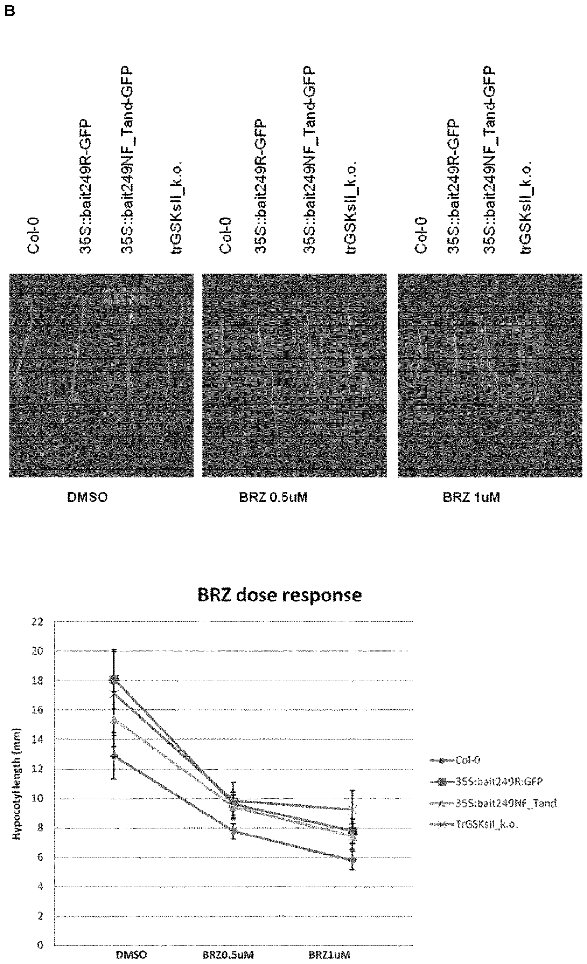

**Figure 82****.** (a) Phenotype of 35S::bait249R-GFP and 35S::bait249NF_Tand-GFP *Arabidopsis* seedlings compared to Col-0 grown vertically *in vitro* for 8 days under long day photoperiod and in soil for 1,5 months. Quantification of roots and hypocotyls lengths on an average of 50 8 D.A.S. seedlings per line is also represented. (b) Brazzinazole resistance dose response assay for 35S::bait249R-GFP and 35S::bait249NF_Tand-GFP *Arabidopsis* 4 D.A.S. seedlings lines compared to Col-0 and triple GSKs group II T-DNA mutant (trGSKsII_k.o.). Corresponding quantification of hypocotyls lengths on an average of 50 seedlings per line is represented in the graph.

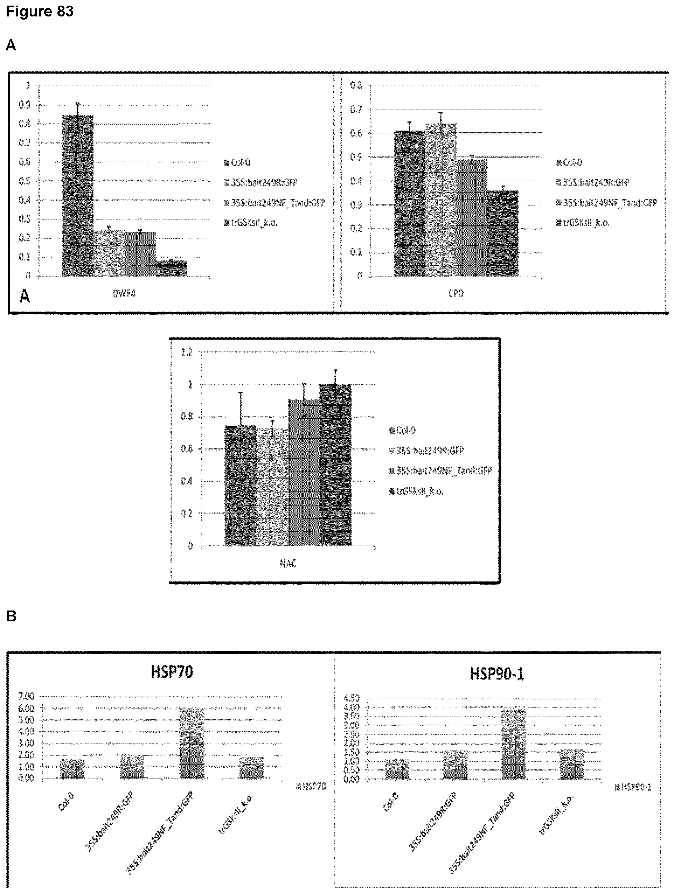

**Figure 83** a) Relative expression levels of the BR-biosynthetic genes *DWF4* and *CPD* and of the gene for the BR-responsive NAC transcription factor (At5g46590) in 8 D.O. *Arabidopsis* seedlings grown *in vitro* under long day conditions. b) Chaperone genes (*HSP70, HSP90-1, HSP101, HSC70-1, HSC70-2* and *HSC70-3*) expression levels measured in the same experimental conditions. In each case the mRNA amount was normalized to the level of *CDKA1* as reference gene.

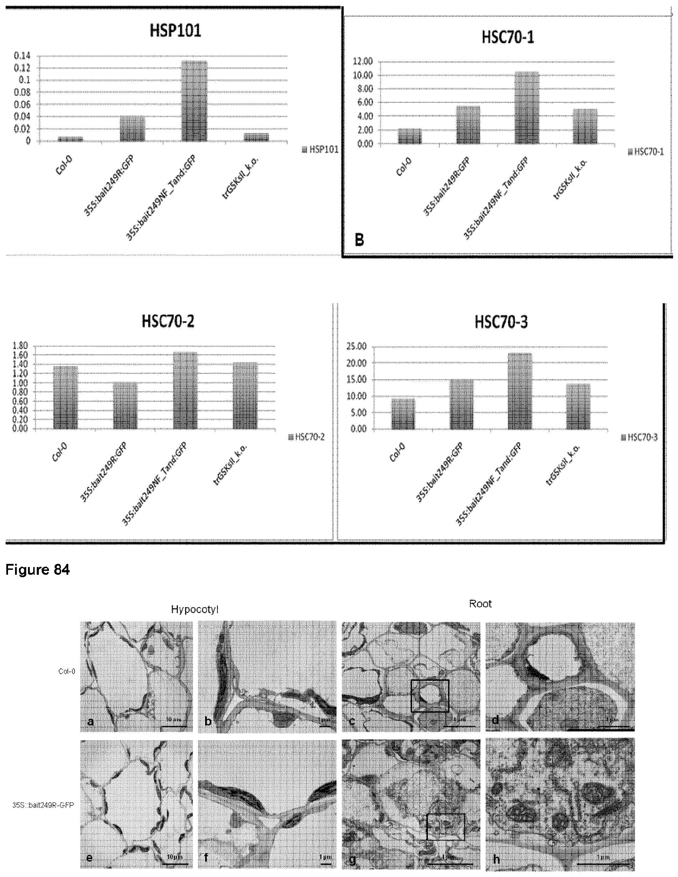

**Figure 84****.** TEM ultrastructural evaluation of 35S::bait249R-GFP cytotoxicity in *Arabidopsis* plants. A low magnification comparison between hypocotyls and root cells in Col-0 (a,c) and the mutant (e,g) is showed. High magnification micrographs of the same areas in Col-0 (b,d) and mutant (f,h). Size bars are indicated.

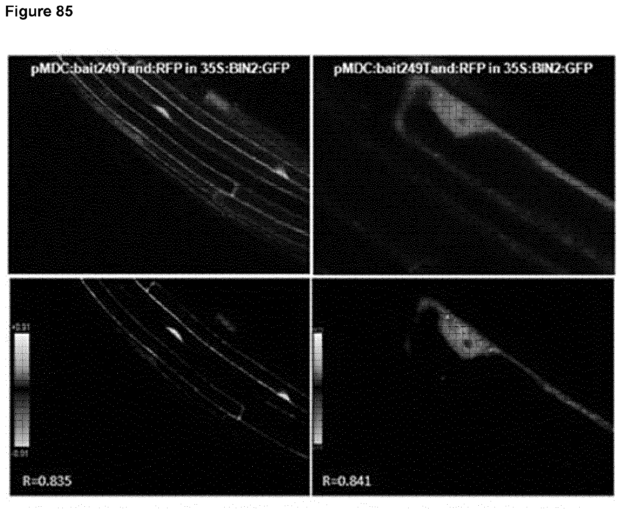

**Figure 85****.** Upper panel: CLSM images of 8 D.A.S. *Arabidopsis* seedlings expressing 35S::BIN2-GFP and pMDC::bait249NF_Tand_RFP after 24 hours of induction. In the bottom panel: corresponding images representing co-localization quantification performed by ImageJ MBF software. Mander's overlap coefficients (0<R<1) for each picture are indicated.

### Detailed description

### Definitions

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

The beta-aggregation inducing or aggregation-nucleating molecules described herein are sometimes referred to as "interferors" or "interferor molecules". With this term, the gatekeeper-containing molecules as described herein (i.e. the molecules with X₂ᵢ₋₁ and X₂ᵢ moieties) are intended, the term is thus not synonymous to the "interferors" as used in WO2007071789. "Interferors" as used herein should thus be read as molecules with the (X₂ᵢ₋₁-Yᵢ-X₂ᵢ-Zᵢ)ₙ structure. The term "interferor peptides" is sometimes used if they are of completely peptidic nature. The term "bait" is also sometimes used as a synonym, although this may also refer to the aggregating region as such. The interferor molecules as described herein are non-naturally occurring molecules. They are synthetic (in the sense of man-made) and do not intend to encompass protein fragments (due to the specific sequence requirements, it is unlikely that natural protein fragments found in organisms will fall under this definition).

As used herein, the term "hydrophobic amino acids" refers to the following 13 amino acids: isoleucine (I), leucine (L), valine (V), phenylalanine (F), tyrosine (Y), tryptophan (W), histidine (H), methionine (M), threonine (T), lysine (K), alanine (A), cysteine (C), and glycine (G). The term "aliphatic amino acids" refers to I, L or V residues. The term "charged amino acids" refers to arginine (R), lysine (K) - both positively charged; and aspartic acid (D), glutamic acid (E) - both negatively charged. Although histidine is sometimes referred to as positively charged, since the nitrogen in its side chain can be protonated in acidic conditions, it is herein not envisaged under the charged amino acids, unless explicitly stated otherwise. This because the positive charge in physiological conditions is not comparable to that of R or K residues, and it is the charge in physiological conditions that is important herein.
Throughout the application, the standard one letter notation of amino acids will be used. Typically, the term "amino acid" will refer to "proteinogenic amino acid", i.e. those those amino acids that are naturally present in proteins. Most particularly, the amino acids are in the L isomeric form. D amino acids are also envisaged, but typically have different aggregation propensities.
The phrase "a stretch of X contiguous amino acids naturally occurring in a protein", wherein X is a number, as used herein, refers to the fact that these X amino acids are present as an uninterrupted stretch, in the same order, in a protein of an organism. In other words, the stretch corresponds to the exact sequence of the protein over a length of X residues.

The "total charge" of a stretch of amino acids is the sum of the number of positively charged amino acids minus the sum of the number of negatively charged amino acids or vice versa. As used herein, it is always an absolute value. Thus, if a stretch contains e.g. one positively charged residue and three negatively charged amino acids, the total charge of that stretch is two.

The term "monomer" as used in the application is an atom or a small molecule that may bind chemically to other monomers to form a polymer. This may refer to natural monomers, such as amino acids (the polymers of which are polypeptides or proteins), nucleotides (the polymers being nucleic acids), or monosaccharides - e.g. glucose, the polymers of which are starches, glycogen or glucose; or xylose, which has xylan as a polymer). Most particularly, however, monomer is used herein to refer to organic molecules outside these three categories, which can form synthetic or other natural polymers, such as e.g. vinyl chloride or isoprene. A most particularly envisaged monomer is ethylene oxide, the oligomer or polymer of which is polyethylene glycol (PEG), sometimes also referred to as polyethylene oxide (PEO) or polyoxyethylene (POE).
In the context of monomers, a "unit of the same nature" is used herein to refer to monomers of the same general structure, but not necessarily identical. For instance, two different amino acids are units of the same nature, while an amino acid and a monosaccharide are units of a different nature.

A "subject" as used herein typically refers to both "animal subjects" and "plant subjects". An "animal subject" is used herein to refer to a vertebrate organism, more particularly a mammal, most particularly a human. Particularly envisaged mammalian subjects are those kept as a pet, such as dogs, cats, rabbits, gerbils, hamsters, chinchillas, mice, rats, guinea pigs, donkeys, mules, ferrets, pygmy goats, pot-bellied pigs, avian pets such as canaries, parakeets, parrots, chickens, turkeys; reptile pets, such as lizards, snakes, tortoises and turtles; and aquatic pets, such as fish, salamanders and frogs. Other particularly envisaged animals are species used for toxicological analysis, such as mice, rats, guinea pigs, rabbits, dogs, pigs, monkeys, ferrets and sheep. Also particularly envisaged are livestock animals such as alpaca, banteng, bison, camel, cattle (cows), deer, donkey, gayal, goat, horse, llama, mule, pig, pony, reindeer, sheep, water buffalo and yak. A "plant subject" as used herein refers to living organisms from the kingdom Plantae. Particularly envisaged plants include cash crops (i.e. crops grown for profit), such as, but not limited to, maize, rice, wheat, soybean, barley, sorghum, millet, oat, rye, triticale, buckwheat, quinoa, fonio, einkorn, durum, potato, coffee, cocoa, cassava, tea, rubber tree, coconut palm, oil palm, sugar cane, sugar beet, banana tree, orange tree, pineapple tree, apple tree, pear tree, lemon tree, olive tree, peanut tree, green bean, lettuce, tomato, carrot, zucchini, cauliflower, rapeseed, jatropha, mustard, jojoba, flax, sunflower, green algae, jute, cotton, hemp (or other strains of Cannabis sativa), canola, or tobacco.
An "organism" as used throughout the application refers to any contiguous living system (such as animal, fungus, micro-organism, or plant), as well as viruses (as these are also separate entitities ('contiguous systems') that contain proteins). Particularly envisaged organisms are subjects (see above), envisaged organisms that are not subjects include non-vertebrate organisms like Drosophila species or Caenorhabditis species. Also particularly envisaged are micro-organisms and/or pathogenic organisms.

An "infection" as used herein refers to the colonization of a host organism (typically a subject) by parasite or pathogenic species (organisms). Infecting pathogens or parasites seek to use the host's resources to reproduce, often resulting in infectious disease. "Infectious disease" is used herein to refer to any type of disease caused by the presence of an external organism (pathogen) in or on the subject or organism with the disease. Infections are usually considered to be caused by microorganisms or microparasites like viruses, prions, bacteria, and viroids, though larger organisms like macroparasites and fungi can also infect. The organisms that can cause infection are herein referred to as "pathogens" (in case they cause disease) and "parasites" (in case they benefit at the expense of the host organism, thereby reducing biological fitness of the host organism, even without overt disease being present) and include, but are not limited to, viruses, bacteria, fungi, protists (e.g. Plasmodium, Phytophthora) and protozoa (e.g. Plasmodium, Entamoeba, Giardia, Toxoplasma, Cryptosporidium, Trichomonas, Leishmania, Trypanosoma) (microparasites) and macroparasites such as worms (e.g. nematodes like ascarids, filarias, hookworms, pinworms and whipworms or flatworms like tapeworms and flukes), but also ectoparasites such as ticks and mites. Parasitoids, i.e. parasitic organisms that sterilize or kill the host organism, are envisaged within the term parasites. According to particular embodiments where the molecules can be administered directly to ectoparasites rather than through the host organism, it is envisaged that ectoparasites are not included within the sense of parasites causing an infection.

Note that although infectious disease often is used for subjects that are animals, particularly mammals, most particularly humans, it is envisaged herein that infectious disease applies to plants as well (especially when referred to disease in an organism). Indeed, plant pathogens can also cause infections in plants and include, but are not limited to, fungi (e.g. Ascomycetes, Basidiomycetes, Oomycetes), bacteria, Phytoplasma, Spiroplasma, viruses, nematodes, protozoa and parasitic plants.

A "pest" or "plant pest" as used in the application refers to organisms that cause damage to plants, particularly plants used in agriculture. Note that the term "plant pest" is used in the meaning that the pest targets plants, it is not necessarily the case that the pest is a plant species. Indeed, pests particularly are animals (vertebrates eating or destroying the plants, or invertebrates), other plants (crop weeds and parasitic plants), micro-organisms and viruses. Most particularly, pests are invertebrate animals (e.g. insects (including agricultural pest insects, insect pests of ornamental plants, insect pests of forests, insect vectors of human, animal or plant pathogens. Examples include, but are not limited to, aphids, caterpillars, flies, wasps, and the like), nematodes (living freely in soil or particularly species that parasitize plant roots, such as root-knot nematode, soybean cyst nematode and potato cyst nematode), mites (such as spider mites, thread-footed mites and gall mites) and gastropods (including slugs such as Deroceras spp., Milax spp., Tandonia sp., Limax spp., Arion spp. and Veronicella spp. and snails such as Helix spp., Cernuella spp., Theba spp., Cochlicella spp., Achatina spp., Succinea spp., Ovachlamys spp., Amphibulima spp., Zachrysia spp., Bradybaena spp., and Pomacea spp.), pathogenic fungi (including Ascomycetes (such as Fusarium spp., Thielaviopsis spp., Verticillium spp., Magnaporthe spp.), Basidiomycetes (such as Rhizoctonia spp., Phakospora spp., Puccinia spp.), and fungal-like Oomycetes (such as Pythium spp. and Phytophthora spp.), bacteria (such as Burkholderia spp. and Proteobacteria such as Xanthomonas spp. and Pseudomonas spp.), Phytoplasma, Spiroplasma, viruses (such as tobacco mosaic virus and cauliflower mosaic virus), and protozoa. The term "pest" shows significant overlap with the infection-related "pathogens" and "parasites", particularly for micro-organisms and viruses. However, for plant pests, ectoparasites and (invertebrate) animals that feed on plants are always included in the definition.

"Non-infectious diseases" are all diseases that are not infectious diseases, i.e. are those diseases that are not caused by a pathogen and cannot be shared from one person to another. Non-infectious diseases may be caused by either the environment, nutritional deficiencies, lifestyle choices, or genetic inheritances, and include for instance most forms of cancer, asthma, and heart disease. A most particularly envisaged class of non-infectious disease are those physiological disorders that are caused by the own proteins of the organism with the disease (e.g. through aberrant expression or through mutation).

As used in the application, the term "drug resistance" refers to a reduction in effectiveness of a drug in curing a disease or condition. Particularly, it applies in the context of resistance acquired by pathogens. When an organism is resistant to more than one drug, it is said to be multidrug-resistant. The term "antibiotic resistance" as used herein is a type of drug resistance where a microorganism is able to survive exposure to an antibiotic. To determine antibiotic resistance in practice, the clinical breakpoint can be used. An antibiotic breakpoint is a maximum MIC (Minimum inhibitory concentration, the lowest concentration of an antimicrobial that will inhibit the visible growth of a microorganism after incubation) threshold for predicting successful antibiotic therapy. During the antibiotic dosing interval, organisms with an MIC at or below this threshold are expected to be (at least) inhibited or to be killed. In other words, if the MIC value of an antibiotic for a given organism is higher than the breakpoint value of that antibiotic for the given micro-organism, the microorganism is said to be resistant. Clinical breakpoints are tested and monitored by the European Committee on Antimicrobial Susceptibility Testing (EUCAST) in Europe, and breakpoint values can be found at their site. Clinical breakpoints are also separately determined by the Clinical and Laboratory Standards Institute (CLSI) in the US, these are recognized by the FDA.
Drug, and particularly antibiotic, resistance can be natural resistance (e.g. because the protein targeted by the drug is not present in the organism) or can be acquired resistance (e.g. through mutations, which can be in the gene targeted by the drug, or by mutations that e.g. increase activity of efflux pumps, so that the drug (e.g. antibiotic) is removed from the microbial organism. Alternatively, resistance can be acquired through horizontal gene transfer, meaning that (for antibiotics) microbial organisms that are resistant to a particular antibiotic exchange genetic information with microbial organisms (from the same or different species) that are sensitive to that antibiotic, thereby making the sensitive organisms resistant).

A "biofilm", as used herein, is an aggregate of microorganisms in which cells adhere to each other and/or to a surface. These adherent cells are frequently embedded within a self-produced matrix generally composed of extracellular DNA, proteins, and polysaccharides in various configurations. Biofilms can contain many different types of microorganism, e.g. bacteria, archaea, protozoa, fungi and algae. However, monospecies biofilms occur as well. Microorganisms living in a biofilm usually have significantly different properties from free-floating (planktonic) microorganisms of the same species, as a result of the dense and protected environment of the film. For example, increased resistance to detergents and antibiotics is often observed, as the dense extracellular matrix and the outer layer of cells protect the interior of the community. Biofilm formation is an often encountered problem with implant surgery, as biofilms can be formed on the inert surfaces of implanted devices such as catheters, stents, prosthetic cardiac valves and intrauterine devices.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, Calif., USA) using standard defaults as used in the reference manual accompanying the software. "Similarity" refers to the percentage number of amino acids that are identical or constitute conservative substitutions. Similarity may be determined using sequence comparison programs such as GAP (Deveraux et al. 1984, Nucleic Acids Research 12, 387-395). In this way, sequences of a similar or substantially different length to those cited herein might be compared by insertion of gaps into the alignment, such gaps being determined, for example, by the comparison algorithm used by GAP.

A "microarray" as used herein refers to a 2D array on a solid substrate. It can serve as a multiplex assay, i.e. simultaneously measure multiple analytes (up to dozens or more) in a single assay. It can be a lab-on-a-chip device that makes use of microfluidics. As solid substrate, typically glass slides or silicon thin film gels are used, but other materials can be used as well (e.g. nitrocellulose, plastics, ...). As the molecules presented herein bind to proteins, a microarray coated with molecules can also be referred to as a protein microarray, protein binding microarray, or protein chip. Protein microarrays are generally used in biomedical applications to determine the presence and/or amount (referred to as relative quantitation) of proteins in biological samples. Typically, in the microarrays described herein, the molecules of the application are used as capture molecules spotted or synthesized on the microarray.

### Structure of the Molecules

### General structure

The present specification describes molecules (which include molecules in accordance with the aspects and embodiments of the invention) that can be described with the following formula:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, K and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is an independently selected linker and Zₙ is either a linker or absent.

This formula thus encompasses the following structures:
X₁-Y₁₋X₂-Z₁ (i.e., n=1),
X₁-Y₁-X₂-Z₁₋X₃-Y₂-X₄-Z₂ (i.e., n=2),
X₁-Y₁-X₂-Z₁-X₃-Y₂-X₄-Z₂-X₅-Y₃-X₆-Z₃ (i.e., n=3),
X₁-Y₁-X₂-Z₁-X₃-Y₂-X₄-Z₂-X₅-Y₃-X₆-Z₃-X₇-Y₄-X₈-Z₄ (i.e., n=4), and
X₁-Y₁-X₂-Z₁-X₃-Y₂-X₄-Z₂-X₅-Y₃-X₆-Z₃-X₇-Y₄-X₈-Z₄-X₉-Y₅-X₁₀-Z₅ (i.e., n=5), wherein each numbered X, Y and Z are as defined above.

### Yᵢ moieties: aggregation-nucleating regions

The numbered Yᵢ moieties in these molecules are beta-aggregation inducing regions. I.e., these regions are responsible for inducing the aggregation of the molecules, particularly when brought into contact with a target protein. Here, we will explain in more detail the sequence constraints of these regions.
Mutational studies of the kinetics of aggregation of full-length proteins revealed simple correlations between aggregation and physico-chemical properties such as β-sheet propensity, hydrophobicity and charge. This prompted the development of computer algorithms that identify aggregation-prone regions in the amino acid sequence of a protein. One of these is the Zyggregator algorithm of Dobson et al. (Pawar et al., J Mol Biol 350: 379-392 (2005)), which identifies aggregation-prone sequences by comparing the aggregation propensity score of a given amino acid sequence with an average propensity calculated for a set of sequences of similar length. The statistical mechanics algorithm TANGO (Fernandez-Escamilla et al., Nat Biotechnol 22:1302-1306 (2004)), on the other hand, balances the physico-chemical parameters mentioned above, supplemented by the assumption that an amino acid is fully buried in the aggregated state: this means it becomes fully desolvated and entropically restricted. From an input sequence, TANGO generates an extensive sample of fragments for which competing structural propensities, such as helix or hairpin formation, are considered. All the fragments are then balanced in a global partition sum, which allows the identification of sequence regions that predominantly form aggregates. The TANGO algorithm has an accuracy of more than 90% for a set of 176 experimentally validated peptides (Fernandez-Escamilla et al., Nat Biotechnol 22:1302-1306 (2004)). Importantly, both the Zyggregator algorithm and TANGO perform well for peptides and denatured proteins.
For globular proteins, a partly folded molecule can either refold to the native state or misfold into an aggregated state. As a result, both reactions are in competition and a precise understanding of the kinetics is essential to predict the final outcome in terms of folding or misfolding/aggregation. Hence, in the context of the present invention, it is important to identify sequences that kinetically favour the induction of aggregation.

Most particularly, the sequences are non-amyloid beta-aggregation sequences (sometimes referred to as amorphous beta-aggregation sequences). Amyloid and non-amyloid beta-aggregation differs in higher-order structure, in aggregation kinetics and in the protein sequences suitable for aggregation (Rousseau et al., Current Opinion in Structural Biology 16:118-126, 2006). Comparing the sequence space of β-aggregation predicted by TANGO or Zyggregator with the sequence space of amyloidosis (e.g. derived from experimental studies such as Lopez de la Paz and Serrano, PNAS 101: 87-92, 2004) reveals the similarities, but also interesting differences between both processes. Indeed, as both amyloid formation and amorphous cross-β aggregation require amino acid compositions that are compatible with a β-strand conformation, an overlap in sequence space is to be expected. However, the structure of amorphous cross-β aggregates is not clearly defined and seems to be characterized by a high degree of flexibility. On the other hand, the structure of amyloid fibers is quasi-crystalline. As a consequence, amino acid preferences will be much more position specific in an amyloid fiber than in amorphous cross-β aggregates. Considering the overlap in sequence space, specific embodiments foresee that the beta-aggregating sequence of the at least one Yi moiety is suitable at least for amorphous beta-aggregation. According to these embodiments, amorphous beta-aggregation is envisaged and amyloid aggregation may or may not occur in addition - this is not vital, as long as at least non-amyloid beta-aggregation is present.
Due to its less stringent conformational requirements, β-aggregation is generally much faster than amyloidosis, although fast amyloidosis has also been observed. As β-aggregates are often observed as precursors on the path to fiber formation, the stability of these precursor aggregates will strongly influence the kinetics of amyloidosis. Polar amylogenic sequences, as observed in yeast prion proteins, will have a much lower β-aggregation propensity and will therefore be much more favorable for the kinetics of amyloidosis. In summary, amorphous cross-β aggregation and amyloidosis can occur in common, and the stability and kinetics of both processes will be determined by the extent to which the structural requirements of both processes are fulfilled.

Thus, in order to kinetically favour the induction of aggregation, it is desirable to not just use beta-aggregation sequences, but non-amyloid beta-aggregation sequences. Accordingly, in particular embodiments, the at least one Yᵢ region is not an amyloid beta-aggregation sequence. This way, fast aggregation of a target protein can be achieved. Generally highly hydrophobic sequences have a strong tendency to form amorphous cross-β aggregates, but do not form amyloid fibers due to steric constraints. On the other hand, generally more polar sequences are more likely to form amyloid fibers. Between these two extremes, a whole spectrum of behaviors will probably be observed. The Tango algorithm, in particular, is very suitable to identify beta-aggregation sequences with high aggregation propensity and low amyloid propensity. Indeed, the algorithm offers separate scores for amyloid propensity and beta-aggregation propensity.
The Tango algorithm has been described in more detail elsewhere (particularly Fernandez-Escamilla et al., Nat. Biotechnol. 22:1302-1306, 2004, especially the Methods section on pages 1305 and 1306 are herein specifically incorporated by reference. See also the Supplementary Notes 1 and 2 of the same article for further details on the methods and the data sets used for the calibration and the testing of the TANGO algorithm; more background can also be found in WO2007071789). Briefly, to predict self-association regions of a peptide, TANGO simply calculates the partition function of the phase-space. To estimate the aggregation tendency of a particular amino acid sequence, the following assumptions are made: (i) in an ordered beta-sheet aggregate, the main secondary structure is the beta-strand. (ii) the regions involved in the aggregation process are fully buried, thus paying full solvation costs and gains, full entropy and optimizing their H-bond potential (that is, the number of H-bonds made in the aggregate is related to the number of donor groups that are compensated by acceptors. An excess of donors or acceptors remains unsatisfied). (iii) complementary charges in the selected window establish favorable electrostatic interactions, and overall net charge of the peptide inside but also outside the window disfavors aggregation. TANGO can be accessed on the World Wide Web.
A high Tango score of a sequence stretch typically corresponds to a sequence with high (and kinetically favourable) beta-aggregation propensity. Thus, the sequence space of "high tango-scoring sequences" which are not too polar and quite hydrophobic defines the ideal Yᵢ (or beta-aggregation inducing, aggregation-nucleating) moieties.

Best aggregation properties are obtained when, in a molecule according to the formula outlined above, each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present. (Although it should be mentioned that 2 R, K, D, E residues may also be present if they are one positively and one negatively charged residue). According to very specific embodiments, the Yi region may contain exactly 2 charged residues (selected from R, K, D, E), as long as the net charge of the Yi region is zero. More particularly however, the Yi region does not contain any charged residue (or proline for that matter).

According to this requirement, at least 50% of the amino acids in the Yi stretch are hydrophobic amino acids, i.e. are amino acids selected from I, L, V, F, Y, W, H, M, T, K, A, C, and G. According to further particular embodiments, at least 60% of the amino acids are hydrophobic amino acids, at least 2/3 of the amino acids are hydrophobic amino acids, at least 70% are hydrophobic amino acids, at least 75% are hydrophobic amino acids, at least 80% are hydrophobic amino acids, at least 85% are hydrophobic amino acids, at least 90% are hydrophobic amino acids, at least 95% are hydrophobic amino acids, or even all amino acids are hydrophobic amino acids. Alternatively, it can be said that at least 3 amino acids in the Yi stretch are hydrophobic amino acids, particularly at least 4 are hydrophobic amino acids, more particularly at least 5 are hydrophobic amino acids, at least 6 or even more than 6 are hydrophobic amino acids.

Some of the listed hydrophobic amino acids are better fitted for inducing beta-aggregation (which is why there are additional requirements to the sequence). According to particular embodiments, the hydrophobic amino acids do not encompass G (since the side chains are too small). According to other particular embodiments, the hydrophobic amino acids do not encompass C (as this may complicate matters as a result of possible disulphide bridge formation). According to yet other particular embodiments, the hydrophobic amino acids do not encompass K (in view of the positive charge of this residue). According to still other particular embodiments, the hydrophobic amino acids do not encompass H (in view of the partly positive charge of this residue). Accordingly, specific embodiments foresee that the hydrophobic amino acids in the Yᵢ stretch are selected from I, L, V, F, Y, W, M, T, and A.

In each Yᵢ stretch, at least one residue selected from I, L, V and F (aliphatic residue or F) is present, most particularly more than one such residue is present. If only one of the residues of the Yᵢ stretch is an I, L, V or F residue, at least one residue in the stretch is selected from Y, W, M, T or A. More particularly, in these embodiments, at least two residues are selected from Y, W, M, T or A. According to very specific embodiments, at least two residues in the Yᵢ stretch are selected from I, L, V, F, Y, and W (i.e., from aliphatic or non-charged aromatic residues). According to other specific embodiments, at least three residues in the Yᵢ stretch are selected from I, L, V, F, Y, W, M, T, and A. According to further specific embodiments, at least four residues in the Yᵢ stretch are selected from I, L, V, F, Y, W, M, T, and A. According to other specific embodiments, at least 40% of the residues in the Yᵢ stretch are selected from I, L, V, F, Y, W, M, T, and A. According to further specific embodiments, at least 50% of the residues in the Yᵢ stretch are selected from I, L, V, F, Y, W, M, T, and A. According to yet further specific embodiments, at least 50% of the residues in the Yᵢ stretch are selected from I, L, V, F, and Y (i.e. are aliphatic residues or F or Y). According to even further specific embodiments, at least 50% of the residues in the Yᵢ stretch are selected from I, L, V, and F (i.e. are aliphatic or F residues). According to yet even further specific embodiments, at least 50% of the residues in the Yᵢ stretch are aliphatic residues, i.e. selected from I, L, and V. According to alternative specific embodiments, at least 60% of the residues in the Yᵢ stretch are selected from I, L, V, F, Y, W, M, T, and A. According to yet further specific embodiments, at least 60% of the residues in the Yᵢ stretch are selected from I, L, V, F, and Y (i.e. are aliphatic residues or F or Y). According to even further specific embodiments, at least 60% of the residues in the Yᵢ stretch are selected from I, L, V, and F (i.e. are aliphatic or F residues). According to yet even further specific embodiments, at least 60% of the residues in the Yᵢ stretch are aliphatic residues, i.e. selected from I, L, and V. According to yet further alternative embodiments, at least two thirds of the residues are selected from I, L, V, F, Y, W, M, T, and A; or are particularly selected from I, L, V, F, and Y; or are more particularly selected from I, L, V, and F (i.e. are aliphatic or F residues); or at least two thirds of the residues in the Yi stretch are aliphatic residues (i.e. selected from I, L and V). Alternatively, it can be said that at least 3 amino acids in the Yi stretch are selected from the above residues, particularly at least 4 amino acids, more particularly at least 5 amino acids, at least 6 or even more than 6 amino acids. According to further particular embodiments, at least 3 amino acids in the Yi stretch are selected from I, L, V or F, particularly at least 4 amino acids are aliphatic residues or F, more particularly at least 5 amino acids are I, L, V or F residues. According to yet even further embodiments, at least 3 amino acids in the Yi stretch are aliphatic residues, at least 4 amino acids in the Yi stretch are aliphatic residues, or at least 5 residues in the Yi stretch are aliphatic residues.

According to other specific embodiments, the number of certain hydrophobic residues is limited in the Yᵢ stretch. For instance, in a (that is, at least one, up to each) Yᵢ stretch, there is no more than one C residue. According to other specific embodiments, there is no more than one H residue in a Yᵢ stretch. According to alternative embodiments, no more than one or two G residues are present in the Yᵢ stretch, particularly no more than one G residue is present. According to alternative embodiments, no more than one or two T residues are present in the Yᵢ stretch, particularly no more than one T residue is present. According to alternative embodiments, no more than one or two M residues are present in the Yᵢ stretch, particularly no more than one M residue is present. According to alternative embodiments, no more than one or two W residues are present in the Yᵢ stretch, particularly no more than one W residue is present. According to alternative embodiments, no more than one or two A residues are present in the Yᵢ stretch, particularly no more than one A residue is present. According to alternative embodiments, no more than one or two Y residues are present in the Yᵢ stretch, particularly no more than one Y residue is present. Note that, as for other embodiments, these embodiments are not exclusive. I.e., a combination of 2 or more of these restrictions may apply to a given Yᵢ stretch.

It goes without saying that when no more than a certain number of particular residues is present, it is always possible that none of these particular residues are present.

Charged amino acids, as well as proline, typically diminish the beta-aggregation potential of a given Yᵢ stretch. Although one charged residue or proline typically can be tolerated, or two charged amino acids if they are of opposite charge, according to particular embodiments, the Yᵢ stretch is free of P, R, K, D and E residues. Alternatively, the stretch contains no more than one P, R, K, D or E residue (i.e., no more than one residue selected from P, R, K, D and E). According to very specific embodiments, the stretch may contain one K residue, but no P, R, D or E residues (in view of the hydrophobic nature of lysine residues). According to particular embodiments, no P, R, K, D, E residues are present in the Yᵢ region. According to further particular embodiments, no P, R, K, D, E or H residues are present in the Yᵢ stretch. This because H residues can be partly positively charged, and neutral Yᵢ regions are generally preferable.

It is particularly envisaged that at least one (and up to each) Yᵢ region carries a maximal charge of 1, and more particularly does not carry a charge. Even more particularly, it is envisaged that the number of charged residues is not higher than one, most particularly, no charged residue is present in at least one (and particularly each) Yᵢ region.

According to other, non-exclusive embodiments, the Yᵢ stretch, even when it does not contain charged residues, also does not contain more than 75% polar non-charged residues, i.e. residues selected from Y, W, T, Q, S, N, and H (C is not regarded as a polar residue here, and H is not considered a charged residue). More particularly, it does not contain more than 67% polar non-charged residues, even more particularly, it does not contain more than 60% polar non-charged residues (or even less than 60% polar, non-charged residues). In other words, even if stretches have a lot of Y, W or T residues (which are polar, non-charged and hydrophobic), there still need to be sufficient apolar residues. According to further particular embodiments, the Yᵢ stretch does not contain more than 50% polar non-charged residues. According to even further particular embodiments, the Yᵢ stretch does not contain more than 40% polar non-charged residues. According to yet even further particular embodiments, the Yᵢ stretch does not contain more than a third polar non-charged residues. According to yet even further particular embodiments, the Yᵢ stretch does not contain more than 25% polar non-charged residues.

Non-hydrophobic, polar non-charged residues (i.e. S, N and Q) may be present in a Yᵢ region, but their number is preferably limited. According to other specific embodiments, there is no more than one Q residue. According to other specific embodiments, there is no more than one N residue. According to alternative embodiments, no more than one or two S residues are present in the Yᵢ stretch, particularly no more than one S residue is present.

Non-aromatic polar non-charged residues (i.e. S, N, T and Q) may be present in a Yᵢ region, but it is particularly envisaged that they are not adjacent to each other (i.e. no 2 contiguous non-aromatic polar non-charged residues are present).

As is evident from the above, it may be beneficial to limit the presence of specific residues for particular reasons. It may also be beneficial to limit the sum of groups of residues. According to particular embodiments, the residues in a Yi stretch do not contain more than 60% residues selected from C, M, N, Q and W. According to further particular embodiments, the residues in a Yi stretch do not contain more than 50% residues selected from C, M, N, Q and W. According to further particular embodiments, the residues in a Yi stretch do not contain more than 40% residues selected from C, M, N, Q and W. According to even further particular embodiments, the residues in a Yi stretch do not contain more than a third of residues selected from C, M, N, Q and W. According to particular embodiments, the residues in a Yi stretch do not contain more than 25% residues selected from C, M, N, Q and W.

According to still other particular embodiments, the Yᵢ stretch, even when it does not contain charged residues or proline, also contains less than 75% small residues other than V (i.e., selected from A, T, C, G, S, N), more particularly less than 67% small residues other than V, even more particularly, less than 60% small residues other than V. In other words, even if stretches have a lot of T and A residues, there still need to be sufficient large hydrophobic residues. According to even further particular embodiments, no more than 50% (but including 50) of the residues in the Yᵢ region are small residues other than V. According to yet even further particular embodiments, no more than 40% of the residues in the Yᵢ region are small residues other than V. According to yet even further particular embodiments, no more than a third (i.e. 33,33...%) of the residues in the Yᵢ region are small residues other than V. According to yet even further particular embodiments, no more than 25% of the residues in the Yᵢ region are small residues other than V.
Particularly, it is envisaged that the number of tiny residues is limited in the Yᵢ stretch. Although C can be considered a tiny residue when it is not involved in disulfide bridge formation, it is particularly envisaged that tiny residues are A, G and S residues. According to particular embodiments, no more than 50% of the residues in the Yᵢ stretch are A, G and S residues. According to further particular embodiments, no more than 40% of the residues in the Yᵢ stretch are selected from A, G and S. According to even further particular embodiments, no more than a third of the residues in the Yᵢ stretch are selected from A, G and S. According to yet even further particular embodiments, no more than 25% of the residues in the Yᵢ stretch are selected from A, G and S residues.
Alternatively or additionally, the number of tiny residues may be limited. This is particularly true for the non-polar A and G residues. According to particular embodiments, the total number of A and G residues in a Yᵢ stretch is no more than 4. According to further particular embodiments, the total number of A and G residues in a Yᵢ stretch is no more than 3. According to even further particular embodiments, the total sum of A and G residues in a Yᵢ stretch is no more than 2. According to yet even further particular embodiments, the total sum of A and G residues in a Yᵢ stretch is no more than one.

To achieve sequence-specific aggregation, it is envisaged that the Yi moieties selected for inducing aggregation have sufficient sequence complexity. By this, it is meant that sequences that have too much of a particular amino acid are not particularly suitable for sequence-specific aggregation. For instance, a poly-leucine stretch will typically not achieve sequence-specific aggregation (even though it is sufficiently hydrophobic and might aggregate). Thus, according to particular embodiments, no more than 3 contiguous identical amino acids are present in a Yi stretch. According to even more particular embodiments, no more than 2 contiguous identical amino acids are present in a Yi stretch. According to specific embodiments, only an aliphatic amino acid may be adjacent to the same amino acid in a Yi stretch. I.e., according to these embodiments, only 2 contiguous (consecutive) I, L or V amino acids can be present in a Yᵢ stretch. According to further particular embodiments, no more than 3 consecutive identical aliphatic amino acids may be present in a Yi region. According to even further particular embodiments, no more than 2 consecutive identical aliphatic amino acids may be present in a Yi region. Thus, according to these embodiments, only two identical aliphatic amino acids may be adjacent to each other in a Yi stretch.
According to alternative, but not exclusive embodiments, no single non-aliphatic amino acid is present more than 3 times in a Yi stretch (i.e. the number of identical amino acids for a non-aliphatic residue is limited to 3 in a Yi stretch). According to further particular embodiments, no single non-aliphatic amino acid is present more than twice (or 2 times) in a Yi stretch. According to further particular embodiments, no single non-aliphatic amino acid is present more than 1 time in a Yi stretch. According to alternative embodiments, no single aliphatic amino acid is present more than 3 times in a Yi stretch. According to further embodiments, no single aliphatic amino acid is present more than 2 times in a Yi stretch.
According to other particular embodiments, no particular amino acid makes up more than 50% of residues in a Yi stretch (i.e., no more than half the Yi stretch is composed of a particular amino acid). According to further particular embodiments, no single amino acid makes up more than 40% of residues in a Yi stretch. According to even further particular embodiments, no amino acid makes up more than one third of residues in a Yi stretch.
According to yet other particular embodiments, the Yi stretch is not composed of, or even does not contain, di-amino acid repeats. With di-amino acid repeats it is meant three or more, or even two or more, repeats of two non-identical residues. According to further particular embodiments, the Yi stretch is not composed of, or even does not contain, tri-amino acid repeats. With tri-amino acid repeats it is meant three or more, or even two or more, repeats of three residues, at least two of which are non-identical. By way of example, while isoleucine and tryptophan are perfectly acceptable in a Yi region, a Yi region that is exclusively built of IW or IIW repeats also will not be particularly suitable for sequence-specific aggregation. Note that sequence-specific aggregation is not ruled out for any of these sequences, but it is preferred to use more complex sequences to achieve specific aggregation.

The length of the aggregating stretch is typically a trade-off between the desired specificity and the cost and ease of synthesis of hydrophobic sequences. As shown in Figure 2, sequences don't need to be very long to be unique within the proteome of a given organism. For instance, 60% of sequences with a length of 5 amino acids that are present in proteins are unique in humans (i.e. only 40% of such sequences occur more than once). For organisms with less complex genomes, such as E. coli, over 80% of sequences of 5 amino acids encoded by the genome are unique. It can be seen from the figure that the increase in specificity levels off, so that it is rarely necessary to use very long sequences to achieve specificity. The specification describes the Yi region which contains at least 5 residues. According to particular embodiments, the Yi region contains at least 6 residues. According to other, non-exclusive, particular embodiments, the Yi region contains at most 12 residues. According to yet further embodiments, the Yi region contains at most 11 residues. According to even further specific embodiments, the Yi region contains at most 10 residues.
In some instances, it may be desirable to work with sequences that are not unique in a given organism. This is for instance the case in applications directed against (non-self) pathogens, where inhibition of growth and/or killing the pathogenic organism is more important than the targeting of only one specific protein. As can be seen from the figure, the beta-aggregating stretches will typically be shorter in these embodiments, so that more proteins can be targeted.

To avoid unnecessary extension of the Yi stretch, according to particular embodiments, it is envisaged that the N- and/or C-terminal residue of the Yi stretch is a residue that is particularly amenable to β-aggregation, particularly a residue selected from I, L, V, F, Y, W, A, M and T. According to further particular embodiments, the N- and/or C-terminal residue (i.e. at least one selected from the N- and C-terminal residue, or the first and/or last residue) of a Yi stretch is selected from I, L, V, F, Y and W.

According to further particular embodiments, the N- and/or C-terminal residue of a Yi stretch is selected from I, L, V, F, and Y. According to even further particular embodiments, the N- and/or C-terminal residue of a Yi stretch is selected from I, L, V, and F. According to yet even further particular embodiments, the N- and/or C-terminal residue of a Yi stretch is selected from I, L, and V (i.e. is an aliphatic residue). According to further particular embodiments, these limitations apply to both the N- and C-terminal residue of the Yi stretch.

Since it is an object to provide molecules that are capable of specifically downregulating proteins, particularly in a sequence-dependent manner, particularly described are those molecules where at least one of the Yᵢ in the molecule is a stretch of 4 to 17 (particularly 4 to 16 or 4 to 15), or in accordance with aspects or embodiments of the invention a stretch of 6 to 16, amino acids is identical to a contiguous stretch naturally occurring in a protein. It can be said that this contiguous stretch in the protein is the cognate region of the Yi moiety. According to further specific aspects, this is the case for more than one Yᵢ in the molecule, particularly for two Yᵢ or at least two Yᵢ in the molecule, most particularly for all Yᵢ in the molecule. According to very specific embodiments, however, Yi is not identical to a stretch naturally occurring in a protein. This is e.g. envisaged for proteins with an artificial tag, where Yi is identical to a sequence in the artificial tag. Such tags can be useful, as artificial sequence design allows more freedom in determining aggregation propensity of the sequence. This way, it becomes feasible to target proteins that do not have a clear core aggregating region.

Since the at least one Yᵢ stretch confers specificity, it is in some embodiments particularly envisaged that this stretch does not correspond to (part of) a repeating stretch of one or two amino acids (e.g. a polyleucine stretch or alternating leucines and valines). This is particularly the case for those embodiments where n=1. According to further particular embodiments, the Yᵢ stretch contains at least 3 different amino acids in its sequence.

However, according to particular embodiments, at least one Yᵢ is identical to a contiguous stretch naturally occurring in a protein, while at least one other Yᵢ in the same molecule is not identical to a stretch in a protein in the organism in the genome of which the target protein is encoded. The latter Yi is typically a 'booster' sequence, i.e. a sequence known to have a very high tendency for aggregation. This sequence can improve the kinetics of aggregation, or make sure aggregation of the target protein occurs/is initiated at lower concentrations. Such sequences can be synthetic, or can be derived from a sequence (i.e. identical or highly similar to a sequence stretch) present in another organism/species.

If specific targeting of one protein is envisaged, the at least one stretch of 4 to 15, or in accordance with aspects or embodiments of the invention the at least one stretch of 6 to 16, contiguous amino acids naturally occurring in a protein should be unique to said protein in the organism (or species) in the genome of which the protein is encoded (i.e., should be unique in the proteome of said organism/species), to ensure that only one protein in the organism is indeed targeted. The uniqueness is typically only necessary in a specific genome (i.e., the genome of the organism wherein the protein to be downregulated is present). Indeed, if the sequence is present in another organism to which the interferor is not administered (or in which it cannot reach its target), this does not matter. This also applies in instances where it does not matter that a protein in a different organism, typically a microorganism or pathogenic organism, is targeted.

Sometimes, particularly when targeting pathogens or treating or stabilizing infections, it is envisaged that more than one protein may be targeted. In these cases, the protein sequence does not need to be unique in the genome of the organism. It may still be unique to the organism or species, however. This may be envisaged when the molecules described herein are administered to more than one different species simultaneously (e.g. a mixture of microorganisms), while only in one species protein(s) need to be downregulated (e.g. to target a pathogenic species, while not interfering with non-pathogenic or beneficial organisms. Note that this for instance also applies when administering e.g. an interferor molecule targeting an antimicrobial protein to a human subject: in such cases, the sequence of the Yᵢ moiety should not be identical to that of a human protein, or at the least not identical to a human protein with which the interferor can come into contact.). According to further particular embodiments, the sequence is unique to the protein and unique to the organism/species.

Instead of species, the above considerations can also apply to a genus, a family, an order or a class of organisms, although the likelihood of sequence conservation, and finding a unique sequence, decreases with increase in taxonomic rank.

The at least two Yᵢ moieties present (in embodiments where n is at least two) may be identical (i.e. targeting the same protein(s), herein sometimes referred to as 'tandem repeat peptides'), may be different but present in the same protein (targeting the same protein in different ways, i.e. "biaggretopic" interferors, wherein at least two different "aggretopes" or aggregating regions are targeted), may be different and present in different proteins (to target more than one protein in an organism (i.e. true bispecific interferors), or to target proteins in different organisms if the interferor is administered to or brought into contact with more than one (micro)organism). Note that "administering to an organism" may be indirect administering. For instance, in the case of pathogens, it is envisaged that the interferor will be administered to the host organism (typically a subject, either plant or animal subject) to target the pathogenic organism. Since the interferor molecule in these instances will contain at least one aggregating sequence present in the pathogenic organism and aims to aggregate the protein in which this sequence is present, it will be clear to the skilled person that this should be interpreted as "administering the molecule to the pathogenic organism" - it is the contacting (reaching the target) that counts in this regard.

Generally, to achieve specific targeting, a perfect match between the Yi region and the sequence stretch in the protein of interest is envisaged. However, in some instances, it is envisaged that non-identical, but closely related, sequences can be used, i.e. sequences which have one or two substitutions. In order to maintain specificity, the specification describes that for non-conservative substitutions, for Yi stretches less than 6 amino acids, only one amino acid difference is tolerated. For sequences of at least 6 amino acids (particularly at least 7 or at least 8 amino acids), one or two amino acids can be substituted. In accordance with aspects or embodiments of the invention, Yi is a stretch identical to or differing with no more than one amino acid substitution from a contiguous stretch of amino acids occurring in a protein of interest. Alternatively, the specification describes that the sequence identity between the Yi and the aggregating stretch in the protein of interest is at least 70%, at least 75%, particularly at least 80%, at least 85%, at least 90% or even higher. In case of conservative substitutions, the sequence similarity between the Yi and the aggregating stretch in the protein of interest is at least 70%, at least 75%, particularly at least 80%, at least 85%, at least 90% or even higher.

As the skilled person will realize, making a (particularly non-conservative) substitution may result in altered specificity (i.e. making the stretch identical to one of another protein in the organism, or to one of a protein in another organism with which the interferor can come into contact), so it should be checked whether this happens if the altered targeting is undesired. (In some instances, targeting more than one protein may be desired, see also below).

Conservative substitution is the substitution of amino acids with other amino acids whose side chains have similar biochemical properties (e.g. are aliphatic, are aromatic, are positively charged, ...) and is well known to the skilled person. Non-conservative substitution is then the substitution of amino acids with other amino acids whose side chains do not have similar biochemical properties (e.g. replacement of a hydrophobic with a polar residue). Conservative substitutions will typically yield sequences which are not identical anymore, but still highly similar.

Reasons to introduce a substitution may vary. According to particular embodiments, the substitution is with a gatekeeper residue, particularly with one selected from R, K, E, D, P, N, S, A, H, G, Q, more particularly selected from R, K, E, D and P, most particularly selected from R, K and P residues. This may be envisaged to improve solubility or reduce self-aggregation (by 'breaking' the beta-sheet forming potential of the hydrophobic Yi region), or to reduce specificity while maintaining aggregation (This is exemplified in Example 3). Although such substitutions in general decrease specificity, in some embodiments, this may be envisaged to provide easier access to the aggregation-inducing sequence (by 'opening up' the hydrophobic region). This is particularly the case for embodiments where aggregation is favored over specificity (e.g. in antimicrobial applications).

According to alternative embodiments, substitution is conservative substitution. This is envisaged when downregulating a family of proteins is envisaged, and the proteins share a conserved, but not identical, sequence motif. In such instances, aggregation of these closely related proteins can be achieved using a consensus sequence motif (i.e., a similar, but not identical sequence, where 'similar' is used in the context of sequence alignment). However, it is possible that aggregation is less efficient when the sequence match is not 100%.

Another reason to consider substitution is to increase the inherent aggregation propensity of the sequence. For instance, one can consider to replace a particular residue with a residue with higher beta-sheet propensity or aggregation potential. This is not necessarily a conservative substitution. Methods to determine beta-sheet propensity or aggregation potential are well known in the art. By way of example, beta-sheet propensity of a particular residue can be determined taking into account the Chou-Fasman parameters (Chen et al., BMC Bioinformatics 7 (Suppl 4): S14, 2006). One or more residues with a P(b-sheet) score lower than 100 can be replaced with residues with a P(b-sheet) > 100 score. High scoring beta-sheet residues in the Chou-Fasman method are (in descending order): valine, isoleucine, tyrosine, phenylalanine, tryptophan, leucine, threonine, cysteine, glutamine and methionine. Particularly substitution with valine or isoleucine is envisaged to increase beta-sheet forming potential. Of course, specificity should always be checked, and here again, substitution is particularly envisaged for applications where aggregation is favored over specificity.

If the Yi moiety needs to be identical to a stretch occurring in a protein, the specific aggregation-inducing sequences can be identified on sight in the protein sequence (using the above guidelines) or using specific algorithms to identify sequence stretches that satisfy these requirements. Using the same or other method or algorithms, it can be checked whether the sequence occurs in other proteins (or is encoded in genomes of other organisms) as well.

One particularly easy way of identifying such sequences in a protein is by using a beta-aggregation-predicting algorithm first (preferably one taking into account biophysical parameters), and selecting the most proper sequences on the basis of the above sequence limitations. Tango and Zyggregator were already listed as examples of such algorithms, but many more have been described in the art, including, but not limited to those described by Bryan et al., PLoS Comput Biol. 5(3):e1000333, 2009; Caflish, Curr Opin Chem Biol. 10(5):437-44, 2006; Conchillo-Sole et al., BMC Bioinformatics 8:65, 2007; Galzitskaya et al., PLoS Comput Biol. 29;2(12):e177, 2006; Goldschmidt et al., PNAS 107(8):3487-92, 2010; Maurer-Stroh et al., Nat Methods 7(3):237-42, 2010; Rojas Quijano et al., Biochemistry 45(14):4638-52, 2006; Saiki et al., Biochem Biophys Res Commun 343(4):1262-71, 2006; Sanchez de Groot et al., BMC Struct Biol 5:18, 2005; Tartaglia et al., Protein Sci. 14(10):2723-34, 2005; Tartaglia et al., J Mol Biol. 380(2):425-36, 2008; Thompson et al., PNAS 103(11):4074-8, 2006; Trovato et al., Protein Eng Des Sel. 20(10):521-3, 2007; Yoon and Welsh, Protein Sci. 13(8):2149-60, 2004; Zibaee et al., Protein Sci. 16(5):906-18, 2007. Note that many of these are primarily involved with amyloid aggregating sequences and not just with amorphous beta-aggregation. As explained before, the sequence space of both forms of aggregation overlaps (Rousseau et al., Current Opinion in Structural Biology 16:118-126, 2006), and both forms of aggregation are envisaged, as long as the kinetics and conditions of the reaction favour aggregation of the protein of interest. Typically however, such algorithms may also identify polar stretches (such as those present in yeast prion proteins) that do not fulfill the Yi sequence limitations defined herein.

### X₂ᵢ₋₁ and X₂ᵢ moieties: gatekeeper residues

In the interferor molecules described herein, the aggregation-inducing sequences are flanked on both sides by 1 to 4 specific amino acids (the Xᵢ moieties) that have low beta-aggregation potential. These are sometimes referred to as gatekeeper residues (Pedersen et al., J Mol Biol 341: 575-588, 2004), and are essential in keeping the interferor molecules from self-aggregation (particularly prior to being in contact with a target protein).

In the native state of proteins, aggregation is often contained or opposed by naturally occurring charged residues but also e.g. prolines and glycines at the flanks of aggregating sequence segments. These effectively act as gatekeeper residues, i.e. residues that do not necessarily stabilize the native state, but which block the formation of unwanted misfolded or aggregated states by, for example, steric or electrostatic clashes. It has also been reported on several occasions that the introduction of charged residues, prolines or glycines in aggregation-prone sequences reduces aggregation. The aggregation-opposing properties of proline and glycine originate primarily from their structure-breaking properties. Identically charged residues are also very effective at opposing aggregation, because of the huge repulsive force generated upon self-assembly. Interestingly, in nature, arginine and lysine are preferred over glutamate and aspartate at the flanks of strongly aggregating sequences (Rousseau et al., J Mol Biol 355:1037-1047, 2006). The reason for this preference might be that, in addition to charge, arginine and lysine also have much larger conformational entropy, making it very costly to immobilize them in densely packed aggregates.
In the WO2007071789 application, it was stated that such gatekeepers reduce aggregation propensity. In order to optimize co-aggregation of interferors with a given target protein, the self association region of the target protein that is included in the interferor can be mutated so that the gatekeeper residues are replaced by aggregation promoting residues. In other words, the presence of gatekeepers was deemed undesirable.

Now, it was surprisingly demonstrated that, for the strong beta-aggregating regions that make up the Yᵢ moieties, flanking them with gatekeepers (i.e. the two numbered X moieties) indeed reduces their self-aggregation propensity, but does not substantially interfere with their capacity of inducing aggregation of the full-length protein. On the one hand, it is surprising that despite the high hydrophobicity and intrinsic aggregation propensity, these molecules still remain in solution; on the other, the effect of the gatekeepers provided in the molecules does not prevent co-aggregation of the (full-length) protein with the molecule. This unique combination of features makes the molecules provided herein particularly suitable for achieving induced protein aggregation.

These gatekeeper residues are particularly selected from R, K, E, D, P, N, S, H, G and Q residues. Alanine residues might also be used, but since these can also be present in the Yi moieties, it is particularly envisaged that a X moiety as used herein is not just composed of A residues. More specific, it is envisaged that A residues are only used as gatekeeper if another part of the X moiety is at least one residue selected from R, K, P, D or E.
Note that, with the exception of G and P, these are all polar residues. G and P residues are good gatekeepers because of their specific side chain structure (proline) or lack thereof (glycine). Although S, H, N and Q are polar residues, they can be tolerated in a beta-aggregating stretch in low numbers (see above). A similar consideration applies for the G residues.
According to particular embodiments, the residues in the flanking X moieties are residues that are not present in the Yᵢ moiety in between these flanking moieties.
In these instances, the X moieties are typically 1 to 4 amino acids selected from R, K, E, D, P and H. More particularly, they are 1 to 4 amino acids selected from R, K, E, D, and P. Even more particularly, they are selected from R, D and P. Note that P remains in this selection of gatekeepers as it is a very efficient breaker of beta sheet structure. However, according to particular embodiments, especially where n is 1, P is not envisaged as gatekeeper.

Normally, one or two amino acids are enough to break the beta-sheet and keep the molecules in solution. Shorter X moieties are beneficial in terms of ease and cost of synthesis, while being as efficient in their 'gatekeeping' function, i.e. in demarcating and/or presenting the hydrophobic Y moieties. Accordingly, in some particular embodiments, each X₂ᵢ₋₁ and X₂ᵢ is 1 or 2 amino acids (which can be independently selected from each other).

Similarly, peptides which are limited in charge may facilitate interaction with proteins of interest: while still providing possibility of H bonds, the risk of electrostatic repulsion is reduced. Thus, according to alternative, but not exclusive, embodiments, each X₂ᵢ₋₁ and X₂ᵢ has a total charge of no more than 2. Alternatively, the total number of amino acids in both X moieties is 5 or less, particularly 4 or less. Alternative embodiments provide that the total charge of both X moieties surrounding the hydrophobic Y region is less than 5, particularly 4 or less. The embodiments in this paragraph are most particularly envisaged for molecules where n is 1, or molecules where n is two.

As it is particularly envisaged that the Yi moieties do not contain charged residues, the total charge of the molecules will typically be not higher than 5 when n is 1, and not higher than 10 when n is 2. For molecules where n is 2, it is particularly envisaged that the total charge of the molecule is between 2 and 10, more particularly between 2 and 8, more particularly between 4 and 8, even more particularly between 2 and 6 (e.g. 3, 4, 5), between 4 and 6, such as 4, 5 or 6. It is particularly envisaged that the charge is more or less evenly distributed among the molecule, i.e. all X moieties have a similar charge (to put it differently: the charge difference between the X moieties contained in a molecule is particularly not more than one). According to specific embodiments, the molecule is not neutral, i.e. contains some charge. This because charged moieties typically will assist in attaining specificity, i.e. in making the Yi moiety of the molecules interact only with almost completely identical or completely identical polypeptide stretches. Moreover, the charge may help to prevent self-association and premature aggregation of the molecules (i.e., without co-aggregation of the target). Thus, in specific embodiments, at least one - and up to each - numbered X moiety has a charge of 1 or 2. In order to be economical, the X moieties are particularly made up predominantly or exclusively of identically charged residues within the moiety. It is possible that a X₂ᵢ₋₁ moiety has a charge with a different sign than that of the corresponding X₂ᵢ moiety.

According to particular embodiments, X₂ᵢ₋₁ and X₂ᵢ surrounding a Yᵢ region are identical. According to further embodiments, each X₂ᵢ₋₁ in the molecule is identical to each X₂ᵢ. According to even further embodiments, all X₂ᵢ₋₁ and X₂ᵢ in the molecule are identical. According to other specific embodiments, the residues of at least one, and particularly all X₂ᵢ₋₁ mirror the sequence order of the residues in the corresponding X₂ᵢ. That is, if the gatekeeper residues of X₂ᵢ₋₁ are for instance N-P (i.e., an asparagine amino acid N-terminal of a proline amino acid), the residues in X₂ᵢ are P-N (the asparagine C-terminal of the proline residue).

According to alternative embodiments, the charge of at least one, and particularly all, X₂ᵢ₋₁ has the same sign as its corresponding X₂ᵢ. According to further specific embodiments, the charge of at least one, and particularly all, X₂ᵢ₋₁ is identical to that of the corresponding X₂ᵢ. Identically charged residues more strongly oppose aggregation through self-association. According to other embodiments, the charge of all X moieties in the molecule is either neutral or has the same sign. This may help to prevent electrostatic attraction between the molecules.

According to some very specific embodiments, at least part of at least one X moiety flanking the Yi region is also present in the protein of interest. Indeed, hydrophobic sequences in proteins are often flanked by gatekeeper residues, to prevent aggregation. According to specific embodiments where at least one Yi is identical to a sequence naturally occurring in a protein, at least one gatekeeper flanking the Yᵢ in the X moieties of the molecules is identical to a gatekeeper residue occurring in the protein. This is particularly the case in instances where the Yᵢ sequence corresponds to the complete sequence between the gatekeepers in the protein, so that the sequence of the molecule corresponds to the sequence of the protein for at least one Yi stretch and at least part of at least one X stretch neighbouring the Yᵢ stretch. It also applies particularly for proteins where the naturally flanking gatekeeper(s) are not that strong, i.e. are residues that can also be part of a Yᵢ stretch, such as e.g. N or S. Thus, the sequence of the molecule and the protein of interest correspond over a contiguous region of at least one amino acid longer than the Yᵢ stretch of the molecule (i.e., at least one flanking gatekeeper of the aggregating sequence in the protein is included in the molecule).

According to alternative particular embodiments, it is envisaged that the gatekeeper moieties facilitate or stabilize the interaction of the Yi moiety with its cognate region in the protein. This can be the case where the residues flanking the aggregating region in the protein (on the N- and/or C-terminal side) carry a charge. To reduce the chance of repulsion by similar charges, and so maximize the chance of interaction, the charge of the X₂ᵢ₋₁ and/or X₂ᵢ gatekeepers can be chosen to be complementary to the charge of the flanking sequence in the protein. To illustrate this with an example, the calcineurin protein in yeast (see example 1.5) has two aggregation-inducing regions which are flanked by charged residues in the protein sequence. The two protein regions are as follows: NKLR FAFNIY DIDRD (SEQ ID NO: 100), and GNGE LFIVM KMMV (SEQ ID NO: 101) (shown are the two aggregating regions (underlined) with 4 or 5 flanking residues N- or C-terminal thereof). NKLR (SEQ ID NO: 102) has two positively charged amino acids, while the net charge of DIDRD (SEQ ID NO: 103) is minus 2 (three negatively charged D residues, 1 positive R residue). Thus, for embodiments where the gatekeeper moieties have complementary charges, the X moiety on the N-terminus of FAFNIY (SEQ ID NO: 104) should be negatively charged, while the X moiety at the C-terminal side should be positively charged. For the LFIVM sequence (SEQ ID NO: 105), the opposite applies: this is flanked in the calcineurin protein by a negative charge at its N-terminal side and by a positive K residue at its carboxyterminal end. Thus, molecules with complementary gatekeepers have a positive charge at the N-terminal end and a negative charge at the other end. In Example 1.5, this is the case for constructs 6 and 7.

As is evident from the DIDRD (SEQ ID NO: 103) example, opposite charges may be present in flanking regions. To determine the net charge, typically no more than 7 amino acids flanking the sequence are taken into account, preferably even less, such as 5, 4 or 3. The net charge is then the sum of the charge of the residues in this stretch. Residues immediately adjacent to the aggregation-inducing stretch often have a more important contribution to the actual charge. Thus, for instance, if a positively charged residue immediately flanks the aggregating sequence, while a negative residue is e.g. four amino acids further down- or upstream, then the flank can be considered positively charged rather than neutral, as the charge effect from the immediately adjacent residue will be much stronger. According to most particular embodiments, only the residue immediately flanking the aggregation-prone sequence is taken into account for determining charge of the natural sequence.

### Zᵢ moieties: linkers

As outlined in the formula above, the molecules described herein also contain linker moieties, Zᵢ. According to particular embodiments, the molecules only contain internal linkers and no N- or C-terminal linkers (remember: the formula (X₂ᵢ₋₁-Yᵢ-X₂ᵢ-Zᵢ)ₙ as used herein is equivalent to the formula (Zᵢ-X₂ᵢ₋₁-Vᵢ-X₂ᵢ)ₙ, wherein each Z₂ to Zₙ is a linker, and Z₁ is independently selected from a linker or nothing). In other words, the molecules have gatekeeper residues (i.e., the X moieties) at both their N- and C-termini. This corresponds to molecules of the following formula: (X₂ᵢ₋₁-Yᵢ-X₂ᵢ)ₙ wherein n, i, X₂ᵢ₋₁ and X₂ᵢ, and Yᵢ are as defined above, wherein the moieties are fused to each other by use of optional linker moieties. Since an external (N- and or C-terminal) linker may be used to e.g. fuse other moieties to the molecules, this can also be written as Z₀-(X₂ᵢ₋₁-Yᵢ-X₂ᵢ-Zᵢ)ₙ, with X₂ᵢ₋₁ and X₂ᵢ, Yᵢ, i and n as defined above, wherein Z₀ is an optional N-terminal linker moiety, Z₁ to Zₙ₋₁ is a linker, and Zₙ is an optional C-terminal linker moiety.

Thus, in specific embodiments, Zₙ is nothing (or is a linker of zero linking units).

The nature of the linker moieties is not vital to the invention, although long flexible linkers are typically not used. According to particular embodiments, each Zᵢ is independently selected from stretch of between 0 and 20 identical or non-identical units, wherein a unit is an amino acid, a monosaccharide, a nucleotide or a monomer. Non-identical units can be non-identical units of the same nature (e.g. different amino acids, or some copolymers). They can also be non-identical units of a different nature, e.g. a linker with amino acid and nucleotide units, or a heteropolymer (copolymer) comprising two or more different monomeric species. According to particular embodiments, the length of at least one, and particularly each Zᵢ other than Zₙ, is at least 1 unit. According to other particular embodiments, Zₙ is 0 units. According to particular embodiments, all Zᵢ linkers other than Zₙ are identical. According to further embodiments, all Zᵢ moieties are identical.

Amino acids, monosaccharides and nucleotides and monomers have the same meaning as in the art. Note that particular examples of monomers include mimetics of natural monomers, e.g. non-proteinogenic or non-naturally occurring amino acids (e.g. carnitine, GABA, and L-DOPA, hydroxyproline and selenomethionine), peptide nucleic acid monomers, and the like. Examples of other suitable monomers include, but are not limited to, ethylene oxide, vinyl chloride, isoprene, lactic acid, olefins such as ethylene, propylene, amides occurring in polymers (e.g. acrylamide), acrylonitrile-butadiene-styrene monomers, ethylene vinyl acetate, and other organic molecules that are capable of polymer formation.

According to alternative embodiments, the linker units are chemical linkers, such as those generated by carbodiimide coupling. Examples of suitable carbodiimides include, but are not limited to, 1-Ethyl-3-(3-dimethylaminopropyl)carbodiimide (EDC), N,N'-Diisopropylcarbodiimide (DIC), and Dicyclohexylcarbodiimide (DCC). Another particularly envisaged chemical linker is 4, 7, 10-trioxatridecan-succinic acid (sometimes also designated as 4, 7, 10-trioxatridecan-succinamic acid) or Ttds.

According to specific embodiments, at least one, and particularly all, Zi are of between 0 and 10 units of the same nature, particularly between 0 and 5 units of the same nature. If the linkers are flexible, it is particularly envisaged to use short linkers. The use of short linkers prevents that two Yᵢ stretches of the same molecule will fold back on itself, as this would make them less accessible to the protein(s) of interest. Also, by making sure the different Yᵢ stretches of one molecule cannot interact with each other, solubility of the molecule is increased. According to particular embodiments, the linkers are so short that they do not allow the folding of the Yᵢ stretches in antiparallel fashion. For instance for amino acids, at least three or four amino acids are required to make a complete turn, so linkers of no more than four or of no more than three amino acids are particularly envisaged. This also depends on the nature of the amino acids, so the use of amino acids that do not have a particular structural propensity, or a propensity for a kinked structure, such as G, S and P is particularly envisaged. According to particular embodiments, at least one Zi moiety, and particularly all Zᵢ moieties except Zₙ, is a peptide or polypeptide linker. Particularly envisaged sequences of such linkers include, but are not limited to, PPP, PP or GS. For Zᵢ moieties that are made up of amino acids, one can take into account the primary structure (e.g. in the sequence of the linker include many amino acids without a penchant for a particular structure), but also the secondary or tertiary structure. For instance, one can choose amino acids that form no particular secondary structure, or form a (linear) alpha helix. Or, amino acids can be chosen so that they do not form a stable tertiary structure, as this might result in the Yᵢ moieties becoming inaccessible. The amino acid linkers may form a random coil. Another particularly envisaged linker is polyethylene glycol (PEG), i.e. an oligomer or polymer of monomeric ethylene oxide groups. PEG oligomers are often abbreviated whilst indicating the number of monomeric units, e.g. PEG₂, PEG₃ or (PEG)4. According to particular embodiments, at least one Zi moiety is a PEG oligomer (PEG in short). According to further particular embodiments, all Zi moieties are PEG moieties. According to yet alternative embodiments, at least one Zi moiety, and particularly all Zᵢ moieties except Zₙ, is a PEG linker.

The linker is preferably short to prevent antiparallel interactions of the Yᵢ moieties of the same molecule. Note however that in many instances, formation of an antiparallel beta sheet is not favored, even though the length of the linker would make this possible. For instance, for molecules where Yᵢ has the same sequence as the next Yᵢ (i.e., Yᵢ₊₁) and this sequence is not symmetric, no beta sheet will be formed. For such molecules, the linker should be short enough to prevent folding of Yᵢ stretches in parallel fashion - the length of such linkers depend on the length of the Yᵢ stretch and the amount of units needed to make two hairpin turns (i.e., minimal length needed to allow the Yi to be arranged in parallel). Of note, this applies to flexible linkers that allow the folding of the molecules. If it can be ensured that the linkers are rigid and the different Yᵢ regions of one molecule cannot be brought into contact with each other (i.e., cannot interact with each other), the length of the linker is not really important. In such instances, it can be more than 20 units, although typically length will be limited for practical reasons.

One particular example where longer linkers are favoured over other linkers are those instances where at least two different proteins, particularly two different proteins in the same organism, are targeted (i.e. the Yi moieties correspond to aggregation-inducing regions of more than one protein). To ensure that the molecule can (e.g. simultaneously) interact with more than one protein, it may be beneficial to increase the distance between the different targeting Yi moieties, so that the interaction is not prevented due to steric hindrance. In these instances, the Zi linker may be a stretch of between 0 and 100 identical or non-identical units, wherein a unit is an amino acid, a monosaccharide, a nucleotide or a monomer; or of between 0 and 90, 0 and 80, 0 and 70, 0 and 60, 0 and 50, 0 and 40, 0 and 30 or 0 and 20. Particularly, the minimal length of the Zi linker is at least 1 unit, at least 2 units, at least 3 units, at least 4 units, or at least 5 units.

When longer linkers are used, they are preferably not identical to sequences of the proteins from which the at least one Yi region is derived. According to very particular embodiments, a linker of more than 20 units is not a peptidic linker, i.e. the units are not amino acids. According to alternative embodiments, longer linkers can be peptidic linkers, but peptidic linkers containing repeat motifs (e.g. GS, GGS, PP linkers or other linkers containg mono-, di- or tri- aminoacid repeats). Particularly, the linker is essentially free of secondary polypeptide structure, for example of stretches of alpha-helix or beta-sheet. Any predisposition of the polypeptide linker toward a motif of polypeptide secondary structure will necessarily limit the degree of spatial freedom enjoyed by the linker's ends.

### General remarks on molecule structure

As myriad peptides have already been described in the art, it is possible that some molecules with a peptidic structure that falls under the general formula have already been described in the art (for a different purpose) - particularly those where n is 1 (although, to the best of our knowledge, this is not the case). This is why we foresee that the scope of the product claim directed to the molecules, particularly when n is 1, will be different from the scope of the uses and methods in which these molecules can be applied. Accordingly, the specification describes that when n is 1, it is foreseen that the X moieties are more stringently selected (e.g. only from R, K, E, D, P; or e.g. excluding K, cf. above), that the X moieties are shorter (e.g. 1 or 2 amino acids) and do not exist exclusively of K residues, that the Y moieties are shorter (e.g. no longer than 13, 11 or 10 amino acids), selected from a different length range (e.g. 5 to 12 amino acids, or 5 to 10 amino acids) or are more stringently selected (e.g. absence of specific residues as P, R, K, D, E, e.g. more than 60% hydrophobic, ...) than is the case for n which is at least two.

Particularly described molecules are those with the following structure: X₁-Y₁-X₂-Z₁ (i.e. n is 1), wherein X₁ and X₂ are in total no more than 5 amino acids; Y₁ is a stretch of between 4 and 10 amino acids and Z₁ is a stretch of 0 units; and X₁-Y₁-X₂-Z₁-X₃-Y₂-X₄-Z₂ (i.e., n is 2), wherein Z₁ is a linker and Z₂ is nothing.

Particular combinations of limitations that are envisaged for embodiments where n=1 include:
- X₁ and X₂ are equal to each other and are 1 or 2 amino acids selected from R, K, E, D and P; and
- Y₁ is a stretch of 6 to 10 contiguous amino acids at least 3 of which are hydrophobic, in which no P, R, K, D, E or H residue is present, in which the total sum of C, M, N, Q, and W residues is no more than 1, of which less than 60% are small amino acids other than V (i.e. selected from A, C, G, S, P, N, T, D), in which no more than 2 consecutive identical amino acids are present, in which no non-alifatic residue is present more than twice, and in which the first and last residue are aliphatic or selected from F, Y, W, A, M and T.

Another particular combination where n = 1 is
- X₁ and X₂ are 1 or 2 amino acids selected from R, K, E, D and P; and
- Y₁ is a stretch of 6 to 11 contiguous amino acids, at least 75% of which are hydrophobic amino acids, in which at least 50% of the amino acids are aliphatic or F residues, in which no P, R, K, D, E or H residue is present, in which no more than one C, M, N, Q, W, G, S, A or T residue is present, in which no more than 3 Y or F residues are present, in which no two contiguous identical non-aliphatic residues are present (i.e., no 2 contiguous Y or F residues are present, or only I, L and V can be contiguous identical residues)and no more than 2 contiguous identical aliphatic residues are present, in which no two consecutive non-aromatic polar residues (i.e. selected from S, N, T and Q) are present, wherein no more than 50% identical residues are present, wherein the 1^{st} and/or last residue is an aliphatic or F residue, wherein the sum of A and G residues is no more than 2, wherein the total percentage of A, G and S residues is no more than 25%, wherein the total percentage of C, M, N, Q and W residues is no more than 25%, and wherein the total percentage of small residues other than V (i.e. selected from A, C, G, S, N, T) is no more than 25%.

According to some particular embodiments, Z₁ is not present (i.e., Z₁ is zero units). According to other embodiments, Z₁ is also present, and may be N- or C-terminal.

Of note, although these combinations are particularly envisaged and indeed describe a sequence space of well-working compounds, these limitations are primarily intended to assure there is no overlap between the presently claimed compounds and what is described in the prior art. It may very well be that these limitations are too stringent, or that some are too stringent and others are not stringent enough. Therefore, it is envisaged that any single one of these criteria may be varied according to the boundaries described above for the individual moieties, and/or that some of the criteria may be omitted or replaced with other limitations, and/or that other limitations may be added.

### Other moieties

The molecule can further comprise (or can be further fused to) other moieties. For all moieties, the nature of the fusion or linker is not vital to the invention, as long as the moiety and the aggregator molecule can exert their specific function. According to particular embodiments, the moieties which are fused to the molecules can be cleaved off (e.g. by using a linker moiety that has a protease recognition site). This way, the function of the moiety and the molecule can be separated, which may be particularly interesting for larger moieties, or for embodiments where the moiety is no longer necessary after a specific point in time (e.g. a tag that is cleaved off after a separation step using the tag).

It is particularly envisaged that the molecule further comprises a detectable label. The detectable label can be N- or C-terminally or even internally fused to the molecule (e.g. through the linker, or the linker can be used as the detectable label). Alternatively, the detectable label can refer to the use of one or more labeled amino acids in one or more of the X-Y-Z moieties of the molecule (e.g. fluorescently or radioactively labeled amino acids).

Note that, for embodiments where Zₙ is present, the detectable label can be fused to the Zₙ linker moiety. (Although this notation would entail that the tag is added at the C-terminus, N-terminal tags are envisaged as well - this corresponds to the equivalent notation of (Zᵢ-X₂ᵢ₋₁-Yᵢ-X₂ᵢ)ₙ, wherein each Z₂ to Zₙ is a linker, and Z₁ is the linker to which the tag is fused).

However, as the nature of the linker to which the detectable label is fused might differ significantly from that used in the molecule, particularly with regard to length restrictions, it might be preferable to refer to molecules labeled in this way as molecules where the Zn moiety is absent, and where a detectable label is fused to the molecule using a separate linker. Indeed, the linkers used to add the tag to the molecules may be both long and flexible. However, the actual way in which the detectable label is attached to the molecules is not vital to the invention and will typically depend on the nature of the label used and/or the purpose of labeling (which may determine the required proximity). Note that in principle any known label for molecules of proteinaceous nature can be used, as long as the label can be detected. Particularly envisaged labels include, but are not limited to, tags, fluorescent labels, enzyme substrates, enzymes, quantum dots, nanoparticles which may be (para)magnetic, radiolabels, optical labels and the like.

As with other moieties, since the molecules have two ends, it is envisaged that the molecules will be fused to another moiety (e.g. a label) at both its N- and C-terminus. These two labels can be identical (yielding a stronger signal) or different (for different detection purposes). Moieties such as labels can be fused through Z₀ and/or Zₙ linkers, or through longer linkers.

According to particular embodiments, the detectable label is not GFP or biotin. According to other particular embodiments, biotin or GFP can be the detectable label.

According to other particular embodiments, the molecules may be fused to other moieties, e.g. to extend their half-life in vivo. Apart from increasing stability, such moieties may also increase solubility of the molecule they are fused to. Although the presence of gatekeepers (the numbered X moieties) is in principle sufficient to prevent premature aggregation of the molecules and keep them in solution, the further addition of a moiety that increases solubility (i.e. prevents aggregation) may provide easier handling of the molecules, and particularly improve stability and shelf-life. A well-known example of such moiety is PEG (polyethylene glycol). This moiety is particularly envisaged, as it can be used as linker as well as solubilizing moiety. Other examples include peptides and proteins or protein domains, or even whole proteins (e.g. GFP). In this regard, it should be noted that, like PEG, one moiety can have different functions or effects. For instance, a flag tag (sequence DYKDDDDK (SEQ ID NO: 106)) is a peptide moiety that can be used as a label, but due to its charge density, it will also enhance solubilisation. PEGylation has already often been demonstrated to increase solubility of biopharmaceuticals (e.g. Veronese and Mero, BioDrugs. 2008; 22(5):315-29). Adding a peptide, polypeptide, protein or protein domain tag to a molecule of interest has been extensively described in the art. Examples include, but are not limited to, peptides derived from synuclein (e.g. Park et al., Protein Eng. Des. Sel. 2004; 17:251-260), SET (solubility enhancing tag, Zhang et al., Protein Expr Purif 2004; 36:207-216), thioredoxin (TRX), Glutathione-S-transferase (GST), Maltose-binding protein (MBP), N-Utilization substance (NusA), small ubiquitin-like modifier (SUMO), ubiquitin (Ub), disulfide bond C (DsbC), Seventeen kilodalton protein (Skp), Phage T7 protein kinase fragment (T7PK), Protein G B1 domain, Protein A IgG ZZ repeat domain, and bacterial immunoglobulin binding domains (Hutt et al., J Biol Chem.; 287(7):4462-9, 2012). The nature of the tag will depend on the application, as can be determined by the skilled person. For instance, for transgenic expression of the molecules described herein, it might be envisaged to fuse the molecules to a larger domain to prevent premature degradation by the cellular machinery. Other applications may envisage fusion to a smaller solubilisation tag (e.g. less than 30 amino acids, or less than 20 amino acids, or even less than 10 amino acids) in order not to alter the properties of the molecules too much.

Apart from extending half-life, molecules may be fused to moieties that alter other or additional pharmacokinetic and pharmacodynamic properties. For instance, it is known that fusion with albumin (e.g. human serum albumin), albumin-binding domain or a synthetic albumin-binding peptide improves pharmacokinetics and pharmacodynamics of different therapeutic proteins (Langenheim and Chen, Endocrinol.; 203(3):375-87, 2009). Another moiety that is often used is a fragment crystallizable region (Fc) of an antibody. The nature of these moieties is not vital to the invention and can be determined by the person skilled in the art depending on the application.

According to particular embodiments, the molecules are not fused to an agarose bead, a latex bead, a cellulose bead, a magnetic bead, a silica bead, a polyacrylamide bead, a microsphere, a glass bead or any solid support (e.g. polystyrene, plastic, nitrocellulose membrane, glass), or the NusA protein. (Note however, that these fusions are possible, and in specific embodiments, they are also envisaged).

Other moieties which are also envisaged in combination with the molecules described herein are targeting moieties. For instance, the molecules may be fused to e.g. an antibody, a peptide or a small molecule with a specificity for a given target, and the molecule initiates aggregation at the site of the target (in this case, the Yᵢ region(s) will have a sequence identical to one present in the same or a different target protein, or the sequence will be one with high aggregating propensity). This is similar to the strategy which is outlined in WO2008148751. An extensive list of possible target moieties (also designated as 'binding regions' or 'binding domains' in WO2008148751) which can be combined with the molecules of the invention is described in WO2008148751 on page 3 (starting on line 26) and page 4 (ending on line 34): the term 'binding region' or 'binding domain' typically refers to a molecule that interacts with the target protein. In certain cases a binding domain is a chemical compound (e.g. a small compound with an affinity for at least one target protein) and in certain other cases a binding domain is a polypeptide, in certain other cases a binding domain is a protein domain. A protein binding domain is an element of overall protein structure that is self-stabilizing and often folds independently of the rest of the protein chain. Binding domains vary in length from between about 25 amino acids up to 500 amino acids and more. Many binding domains can be classified into folds and are recognizable, identifiable, 3-D structures. Some folds are so common in many different proteins that they are given special names. Non-limiting examples are Rossman folds, TIM barrels, armadillo repeats, leucine zippers, cadherin domains, death effector domains, immunoglobulin-like domains, phosphotyrosine-binding domain, pleckstrin homology domain, src homology 2 domain, the BRCT domain of BRCA1 , G-protein binding domains, the Eps 15 homology (EH) domain and the protein-binding domain of p53. Antibodies are the natural prototype of specifically binding proteins with specificity mediated through hypervariable loop regions, so called complementary determining regions (CDR). Although in general, antibody-like scaffolds have proven to work well as specific binders, it has become apparent that it is not compulsory to stick strictly to the paradigm of a rigid scaffold that displays CDR-like loops. In addition to antibodies, many other natural proteins mediate specific high-affinity interactions between domains. Alternatives to immunoglobulins have provided attractive starting points for the design of novel binding (recognition) molecules. The term scaffold, as used in this invention, refers to a protein framework that can carry altered amino acids or sequence insertions that confer binding to specific target proteins. Engineering scaffolds and designing libraries are mutually interdependent processes. In order to obtain specific binders, a combinatorial library of the scaffold has to be generated. This is usually done at the DNA level by randomizing the codons at appropriate amino acid positions, by using either degenerate codons or trinucleotides. A wide range of different non- immunoglobulin scaffolds with widely diverse origins and characteristics are currently used for combinatorial library display. Some of them are comparable in size to a scFv of an antibody (about 30kDa), while the majority of them are much smaller. Modular scaffolds based on repeat proteins vary in size depending on the number of repetitive units. A non-limiting list of examples comprise binders based on the human 10th fibronectin type III domain, binders based on lipocalins, binders based on SH3 domains, binders based on members of the knottin family, binders based on CTLA-4, T-cell receptors, neocarzinostatin, carbohydrate binding module 4-2, tendamistat, kunitz domain inhibitors, PDZ domains, Src homology domain (SH2), scorpion toxins, insect defensin A, plant homeodomain finger proteins, bacterial enzyme TEM- 1 beta-lactamase, Ig-binding domain of Staphylococcus aureus protein A, E. coli colicin E7 immunity protein, E. coli cytochrome b562, ankyrin repeat domains. Also included as binding domains are compounds with a specificity for a given target protein, cyclic and linear peptide binders, peptide aptamers, multivalent avimer proteins or small modular immunopharmaceutical drugs, ligands with a specificity for a receptor or a co-receptor, protein binding partners identified in a two-hybrid analysis, binding domains based on the specificity of the biotin-avidin high affinity interaction, binding domains based on the specificity of cyclophilin-FK506 binding proteins. Also included are lectins with an affinity for a specific carbohydrate structure.

Of note, for those embodiments where the molecules are fused to a targeting moiety, it is specifically envisaged that at least one, but up to each Yi moiety is a synthetic sequence, more particularly a sequence that is not present in a protein of the organism to which the molecule is administered. Indeed, in such instances, it may be envisaged to nucleate aggregation in situ (upon reaching of the target protein) by the nucleated aggregation of the targeting-moiety fused interferor molecules.

Note however that targeting moieties are not necessary, as the molecules themselves are able to find their target through specific sequence recognition. Thus, according to alternative embodiments, the molecules can effectively be used as targeting moiety and be further fused to other moieties such as drugs, toxins or small molecules. By targeting the molecules to specific proteins (e.g. proteins only occurring in a particular cell type or cell compartment), these compounds can be targeted to the specific cell type/compartment. Thus, for instance, toxins can selectively be delivered to cancer cells, or drugs can be delivered in the cytoplasm.

According to yet other embodiments, the molecules can further comprise a sequence which mediates cell penetration (or cell translocation), i.e. the molecules are further modified through the recombinant or synthetic attachment of a cell penetration sequence. The interferor molecule (e.g. as a polypeptide) may be further fused or chemically coupled to a sequence facilitating transduction of the fusion or chemical coupled proteins into prokaryotic or eukaryotic cells. Cell-penetrating peptides (CPP) or protein transduction domain (PTD) sequences are well known in the art and include, but are not limited to the HIV TAT protein, a polyarginine sequence, penetratin and pep-1. Still other commonly used cell-permeable peptides (both natural and artificial peptides) are disclosed e.g. in Sawant and Torchilin, Mol Biosyst. 6(4):628-40, 2010; Noguchi et al., Cell Transplant. 19(6):649-54, 2010 and Lindgren and Langel, Methods Mol Biol. 683:3-19, 2011.

Typical for CPP is their charge, so it is possible that some charged molecules described herein do not need a CPP to enter a cell. Indeed, as will be shown in the examples, it is possible to target signal peptides or intracellular regions, which require that the molecules are taken up by the cell, and this happens without fusion to a CPP.

In those instances where other moieties are fused to the molecules, it is envisaged in particular embodiments that these moieties can be removed from the molecule. Typically, this will be done through incorporating a specific protease cleavage site or an equivalent approach. This is particularly the case where the moiety is a large protein: in such cases, the moiety may be cleaved off prior to using the molecule in any of the methods described herein (e.g. during purification of the molecules). The cleavage site may be incorporated separately or may be an integral part of the external Zₙ linker (or external Z₁ linker if the moiety is N-terminal). According to very specific embodiments, the moiety may be part of an internal Zi linker, or may even be the whole Zi linker. By way of example, a molecule with n=2 could have the following structure: X₁-Y₁-X₂-Z₁-X₃-Y₂-X₄, wherein Z₁ (in part or in whole) is a hexahistidine sequence: this is then both the linker and detection sequence. Although it is possible, in those instances normally no cleavage site will be built in, as this would lead to cleaving of the molecule itself. Note that according to the embodiments where the additional moiety is fused internally, only non-proteinaceous (e.g. PEG) or peptide sequences with limited length (less than 30, 20 or 10 amino acids, cf. above) are envisaged as solubilization moieties. Otherwise, the protein domain might interfere with induction of aggregation.

According to specific embodiments, the total length of the molecules described herein does not exceed 50 amino acids. More particularly, the length does not exceed 40 amino acids, 30 amino acids, 25 amino acids or even 20 amino acids.. According to further specific embodiments where the molecules are fused to further moieties, the length limitation only applies to the (X₂ᵢ₋₁-Yᵢ-X₂ᵢ-Zᵢ)ₙ part of the total molecule (and thus not e.g. to the label). Thus, if a cleavage site has been built in the molecule, the length restriction typically applies to the length after cleavage.

For molecules that are completely proteinaceous, it is envisaged that they can be provided as nucleic acids, e.g. as a recombinant vector including a sequence encoding at least one molecule described herein.

### Particular applications of the molecules

According to a further aspect, the molecules described herein can be used for downregulating or inhibiting the function of a protein. Typically, this is achieved by inducing aggregation of that protein. The present specification describes methods for downregulating the function of a protein comprising contacting said protein with a molecule of the following structure: (X₂ᵢ₋₁-Yᵢ-X₂ᵢ-Zᵢ)ₙ, wherein:
- n is an integer from 1to 5 and i increases from 1to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, K and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing.

Most particularly, the specification describes that when n is 1, Y₁ may be a stretch of 4 to 11 contiguous amino acids. According to theseexamples, Z₁ may be nothing (i.e. a stretch of 0 units) or a linker other than an amino acid linker or any linker envisaged herein (i.e. no further limitations apply to Z₁).

For the molecules, the same considerations and limitations as above apply. Particularly, it should be noted that, as long as downregulation of the function is achieved, one or two substitutions in the Yi occurring in the protein can be tolerated, as described earlier. Typically, Yᵢ will be identical however.

The molecules can be used across a whole range of fields, including white biotechnology (or industrial biotechnology), red or medical biotechnology, green or agricultural biotechnology, blue (or aquatic) biotechnology. They can be used to inhibit proteins, as well as to detect proteins, and this in all of these fields. As will be seen, applications in which the molecules are administered to a subject show significant similarities across fields, i.e. both in medical (animal subjects) and in agricultural applications (plant subjects), a subdivision can be made to infectious and non-infectious applications.

### Medical applications for interferors

There are two large fields of medical applications for the molecules described herein, namely infectious disease and non-infectious disease. The big difference between the applications is that, in infectious disease settings, the interferor molecule will typically target at least one protein of the pathogen(s) causing the infection, while it is normally administered to a subject whose proteome is not targeted (i.e., the Yi stretch corresponds to one in the proteome of a pathogen, but does not occur in the proteome of the subject with the infection). Direct targeting of the pathogen is however also envisaged (e.g. when treating infections with ectoparasites).

In non-infectious disorders, the interferor molecule will typically target at least one protein present in the subject to which the interferor is administered.

The same consideration (administration to an organism and presence in the proteome of the Yi stretch in said organism) of non-infectious and infectious disease applies for application of interferors in plants, but these are typically not regarded as medical applications. Thus, while similar methods can be practiced on plants (see further), the methods described in this section are considered medical methods as they typically involve an animal subject rather than a plant subject.

According to further particular embodiments, these methods are provided for downregulating a protein in a disease setting, or for making a diagnosis. The specification describes a molecule of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing;
wherein if n is 1, Y₁ is a stretch of 4 to 11 contiguous amino acids (and according to further particular embodiments, Z₁ is not an amino acid linker);
for use as a medicament or diagnostic.

As used herein, methods and uses are often interchangeable, i.e. a molecule for use as a medicament, or a molecule for use in treatment of a specific disease is equivalent to a method for treating the disease comprising the use of (e.g. contacting with) the molecule.

According to particular embodiments, if the molecule is used as medicament or diagnostic, it is envisaged that they are used as exogenous molecules that are administered, and not as a transgene. This also applies for molecules not used as medicament. According to alternative embodiments, the molecules can be administered as a transgene as well as an exogenous molecule.
If the molecules are administered as transgenes (i.e. as nucleic acids encoding the molecules), it goes without saying that the interferor molecules according to these embodiments are entirely of polypeptide nature, since they need to be able to be encoded. I.e., all numbered X, Y and Z moieties present in the interferor molecules are of polypeptide nature. Medical applications in which transgenic delivery is envisaged include, but are not limited to, gene therapy methods (e.g. using lentiviruses) or stem cell applications.

Thus, the present specification describes a nucleic acid sequence that encodes a molecule having the following structure:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing.

It is particularly envisaged that the nucleic acid sequences encode the molecules with all the limitations and variations described herein, *mutatis mutandis.* Thus, the encoded polypeptide is in essence as described herein, that is too say, the variations mentioned for the interferor molecules that are compatible with this aspect are also envisaged as variations for the polypeptides encoded by the nucleic acid sequences. By way of example, embodiments specifying the sequence or the length of the X or Y moieties are compatible with being encoded in nucleic acids, embodiments wherein the Z moiety is of a non-amino acid nature are not.

According to specific embodiments, the encoded polypeptide sequence is a non-naturally occurring polypeptide. According to further particular embodiments, the nucleic acid sequence is an artificial gene. Since the nucleic acid aspect is most particularly suitable in applications making use of transgenic expression, particularly envisaged embodiments are those where the nucleic acid sequence (or the artificial gene) is fused to another moiety, particularly a moiety that increases solubility and/or stability of the gene product. Indeed, transgenic expression of peptides sometimes may be difficult due to rapid degradation of the product.

It should be noted that all methods and uses involving the molecules of the application thus also encompass methods and uses where the molecules are provided as the nucleic acid sequence encoding them, and the molecules are expressed from the nucleic acid sequence.

The specification also describes recombinant vectors comprising such a nucleic acid sequence encoding a molecule as herein described. These recombinant vectors are ideally suited as a vehicle to carry the nucleic acid sequence of interest inside a cell where the protein to be downregulated is expressed, and drive expression of the nucleic acid in said cell. The recombinant vector may persist as a separate entity in the cell (e.g. as a plasmid), or may be integrated into the genome of the cell. Recombinant vectors include i.a. plasmid vectors, binary vectors, cloning vectors, expression vectors, shuttle vectors and viral vectors. Thus, also encompassed herein are methods and uses where the molecules are provided as recombinant vectors with a nucleic acid sequence encoding the molecules, and the molecules are expressed from the nucleic acid sequence provided in the recombinant vector.

The specification also describes cells comprising a nucleic acid sequence encoding a molecule as herein described, or comprising a recombinant vector that contains a nucleic acid sequence encoding such interferor molecule. The cell may be a prokaryotic or eukaryotic cell. In the latter case, it may be a yeast, algae, plant or animal cell (e.g. insect, mammal or human cell). Thus, also encompassed herein are methods and uses where the molecules are provided as cells with a nucleic acid sequence encoding the molecules, and the molecules are expressed from the nucleic acid sequence provided in the cells. This can e.g. be the case in stem cell therapy.

Note that the transgenic approach is not limited to medical applications. According to very particular embodiments, the the provision of interferor molecules encoded in nucleic acid instead of directly as polypeptides is particularly suited for use in plants, as will be discussed below.
Accordingly, the specification describes plants, or plant cells, or plant seeds, that contain a nucleic acid sequence, artificial gene or a recombinant vector as described herein.
In specific embodiments the invention provides a method for the production or manufacture of a medicament or a pharmaceutical composition comprising at least one interferor molecule and furthermore mixing said at least one interferor molecule with a pharmaceutically acceptable carrier. I.e., the interferor is provided for use as a medicament, or pharmaceutical compositions containing interferors are provided.

In a preferred embodiment the interferor molecule is a polypeptide (which means that all linker moieties, Zᵢ, present are of polypeptide nature) and can be made by chemical synthesis or alternatively as a recombinant protein. (Note that the features of the interferor molecules for medical use are also envisaged for non-medical use, where applicable).

A "Polypeptide" refers to a polymer in which the monomers are amino acids and are joined together through amide bonds, alternatively referred to as a peptide. When the amino acids are alpha-amino acids, either the L-optical isomer or the D-optical isomer can be used. Additionally, unnatural amino acids, for example, beta-alanine, phenylglycine and homoarginine are also included. Commonly encountered amino acids that are not gene-encoded may also be used in the present invention. All or part of the amino acids used in the interferors may be either the D- or L-isomer. In addition, other peptidomimetics are also useful in the present invention. We specifically refer and incorporate herein the review of the development and use of peptidomimetics as antagonists for protein-protein interactions from Sillerud LO and Larson RS (2005) Curr Protein Pept Sci. 6(2):151-69. Furthermore, D-amino acids can be added to the peptide sequence to stabilize turn features (especially in the case of glycine). In another approach alpha, beta, gamma or delta turn mimics (such as alpha, beta, gamma, or delta di-peptides can be employed to mimic structural motifs and turn features in a peptide and simultaneously provide stability from proteolysis and enhance other properties such as, for example, conformational stability and solubility.

A recombinant interferor may be manufactured using suitable expression systems comprising bacterial cells, yeast cells, animal cells, insect cells, plant cells or transgenic animals or plants. The recombinant interferor may be purified by any conventional protein or peptide purification procedure close to homogeneity and/or be mixed with additives. In yet another embodiment said interferor is a chemically modified polypeptide. Chemical synthesis enables the conjugation of other small molecules or incorporation of non-natural amino acids by design. In a particular embodiment the conjugation of small molecules to a peptide interferor might lead to a potential application of these molecules in in the growing area of targeted cytotoxic agents for anti-tumor therapy. Incorporation of non-natural amino acids into the peptide opens up the possibility for greater chemical diversity, analogous to small-molecule medicinal chemistry approaches for developing high-affinity, high-specificity molecular recognition. Non-natural amino acids can also prevent rapid degradation of the peptide interferor by rendering the peptide unrecognizable to proteases (e.g. serum or stomach). In yet another embodiment the interferor molecules of the invention comprise modified amino acids such as a D-amino acid or a chemically modified amino acid. In yet another embodiment said interferor consists of a mixture of natural amino acids and unnatural amino acids. In yet another embodiment the half-life of a peptide can be extended by modifications such as glycosylation (Haubner R. et al (2001) J. Nucl. Med. 42, 326-336), conjugation with polyethylene glycol (PEGylation, see Kim TH et al (2002) Biomaterials 23, 2311-2317), or engineering the peptide to associate with serum albumin (see Koehler MF et al (2002) Bioorg. Med. Chem. Lett. 12, 2883-2886). The administration of a pharmaceutical composition comprising an interferor molecule may be by way of oral, inhaled, transdermal or parenteral (including intravenous, intraperitoneal, intramuscular, intracavity, intrathecal, and subcutaneous) administration. Particularly preferred examples of delivery methods for interferors are a transdermal patch (Henry S et al (1998) J. Pharm. Sci. 87, 922-925), iontophoresis (Suzuki Y et al (2002) J. Pharm. Sci. 91, 350-361), sonophoresis (Boucaud A et al (2002) J. Control. Release 81, 113-119), aerosols (Duddu SP et al (2002) Pharm. Res. 19, 689-695), transfersomes or liposomes (Guo J et al (2000) Drug Deliv. 7,113-116). The interferor may be administered alone or preferably formulated as a pharmaceutical composition. (This means methods are provided comprising administering the interferor alone, or formulated as pharmaceutical composition).

It is preferred that the interferor or a pharmaceutically acceptable salt thereof is administered in the form of a unit-dose composition, such as a unit dose oral, parenteral, transdermal or inhaled composition. Such compositions are prepared by admixture and are suitably adapted for oral, inhaled, transdermal or parenteral administration, and as such may be in the form of tablets, capsules, oral liquid preparations, powders, granules, lozenges, reconstitutable powders, injectable and infusable solutions or suspensions or suppositories or aerosols.

Since the molecules are provided for use as a medicament or diagnostic, or in methods of treatment or diagnosis, it is also envisaged that they can be provided as pharmaceutical. Accordingly, the specification describes pharmaceutical compositions comprising the molecules as described herein. Particularly, the pharmaceutical compositions comprise at least one molecule having the following structure:
(X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing;
and a pharmaceutically acceptable carrier.
More particularly, pharmaceutical compositions are provided comprising at least one molecule having the following structure: (X₂ᵢ₋₁-Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; wherein at least one Yᵢ is a stretch of 4 to 16 contiguous amino acids naturally occurring in a protein; and
- each Zᵢ is a linker and Zₙ is independently selected from a linker or nothing;
and a pharmaceutically acceptable carrier.

The specification describes that wherein n is 1, Y₁ typically will be a stretch of 4 to 11 contiguous amino acids and Z₁ is not an amino acid linker (or can even be nothing).

Most particularly, in the pharmaceutical compositions, at least one Yᵢ of the molecules is present in a protein to be downregulated in the subject to which the composition will be administered. Note that this does not imply that this protein is encoded in the genome of that subject. Indeed, for subjects suffering from infection, it is envisaged that molecules are administered that target proteins of the infectious organism and not of the subject itself. (Again, note that similar considerations apply for compositions to be administered to plant subjects, i.e. agrochemical compositions. These may target either plant proteins, or proteins of infectious organisms).

This invention also relates to pharmaceutical compositions containing one or more interferors of the present invention. These compositions can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions that are comprised of a pharmaceutically acceptable carrier and a pharmaceutically effective amount of an interferor, or salt thereof, of the present invention. A pharmaceutically acceptable carrier is preferably a carrier that is relatively non-toxic and innocuous to a patient at concentrations consistent with effective activity of the active ingredient so that any side effects ascribable to the carrier do not vitiate the beneficial effects of the active ingredient. A pharmaceutically effective amount of interferor is preferably that amount which produces a result or exerts an influence on the particular condition being treated. The interferors of the present invention can be administered with pharmaceutically-acceptable carriers well known in the art using any effective conventional dosage unit forms, including immediate, slow and timed release preparations, orally, parenterally, topically, nasally, ophthalmically, optically, sublingually, rectally, vaginally, and the like.

For oral administration, the interferors can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule that can be of the ordinary hard- or soft-shelled gelatin type containing, for example, surfactants, lubricants, and inert fillers such as lactose, sucrose, calcium phosphate, and corn starch.

In another embodiment, the interferors of this invention may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

Dispersible powders and granules are suitable for the preparation of an aqueous suspension. They provide the active ingredient (i.e. the at least one interferor) in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The pharmaceutical compositions of this invention may also be in the form of oil-in-water emulsions. The oily phase may be a vegetable oil such as liquid paraffin or a mixture of vegetable oils. Suitable emulsifying agents may be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions may be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or n-propyl p-hydroxybenzoate; one or more coloring agents; one or more flavoring agents; and one or more sweetening agents such as sucrose or saccharin. Syrups and elixirs may be formulated with sweetening agents such as, for example, glycerol, propylene glycol, sorbitol or sucrose. Such formulations may also contain a demulcent, and preservative, such as methyl and propyl parabens and flavoring and coloring agents. The interferors of this invention may also be administered parenterally, that is, subcutaneously, intravenously, intraocularly, intrasynovially, intramuscularly, or intraperitoneally, as injectable dosages of the interferor in preferably a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1 ,1 - dioxolane-4- methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) preferably of from about 12 to about 17. The quantity of surfactant in such formulation preferably ranges from about 5% to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethyl-cellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived from a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

A composition of the invention may also be administered in the form of suppositories for rectal administration of the drug. These compositions can be prepared by mixing the drug with a suitable non-irritation excipient which is solid at ordinary temperatures but liquid at the rectal temperature and will therefore melt in the rectum to release the drug. Such materials are, for example, cocoa butter and polyethylene glycol.

Another formulation employed in the methods of the present invention employs transdermal delivery devices ("patches"). Such transdermal patches may be used to provide continuous or discontinuous infusion of the interferors of the present invention in controlled amounts. The construction and use of transdermal patches for the delivery of pharmaceutical agents is well known in the art (see for example US 5,023,252). Such patches may be constructed for continuous, pulsatile, or on demand delivery of pharmaceutical agents. Controlled release formulations for parenteral administration include liposomal, polymeric microsphere and polymeric gel formulations that are known in the art. It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US 5,011 ,472.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M. F. et al., "Compendium of Excipients for Parenteral Formulations" PDA Journal of Pharmaceutical Science & Technology 1998, 52(5), 238-311 ; Strickley, R.G "Parenteral Formulations of Small Molecule Therapeutics Marketed in the United States (1999)-Part-1" PDA Journal of Pharmaceutical Science & Technology 1999, 53(6), 324-349 ; and Nema, S. et al., "Excipients and Their Use in Injectable Products" PDA Journal of Pharmaceutical Science & Technology 1997, 51 (4), 166-171.

Commonly used pharmaceutical ingredients that can be used as appropriate to formulate the composition for its intended route of administration include:
- acidifying agents (examples include but are not limited to acetic acid, citric acid, fumaric acid, hydrochloric acid, nitric acid); alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine); adsorbents (examples include but are not limited to powdered cellulose and activated charcoal); aerosol propellents (examples include but are not limited to carbon dioxide, CCl₂F₂, F₂ClC-CClF₂ and CClF₃) air displacement agents (examples include but are not limited to nitrogen and argon); antifungal preservatives (examples include but are not limited to benzoic acid, butylparaben, ethylparaben, methylparaben, propylparaben, sodium benzoate); antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal); antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite); binding materials (examples include but are not limited to block polymers, natural and synthetic rubber, polyacrylates, polyurethanes, silicones, polysiloxanes and styrene-butadiene copolymers); buffering agents (examples include but are not limited to potassium metaphosphate, dipotassium phosphate, sodium acetate, sodium citrate anhydrous and sodium citrate dihydrate) carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection) chelating agents (examples include but are not limited to edetate disodium and edetic acid) colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red) ;
- clarifying agents (examples include but are not limited to bentonite);
- emulsifying agents (examples include but are not limited to acacia, cetomacrogol, cetyl alcohol, glyceryl monostearate, lecithin, sorbitan monooleate, polyoxyethylene 50 monostearate);
- encapsulating agents (examples include but are not limited to gelatin and cellulose acetate phthalate);
- flavorants (examples include but are not limited to anise oil, cinnamon oil, cocoa, menthol, orange oil, peppermint oil and vanillin);
- humectants (examples include but are not limited to glycerol, propylene glycol and sorbitol);
- levigating agents (examples include but are not limited to mineral oil and glycerin);
- oils (examples include but are not limited to arachis oil, mineral oil, olive oil, peanut oil, sesame oil and vegetable oil);
- ointment bases (examples include but are not limited to lanolin, hydrophilic ointment, polyethylene glycol ointment, petrolatum, hydrophilic petrolatum, white ointment, yellow ointment, and rose water ointment);
- penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
- plasticizers (examples include but are not limited to diethyl phthalate and glycerol);
- solvents (examples include but are not limited to ethanol, corn oil, cottonseed oil, glycerol, isopropanol, mineral oil, oleic acid, peanut oil, purified water, water for injection, sterile water for injection and sterile water for irrigation);
- stiffening agents (examples include but are not limited to cetyl alcohol, cetyl esters wax, microcrystalline wax, paraffin, stearyl alcohol, white wax and yellow wax);
- suppository bases (examples include but are not limited to cocoa butter and polyethylene glycols (mixtures);
- surfactants (examples include but are not limited to benzalkonium chloride, nonoxynol 10, oxtoxynol 9, polysorbate 80, sodium lauryl sulfate and sorbitan mono-palmitate);
- suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
- sweetening agents (examples include but are not limited to aspartame, dextrose, glycerol, mannitol, propylene glycol, saccharin sodium, sorbitol and sucrose);
- tablet anti-adherents (examples include but are not limited to magnesium stearate and talc);
- tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
- tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
- tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac) ;
- tablet direct compression excipients (examples include but are not limited to dibasic calcium phosphate);
- tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
- tablet glidants (examples include but are not limited to colloidal silica, corn starch and talc);
- tablet lubricants (examples include but are not limited to calcium stearate, magnesium stearate, mineral oil, stearic acid and zinc stearate);
- tablet/capsule opaquants (examples include but are not limited to titanium dioxide);
- tablet polishing agents (examples include but are not limited to carnuba wax and white wax);
- thickening agents (examples include but are not limited to beeswax, cetyl alcohol and paraffin);
- tonicity agents (examples include but are not limited to dextrose and sodium chloride);
- viscosity increasing agents (examples include but are not limited to alginic acid, bentonite, carbomers, carboxymethylcellulose sodium, methylcellulose, polyvinyl pyrrolidone, sodium alginate and tragacanth); and
- wetting agents (examples include but are not limited to heptadecaethylene oxycetanol, lecithins, sorbitol monooleate, polyoxyethylene sorbitol monooleate, and polyoxyethylene stearate).

Non-limited examples of pharmaceutical compositions according to the present invention can be illustrated as follows:
- Sterile IV Solution: A 5 mg/mL solution of the desired interferor of this invention can be made using sterile, injectable water, and the pH is adjusted if necessary. The solution is diluted for administration to 1 - 2 mg/mL with sterile 5% dextrose and is administered as an IV infusion over about 60 minutes.
- Lyophilised powder for IV administration: A sterile preparation can be prepared with (i) 100 - 1000 mg of the desired interferor of this invention as a lyophilised powder, (ii) 32- 327 mg/mL sodium citrate, and (iii) 300 - 3000 mg Dextran 40. The formulation is reconstituted with sterile, injectable saline or dextrose 5% to a concentration of 10 to 20 mg/mL, which is further diluted with saline or dextrose 5% to 0.2 - 0.4 mg/mL, and is administered either IV bolus or by IV infusion over 15 - 60 minutes.
- Intramuscular suspension: The following solution or suspension can be prepared, for intramuscular injection:
   50 mg/mL of the desired, water-insoluble (or water soluble) interferor of this invention
   5 mg/mL sodium carboxymethylcellulose
   4 mg/mL TWEEN 80
   9 mg/mL sodium chloride
   9 mg/mL benzyl alcohol

Hard Shell Capsules: A large number of unit capsules are prepared by filling standard two-piece hard galantine capsules each with 100 mg of powdered active ingredient, 150 mg of lactose, 50 mg of cellulose and 6 mg of magnesium stearate.

Soft Gelatin Capsules: A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.

Tablets: A large number of tablets are prepared by conventional procedures so that the dosage unit is 100 mg of active ingredient, 0.2 mg of colloidal silicon dioxide, 5 mg of magnesium stearate, 275 mg of microcrystalline cellulose, 11 mg. of starch, and 98.8 mg of lactose. Appropriate aqueous and nonaqueous coatings may be applied to increase palatability, improve elegance and stability or delay absorption.

Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid state extraction techniques. The drug interferors may be compressed with viscoelastic and thermoelastic sugars and polymers or effervescent components to produce porous matrices intended for immediate release, without the need of water.

In a particular embodiment the interferors are formulated in controlled release parenteral formulations. A non-limiting list of such controlled release parenteral delivery systems which can be applied for the administration of interferors include oil-based injections, implants, liposomes, nanoparticles, PEGylation, mircrospheres and pumps. In a particular embodiment solid lipid nanoparticles can be developed as carrier systems for interferors. In another particular embodiment interferors can be microencapsulated. Microspheres are free-flowing powders, ideally less than about 125 µm in diameter, which can be suspended in suitable aqueous vehicles for injection with a conventional syringe using an 18 or 20-gauge needle. The most promising polymers for such use are lactide/glycolide co-polymers, poly(ortho esters) and polyanhydrides. Poly (D,L-lactide-co-glycolide) (PLGA) is a biodegradable polymer that hydrolyses with an acid or base-catalysed reaction to form the natural metabolites glycolic acid and lactic acid. PLGA is FDA-approved.

According to particular embodiments, the molecule can be provided in lyophilized form with a physiological buffer. According to particular embodiments, the pharmaceutical compositions, if a liquid, are provided at physiological pH, particular between pH 5 and 9, more particular between pH 6 and pH 8.

The term 'administering' as used herein has the object of contacting the protein(s) of interest with the molecules. Note that, e.g. in the case of infectious disease, the administering may be to a different organism than the one wherein the protein(s) should be aggregated. Intracellular administration can be through carrier-mediated delivery, e.g. by liposomal carriers or nano-particles or by injection. In yet another alternative embodiment the aggregator molecule can enter a cell through a sequence which mediates cell penetration (or cell translocation). In the latter case the aggregator molecule is further modified through the recombinant or synthetic attachment of a cell penetration sequence.

### Combination therapies

In a particular embodiment the interferors of this invention can be administered as the sole pharmaceutical agent or in combination with one or more other pharmaceutical agents where the combination causes no unacceptable adverse effects. The present invention relates also to such combinations. For example, the interferors of this invention can be combined with known antimicrobial agents (e.g. anti-fungals or antibiotics) or other indication agents, and the like, as well as with admixtures and combinations thereof.

The term "treating" or "treatment" as stated throughout this document is used conventionally, e.g. , the management or care of a subject for the purpose of combating, alleviating, reducing, relieving, improving the condition of, etc., of a disease or disorder, such as a carcinoma. Treating can be applied both for infectious diseases as for non-infectious disorders.

### Dose and administration:

Based upon standard laboratory techniques known to evaluate interferors useful for the treatment of infectious disease (as shown in the examples, in particular fungal infections, bacterial infections, or viral infections, in particular *Candida albicans* infections, *Staphylococcus sp.* infections and influenza virus infections), , or for the treatment of non-infectious disease (such as cancer, AMD and inflammation, as shown in the Examples) by standard toxicity tests and by standard pharmacological assays for the determination of treatment of the conditions identified above in mammals, and by comparison of these results with the results of known medicaments that are used to treat these conditions, the effective dosage of the interferors of this invention can readily be determined for treatment of each desired indication. The amount of the active ingredient to be administered in the treatment of one of these conditions can vary widely according to such considerations as the particular interferor and dosage unit employed, the mode of administration, the period of treatment, the age and sex of the patient treated, and the nature and extent of the condition treated.

The total amount of the active ingredient to be administered will generally range from about 0.001 mg/kg to about 200 mg/kg body weight per day, and preferably from about 0.01 mg/kg to about 50 mg/kg body weight per day. Clinically useful dosing schedules will range from one to three times a day dosing to once every four weeks dosing. In addition, "drug holidays" in which a patient is not dosed with a drug for a certain period of time, may be beneficial to the overall balance between pharmacological effect and tolerability. A unit dosage may contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight. Preferably, compositions for inhalation are presented for administration to the respiratory tract as a snuff or an aerosol or solution for a nebulizer, or as a microfine powder for insufflation, alone or in combination with an inert carrier such as lactose. In such a case the particles of active interferor suitably have diameters of less than 50 microns, preferably less than 10 microns, for example between 1 and 5 microns, such as between 2 and 5 microns. Alternatively, coated nanoparticles can be used, with a particle size between 30 and 500 nm. A favored inhaled dose will be in the range of 0.05 to 2 mg, for example 0.05 to 0.5 mg, 0.1 to 1 mg or 0.5 to 2 mg.

It is evident for the skilled artisan that the specific initial and continuing dosage regimen for each patient will vary according to the nature and severity of the condition as determined by the attending diagnostician, the activity of the specific interferor employed, the age and general condition of the patient, time of administration, route of administration, rate of excretion of the drug, drug combinations, and the like. The desired mode of treatment and number of doses of an interferor of the present invention or a pharmaceutically acceptable salt or ester or composition thereof can be ascertained by those skilled in the art using conventional treatment tests.

In certain embodiments, the medicaments of the invention are administered to the class of mammalian, including the orders carnivore (e.g., dogs and cats), rodentia (e.g., mice, guinea pigs, and rats), lagomorpha (e.g. rabbits) and primates (e.g., humans, chimpanzees, and monkeys).

As is common practice, the compositions will usually be accompanied by written or printed directions for use in the medical treatment concerned.

The present invention also includes isotopically labelled interferors, which are identical to those defined herein, but for the fact that one or more atoms are replaced by an atom having an atomic mass or mass number different from the atomic mass or mass number usually found in nature. Examples of isotopes that may be incorporated into interferors of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Interferors of the present invention and pharmaceutically acceptable salts of said interferors or which contain the aforementioned isotopes and/or other isotopes of other atoms are within the scope of this invention. Certain isotopically labeled interferors of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, may afford certain therapeutic advantages resulting from greater metabolic stability, for example increased in vivo half-life or reduced dosage requirements and, hence, may be preferred in some circumstances, Isotopically labelled interferors of formula I of this invention may generally be prepared by carrying out the procedures disclosed in the Examples below, by substituting a readily available isotopically labelled reagent for a non-isotopically labelled reagent.

As mentioned, the molecules may be applied to treat both non-infectious and infectious disease. According to a first embodiment,the molecules of the invention can be used for the treatment of (non-infectious) diseases (alternatively for the manufacture of a medicament to treat (non-infectious) diseases), such as cancer, an inflammatory disease or an immune related disorder, associated with the aberrant expression of a particular protein (e.g. through the overexpression of a particular protein or the (over)-expression of a splice variant of a particular protein or a mutant version of a particular protein, the (over)-expression of a membrane-anchored protein, the (over)-expression of a (mutant) transmembrane protein, the (over)-expression of a secreted protein (e.g. a protease, an antibody or a cytokine present in the blood or plasma), the (over)-expression of a protein in the extracellular matrix (e.g. a matrix metalloprotein or a transmembrane protein (e.g. a growth factor receptor). The term 'aberrant expression' refers to for example the (over)expression of an oncogenic growth factor in the case of cancer, it also includes the expression of a dominant negative receptor or a mutant receptor or the occurrence of a shedded receptor in the blood or the expression (or over-expression) of a cytokine or a growth factor in a body fluid. In a particular embodiment the "aberrant expression" refers to the unwanted presence of a post-translationally modified protein or to the undesired presence of a non-post-translationally modified protein. Post-translational modifications alter the physico-chemical properties of the modified amino acids, and as such they have the potential of altering the aggregation tendency of a given polypeptide segment that can be exploited to specifically target the form that has the strongest aggregation tendency. So if a post-translational modification significantly decreases the aggregation tendency of the beta-aggregation region, then interference will be most efficient with the unmodified protein. In contrast, in case of post-translational modifications that increase the aggregation tendency of the beta-aggregation region, then interference will be most efficient with the modified protein. Based on the hydrophobicity alone, it is assumed that modifications such as phosphorylation and glycosylation will decrease aggregation tendency, whereas lipid attachment will increase aggregation tendency.

In specific embodiments, the molecules can be used to downregulate a (one or more) protein that needs to be downregulated in a disease setting. Such a protein typically is a protein whose overexpression is associated with, and preferably causative of, the disease (for instance, VEGFR-2 is a protein whose overexpression is causatively linked with cancer and AMD, EGFR is a protein whose overexpression is causatively linked to cancer, TNF is a protein whose overexpression is causatively linked to inflammation). However, it can also be a protein that is only expressed in disease settings and is normally not expressed in the cells/tissue/subject to be targeted when the cells/tissue/subject is healthy (e.g. PIGF is a protein not normally expressed in adult tissue, but it is expressed in tumours). A protein to be downregulated may also be a protein that is (typically downstream) in the same signaling pathway of a protein causative of the disease (e.g. a receptor when the ligand is associated with disease) so that the aberrant signal can be stopped.

Accordingly, the specification describes molecules of the structure (X₂ᵢ₋₁-Yᵢ-X₂ᵢ-Zᵢ)ₙ for use in treatment, stabilization or prevention of a disease, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with said disease; and
- each Zᵢ is a linker and Zₙ is independently selected from a linker or nothing.

Particularly envisaged diseases to be treated include, but are not limited to, cancer, AMD and inflammatory disease. Thus, the specification describes molecules of the structure (X₂ᵢ₋₁-Yᵢ-X₂ᵢ-Zᵢ)ₙ, for use in treatment, stabilization or prevention of cancer, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with cancer; and
- each Zᵢ is a linker and Zₙ is independently selected from a linker or nothing.

Similarly, the specification describes molecules of the structure (X₂ᵢ₋₁-Yᵢ-X₂ᵢ-Zᵢ)ₙ, for use in treatment, stabilization or prevention of age-related macular degeneration (AMD), wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with AMD; and
- each Zᵢ is a linker and Zₙ is independently selected from a linker or nothing.

Likewise, the specification describes molecules of the structure (X₂ᵢ₋₁-Yᵢ-X₂ᵢ-Zᵢ)ₙ, for use in treatment, stabilization or prevention of inflammatory disease, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with inflammatory disease; and
- each Zᵢ is a linker and Zₙ is independently selected from a linker or nothing.

Most particularly, the protein to be downregulated in cancer or AMD is VEGFR-2. Alternatively, the protein to be downregulated in cancer is EGFR2. Particular proteins that can be downregulated in inflammatory disease include TNF-α and IL-1β.

As mentioned before, this is equivalent as saying that methods are described to treat, stabilize or prevent a disease (particularly cancer, AMD or inflammatory disease, respectively) in a subject in need thereof, comprising: administering to the subject a molecule having the structure (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, K and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with the disease (particularly cancer, AMD or inflammatory disease, respectively); and
- each Zᵢ is a linker and Zₙ is independently selected from a linker or nothing.

Or, in Swiss-type format, the use of molecules of the structure (X₂ᵢ₋₁-Yᵢ-X₂ᵢ-Zᵢ)ₙ is described for the manufacture of a medicament for treatment, stabilization or prevention of disease (particularly cancer, AMD or inflammatory disease, respectively), wherein:
n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, K and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein whose expression or overexpression is associated with the disease (particularly cancer, AMD or inflammatory disease, respectively); and
- each Zᵢ is a linker and Zₙ is independently selected from a linker or nothing.

These three equivalent formulations are included to comply with different national requirements of patent law.

The subject particularly is an animal, more particularly a mammal (e.g. cat, dog, rabbit, horse, cow, pig, sheep, goat, llama, mouse, rat, ...), most particularly a human. Other subjects are envisaged as well (see definitions).

Note however that these methods can also be applied to organisms other than mammals, particularly to treat disease in plants (see further).

In a particular embodiment, the interferor molecule is directed against a growth factor or growth factor receptor, or a transmembrane receptor kinase (e.g. a tyrosine kinase receptor). A list of non-limiting examples comprises PDGF-beta protein (and specific interferors can be used to treat a subject having a disorder characterized by unwanted PDGF-beta expression, e.g. testicular and lung cancers), the Erb-B protein (and specific interferors can be used to treat a subject having a disorder characterized by unwanted Erb-B expression, e. g. breast cancer), the VEGF protein (and specific interferors can be used to treat a subject having unwanted VEGF expression, e.g. esophageal, colon cancers or pathological angiogenesis), the EGFR protein (and specific interferors can be used to treat a subject having a disorder characterized by unwanted EGFR expression, e.g. breast cancer), the WNT-1 protein (and specific interferors can be used to treat a subject having unwanted WNT-1 expression, e.g. basal cell carcinoma), the Her2/Neu protein (and interferors can be used to treat a subject having a disorder characterized by unwanted Her2/Neu expression, e.g. breast cancer), the alpha v-integrin protein (and interferors can be used to treat a subject having a disorder characterized by unwanted alpha v-integrin, e.g. brain tumors or tumors of epithelial origin), the Flt-1 receptor protein (and interferors can be used to treat a subject having a disorder characterized by unwanted Flt-I receptors e.g. cancer and rheumatoid arthritis). Still other examples of specific interferors can be designed with a specificity for co-ligands of integrins e.g. VLA4, VCAM, ICAM, selectin or co-ligand thereof, e.g. P-selectin, E-selectin (ELAM), L-selectin, or P-selectin glycoprotein-(PSGLl), a component of the complement system, e.g., C3, C5, C3aR, C5aR, C3 convertase, C5 convertase, the function of a chemokine or receptor thereof e.g. TNF-α, IL-1α, IL-1, IL-2, IL-2R, IL-4, IL-4R, IL-5, IL-6, IL-8, TNFRI, TNFRII, IgE, SCYA11 or CCR3, a component of an ion channel, a component of a G protein coupled receptor or with the function of a neurotransmitter receptor or ligand thereof.

According to another aspect, particularly envisaged medical applications are the use of the molecules provided herein as a medicament in infectious disease.
Thus, the specification describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pathogenic organism; and
- each Zᵢ is a linker and Zₙ is independently selected from a linker or nothing;
for use as an anti-pathogenic (or anti-infectious) drug.

According to a particular embodiment, the molecules are used as antimicrobial agents. Thus, the specification describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism; and
- each Zᵢ is a linker and Zₙ is independently selected from a linker or nothing;
for use as an antimicrobial.

The use of the interferors thus is contemplated for the treatment of infection with animal and human bacterial pathogens, for animal and human fungal pathogens and for animal and human parasitic pathogens. A non-exclusive list of animal and human pathogens is found in Table 1A of US patent 8,088,888 (starting on page 8, ending on page 15). A non-exclusive list of animal and fungal pathogens is found in Table 1B of US patent 8,088,888 (starting on page 15, ending on page 18). A non-exclusive list of animal and human parasitic pathogens is found in Table 1C of US patent 8,088,888 (starting on page 18, ending on page 20). Tables 1A, 1B and 1C of US patent 8,088,000 are hereby incorporated in its entirety by reference.

According to another particular embodiment, the molecules are used as antiviral agents. Thus, the specification describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a viral organism; and
- each Zᵢ is a linker and Zₙ is independently selected from a linker or nothing;
for use as an antiviral.

Another equivalent formulation is that the use of these molecules is provided for the manufacture of a medicament for treatment of pathogen infection (or microbial or viral infection, respectively), i.e. a Swiss-type claim.

This is also equivalent as saying that methods are described to prevent or treat pathogen infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pathogen; and
- each Zᵢ is a linker and Zₙ is independently selected from a linker or nothing.

Or, in the case of antimicrobials, it is equivalent to methods described to treat microbial infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism; and
- each Zᵢ is a linker and Zₙ is independently selected from a linker or nothing.

Or, in the case of antivirals, it is equivalent to methods described to treat viral infection in a subject in need thereof, comprising:
administering to the subject a molecule having the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a viral organism; and
- each Zᵢ is a linker and Zₙ is independently selected from a linker or nothing.

Further steps of the methods may include the evaluation of (or the measuring of) the presence of the pathogenic (e.g. microbial, viral) organism (e.g. monitoring growth, reproduction or survival of the pathogenic organism).

"Microbial organism" as used herein may refer to bacteria, such as Gram-positive bacteria (e.g. cocci such as Staphylococcus sp., Enterococcus sp., bacilli such as Bacillus sp.), Gram-negative bacteria (e.g. Escherichia species, Yersinia species), Spirochaetes (e.g. Treponema species such as Treponema pallidum, Leptospira species, Borrelia species, such as Borrelia burgdorferi), Mollicutes (i.e. bacteria without a cell wall, such as Mycoplasma species), acid-fast bacteria (e.g. Mycobacterium species such as Mycobacterium tuberculosum, Nocardia species). "Microbacterial organisms" also encompasses fungi (such as yeasts and moulds, e.g. Candida species, Aspergillus species, Coccidioides species, Cryptococcus species, Histoplasma species, Pneumocystis species, or Trichophyton species), protozoa (e.g. Plasmodium species, Entamoeba species, Giardia species, Toxoplasma species, Cryptosporidium species, Trichomonas species, Leishmania species, Trypanosoma species) and Archaea. According to particular embodiments, the protein of a microbial organism is a protein of a microbial organism selected from bacteria, fungi or protozoa. More particularly, the protein is one of Gram-positive bacteria, Gram-negative bacteria, mycobacteria, Mollicutes, protozoa, or fungi. More particularly, it is a protein of Gram-positive bacteria, Gram-negative bacteria, mycobacteria, or fungi such as yeasts and moulds.

"Viral organism" or "virus", which are used as equivalents herein, are small infectious agents that can replicate only inside the living cells of organisms. They include dsDNA viruses (e.g. Adenoviruses, Herpesviruses, Poxviruses), ssDNA viruses (e.g. Parvoviruses), dsRNA viruses (e.g. Reoviruses), (+)ssRNA viruses (e.g. Picornaviruses, Togaviruses), (-)ssRNA viruses (e.g. Orthomyxoviruses, Rhabdoviruses), ssRNA-RT (reverse transcribing) viruses, i.e. viruses with (+)sense RNA with DNA intermediate in life-cycle (e.g. Retroviruses), and dsDNA-RT viruses (e.g. Hepadnaviruses). A particular class of viruses are bacteriophages (or phages in short), viruses which infect bacteria and inject their genetic material (ssRNA, dsRNA, ssDNA, or dsDNA). Phages are particularly abundant in sea water and many marine bacteria are infected with phages. Bacteriophages might represent a problem in industrial processes where bacteria are used (e.g. in food production such as yoghurt, cheese, and the like).

In methods directed to treating a viral infection or inhibiting viral infectivity in a non-human animal, the animal virus is preferably selected from a picornavirus, such as a bovine enterovirus, a porcine enterovirus B, a foot-and-mouth disease virus, an equine rhinitis A virus, a bovine rhinitis B virus, a Ijungan virus, equine rhinitis B virus, an aichi virus, a bovine kobuvirus, a porcine teschovirus, a porcine sapelovirus, a simian sapelovirus, an avian sapelovirus, an avian encephalomyelitis virus, a duck hepatitis A virus, or a simian enterovirus A; a pestivirus, such as border disease virus, a bovine virus diarrhea, or a classical swine fever virus; an arterivirus, such as an equine arteritis virus, a porcine reproductive and respiratory syndrome virus, a lactate dehydrogenase elevating virus, or a simian haemorrhagic fever virus; a coronavirus, such as a bovine coronavirus, a porcine coronavirus, a feline coronavirus, or a canine coronavirus; a paramyxovirus, such as a hendra virus, a nipah virus, a canine distemper virus, a rinderpest virus, a Newcastle disease virus, and a bovine respiratory syncytial virus; an orthomyxovirus, such as an influenza A virus, an influenza B virus, or an influenza C virus; a reovirus, such as a bluetongue virus; a porcine circovirus, a herpesvirus, such as a pseudorabies virus or a bovine herpesvirus 1; an asfarvirus, such as an African swine fever virus; a retrovirus, such as a simian immunodeficiency virus, a feline immunodeficiency virus, a bovine immunodeficiency virus, a bovine leukemia virus, a feline leukemia virus, a Jaagsiekte sheep retrovirus, or a caprine arthritis encephalitis virus; a flavivirus, such as a yellow fever virus, a West Nile virus, a dengue fever virus, a tick borne encephalitis virus, or a bovine viral diarrhea; or a rhabdovirus, such as a rabies virus.

In methods directed to treating a viral infection or inhibiting viral infectivity in a human, the human virus is preferably selected from an adenovirus, an astrovirus, a hepadnavirus, a herpesvirus, a papovavirus, a poxvirus, an arenavirus, a bunyavirus, a calcivirus, a coronavirus, a filovirus, a flavivirus, an orthomyxovirus, a paramyxovirus, a picornavirus, a reovirus, a retrovirus, a rhabdovirus, or a togavirus. In preferred embodiments, the adenovirus includes, but is not limited to, a human adenovirus. In preferred embodiments, the astrovirus includes, but is not limited to, a mamastrovirus. In preferred embodiments, the hepadnavirus includes, but is not limited to, the hepatitis B virus. In preferred embodiments, the herpesvirus includes, but is not limited to, a herpes simplex virus type I, a herpes simplex virus type 2, a human cytomegalovirus, an Epstein-Barr virus, a varicella zoster virus, a roseolovirus, and a Kaposi's sarcoma-associated herpesvirus. In preferred embodiments, the papovavirus includes, but is not limited to, human papilloma virus and a human polyoma virus. In preferred embodiments, the poxvirus includes, but is not limited to, a variola virus, a vaccinia virus, a cowpox virus, a monkeypox virus, a smallpox virus, a pseudocowpox virus, a papular stomatitis virus, a tanapox virus, a yaba monkey tumor virus, and a molluscum contagiosum virus. In preferred embodiments, the arenavirus includes, but is not limited to lymphocytic choriomeningitis virus, a lassa virus, a machupo virus, and a junin virus. In preferred embodiments, the bunyavirus includes, but is not limited to, a hanta virus, a nairovirus, an orthobunyavirus, and a phlebovirus. In preferred embodiments, the calcivirus includes, but is not limited to, a vesivirus, a norovirus, such as the Norwalk virus and a sapovirus. In preferred embodiments, the coronavirus includes, but is not limited to, a human coronavirus (etiologic agent of sever acute respiratory syndrome (SARS)). In preferred embodiments, the filovirus includes, but is not limited to, an Ebola virus and a Marburg virus. In preferred embodiments, the flavivirus includes, but is not limited to, a yellow fever virus, a West Nile virus, a dengue fever virus, a hepatitis C virus, a tick borne encephalitis virus, a Japanese encephalitis virus, a Murray Valley encephalitis virus, a St. Louis encephalitis virus, a Russian spring-summer encephalitis virus, a Omsk hemorrhagic fever virus, a bovine viral diarrhea virus, a Kyasanus Forest disease virus, and a Powassan encephalitis virus. In preferred embodiments, the orthomyxovirus includes, but is not limited to, influenza virus type A, influenza virus type B, and influenza virus type C. In preferred embodiments, the paramyxovirus includes, but is not limited to, a parainfluenza virus, a rubula virus (mumps), a morbillivirus (measles), a pneumovirus, such as a human respiratory syncytial virus, and a subacute sclerosing panencephalitis virus. In preferred embodiments, the picornavirus includes, but is not limited to, a poliovirus, a rhinovirus, a coxsackievirus A, a coxsackievirus B, a hepatitis A virus, an echovirus, and an eneterovirus. In preferred embodiments, the reovirus includes, but is not limited to, a Colorado tick fever virus and a rotavirus. In preferred embodiments, the retrovirus includes, but is not limited to, a lentivirus, such as a human immunodeficiency virus, and a human T-lymphotrophic virus (HTLV). In preferred embodiments, the rhabdovirus includes, but is not limited to, a lyssavirus, such as the rabies virus, the vesicular stomatitis virus and the infectious hematopoietic necrosis virus. In preferred embodiments, the togavirus includes, but is not limited to, an alphavirus, such as a Ross river virus, an O'nyong'nyong virus, a Sindbis virus, a Venezuelan equine encephalitis virus, an Eastern equine encephalitis virus, and a Western equine encephalitis virus, and a rubella virus.

According to particular embodiments, the protein of a pathogenic organism is a protein from a drug-resistant pathogen, strain or variant. Drug resistance is known to arise in several pathogens, particularly in viruses and micro-organisms.

A particular example of drug resistance is antibiotic resistance, thus according to further particular embodiments, the protein of a microbial organism is a protein from an antibiotic-resistant organism, strain or variant, or also occurring in an antibiotic-resistant organism. See also the Examples section. Since the present molecules have a completely different mechanism of action than known antibiotics (i.e., they aggregate a target protein of the microbial organism), it is envisaged that antibiotic-resistant organisms are not resistant to these molecules. Moreover, as most hydrophobic regions of proteins are in conserved parts of the protein, it is envisaged that antibiotic resistance (or antimicrobial resistance) will develop slower towards these molecules than towards conventional antibiotics. Indeed, according to particular embodiments, the methods comprise prolonged administration of the molecules without a significant increase in MIC values. Thus, methods for administration are provided wherein the molecules as described herein are administered at least once a day for at least ten days, or at least once a day for fourteen days. Particularly, this treatment regimen is not accompanied by the development of antibiotic resistance. Thus, these embodiments foresee that the MIC values over this range in time do not increase more than fourfold, or do not double. Most particularly, it is envisaged that, during prolonged administration, the MIC value of the interferor molecule for the specific microbial organism remains below the clinical breakpoint of the interferor molecule for the specific microbial organism.

According to specific embodiments, the molecules can be used to kill the pathogenic (e.g. microbial) organism, or the methods result in killing of the pathogenic (e.g. microbial) organism. According to yet further specific embodiments, the molecules succeed in quickly killing the pathogenic (microbial) organism, particularly within an hour or less, or within 30 minutes or less. A fast bactericidal effect (or a fast killing effect on microorganisms) not only yields better clinical results even in monotherapy (Finch et al., Antimicrob Agents Chemother; 46(6):1746-54, 2002), but may also shorten the duration of antimicrobial therapy and length of hospital stay, as well as contribute to the decreased development of resistance. This was for instance shown for the fast-killing fluoroquinolone antibiotics (Albertson et al., Int J Clin Pract.;64(3):378-88, 2010).

According to alternative embodiments, the methods using the molecules inhibit growth and/or reproduction of the pathogenic (e.g. microbial or viral) organism without killing it.

According to particular embodiments, the protein of the microbial organism is an essential protein, i.e. the organism cannot survive if it is depleted of the protein. According to other (non-exclusive) particular embodiments, the protein of the microbial organism is involved in biofilm formation. Biofilm formation is a well-characterized phenomenon, and multiple proteins involved in biofilm formation have been identified and/or characterized, as the skilled person will be aware of. These proteins may differ among microbial species (or may be homologous, but have a different name). Examples of such proteins include, but are not limited to, Hwp1, the Als family of proteins (particularly in C. albicans), proteins encoded by the EPA gene family, the AWP1-4 gene family and the PWP gene (particularly in C. glabrata), In *Yersinia* proteins encoded by the hmsHFRS, gmhA, yrbH, waaAE-coaD, hmsT, hmsP, speA, speC, nghA , rcsA, rcsC , rcsDB, phoPQ genes (see table 1 of Zhou and Yang, Protein Cell 2011), in *Staphylococcus* proteins encoded by icaADBC, icaR, sar, agr, rbf, sigma(B) genes.

According to further embodiments, methods are provided to treat or prevent biofilm formation, wherein the protein of the microbial organism is involved in biofilm formation. According to yet further embodiments, the biofilm formation is on an object, particularly an implantable device, such as a catheter or stent.

Thus, the specification describes methods to prevent, inhibit or reverse microbial biofilm growth on a surface, comprising contacting the surface with a molecule of the structure (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; wherein at least one Yᵢ is a stretch present in a protein of a microbial organism; and
- each Zᵢ is a linker and Zₙ is independently selected from a linker or nothing.

Most particularly, the protein of the microbial organism is a protein involved in biofilm formation. The surface on which biofilm formation is prevented, inhibited or reversed can be a biotic or abiotic surface. Particularly, the surface can be on or within the subject's body, either from the body itself, or from an exogenous object within the body, such as from an implanted device.

Although it is primarily envisaged to prevent or treat microbial infections in subjects, it should be noted that the molecules might be used as an antimicrobial agent to prevent or remedy the presence of (pathogenic) microorganisms on surfaces or objects outside of the subject's body as well. This application is mainly intended for coating of high-value or sterile material, e.g. materials used for implantable devices. Accordingly, methods are provided to prevent, inhibit or reverse microbial biofilm growth on a surface of an implantable device, such as a catheter or stent.

Consequently, the coated devices are also envisaged within the scope of the invention. Coating of the devices can be done directly by applying the molecules to the device. Alternatively, they can be coated on the device using (cross-)linkers. The devices can be fully coated (e.g. by submersion of the device in a solution of the molecules), or only parts of the device can be coated. It is particularly envisaged that the devices are coated with molecules against a protein present in a microbial organism, particularly a protein involved in biofilm formation. However, it is also envisaged that the devices are coated with molecules directed against other proteins (or with molecules that are bispecific and target a protein of a microbial organism and another protein through different Yᵢ moieties). For instance, implantation of devices often may cause localized adverse immune reactions. These can be countered by coating at least part of the device with molecules targeted against proteins involved in these immune reactions (or with e.g. bispecific molecules against the two different proteins). As another example, to counter co-infection of (particular biofilm-forming) fungi and bacteria, at least part of the device may be coated with molecules targeted against fungal proteins and part coated with molecules targeted against bacterial proteins (or with molecules that are (at least bi)specific to a fungal protein and a bacterial protein).

Thus, the specification describes devices (particular implantable devices) that are at least partly coated with molecules of the structure (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present; and
- each Zᵢ is a linker and Zₙ is independently selected from a linker or nothing.

Most particularly, at least one Yᵢ is a stretch naturally occurring in a protein of a microbial organism, specifically a protein involved in biofilm formation.

### Screening methods

According to a further aspect, methods to screen for new compounds in cell lines (including, but not limited to, human, mammalian, insect and plant cell lines), pathogens or microbial organisms are provided. These methods allow rapid identification of compounds which have effect on growth, reproduction or survival of the cell line or organism under study, or of compounds which inhibit protein function and/or aggregate proteins in said cell line or protein, even without prior knowledge of the target. As downregulation by aggregation is sequence-specific however, the sequence of the working compounds will allow rapid identification of the target. Thus, not only can new compounds be obtained using these screening methods, they also allow identification of new drug targets. For this reason, it may also be particularly interesting to use cell lines that model disease (such as e.g. cancer cell lines, or even cells directly isolated from a tumor).

The specification describes screening methods comprising the steps of:
a) identifying in at least one protein at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P, most particularly selected from R, K and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in the at least one protein identified in step a); and
   - each Zᵢ is a linker and Zₙ is independently selected from a linker or nothing;
c) adding the at least one molecule made in step b) to the protein of step a); and
d) assessing the function and/or aggregation of the protein.

Particularly, the screening methods will be performed in cellular systems, or directly on pathogens. The specification describes such methods involving the steps of:
a) identifying in at least one protein of the cell line, pathogen or microbial organism at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P, most particularly selected from R, K and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in the at least one protein of the cell line, pathogen or microbial organism identified in step a); and
   - each Zᵢ is a linker and Zₙ is independently selected from a linker or nothing;
c) adding the at least one molecule made in step b) to the cell line, pathogen, or microbial organism in the genome of which the protein of step a) is encoded; and
d) assessing survival and/or growth or reproduction of the cell line, pathogen or microbial organism.

As mentioned above, if desired, the methods may further comprise a step of correlating the Yi sequence of a molecule (synthesized in step b) that affects survival, growth and/or reproduction of the cell line, pathogen or microbial organism with a protein identified in step a) to identify the protein that is targeted. However, this correlation step is not required - for antipathogenic compounds, it may be more important that they succeed in inhibiting survival, reproduction or growth of the pathogen than to know the exact protein that is targeted.

As inhibiting survival, growth or reproduction of the targeted organism is primarily envisaged for pathogenic organisms (e.g. microbial or viral organisms), these methods are particularly suited to screen for new antipathogenic (e.g. antimicrobial or antiviral) compounds.

Thus, according to specific embodiments, methods to screen for new antipathogenic compounds are provided. The specification describes such methods comprising the steps of:
a) identifying in at least one protein of a pathogen at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P, most particularly selected from R, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pathogenic organism identified in step a); and
   - each Zᵢ is a linker and Zₙ is independently selected from a linker or nothing;
c) adding the at least one molecule made in step b) to the pathogenic organism in the genome of which the protein of step a) is encoded; and
d) assessing survival and/or growth or reproduction of the pathogenic organism.

According to specific embodiments, methods to screen for new antimicrobial compounds are provided. The specification describes such methods comprising the steps of:
a) identifying in at least one microbial protein at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P, most particularly selected from R, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a microbial organism identified in step a); and
   - each Zᵢ is a linker and Zₙ is independently selected from a linker or nothing;
c) adding the at least one molecule made in step b) to the microbial organism in the genome of which the protein of step a) is encoded; and
d) assessing survival and/or growth or reproduction of the microbial organism.

In assessing the survival, the MIC values can be determined. In order to identify a compound with antimicrobial activity, i.e. to classify a molecule as a hit, the MIC should particularly be lower than 100 µg/ml, more particularly lower than 50µg/ml, 20µg/ml or most particularly lower than 10 µg/ml (such as e.g. 5 µg/ml, 2 µg/ml or 1 µg/ml.

Although MIC values are typically expressed in µg/ml, it should be noted that the molecular weight of the present compounds is on average significantly higher than those of classic antibiotic compounds. Indeed, whereas an antibiotic typically has a molecular mass in the range of 200-700 Dalton, due to the presence of amino acids in at least the X and Y moieties, and due to the fact that more than one 'unit' may be present in the molecules (i.e., n may be higher than one), the molecular weight will typically be (much) higher than 1000 Dalton. As a result, a similar molar concentration will give much higher molecular masses, and thus much higher MIC values. In other words, to determine effective antimicrobial effect, it may be interesting to calculate the molar equivalent of the µg/ml values (and e.g. compare these with molar values for other antibiotics).

According to further specific embodiments, methods to screen for new antiviral compounds are provided. The specification describes such methods comprising the steps of:
a) identifying in at least one viral protein at least one region of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing at least one molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P, most particularly selected from R, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a viral organism identified in step a); and
   - each Zᵢ is a linker and Zₙ is independently selected from a linker or nothing;
c) adding the at least one molecule made in step b) to the viral organism in the genome of which the protein of step a) is encoded; and
d) assessing survival and/or growth or reproduction of the viral organism.

Typically, for viruses, survival may be measured indirectly by assessing effect (e.g. survival) on infected cells. This indirect method can also be applied for other pathogenic organisms. However, direct methods are usually preferred, as they are more quantitative. For viruses, viral titer can be determined. Alternatively, a minireplicon system can be set up in which only particular viral proteins are present, and where a reporter protein is an indication of the effect on virus reproduction or 'survival'.

Of note, although the screening methods are described herein as methods to identify new antipathogenic compounds, the skilled person will readily appreciate that these methods can also be used to identify new compounds directed against non-pathogenic proteins to select the most effective compound. For instance, molecules envisaged to treat a disease by downregulating a protein can first be screened for effectiveness in a cell or animal model with a suitable readout (provided that the sequence conservation of the region corresponding to the at least one Yᵢ stretch is sufficient to use the same compound in the cell or animal model and the organism to which the compound should be administered). This may be done e.g. for proteins that contain different beta-aggregating regions, or to identify the best combination of beta-aggregating regions.

Indeed, similar to screening methods for molecules that affect growth, reproduction and/or survival of pathogenic organisms (and that as a consequence can be used in treatment of infectious disease), the methods can be used to screen for molecules that can be used in non-infectious disease. The methods can be used to identify the most effective compound against a known target (e.g. by screening in a cell line that has a reporter linked to the target activity, or that depends on the presence of the target for its survival), or to identify new targets. The latter is particularly true for applications where cell death can be a read-out, as they don't depend on a reporter assay. However, the methods can be used to identify new targets in a pathway for which a reporter assay is available as well.

The screening methods presented herein provide considerable advantages over existing screening methods. On the one hand, they are not random screens, and thus are more likely to yield successful compounds. On the other hand, they do not require 3D structures of target proteins to be available for efficient screening, as the targeting is entirely sequence-based. Identifying new targets is known to be a particular problem for antimicrobials or antibiotics.

In order to make sure that the identified compound is effective and without side effects, toxicity of the compound should be tested, particularly on vertebrate, most particularly in mammalian systems. Also, to avoid targeting of non-relevant proteins (e.g. non-microbial proteins in case a new antibiotic molecule is identified), cross-reactivity should be tested. This can in first instance easily be done by comparing the sequence identified in step a) to sequences of organisms/species to which the compound (e.g. antimicrobial or antiviral compound) is to be administered, e.g. by sequence alignment programs such as BLAST. For molecules directed against pathogens (to be used in anti-infectious applications), it is particularly envisaged that the one or more Yi moieties that are identical to stretches in pathogenic (e.g. microbial) proteins present in the molecules are not encoded in the genome of the (typically mammalian, particularly human) organism/subject to which the molecules are administered.

In embodiments covering methods to identify new targets for antipathogenic (e.g. antimicrobial) compounds, it is particularly envisaged that the pathogenic (e.g. microbial) protein in step a) is not a known target for drugs (e.g. antibiotics). This would make the identified compounds particularly useful in combination therapy, as different targets are tackled. Moreover, if the target is not a known target for drugs (such as antibiotics), no drug (antibiotic) resistance has been developed yet.

Another particular advantage is that the screening methods can be performed without selection bias for a particular protein as an interesting target. Indeed, the entire proteome of the organism (or cell line) can be analyzed, and the suitable sequences can be used into molecules described herein, and tested for effectiveness. This has a high chance of yielding new targets. Moreover, when such analysis is done, particularly for antipathogenic applications it is envisaged that the regions identified in step a) are regions that occur more than once in the proteome. More particularly, the at least one region of 4 to 16 contiguous amino acids identified in step a) occurs in more than one protein of the pathogenic (e.g. microbial or viral) organism.

Indeed, when confronted with infection, in many instances, it is desirable to target more than one pathogenic organism. This is e.g. evident in infections with microbial organisms, where often broad spectrum antimicrobials are used. This allows fighting infection even without knowing the precise identity of the infectious organism. In the present methods, it can also be ensured that the at least one region of 4 to 16 contiguous amino acids identified in step a) occurs in a protein, or at least one protein, of more than one microbial organism.

Most particularly, the microbial organisms targeted herein are pathogenic microbial organisms. In order to make sure that no beneficial microbial organisms are killed (e.g. beneficial microorganisms in the gut flora of a subject), it can be checked whether the stretch identified in the microbial organism is specific to pathogenic organism(s) and does not occur in beneficial microorganisms.

### Isolation, depletion, detection and diagnosis

The specification describes a method to isolate a specific protein from a sample comprising contacting said sample with at least one interferor molecule and isolating the resulting co-aggregated interferor-protein complex from said sample. That is to say, the interferor molecule acts as a binding agent that catches away the target protein. This can be used in all fields where detection is necessary (e.g. in white biotech to measure levels of pollutants, in red or green biotech to measure e.g. levels of biomarkers or metabolites, etc.).

The method for the isolation of a specific protein from a sample may further comprise the separation of said at least one protein from the sample. One application of the separation of at least one protein from a sample is the removal (or depletion) of highly abundant proteins from a sample. Indeed, a major challenge in protein target discovery and validation is how to specifically dissect complex protein samples (e.g. plasma, urine, cerebrospinal fluid) and to measure trace targets (i.e. very low abundant targets). Typically, abundant proteins are often 6-10 orders of magnitude more concentrated than low abundant proteins. Thus in certain occasions highly abundant proteins must be removed to detect and measure trace proteins of medical importance. Since albumin, IgG, antitrypsin, IgA, transferrin and haptoglobin make up approximately 90% of the total protein content in human serum, there is a critical need for diagnostic tools to rapidly deplete these unwanted abundant proteins and unmask the less abundant, low molecular weight protein biomarkers. Several methods are already used in the art: 1) immunoglobulin G (IgG) as affinity reagents to capture and separate abundant protein targets, 2) immunoglobulin yolk (IgY) are IgG-like antibodies isolated from egg yolks of immunized birds, 3) pre-fractionation is used to separate a mixture of proteins into different fractions to remove certain proteins in the original mixture, and 4) protein A and protein G are bacterial cell wall proteins with a specificity to IgG antibodies, hence protein A and G affinity resins provide a removal of IgG and 5) IgG- and IgY-microbeads are used for protein detection.

The method for the isolation of at least one protein may further comprise the detection of at least one protein in said molecule-protein complex. Detection can be carried out by separating the interferor molecule-target protein complex(es) by for example electrophoresis, column chromatography, filtration, electrostatic attraction, magnetic or paramagnetic attraction, mass spectrometry and the like.

According to a further aspect, the interferor molecules, as further defined in the claims, are used in detection or as a diagnostic. Thus the interferor molecules, as further defined in the claims, can be used in a diagnostic method. Accordingly, methods for detection and diagnosis are provided.

The specification describes methods to detect a protein in a sample, comprising the steps of:
a) contacting a sample suspected of containing the protein with a molecule of the following structure: (X₂ᵢ₋₁-Yᵢ-X₂ᵢ-Zᵢ)ₙ, wherein:
   - n is 1 to 5 and i runs from 1 to n;
   - each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
   - each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in the protein to be detected; and
   - each Zᵢ is a linker and Zₙ is independently selected from a linker or nothing;
b) detecting the presence of molecules reacted with the protein.

The sample can be provided as such or can be pre-processed. The 'molecules reacted with the protein' in step b) envisages both molecules reacting with the protein (i.e., real-time detection) as molecules that have reacted with the protein. These molecules may still be in contact with the protein or may not be in contact anymore. This detection can also be done through the protein, i.e. by detecting the presence of protein reacted with the molecules. Detection can in both instances be direct (by measuring the molecules or proteins that have reacted) or indirect (by measuring the fraction that has not reacted).
According to particular embodiments, the at least one Yᵢ naturally occurring in the protein to be detected is unique to said protein in said sample. Thus, for instance, in a sample of human origin, the sequence also occurring in the protein to be detected is encoded only once in the human genome (or occurs only once in the human proteome), namely in the (sequence encoding the) protein to be detected. Although this uniqueness is not always necessary - e.g. when different proteins have the same sequence and are detected together, they can still be further discriminated, e.g. based on size - it is particularly envisaged to facilitate detection. The 'in said sample' part is important in determining the uniqueness of the protein stretch: e.g. if the sample is pre-processed, it is likely to contain less different proteins than detection in complex mixtures or in samples from different (micro-)organisms.
It should be noted that the present detection methods are highly similar to established methods, typically antibody-based detection methods. The significant difference is the use of the particular molecules. In fact, it should be noted that in the detection methods described herein, the molecules as defined herein fulfill a similar role as antibodies. Thus, in detection methods which are normally antibody-based, the molecules described herein can replace at least one antibody. For instance, molecules where at least one Yᵢ is a stretch naturally occurring in the protein to be detected can replace a primary antibody in detection assays (and can be labeled 'primary interferors'), molecules where Yi is a stretch naturally occurring in a primary antibody used for detection can replace a secondary antibody. Alternatively, the secondary interferor molecule can be directed to a tag or label fused to a primary antibody, or can be directed to a 'primary interferor' or moiety fused thereto.

According to specific embodiments, the molecule used for detection comprises a detectable label. According to further specific embodiments, the detecting in step b) is through detection of the detectable label.

According to particular embodiments, the methods additionally comprise a separation of molecules reacted with the protein and molecules not reacted with the protein prior to detection in step b). The opposite can also be done: separating the proteins reacted with the molecules from the proteins not reacted with the molecules prior to detection. Which of the two is separated typically depends on the set-up of the experiment, and e.g. if proteins or molecules are immobilized.

The detection in step b) can be direct or indirect, e.g. through detection of the non-reacted fraction of molecules. The detection can be qualitative (e.g. is the protein present or not), semi-quantitative (e.g. is there more or less of the protein present) or quantitative (e.g. how much of the protein is present).

According to particular embodiments, the sample is from a subject, particularly from a mammalian subject, most particularly from a human subject. However, in principle, any protein containing an aggregation nucleating region can be detected, thus the source of the sample can be from any organism (e.g. plants, insects, pathogens, mammals). The sample doesn't even need to be from an organism, as long as it contains proteins. For instance, it can be a fluid (e.g. water, beer) sample where the presence of proteins (e.g. hormones, estrogens) is measured, or it can be a food or feed sample. It is important to remark that, for samples coming from an organism, the sample and the protein to be detected need not to be from the same species. E.g., in case of detecting the presence of a pathogen, it is envisaged that a sample will be taken from a subject or plant, while the protein to be detected is from a pathogen (e.g. virus or microorganism).

Generally spoken a diagnostic method includes the following steps: i) an examination phase (the collection of data, i.e. the qualitative or quantitative detection of a protein biomarker in a sample), ii) the comparison of the obtained data with standard values (e.g. from samples from non-diseased subjects), iii) the finding of any significant deviation between the obtained data and the reference data (i.e. by comparing the data) and iv) attributing the deviation to a particular clinical picture (animal subject) or status (plant subject). Note that these steps can also be taken for regular detection methods. In this case, the status obtained in step iv) is not of a disease status, but e.g. the presence of pollutants.

Accordingly, in specific embodiments, the presence, absence or amount of protein detected in the sample is indicative of a disease status (or of health). Such a disease status can e.g. be presence of disease, absence of disease (i.e. the finding of health), progression of disease (e.g. malignancy, metastasis, response to therapy). Examples of proteins associated with disease status, e.g. biomarkers, are well documented in the art. Thus, methods are provided for the detection of a protein biomarker (i.e., a protein indicative of disease status) in a sample comprising contacting said sample with at least one interferor, with a specificity for said protein biomarker, optionally isolating the resulting co-aggregated interferor-protein biomarker complex from said sample and detecting said interferor-protein biomarker. Protein biomarkers are increasingly used in the clinic to predict the onset of disease, diagnose it, monitor its progression, and provide prognosis as to its responsiveness to therapeutics (i.e. in predicting of response to therapeutics, adverse events and drug interactions and in establishing baseline risk). Well known examples of biomarkers for human subjects include prostate specific antigen (PSA) for (early detection of) prostate cancer, carcinoembryonic antigen for gastrointestinal cancer, C-reactive protein (CRP) for systemic inflammation, rheumatoid factor, anti-cyclic citrullinated peptide antody (anti-CCP) for rheumatoid arthritis, MMP-3 for joint damage and amyloid beta antibodies for Alzheimer's disease. Protein biomarkers are theoretically better than mRNA markers because of their increased stability and the broader range of technologies to study them. The explosion of interest in biomarker research is driving the development of new predictive, diagnostic and prognostic products in modern medical practice, and biomarkers are also playing an increasingly important role in the discovery and development of new drugs. Protein biomarkers can be identified in body fluids (e.g. serum, urine, blood, saliva, tears, CSF, synovial fluid, blood, nipple aspiration fluid, ascites, sperm, sweat, a tumour biopt). Biomarkers can be divided into the categories of predictive or prognostic. A prognostic biomarker is associated with the likelihood of an outcome such as survival, response (e.g. to a particular therapy) and recurrence. A predictive biomarker is a biomarker that is present prior to an event occurring and which predicts that outcome. A predictive biomarker can be either positive or negative. Biomarkers may not only be used to diagnose a disease but also for patient selection or follow-up. As research continues, our understanding of the role biomarkers can play in the management of disease areas such as cancer, cardiology, neurology, metabolic, autoimmune and inflammatory diseases has evolved.

A non-limiting list of protein biomarkers which can be detected with the interferor molecules and methods of the present invention comprises the diagnosis of lung cancer biomarkers (as described in WO2005098445), the diagnosis of head and neck squamous cell carcinoma (as WO2005034727), the diagnosis of abdominal aortic aneurysm (as an WO2009046267), the diagnosis of HTLV-mediated diseases (as in WO2004029575), the diagnosis of ductal carcinoma of the breast (as in WO2009039023), the diagnosis of prostate cancer (as in WO2004030511), the diagnosis of obstructive sleep apnea (as in WO2006020567), the prognosis of Gefitinib-treated GBM patients (as in WO2010033993), the diagnosis of the onset and/or progression of amyotrophic lateral sclerosis (as in WO2006060799), the diagnosis of mild cognitive impairment and Alzheimer's disease (as in WO2008014314), the diagnosis of liver fibrosis (as in US20100323374), the diagnosis of nervous system injury (as in EP2207033), the detection of skeletal muscle damage (as in WO2007066129), the diagnosis of obstructive sleep apnea (as in WO2006020567), the diagnosis of malignant breast carcinomas (as in WO20052463), the diagnosis of urological disorders (as in WO2010078403), the *in vitro* testing of developmental toxicity and embryotoxicity (as in WO2009146915), the prognosis and treatment outcome of glioblastoma (as in WO2009102729), the assessment of radiation injury and exposure (as in WO2008140463), the detection of liver cancer (as in US20050202485), the detection of nasopharyngeal carcinoma (as in US20050158745), the detection of ovarium cancer (as in WO2003057014), the prediction of the clinical response to anti-TNF-alpha antibodies in patients with psoriatic arthritis (as in WO2011014349), the detection of salivary biomarkers for the identification of oral cancer and periodontal disease (as in US20110021370), the diagnosis of Barrett's esophagus and esophageal adenocarcinoma (as in WO2010115077), the diagnosis of atrial fibrillation and stroke (as in WO2010113185), the prediction of allograft rejection (as in WO2010093869), the prediction of the clinical response to anti-TNF antibodies in patients with ankylosing spondylitis (as in WO2010077722), the diagnosis of interstitial cystitis (as in WO2010068747), the diagnosis and prognosis of sepsis (as in US20100292131), the prognosis for metachronous colorectal cancer (as in US20090155842), the monitoring of the treatment of ALS with nimesulide (as in WO2004043444).

According to further specific embodiments where a biomarker protein is detected (i.e. at least one Yᵢ in the molecules is a stretch naturally occurring in a protein which is indicative of a disease status), these methods comprise in addition to steps a) and b) outlined above a step c) correlating the presence, absence or amount of protein detected in the sample with a disease status in the subject.

The specification describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein which is indicative of a disease status; and
- each Zᵢ is a linker and Zₙ is independently selected from a linker or nothing;
for use in diagnosis of said disease status.

Note that the same methods can be used in plants, to determine the disease status of the plant.

A specific embodiment of the above methods is diagnosing the presence of infection. According to this embodiment, the protein indicative of disease status (i.e. infection) is a protein of a pathogen (cause of the infection, and thus indicative of infection). Accordingly, the specification describes molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a pathogen; and
- each Zᵢ is a linker and Zₙ is independently selected from a linker or nothing;
for use in diagnosis of infection with said pathogen.

Again, these diagnostic methods can be applied to subjects as well as plants (or other organisms in which an infection can be present).

Diagnosis does not always need to be a disease status however. As mentioned, the detection methods can be used to determine protein levels in samples not derived from a subject (as typically encountered in industrial biotechnology), or it can be used to determine protein (e.g. metabolite) levels in samples of organisms without this being linked to disease. This is particularly the case in plant biotechnology, most particularly for transgenic plants. Indeed, it can be useful to measure increased or decreased protein levels, e.g. to evaluate which plants have the highest expression levels of a useful protein, or which plants have less expression of harmful proteins or proteins which make the plant unpalatable, or which plants have a better response to stress conditions.

As in vitro diagnostics are envisaged herein, also provided are kits comprising at least one molecule as described herein and at least a suitable buffer. Such kit will ideally be adapted for the specific detection protocol used. The kits may contain any of the pharmaceutical compositions or agrochemical compositions described herein. Also, the kit may contain one or more devices, typically devices used for detection of the protein, such as solid supports (e.g. plates, micro-arrays, nanoparticles, ...), optionally precoated with the molecules described herein. The kit may additionally contain instructions for use. Obviously, kits need not to be limited to diagnostic or detection applications. However, in case of kits for provided for treatment of plants or animal subjects, the kits will optionally contain devices for administration purposes (e.g. a syringe, a container with spraying nozzle) rather than lab devices.

In the prior art a variety of different manners of assaying for protein levels (or protein biomarkers) are known, one representative and convenient type of protocol for assaying protein levels is ELISA. In ELISA and ELISA-based assays, one or more antibodies specific for the proteins of interest (or protein biomarkers) may be immobilized onto a selected solid surface, preferably a surface exhibiting a protein affinity such as the wells of a polystyrene microtiter plate. After washing to remove incompletely adsorbed material, the assay plate wells are coated with a non-specific "blocking" protein that is known to be antigenically neutral with regard to the test sample such as bovine serum albumin (BSA), casein or solutions of powdered milk. This allows for blocking of non-specific adsorption sites on the immobilizing surface, thereby reducing the background caused by non-specific binding of antigen onto the surface. After washing to remove unbound blocking protein, the immobilizing surface is contacted with the sample to be tested under conditions that are conducive to immune complex (antigen/antibody) formation. Such conditions include diluting the sample with diluents such as BSA or bovine gamma globulin (BGG) in phosphate buffered saline (PBS)/Tween or PBS/Triton-X 100, which also tend to assist in the reduction of nonspecific background, and allowing the sample to incubate for about 2-4 hrs at temperatures on the order of about 25°-27°C (although other temperatures may be used). Following incubation, the antisera-contacted surface is washed so as to remove non-immunocomplexed material. An exemplary washing procedure includes washing with a solution such as PBS/Tween, PBS/Triton-X 100, or borate buffer. The occurrence and amount of immunocomplex formation may then be determined by subjecting the bound immunocomplexes to a second antibody having specificity for the target that differs from the first antibody and detecting binding of the second antibody. In certain embodiments, the second antibody will have an associated enzyme, e.g. urease, peroxidase, or alkaline phosphatase, which will generate a color precipitate upon incubating with an appropriate chromogenic substrate. For example, a urease or peroxidase-conjugated anti-human IgG may be employed, for a period of time and under conditions which favor the development of immunocomplex formation (e.g., incubation for 2 hr at room temperature in a PBS-containing solution such as PBS/Tween). After such incubation with the second antibody and washing to remove unbound material, the amount of label is quantified, for example by incubation with a chromogenic substrate such as urea and bromocresol purple in the case of a urease label or 2,2'-azino-di-(3-ethyl-benzthiazoline)-6-sulfonic acid (ABTS) and H₂O₂, in the case of a peroxidase label. Quantitation is then achieved by measuring the degree of color generation, e.g., using a visible spectrum spectrophotometer. Alternatively, the preceding format may be altered by first binding the sample to the assay plate. Then, primary antibody is incubated with the assay plate, followed by detecting of bound primary antibody using a labeled second antibody with specificity for the primary antibody. Alternatively, non-ELISA based-methods for measuring the levels of one or more protein biomarkers in a sample may be employed. Representative examples include but are not limited to mass spectrometry, proteomic arrays, xMAP™ microsphere technology, flow cytometry, western blotting, and immunohistochemistry. According to the methods of the invention, and further exemplified in the examples section, the primary antibody is replaced by a specific interferor which binds to the protein biomarker. In a specific embodiment such interferor is labeled with a detection molecule.

In a specific embodiment the solid substrate upon which the specific interferor or interferors are immobilized can be made of a wide variety of materials and in a wide variety of shapes, e.g., microtiter plate, microbead, dipstick, resin particle, etc. The substrate may be chosen to maximize signal to noise ratios, to minimize background binding, as well as for ease of separation and cost. Washes may be effected in a manner most appropriate for the substrate being used, for example, by removing a bead or dipstick from a reservoir, emptying or diluting a reservoir such as a microtiter plate well, or rinsing a bead, particle, chromatograpic column or filter with a wash solution or solvent.

In a particular embodiment the detection of a specific protein biomarker is quantitative. In another particular embodiment the detection of a specific protein biomarker is qualitative. As such, where detection is qualitative, the methods provide a reading or evaluation, e.g. assessment, of whether or not the protein biomarker is present in the sample being assayed. In yet other embodiments, the methods provide a quantitative detection of whether the protein biomarker is present in the sample being assayed, i.e., an evaluation or assessment of the actual amount or relative abundance of the target analyte. In such embodiments, the quantitative detection may be absolute or, if the method is a method of detecting two or more different protein biomarkers in a sample, relative. As such, the term "quantifying" when used in the context of quantifying a protein biomarker in a sample can refer to absolute or to relative quantification. Absolute quantification may be accomplished by inclusion of known concentration(s) of one or more control protein biomarkers and referencing the detected level of the protein biomarker with the known control protein biomarkers (e.g. through generation of a standard curve). Alternatively, relative quantification can be accomplished by comparison of detected levels or amounts between two or more different protein biomarkers to provide a relative quantification of each of the two or more different protein biomarkers, e.g. relative to each other.

In certain embodiments the detection of only one protein biomarker is evaluated. In yet other embodiments, the expression of two or more, e.g. about 3 or more, about 10 or more, about 15 or more protein biomarkers is evaluated. A typical way in which this can be done is using a protein binding microarray (see below).

In such embodiments, the prediction, diagnosis, or characterization may be provided by providing, i.e. generating, a written report that includes the artisan's monitoring assessment, i.e. the artisan's prediction of the onset of a particular disease, the artisan's diagnosis of the subject's disease, or the artisan's characterization of the subject's prognosis of the disease. Thus, a subject method may further include a step of generating or outputting a report providing the results of a monitoring assessment, which report can be provided in the form of an electronic medium (e.g., an electronic display on a computer monitor), or in the form of a tangible medium (e.g., a report printed on paper or other tangible medium). A "report," as described herein, is an electronic or tangible document which includes report elements that provide information of interest relating to a subject monitoring assessment and its results. A subject report can be completely or partially electronically generated. A subject report can further include one or more of: 1) information regarding the testing facility; 2) service provider information; 3) patient data; 4) sample data; 5) an assessment report, which can include various information including: a) reference values employed, and b) test data, where test data can include, e.g., a protein level determination; 6) other features.

The report may include information about the testing facility, which information is relevant to the hospital, clinic, or laboratory in which sample gathering and/or data generation was conducted. Sample gathering can include obtaining a fluid sample, e.g. blood, saliva, urine etc. a tissue sample, e.g. a tissue biopsy, etc. from a subject. Data generation can include measuring the level of polypeptide biomarker concentration for one or more biomarkers that are differentially expressed or present at different levels in different subjects.

In many embodiments, the subjects are within the class of mammalian, including the orders carnivore (e.g., dogs and cats), rodentia (e.g., mice, guinea pigs, and rats), lagomorpha (e.g. rabbits) and primates (e.g., humans, chimpanzees, and monkeys). In certain embodiments, the animals or hosts, i.e., subjects (also referred to herein as patients) are humans.

As herein explained before, the most broadly used bio-detection technologies are based on the use of antibodies. Antibodies recognize and bind to other molecules based on their shape and physicochemical properties. Antibodies are highly suited for detecting small quantities of target proteins in the presence of complex mixtures of proteins. The present invention shows that the use of interferor molecules is an alternative for the use of antibodies (as the recognition element) for the specific capture of target proteins. Indeed, interferor molecules can be used in numerous applications in which antibodies typically are used. To name only a few, applications are envisaged in diagnosis, micro- analytics, forensics and in the specific detection of pathogens. For the detection and separation applications of the invention it can be convenient that the interferor molecule is bound to a carrier (or a support). A support can be a flat surface such as plastic or nitrocellulose or a chromatographic column but is preferably a bead such as microsphere beads. A general discussion on various types of beads and microspheres, which serve the purpose of binding interferor molecules, is described on pages 9 and 10 of US6682940 and is herein specifically incorporated by reference. In a particular embodiment the interferor molecule is bound to a carbohydrate type of carrier, e.g. cellulose or agarose. An interferor can be bound to said carbohydrate carrier with a cross-linking agent such as glutaraldehyde. In another particular embodiment the carrier can be cellulose, glass or a synthetic polymer. Covalent attachment between the interferor and the carrier can be carried out via amino acid residues of the interferor and an azide, carbodiimide, isocyanate or other chemical derivatives present on the interferor.

In yet another particular the carrier is a porous silanised glass micro bead. The interferor can be covalently bonded to the carrier via its peptide amine groups (by Schiff reaction followed by reduction with sodium borohydride) to aldehyde groups formed by periodate oxidation of glycidoxypropylsilane groups chemically linked to the silica atoms (this coupling is described in Sportsman and Wilson (1980) Anal. Chem. 52, 2013-2018).

In a specific embodiment the carrier is enveloped by a proteinaceous film to which the interferor is crosslinked (see claims 1-50 and examples relating to the carrier in US4478946).

In another specific embodiment the support is a fluorescent bead such as a fluorescent latex particle. The patent US4550017, and especially page 4 therein, describes fluorescent compounds which can be used for the manufacturing of fluorescent beads.

In another specific embodiment the beads vary in size and may also contain or be impregnated with fluorescent dyes. Because of varying sizes and dyes of the beads multiple proteins can be detected and quantitated in a single reaction. Procedures for the development of such beads are described in US6159748.

In yet another particular embodiment the coupling between the bead and the interferor is via a poly(threonine), a poly(serine), dextran or poly(ethylene glycol). Examples 6, 7, 8 and 9 of US6399317 illustrate how this coupling can be carried out.

In yet another particular embodiment the support is a magnetic bead. Magnetic beads, coupling between the magnetic beads and a protein agent and their uses are described on page 8 of application US6489092.

According to a further aspect, the support is a microarray. These are particularly envisaged for multiplexed detection of proteins. Accordingly, microarrays are provided comprising molecules described herein. The specification describes microarrays comprising at least two molecules of the following structure: (X₂ᵢ₋₁-Yᵢ-X₂ᵢ-Zᵢ)ₙ, wherein:
- n is 1 to 5 and i runs from 1 to n;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein; and
- each Zᵢ is a linker and Zₙ is independently selected from a linker or nothing.

According to particular embodiments, the molecules are linked to the microarray through a linker (e.g. a Z₀ moiety as described herein). The microarray may be an array, chip, bead, plate, blot, or the like.

According to other particular embodiments, the at least two molecules are at least two different molecules. Particularly, at least one Yi region of the molecules differs between the at least two molecules. More particularly, at least one Yi region of a first of the molecules is identical to a stretch in a protein that is a different protein than the stretch to which at least one other Yi region of a second of the molecules corresponds.

### Application in plants

The molecules described herein can also be used to downregulate the functions of proteins in plants, plant cells or plant seeds. Not that here also, this applies to non-infectious and infectious settings, i.e. targeting plant proteins or targeting pathogen proteins of pathogens in or on the plant. Accordingly, the specification describes methods for down-regulating the biological function of a protein in a plant or plant cell or plant seed, comprising contacting said protein with a molecule of the following structure: (X₂ᵢ₋₁-Yᵢ-X₂ᵢ₋Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein in or on said plant, plant cell or plant seed; and
- each Zᵢ is a linker and Zₙ is independently selected from a linker or nothing.
Further steps of the method may include intermolecular beta-aggregation occuring between the protein and the non-naturally occurring molecule, thereby downregulating the biological function of the protein. As plant subjects, contrary to animal subjects, are immobile, they are particularly vulnerable to infections with ectoparasites and -pathogens (i.e. pests). Indeed, many infectious species do not necessarily enter a plant to exert their pathogenic effect, or do not enter during initial stages of infection (e.g. also because plants don't have a digestive system and a cell wall, making it harder for pathogens to go inside a plant). Thus, a "protein on a plant, plant cell or plant seed" typically refers to a protein of another organism that is present on the plant, plant cell or plant seed. Examples include, but are not limited to, proteins of nematodes, aphids, mites, caterpillars, slugs, moulds, and the like. Note that these organisms can be present on any part of the plant (e.g. nematodes typically infect through plant roots, while aphids will generally be present on green parts of the plant, such as stalk and leaves).

Thus, the methods can be subdivided in methods to downregulate the biological function of a plant protein in a plant (e.g. to obtain a particular property such as increased yield or stress tolerance, or in non-infectious disease settings) and in methods to downregulate the biological function of a protein of a pest organism in or on a plant (as is the case in infectious disease).

The specification describes such former methods (provided for down-regulating the biological function of a plant protein in a plant or plant cell or plant seed), which include the step of contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of said plant, plant cell or plant seed; and
- each Zᵢ is a linker and Zₙ is independently selected from a linker or nothing.

The specification describes such latter methods (for down-regulating the biological function of a protein of a plant pest organism in a plant or plant cell or plant seed), which comprise contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein of a pest organism present in or on said plant, plant cell or plant seed; and
- each Zᵢ is a linker and Zₙ is independently selected from a linker or nothing.

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.
Plants that are particularly useful in the methods of the invention include in particular monocotyledonous and dicotyledonous plants including fodder or forage legumes, ornamental plants, food crops, trees or shrubs selected from the list comprising Acer spp., Actinidia spp., Abelmoschus spp., Agave sisalana, Agropyron spp., Agrostis stolonifera, Allium spp., Amaranthus spp., Ammophila arenaria, Ananas comosus, Annona spp., Apium graveolens, Arachis spp, Artocarpus spp., Asparagus officinalis, Avena spp. (e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica spp. (e.g. Brassica napus, Brassica rapa ssp. [canola, oilseed rape, turnip rape]), Cadaba farinosa, Camellia sinensis, Canna indica, Cannabis sativa, Capsicum spp., Carex elata, Carica papaya, Carissa macrocarpa, Carya spp., Carthamus tinctorius, Castanea spp., Ceiba pentandra, Cichorium endivia, Cinnamomum spp., Citrullus lanatus, Citrus spp., Cocos spp., Coffea spp., Colocasia esculenta, Cola spp., Corchorus sp., Coriandrum sativum, Corylus spp., Crataegus spp., Crocus sativus, Cucurbita spp., Cucumis spp., Cynara spp., Daucus carota, Desmodium spp., Dimocarpus longan, Dioscorea spp., Diospyros spp., Echinochloa spp., Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum spp., Fagus spp., Festuca arundinacea, Ficus carica, Fortunella spp., Fragaria spp., Ginkgo biloba, Glycine spp. (e.g. Glycine max, Soja hispida or Soja max), Gossypium hirsutum, Helianthus spp. (e.g. Helianthus annuus), Hemerocallis fulva, Hibiscus spp., Hordeum spp. (e.g. Hordeum vulgare), Ipomoea batatas, Juglans spp., Lactuca sativa, Lathyrus spp., Lens culinaris, Linum usitatissimum, Litchi chinensis, Lotus spp., Luffa acutangula, Lupinus spp., Luzula sylvatica, Lycopersicon spp. (e.g. Lycopersicon esculentum, Lycopersicon lycopersicum, Lycopersicon pyriforme), Macrotyloma spp., Malus spp., Malpighia emarginata, Mammea americana, Mangifera indica, Manihot spp., Manilkara zapota, Medicago sativa, Melilotus spp., Mentha spp., Miscanthus sinensis, Momordica spp., Morus nigra, Musa spp., Nicotiana spp., Olea spp., Opuntia spp., Ornithopus spp., Oryza spp. (e.g. Oryza sativa, Oryza latifolia), Panicum miliaceum, Panicum virgatum, Passiflora edulis, Pastinaca sativa, Pennisetum sp., Persea spp., Petroselinum crispum, Phalaris arundinacea, Phaseolus spp., Phleum pratense, Phoenix spp., Phragmites australis, Physalis spp., Pinus spp., Pistacia vera, Pisum spp., Poa spp., Populus spp., Prosopis spp., Prunus spp., Psidium spp., Punica granatum, Pyrus communis, Quercus spp., Raphanus sativus, Rheum rhabarbarum, Ribes spp., Ricinus communis, Rubus spp., Saccharum spp., Salix sp., Sambucus spp., Secale cereale, Sesamum spp., Sinapis sp., Solanum spp. (e.g. Solanum tuberosum, Solanum integrifolium or Solanum lycopersicum), Sorghum bicolor, Spinacia spp., Syzygium spp., Tagetes spp., Tamarindus indica, Theobroma cacao, Trifolium spp., Tripsacum dactyloides, Triticosecale rimpaui, Triticum spp. (e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium spp., Vicia spp., Vigna spp., Viola odorata, Vitis spp., Zea mays, Zizania palustris, Ziziphus spp., amongst others.

Plant pests as used herein are in particular insects, arachnids, helminths, fungi, viruses, bacteria, nematodes and molluscs encountered in agriculture, in horticulture, in forests, in gardens and in leisure facilities. The compositions according to the invention are active against normally sensitive and resistant species and against all or some stages of development. These plant pests include: pests from the phylum: Arthropoda, in particular from the class of the arachnids, for example Acarus spp., Aceria sheldoni, Aculops spp., Aculus spp., Amblyomma spp., Amphitetranychus viennensis, Argas spp., Boophilus spp., Brevipalpus spp., Bryobia praetiosa, Centruroides spp., Chorioptes spp., Dermanyssus gallinae, Dermatophagoides pteronyssius, Dermatophagoides farinae, Dermacentor spp., Eotetranychus spp., Epitrimerus pyri, Eutetranychus spp., Eriophyes spp., Halotydeus destructor, Hemitarsonemus spp., Hyalomma spp., Ixodes spp., Latrodectus spp., Loxosceles spp., Metatetranychus spp., Nuphersa spp., Oligonychus spp., Ornithodorus spp., Ornithonyssus spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vaejovis spp., Vasates lycopersici.
Other examples are from the order of the Anoplura (Phthiraptera), for example, Damalinia spp., Haematopinus spp., Linognathus spp., Pediculus spp., Ptirus pubis, Trichodectes spp.
Still other examples are from the order of the Chilopoda, for example, Geophilus spp., Scutigera spp. Still other examples are from the order of the Coleoptera, for example, Acalymma vittatum, Acanthoscelides obtectus, Adoretus spp., Agelastica alni, Agriotes spp., Alphitobius diaperinus, Amphimallon solstitialis, Anobium punctatum, Anoplophora spp., Anthonomus spp., Anthrenus spp., Apion spp., Apogonia spp., Atomaria spp., Attagenus spp., Bruchidius obtectus, Bruchus spp., Cassida spp., Cerotoma trifurcata, Ceutorrhynchus spp., Chaetocnema spp., Cleonus mendicus, Conoderus spp., Cosmopolites spp., Costelytra zealandica, Ctenicera spp., Curculio spp., Cryptorhynchus lapathi, Cylindrocopturus spp., Dermestes spp., Diabrotica spp., Dichocrocis spp., Diloboderus spp., Epilachna spp., Epitrix spp., Faustinus spp., Gibbium psylloides, Hellula undalis, Heteronychus arator, Heteronyx spp., Hylamorpha elegans, Hylotrupes bajulus, Hypera postica, Hypothenemus spp., Lachnosterna consanguinea, Lema spp., Leptinotarsa decemlineata, Leucoptera spp., Lissorhoptrus oryzophilus, Lixus spp., Luperodes spp., Lyctus spp., Megascelis spp., Melanotus spp., Meligethes aeneus, Melolontha spp., Migdolus spp., Monochamus spp., Naupactus xanthographus, Niptus hololeucus, Oryctes rhinoceros, Oryzaephilus surinamensis, Oryzaphagus oryzae, Otiorrhynchus spp., Oxycetonia jucunda, Phaedon cochleariae, Phyllophaga spp., Phyllotreta spp., Popillia japonica, Premnotrypes spp., Prostephanus truncatus, Psylliodes spp., Ptinus spp., Rhizobius ventralis, Rhizopertha dominica, Sitophilus spp., Sphenophorus spp., Stegobium paniceum, Sternechus spp., Symphyletes spp., Tanymecus spp., Tenebrio molitor, Tribolium spp., Trogoderma spp., Tychius spp., Xylotrechus spp., Zabrus spp.
Still other examples are from the order of the Collembola, for example, Onychiurus armatus.
Still other examples are from the order of the Diplopoda, for example, Blaniulus guttulatus.
Still other examples are from the order of the Diptera, for example, Aedes spp., Agromyza spp., Anastrepha spp., Anopheles spp., Asphondylia spp., Bactrocera spp., Bibio hortulanus, Calliphora erythrocephala, Ceratitis capitata, Chironomus spp., Chrysomyia spp., Chrysops spp., Cochliomyia spp., Contarinia spp., Cordylobia anthropophaga, Culex spp., Culicoides spp., Culiseta spp., Cuterebra spp., Dacus oleae, Dasyneura spp., Delia spp., Dermatobia hominis, Drosophila spp., Echinocnemus spp., Fannia spp., Gasterophilus spp., Glossina spp., Haematopota spp., Hydrellia spp., Hylemyia spp., Hyppobosca spp., Hypoderma spp., Liriomyza spp., Lucilia spp., Lutzomia spp., Mansonia spp., Musca spp., Nezara spp., Oestrus spp., Oscinella frit, Pegomyia spp., Phlebotomus spp., Phorbia spp., Phormia spp., Prodiplosis spp., Psila rosae, Rhagoletis spp., Sarcophaga spp., Simulium spp., Stomoxys spp., Tabanus spp., Tannia spp., Tetanops spp., Tipula spp.
Still other examples are from the order of the Heteroptera, for example, Anasa tristis, Antestiopsis spp., Boisea spp., Blissus spp., Calocoris spp., Campylomma livida, Cavelerius spp., Cimex spp., Collaria spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Monalonion atratum, Nezara spp., Oebalus spp., Pentomidae, Piesma quadrata, Piezodorus spp., Psallus spp., Pseudacysta persea, Rhodnius spp., Sahlbergella singularis, Scaptocoris castanea, Scotinophora spp., Stephanitis nashi, Tibraca spp., Triatoma spp.
Still other examples are from the order of the Homoptera, for example, Acyrthosipon spp., Acrogonia spp., Aeneolamia spp., Agonoscena spp., Aleurodes spp., Aleurolobus barodensis, Aleurothrixus spp., Amrasca spp., Anuraphis cardui, Aonidiella spp., Aphanostigma pin, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Drosicha spp., Dysaphis spp., Dysmicoccus spp., Empoasca spp., Eriosoma spp., Erythroneura spp., Euscelis bilobatus, Ferrisia spp., Geococcus coffeae, Hieroglyphus spp., Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva spp., Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes spp., Trioza spp., Typhlocyba spp., Unaspis spp., Viteus vitifolii, Zygina spp.
Still other examples are from the order of the Hymenoptera, for example, Acromyrmex spp., Athalia spp., Atta spp., Diprion spp., Hoplocampa spp., Lasius spp., Monomorium pharaonis, Solenopsis invicta, Tapinoma spp., Vespa spp.
Still other examples are from the order of the Isopoda, for example, Armadillidium vulgare, Oniscus asellus, Porcellio scaber.
Still other examples are from the order of the Isoptera, for example, Coptotermes spp., Cornitermes cumulans, Cryptotermes spp., Incisitermes spp., Microtermes obesi, Odontotermes spp., Reticulitermes spp.
Still other examples are from the order of the Lepidoptera, for example, Acronicta major, Adoxophyes spp., Aedia leucomelas, Agrotis spp., Alabama spp., Amyelois transitella, Anarsia spp., Anticarsia spp., Argyroploce spp., Barathra brassicae, Borbo cinnara, Bucculatrix thurberiella, Bupalus piniarius, Busseola spp., Cacoecia spp., Caloptilia theivora, Capua reticulana, Carpocapsa pomonella, Carposina niponensis, Chematobia brumata, Chilo spp., Choristoneura spp., Clysia ambiguella, Cnaphalocerus spp., Cnephasia spp., Conopomorpha spp., Conotrachelus spp., Copitarsia spp., Cydia spp., Dalaca noctuides, Diaphania spp., Diatraea saccharalis, Earias spp., Ecdytolopha aurantium, Elasmopalpus lignosellus, Eldana saccharina, Ephestia spp., Epinotia spp., Epiphyas postvittana, Etiella spp., Eulia spp., Eupoecilia ambiguella, Euproctis spp., Euxoa spp., Feltia spp., Galleria mellonella, Gracillaria spp., Grapholitha spp., Hedylepta spp., Helicoverpa spp., Heliothis spp., Hofmannophila pseudospretella, Homoeosoma spp., Homona spp., Hyponomeuta padella, Kakivoria flavofasciata, Laphygma spp., Laspeyresia molesta, Leucinodes orbonalis, Leucoptera spp., Lithocolletis spp., Lithophane antennata, Lobesia spp., Loxagrotis albicosta, Lymantria spp., Lyonetia spp., Malacosoma neustria, Maruca testulalis, Mamestra brassicae, Mocis spp., Mythimna separata, Nymphula spp., Oiketicus spp., Oria spp., Orthaga spp., Ostrinia spp., Oulema oryzae, Panolis flammea, Parnara spp., Pectinophora spp., Perileucoptera spp., Phthorimaea spp., Phyllocnistis citrella, Phyllonorycter spp., Pieris spp., Platynota stultana, Plodia interpunctella, Plusia spp., Plutella xylostella, Prays spp., Prodenia spp., Protoparce spp., Pseudaletia spp., Pseudoplusia includens, Pyrausta nubilalis, Rachiplusia nu, Schoenobius spp., Scirpophaga spp., Scotia segetum, Sesamia spp., Sparganothis spp., Spodoptera spp., Stathmopoda spp., Stomopteryx subsecivella, Synanthedon spp., Tecia solanivora, Thermesia gemmatalis, Tinea pellionella, Tineola bisselliella, Tortrix spp., Trichophaga tapetzella, Trichoplusia spp., Tuta absoluta, Virachola spp.
Still other examples are from the order of the Orthoptera, for example, Acheta domesticus, Blatta orientalis, Blattella germanica, Dichroplus spp., Gryllotalpa spp., Leucophaea maderae, Locusta spp., Melanoplus spp., Periplaneta spp., Pulex irritans, Schistocerca gregaria, Supella longipalpa.
Still other examples are from the order of the Siphonaptera, for example, Ceratophyllus spp., Ctenocephalides spp., Tunga penetrans, Xenopsylla cheopis.
Still other examples are from the order of the Symphyla, for example, Scutigerella spp.
Still other examples are from the order of the Thysanoptera, for example, Anaphothrips obscurus, Baliothrips biformis, Drepanothris reuteri, Enneothrips flavens, Frankliniella spp., Heliothrips spp., Hercinothrips femoralis, Rhipiphorothrips cruentatus, Scirtothrips spp., Taeniothrips cardamoni, Thrips spp.
Still other examples are from the order of the Zygentoma (=Thysanura), for example, Lepisma saccharina, Thermobia domestica.
In another embodiment pests of the phylum Mollusca, in particular from the class of the Bivalvia, for example Dreissena spp. are also important plant pests.

In another embodiment pests of the class of the Gastropoda are important plant pests, for example, Anion spp., Biomphalaria spp., Bulinus spp., Deroceras spp., Galba spp., Lymnaea spp., Oncomelania spp., Pomacea spp., Succinea spp.
In yet another embodiment plant pests are from the phylum Nematoda are important plant pests, i.e. phytoparasitic nematodes, thus meaning plant parasitic nematodes that cause damage to plants. Plant nematodes encompass plant parasitic nematodes and nematodes living in the soil. Plant parasitic nematodes include, but are not limited to, ectoparasites such as Xiphinema spp., Longidorus spp., and Trichodorus spp.; semiparasites such as Tylenchulus spp.; migratory endoparasites such as Pratylenchus spp., Radopholus spp., and Scutellonerna. spp.; sedentary parasites such as Heterodera spp., Globodera spp., and Meloidogyne spp., and stem and leaf endoparasites such as Ditylenchus spp., Aphelenchoides spp., and Hirshmaniella spp. In addition, harmful root parasitic soil nematodes are cyst-forming nematodes of the genera Heterodera or Globodera, and/or root knot nematodes of the genus Meloidogyne. Harmful species of these genera are for example Meloidogyne incognata, Heterodera glycines (soybean cyst nematode), Globodera pallida and Globodera rostochiensis (potato cyst nematode). Still other important genera of importance as plant pests comprise Rotylenchulus spp., Paratriclodorus spp., Pratylenchus penetrans, Radolophus simuli, Ditylenchus dispaci, Tylenchulus semipenetrans, Xiphinema spp., Bursaphelenchus spp., and the like. in particular Aphelenchoides spp., Bursaphelenchus spp., Ditylenchus spp., Globodera spp., Heterodera spp., Longidorus spp., Meloidogyne spp., Pratylenchus spp., Radopholus similis, Trichodorus spp., Tylenchulus semipenetrans, Xiphinema spp.
In yet another embodiment plant pests are viruses and the methods of the invention are directed to treating a viral infection or inhibiting viral infectivity in a plant, the plant virus is selected from an alfamovirus, an allexivirus, an alphacryptovirus, an anulavirus, an apscaviroid, an aureusvirus, an avenavirus, an aysunviroid, a badnavirus, a begomovirus, a benyvirus, a betacryptovirus, a betaflexiviridae, a bromovirus, a bymovirus, a capillovirus, a carlavirus, a carmovirus, a caulimovirus, a cavemovirus, a cheravirus, a closterovirus, a cocadviroid, a coleviroid, a comovirus, a crinivirus, a cucumovirus, a curtovirus, a cytorhabdovirus, a dianthovirus, an enamovirus, an umbravirus & B-type satellite virus, a fabavirus, a fijivirus, a furovirus, a hordeivirus, a hostuviroid, an idaeovirus, an ilarvirus, an ipomovirus, a luteovirus, a machlomovirus, a macluravirus, a marafivirus, a mastrevirus, a nanovirus, a necrovirus, a nepovirus, a nucleorhabdovirus, an oleavirus, an ophiovirus, an oryzavirus, a panicovirus, a pecluvirus, a petuvirus, a phytoreovirus, a polerovirus, a pomovirus, a pospiviroid, a potexvirus, a potyvirus, a reovirus, a rhabdovirus, a rymovirus, a sadwavirus, a SbCMV-like virus, a sequivirus, a sobemovirus, a tenuivirus, a TNsatV-like satellite virus, a tobamovirus, a topocuvirus, a tospovirus, a trichovirus, a tritimovirus, a tungrovirus, a tymovirus, an umbravirus, a varicosavirus, a vitivirus, or a waikavirus.
In yet another embodiment plant pests can be plant pathogenic fungi, Such plant fungi include, but are not limited to, those selected from the group consisting of the Genera: Alternaria; Ascochyta; Botrytis; Cercospora; Colletotrichum; Diplodia; Erysiphe; Fusarium; Leptosphaeria; Gaeumanomyces; Helminthosporium; Macrophomina; Nectria; Peronospora; Phoma; Phymatotrichum; Phytophthora; Plasmopara; Podosphaera; Puccinia; Puthium; Pyrenophora; Pyricularia; Pythium; Rhizoctonia; Scerotium; Sclerotinia; Septoria; Thielaviopsis; Uncinula; Venturia; and Verticillium. Specific examples of plant fungi infections which may be treated with the interferors of the present invention include, Erysiphe graminis in cereals, Erysiphe cichoracearum and Sphaerotheca fuliginea in cucurbits, Podosphaera leucotricha in apples, Uncinula necator in vines, Puccinia sp. in cereals, Rhizoctonia sp. in cotton, potatoes, rice and lawns, Ustilago sp. in cereals and sugarcane, Venturia inaequalis (scab) in apples, Helminthosporium sp. in cereals, Septoria nodorum in wheat, Septoria tritici in wheat, Rhynchosporium secalis on barley, Botrytis cinerea (gray mold) in strawberries, tomatoes and grapes, Cercospora arachidicola in groundnuts, Peronospora tabacina in tobacco, or other Peronospora in various crops, Pseudocercosporella herpotrichoides in wheat and barley, Pyrenophera teres in barley, Pyricularia oryzae in rice, Phytophthora infestans in potatoes and tomatoes, Fusarium sp. (such as Fusarium oxysporum) and Verticillium sp. in various plants, Plasmopara viticola in grapes, Alternaria sp. in fruit and vegetables, Pseudoperonospora cubensis in cucumbers, Mycosphaerella fijiensis in banana, Ascochyta sp. in chickpeas, Leptosphaeria sp. on canola, and Colleotrichum sp. in various crops.

In yet another particular embodiment plant pests are plant pathogenic bacteria including, but not limited to, Acidovorax avenae subsp. avenae (causing bacterial brown stripe of rice), Acidovorax avenae subsp. cattleyae (causing bacterial brown spot of cattleya), Acidovorax konjaci Konnyaku (causing bacterial leaf blight), Agrobacterium rhizogenes (causing hairy root of melon), Agrobacterium tumefaciens (causing crown gall), Burkholderia andropogonis (causing bacterial spot of carnation), Burkholderia caryophylli (causing bacterial wilt of carnation), Burkholderia cepacia (causing bacterial brown spot of cymbidium), Burkholderia gladioli pv. gladioli (causing neck rot of gladiolus), Burkholderia glumae (causing bacterial grain rot of rice), Burkholderia plantarii (causing bacterial seedling blight of rice), Clavibacter michiganensis subsp. michiganensis (causing bacterial canker of tomato), Clavibacter michiganensis subsp. sepedonicus (causing ring rot of potato), Clostridium spp. (causing slimy rot of potato), Curtobacterium flaccumfaciens (causing bacterial canker of onion), Erwinia amylovora (causing fire blight of pear), Erwinia ananas (causing bacterial palea browning of rice), Erwinia carotovora subsp. atroseptica (causing black leg of potato), Erwinia carotovora subsp. carotovora (causing bacterial soft rot of vegetables), Erwinia chrysanthemi (causing bacterial seedling blight of taro), Erwinia chrysanthemi pv. zeae (causing bacterial foot rot of rice), Erwinia herbicola pv. millettiae (causing bacterial gall of wisteria), Pseudomonas cichorii (causing bacterial spot of chrysanthemum), Pseudomonas corrugate Pith (causing necrosis of tomato), Pseudomonas fuscovaginae (causing sheath brown rot of rice), Pseudomonas marginalis pv. marginalis (causing soft rot of cabbage) Pseudomonas rubrisubalbicans (causing mottled stripe of sugar cane), Pseudomonas syringae pv. aptata (causing bacterial blight of sugar beet), Pseudomonas syringae pv. atropurpurea (causing halo blight of ryegrass), Pseudomonas syringae pv. castaneae (causing bacterial canker of chestnut), Pseudomonas syringae pv. glycinea (causing bacterial blight of soybean), Pseudomonas syringae pv. lachrymans (causing bacterial spot of cucumber), Pseudomonas syringae pv. maculicola (causing bacterial black spot of cabbage), Pseudomonas syringae pv. mori (causing bacterial blight of mulberry), Pseudomonas syringae pv. morsprunorum (causing bacterial canker of plums), Pseudomonas syringae pv. oryzae (causing halo blight of rice), Pseudomonas syringae pv. phaseolicola (causing halo blight of kidney bean), Pseudomonas syringae pv. pisi (causing bacterial blight of garden pea), Pseudomonas syringae pv. sesame (causing bacterial spot of sesame), Pseudomonas syringae pv. striafaciens (causing bacterial stripe blight of oats), Pseudomonas syringae pv. syringae (causing bacterial brown spot of small red bead), Pseudomonas syringae pv. tabaci (causing wild fire of tobacco), Pseudomonas syringae pv. theae (causing bacterial shoot blight of tea), Pseudomonas syringae pv. tomato (causing bacterial leaf spot of tomato), Pseudomonas viridiflava (causing bacterial brown spot of kidney bean), Ralstonia solanacearum (causing bacterial wilt), Rathayibacter rathayi (causing bacterial head blight of orchardgrass), Streptomyces scabies (causing common scab of potato), Streptomyces ipomoea (causing soil rot of sweet potato), Xanthomonas albilineans (causing white streak of sugar cane), Xanthomonas campestris pv. cerealis (causing bacterial streak of rye), Xanthomonas campestris pv. campestris (causing black rot), Xanthomonas campestris pv. citri (causing canker of citrus), Xanthomonas campestris pv. cucurbitae (causing bacterial brown spot of cucumber), Xanthomonas campestris pv. glycines (causing bacterial pastule of soybean), Xanthomonas campestris pv. incanae (causing black rot of stock), Xanthomonas campestris pv. (causing angular leaf spot of cotton malvacearum), Xanthomonas campestris pv. (causing bacterial canker of mango), Mangiferaeindicae Xanthomonas campestris pv. mellea (causing wisconsin bacterial leaf spot of tobacco), Xanthomonas campestris pv. (causing bacterial spot of great nigromaculans burdock), Xanthomonas campestris pv. phaseoli (causing bacterial pastule of kidney bean), Xanthomonas campestris pv. pisi (causing bacterial stem-rot of kidney bean), Xanthomonas campestris pv. pruni (causing bacterial shot hole of peach), Xanthomonas campestris pv. raphani (causing bacterial spot of Japanese radish), Xanthomonas campestris pv. ricini (causing bacterial spot of castor-oil plant), Xanthomonas campestris pv. theicola (causing canker of tea), Xanthomonas campestris pv. translucens (causing bacterial blight of orchardgrass), Xanthomonas campestris pv. vesicatoria (causing bacterial spot of tomato), Xanthomonas oryzae pv. oryzae (causing bacterial leaf blight of rice).
While the molecule may be administered as such, in plants, it is particularly envisaged to use a transgenic approach. In these cases, the methods make use of non-naturally occurring nucleic acid sequences encoding a molecule having the following structure:
(X₂ᵢ₋₁Yᵢ-X₂ᵢ-Zᵢ)ₙ, wherein n is an integer from 1 to 5 and i increases from 1 to n with each repeat; and wherein
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P; most particularly 1 to 4 amino acids selected from R, D and P;
- each Yᵢ is independently selected from a stretch of 4 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch naturally occurring in a protein in a plant, plant cell or plant seed (or in a protein of a plant pathogen); and
- each Zᵢ is a linker and Zₙ is independently selected from a linker or nothing.

The methods above can then be reformulated as methods for down-regulating the biological function of a plant protein in a plant or plant cell or plant seed (or for down-regulating the biological function of a protein of a pest organism in a plant or plant cell or plant seed, respectively), comprising transforming said plant, plant cell or plant seed, with a (i.e. at least one) non-naturally occurring nucleic acid sequence encoding a molecule having the structure as outlined above.

Alternatively, the methods can be reformulated as methods for down-regulating the biological function of a plant protein in a plant or plant cell or plant seed (or for down-regulating the biological function of a protein of a pest organism in a plant or plant cell or plant seed, respectively), comprising expressing, in said plant, plant cell or plant seed, a non-naturally occurring nucleic acid sequences encoding a molecule having the structure as outlined above.

According to specific embodiments, the encoded polypeptide sequence is a non-naturally occurring polypeptide. According to further particular embodiments, the nucleic acid sequence is an artificial gene. An artificial gene typically comprises the following operably linked DNA elements: a) a plant expressible promoter b) a nucleic acid (particularly DNA) sequence encoding the molecule described above and c) a 3' end region comprising transcription termination and polyadenylation signals functioning in cells of said plant.

Since the nucleic acid aspect is most particularly suitable in applications making use of transgenic expression, particularly envisaged embodiments are those where the nucleic acid sequence (or the artificial gene) is fused to another moiety, particularly a moiety that increases solubility and/or stability of the gene product. Indeed, transgenic expression of peptides sometimes may be difficult due to rapid degradation of the product. Thus, artificial gene comprising the following operably linked DNA elements: a) a plant expressible promoter b) a nucleic acid encoding an interferor molecule fused to a moiety that enhances solubility (prevents aggregation) of the interferor molecule and c) a 3' end region comprising transcription termination and polyadenylation signals functioning in cells of said plant.

The specification describes recombinant vectors comprising such a nucleic acid sequence encoding a molecule as herein described. These recombinant vectors are ideally suited as a vehicle to carry the nucleic acid sequence of interest inside a cell where the protein to be downregulated is expressed, and drive expression of the nucleic acid in said cell. The recombinant vector may persist as a separate entity in the cell (e.g. as a plasmid), or may be integrated into the genome of the cell.

Most particularly, the molecule is a polypeptide encoded by a nucleotide sequence present on a recombinant vector and which, upon introduction into the plant cell, plant seed or plant, produces said polypeptide in said plant cell, plant seed or plant. In these instances, the contacting between the protein and the non-naturally occurring molecule is produced by expression of said non-naturally occurring molecule in said plant or plant cell. Biological function of the protein thus is downregulated by expression of the interferor molecule.

Accordingly, the specification describes plants, or plant cells, or plant seeds, that contain a nucleic acid sequence, artificial gene or a recombinant vector as described herein. Also plant protoplasts containing such sequences are envisaged herein.

In the present invention a "plant expressible promoter" comprises regulatory elements, which mediate the expression of a coding sequence segment in plant cells. For expression in plants, the nucleic acid molecule must be linked operably to or comprise a suitable promoter which expresses the gene at the right point in time and with the required spatial expression pattern. For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT- PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.
The term "operably linked" as used herein refers to a functional linkage between the promoter sequence and the gene of interest, such that the promoter sequence is able to initiate transcription of the gene of interest.
A "constitutive promoter" refers to a promoter that is transcriptionally active during most, but not necessarily all, phases of growth and development and under most environmental conditions, in at least one cell, tissue or organ. An "ubiquitous" promoter is active in substantially all tissues or cells of an organism. A developmentally-regulated promoter is active during certain developmental stages or in parts of the plant that undergo developmental changes. An inducible promoter has induced or increased transcription initiation in response to a chemical (for a review see Gatz 1997, Annu. Rev. Plant Physiol. Plant Mol. Biol., 48:89- 108), environmental or physical stimulus, or may be "stress-inducible", i.e. activated when a plant is exposed to various stress conditions, or a "pathogen-inducible" i.e. activated when a plant is exposed to exposure to various pathogens. An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific". A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are given in Qing Qu and Takaiwa (Plant Biotechnol. J. 2, 1 13-125, 2004), which disclosure is incorporated by reference herein as if fully set forth. A green tissue-specific promoter as defined herein is a promoter that is transcriptionally active predominantly in green tissue, substantially to the exclusion of any other parts of a plant, whilst still allowing for any leaky expression in these other plant parts.
The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.
"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptll that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.
It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).
Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co- transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible.
For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention.
A transgenic plant for the purposes of the invention is thus understood as meaning, as above, that the nucleic acids used in the method of the invention (e.g. the artificial genes) are not present in, or originating from, the genome of said plant, or are present in the genome of said plant but not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, heterologous expression of the nucleic acids takes place. Preferred transgenic plants are mentioned herein.
The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.
The term "increased expression" or "overexpression" as used herein means any form of expression that is additional to the original wild-type expression level. For the purposes of this invention, the original wild-type expression level might also be zero, i.e. absence of expression or immeasurable expression.
Methods for increasing expression of genes or gene products are well documented in the art and include, for example, overexpression driven by appropriate promoters (as described herein before), the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.
An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1 :1 183-1200). Such intron enhancement of gene expression is typically greatest when placed near the 5' end of the transcription unit. Use of the maize introns Adh1 -S intron 1, 2, and 6, the Bronze-1 intron are known in the art. For general information see: The Maize Handbook, Chapter 1 16, Freeling and Walbot, Eds., Springer, N.Y. (1994).
The term "introduction" or "transformation" as referred to herein encompass the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.
The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363- 373); electroporation of protoplasts (Shillito R.D. et al. (1985) Bio/Technol 3, 1099-1 102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation in planta. To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for Agrobacterium-mediated transformation of rice include well known methods for rice transformation, such as those described in any of the following: European patent application EP1198985, Aldemita and Hodges (Planta 199: 612-617, 1996); Chan et al. (Plant Mol Biol 22 (3): 491 -506, 1993), Hiei et al. (Plant J 6 (2): 271 -282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming Agrobacterium tumefaciens, for example pBin19 (Bevan et al (1984) Nucl. Acids Res. 12-8711). Agrobacteria transformed by such a vector can then be used in known manner for the transformation of plants, such as plants used as a model, like Arabidopsis or crop plants such as, by way of example, tobacco plants, for example by immersing bruised leaves or chopped leaves in an agrobacterial solution and then culturing them in suitable media. The transformation of plants by means of Agrobacterium tumefaciens is described, for example, by Hofgen and Willmitzer in Nucl. Acid Res. (1988) 16, 9877 or is known inter alia from F.F. White, Vectors for Gene Transfer in Higher Plants; in Transgenic Plants, Vol. 1 , Engineering and Utilization, eds. S.D. Kung and R. Wu, Academic Press, 1993, pp. 15-38.
In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of Arabidopsis are treated with agrobacteria and seeds are obtained from the developing plants of which a certain proportion is transformed and thus transgenic [Feldman, KA and Marks MD (1987). Mol Gen Genet 208:1 -9; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551 -558; Katavic (1994). Mol Gen Genet, 245: 363-370). However, an especially effective method is the vacuum infiltration method with its modifications such as the "floral dip" method. In the case of vacuum infiltration of Arabidopsis, intact plants under reduced pressure are treated with an agrobacterial suspension [Bechthold, N (1993). CR Acad Sci Paris Life Sci, 316: 1 194-1 199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21 , 20-28. Further biotechnological progress has recently been reported in form of marker free plastid transformants, which can be produced by a transient co-integrated maker gene (Klaus et al., 2004, Nature Biotechnology 22(2), 225-229).
The genetically modified plant cells can be regenerated via all methods with which the skilled worker is familiar. Suitable methods can be found in the abovementioned publications by S.D. Kung and R. Wu, Potrykus or Hofgen and Willmitzer.
Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.
Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.
The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).
The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.
The term "expression cassette" refers to any recombinant expression system for the purpose of expressing a nucleic acid sequence of the invention in vitro or in vivo, constitutively or inducibly, in any cell, including, in addition to plant cells, prokaryotic, yeast, fungal, insect or mammalian cells. The term includes linear and circular expression systems. The term includes all vectors. The cassettes can remain episomal or integrate into the host cell genome. The expression cassettes can have the ability to self-replicate or not (i.e., drive only transient expression in a cell). The term includes recombinant expression cassettes that contain only the minimum elements needed for transcription of the recombinant nucleic acid.

According to alternative embodiments, the molecules are administered to the plants as such, and not as a transgene. As plants do not have a digestive system, the term administering here particularly also envisages topical applications of the molecules (providing the molecules on the plant rather than in the plant), or indirect administration routes (e.g. providing molecules in the soil, so that they can be taken up by the roots of the plant). Although this administration route is particularly suited to tackle plant infections, it can also be applied to downregulate plant proteins.

The molecules may be provided as such, as an agrochemical. Typically however, the molecules for use in plants (or the nucleic acids encoding them) may be provided as a composition together with an agronomically acceptable carrier (rather than a pharmaceutically acceptable carrier). By "agronomically acceptable carrier" is meant a solid or liquid filler, diluent or encapsulating substance that can be safely used in topical or systemic administration of an interferor molecule to a plant, plant seed, plant cell or plant protoplast. The molecules for applications in plants described herein, combined with an agrochemically acceptable carrier, are also referred to as an agrochemical formulation or agrochemical composition. An "agrochemical formulation" as used herein means a composition for agricultural or horticultural use, comprising an active ingredient (i.e. at least a molecule as described herein), optionally with one or more additives favoring optimal dispersion, atomization, leaf wetting, distribution, retention and/or uptake of agrochemicals. As a non-limiting example such additives are diluents, solvents, adjuvants, surfactants, wetting agents, spreading agents, oils, stickers, penetrants, buffering agents, acidifiers, defoaming agents or drift control agents. An "agrochemical" as used herein includes not only compounds or compound formulations that are ready to use, but also precursors in an inactive form, which may be activated by outside factors. As a non limiting example, the precursor can be activated by pH changes, caused by plant wounds upon insect damage, by enzymatic action caused by fungal attack, or by temperature changes or changes in humidity. Agrochemicals, as used herein, not only includes agents (for example, pesticides, growth regulators, nutrients/fertilizers, repellants, defoliants etc.) that are suitable and/or intended for use in field crops (agriculture), but also includes agents (for example, pesticides, growth regulators, nutrients/fertilizers, repellants, defoliants etc.) that are meant for use in greenhouse crops (horticulture/floriculture) and even agents that are suitable and/or intended for non-crop uses such as uses in private gardens, household uses (for example, herbicides or insecticides for household use), or uses by pest control operators (for example, weed control etc.). Preferably, said agrochemical or combination of agrochemicals is selected from the group consisting of herbicides (e.g. a molecule with a Yi region directed against proteins of weeds), insecticides (e.g. a molecule with a Yi region directed against proteins of insects), fungicides (e.g. a molecule with a Yi region directed against proteins of moulds), nematicides (e.g. a molecule with a Yi region directed against proteins of nematodes), biocides (e.g. a molecule with a Yi region directed against proteins of pathogens), or plant growth regulating compounds (e.g. a molecule with a Yi region directed against proteins of the plant to which it is administered). Additional active ingredients (that are not molecules as described herein) are particularly selected from herbicides, insecticides, fungicides, nematicides, biocides, fertilizers, micro-nutrients or plant growth regulating compounds.
In particular, such an agrochemical composition of the invention may comprise a microcapsule, microsphere, nanocapsule, nanosphere, liposomes or vesicles etc. in which the one or more agrochemicals are suitably encapsulated, enclosed, embedded, incorporated or otherwise included; and one or more targeting agents that each comprise one or more binding domains for binding to one or more antigens present at or in said binding site or that form said one or more binding sites on a plant or parts of a plant, such as a leaf, stem, flower, fruit, bulb or tuber of a plant). The carrier with the one or more targeting agents bound, coupled or otherwise attached thereto or associated therewith may be dissolved, emulsified, suspended or dispersed or otherwise included into a suitable liquid medium (such as water or another aqueous, organic or oily medium) so as to provide a (concentrated) solution, suspension, dispersion or emulsion that can be stored and (where necessary after further dilution) be applied to a plant, to one or more parts of a plant (such as leaves, stem, roots, fruits, cones, flowers, bulbs or tubers), or to the surroundings of a plant (e.g. to the soil in which the plant grows), e.g. by spraying, pouring, dripping, brushing, drip-coating or any other suitable technique. Thereupon, the composition can bind at or to the binding site (or to one or more antigens present at or in said binding site or that form said binding site, such as trichomes, stomata, cuticle, lenticels, thorns, spines or wax layer) via one or more binding domains that form part of the targeting agent(s) comprised in the composition, preferably in a targeted manner. Thereupon, the agrochemicals are released from the carrier (e.g. due to degradation of the carrier or passive transport through the wall of the microcapsule, microsphere, nanocapsule, nanosphere, liposome or vesicle etc.) in such a way that they can provide the desired agrochemical action(s). As an alternative to the use of a carrier, the agrochemical or combination of agrochemicals may also be provided in the form of (small) particles which are provided with a suitable coating or (outside) layer to which the targeting agent is coupled or can bind and which may also serve to stabilize or improve the physical integrity or stability of the particles. As another alternative, the agrochemical or combination of agrochemicals may be suitably mixed with an excipient or binder to which the targeting agent is coupled or can bind, and which may again also serve to stabilize or improve the physical integrity or stability of the particles. Such coated or composite particles are preferably in the form of a slurry, wet cake or free-flowable powder, tablet, capsule or liquid concentrate (such as an emulsion, suspension, dispersion).

It is to be understood that although particular embodiments, specific configurations as well as materials and/or molecules, have been discussed herein for cells and methods according to the present invention, various changes or modifications in form and detail may be made without departing from the scope of this invention. The following examples are provided to better illustrate particular embodiments, and they should not be considered limiting the application. The application is limited only by the claims.

### Examples

In the following examples, different applications of the interferor technology are highlighted. The usefulness of the present molecules in treating infectious disease is illustrated in Examples 1 to 3, relating to antifungal, antibacterial and antiviral applications, respectively. Example 1 details how biofilm growth can be treated or prevented, and also shows downregulation of stress resistance in yeast. Example 2 shows that multiple bacterial species, including antibiotic-resistant species, can be efficiently killed while not being toxic to mammalian cells. Moreover, Example 2 shows the feasibility of in vivo administration of the peptides to treat infection. Example 3 characterizes the identification of interferor molecules directed against different influenza viral proteins.

Different ways in which the present molecules can be used in detection of proteins (ranging from bacterial to human proteins), and how this can be applied in diagnosis of disease by detection of biomarkers in complex samples, are illustrated in Example 4.

Example 5 details applications for non-infectious disease, by showing specific aggregation of two well-known receptor tyrosine kinases, involved in cancer and AMD. Also shown is the concept of modulating immune function using the interferor molecules described herein.

Finally, Example 6 shows that the present molecules can also be applied to achieve protein downregulation in plants.

### 1. Anti-fungal applications

### 1.1 Introduction

*Candida albicans* forms part of the normal human flora, and it grows on mucosal surfaces in healthy individuals. In susceptible hosts, this fungal organism can cause both mucosal and hematogenously disseminated disease. For *C. albicans* to persist in the host and induce disease, it must be able to adhere to biotic and abiotic surfaces, invade host cells, and obtain iron. The *C. albicans* hypha-specific surface protein Als3 is a member of the agglutinin-like sequence (Als) family of proteins and is important in all of these processes (Hoyer LL et al (1998) Curr. Genet. 33(6): 451-9) and Liu Y et al (2011) Eukaryotic Cell. 10(2):168-73. Functioning as an adhesin, Als3 mediates attachment to epithelial cells, endothelial cells, and extracellular matrix proteins. It also plays an important role in biofilm formation on prosthetic surfaces. Als3 is one of the two known *C. albicans* invasins. It binds to host cell receptors such as E-cadherin and N-cadherin and thereby induces host cells to endocytose the organism. Als3 also binds to host cell ferritin and enables *C. albicans* to utilize this protein as a source of iron. Because of its multiple functions and its high expression level *in vivo,* Als3 is considered as a promising target to combat *Candida* infections. In the present example we applied the products and the methods of the invention to specifically target the Als3 protein.

### 1.2 Intracellular expression of specific Als3 interferors

The beta-aggregation prediction algorithm TANGO was used to identify the sequence, LQQYTLLLIYLSVATAK (SEQ ID NO: 1) derived from Als3 (depicted in SEQ ID NO: 2), as a sequence with a high aggregation forming potential. This 17 amino acid residue (SEQ ID NO: 1), corresponding with amino acids 2-18 in SEQ ID NO: 2, was employed as the bait sequence in intracellular interferor constructs. QQ and K act as natural gatekeeper residues (X1 and X2 moieties), YTLLLIYLSVATA (SEQ ID NO: 107) is the Y1 region in this case. The DNA sequence, coding for SEQ ID NO: 1, was cloned in the *C. albicans* expression vector pPCK1-GFP (Barelle et al., (2004) Yeast 21(4): 333-40) in frame with the *C. albicans* optimized GFP (Green Fluorescent Protein) gene and a polylinker sequence, further genetically coupled to a DNA sequence encoding for an aggregator booster and an HA-tag. This so called booster sequence (RKLLFNL (SEQ ID NO: 68)) is an artificial sequence with high propensity to aggregate. The sequence RKLLFNL (SEQ ID NO: 68) shows DnaK binding and is an adapted sequence (wild type sequence is RKLFFNL (SEQ ID NO: 69)) derived from sigma factor 32 (see McCarty J. et al (1996) J. Mol. Bio. 256, 829-837). The expression of the complete fusion construct was under the control of the *PCK1* promoter. The *PCK1* gene is repressed by glucose and encodes PEP carboxykinase by gluconeogenetic carbon sources such as acids or succinate (Leuker et al (1997) Gene 192: 235-240).

SEQ ID NO: 2: amino acid sequence of Als3 of *Candida albicans*

The plasmid, comprising the expression cassette, was linearized at the RPS10 locus before transformation of the *C. albicans* CAI4 strain (Fonzi and Irwin (1993) Genetics 134: 717-728). Transformants were generated and confirmed by PCR analysis to comprise the expression cassette. Three independent transformants AFA16a, AFA16b and AFA16c (expressing the GFP + boosters + bait sequence) were used in subsequent experiments. As a negative control, a recombinant comprising the same plasmid but lacking the specific bait sequence was used. This resulted in the control strain, AFA15a (expressing the GFP + boosters). To identify the localization of the bait peptide the expression of GFP was used. All strains were grown overnight under repressing conditions. Next, the cells were transferred to YNB medium, pH 6.5 supplemented with 4% D-glucose (promoter repressing conditions) or to the same medium supplemented with casamino acids (promoter inducing conditions). We followed the expression of the interferor construct via GFP fluorescence assay after 1 h, 2 h, 3 h and 5 h. Under repressing conditions, no GFP fluorescence could be detected (data not shown).

*C. albicans* AFA15a displayed the presence of an intracellular, diffuse GFP signal, rather than a straightforward localization inside the cell compartment. However, recombinant *C. albicans* strains comprising the interferor constructs displayed punctuated loci, specified as anti-Als3 protein aggregates inside the cytosol. This phenomenon was shown already 2h post induction and proliferated within later time points. Over time, the amount of puncta increased in a range from 1 to 3 per cell. Later time points indicated the same proportion of foci, similarly to that obtained after 5 h (data not shown). This indicated, that the presence of the short interfering bait sequences results in an induced aggregation.

### 1.2.1 In vitro biofilm formation

Since it is known that Als3 is important for adhesion and biofilm formation, we also used the recombinant strains in biofilm experiments which were performed *in vitro.* The starting hypothesis was that *C. albicans* strains expressing the interferor construct had similar phenotypes in adhesion and biofilm formation to the ones obtained with the *C. albicans ALS3* deletion mutant. Recombinant strains expressing the interferors were tested for their ability to form *in vitro* biofilm on silicone square discs. Mature biofilms (48 h) were obtained after growth in YNB, pH 6.5 with either succinate or 4% D-glucose as a carbon source. Biofilm architecture was subsequently assessed by fluorescence microscopy. It was observed that *C. albicans* AFA15a proliferated within 48 h resulting in robust attachment and mature biofilm architecture across the whole silicone surface, independent of growth in induced or repressed conditions. Importantly, *C. albicans* SC5314 (wild type *C. albicans* as referenced by Gillum et al (1984) Mol Gen Genet 198:179-182) developed equivalent mature biofilm to AFA15a (data not shown). On the other hand, induction of expression of Als3 interferor constructs resulted in a strongly reduced adhesion and biofilm formation characterized by black areas without any attached cells. Such rudimentary biofilm development is similar to the one produced by the homozygous Als3 mutant *C*. *albicans als3Δ*/*als3Δ.* All three independent transformants were able to form mature biofilms akin to AFA15a, when grown in YNB, 4% D-glucose. Additionally to fluorescence microscopy, the biofilms were quantified by colony forming units (CFU). The data are presented in figure 3.

Quantification of biofilms was in clear agreement with the observations obtained from the GFP fluorescence microscopy. As expected, the control strains (*C*. *albicans* SC5314 and AFA15a) produced mature biofilms under the two growth conditions. In contrast, recombinants expressing the *C*. *albicans* Als3 interferors significantly failed to form biofilm (*p* < 0.001) (almost 80% inhibition was observed) compared to the wild type strain, whereas when grown under repressing conditions, they gained their function and produced similar biofilms as the control strains (almost 100% biofilm formation). These results support the fact, that induced protein aggregation of Als3 resulted in loss of its function and the recombinants expressing the interferors have a similar phenotype to the one obtained with the *C. albicans als3Δ*/*als3Δ* strain.

### 1.2.2 Adherence and invasion of human epithelial cells

In addition to having a role in adhesion, Als3 is also involved in invasion to human epithelial and endothelial cells. Therefore in a next step we studied the ability of the recombinant *C. albicans* strains to adhere and invade human epithelial cells of *C. albicans* control strains (SC5314 and AFA15a) and recombinant strains expressing the interferors (AFA16a, AFA16b and AFA16c) were grown in inducing/repressing conditions overnight. *C. albicans als3Δ*/*als3Δ* was used as a control. Contact of *Candida* with epithelial cells was assessed after 1 h, whereas invasion was monitored after 3 h. The percentage of adhered and invaded fungal cells is shown in figure 4A and B.

The ability of *Candida albicans* strains expressing the Als3 interferor construct to (A) adhere and (B) to invade epithelial cell line TR-146. Strains were incubated overnight in YNB, pH 6.5 supplemented with succinate, (white bars) or in YNB, pH 6.5 4% D-glucose (black bars). Both assays were performed in DMEM containing NaHCO₃, *D*-glucose, Na-pyruvate and glutamine without FCS. Adherence was performed for 1 h, whereas invasion for 3 h, both at 37 °C, 5% CO₂. Adhered *Candida* cells were stained with calcofluor white and the percentage of adhered cells was calculated as an average of attached *Candida* cells on one hundred areas spread over the entire surface of the coverslip. The percentage of invading C. *albicans* cells was determined by dividing the number of internalized cells by the total number of adherent cells. At least 100 fungal cells were counted on each coverslip. *C. albicans* SC5314 was used as a control strain (100%) and *C. albicans als3Δ*/*als3Δ* as a negative control. A significant difference between the adhesion/invasion of strains expressing interferor construct to epithelial cells compared to control is determined by *p*<0.001(*).Standard deviations were calculated from two independent experiments.

The induction of the Als3 specific interferor in *C. albicans* led to a reduced adhesion to epithelial cells in a range from 40% to 50%, as shown in figure 4A. This difference is statistically significant (*p* < 0.001). It is striking that the recombinant strain AFA16a adhered to the same low level as *als3Δ*/*als3Δ* (see Fig. 3A). As expected recombinant grown under repressed conditions (i.e. no Als3 protein interferors produced) behaved similarly to the control strains. Strain AFA15a showed comparable adhesion capacity as *C. albicans* SC5314, regardless of the conditions used. Next, the recombinants carrying Als3 interferor constructs, showed approximately 40% reduction in invasion, when grown in induced conditions, whereas grown in repressing conditions they gained their function (see figure 4B). As expected, *C. albicans als3Δ*/*als3Δ* failed in invading epithelial cells (*p* < 0.001).

In the present example we show that the recombinant approach proves to be a reliable system that can be further used to study targeted protein aggregation of particular proteins of interest.

### 1.3 External application of Als3 interferor peptides to C. albicans reduces adhesion and biofilm formation

Since the Als3 protein is located in the cell wall, we investigated the possibility to induce specific Als3 aggregation by exogenous application of Als3-specific interferors to the *Candida* cells. In addition to the sequence used in the recombinant expression cassette (YTLLLIYLSV (SEQ ID NO: 70)), the TANGO algorithm revealed additional amino acid sequences with strong aggregation propensity in Als3. Thus, also nonapeptide NGIVIVATT (SEQ ID NO: 71) and peptide TWLCGLITLLSLF (SEQ ID NO: 72) were predicted to have high aggregation potentials (range from 50% to 99% aggregation propensity). Several derivatives of these TANGO sequences were designed, synthesized and tested. Table 1 depicts the 23 different peptides used.

To clarify the design of the molecules by referring to the general formula as proposed in the application, E1 and E2 are molecules were n=1. For E1, X₁ and X₂ are each an arginine residue, Y₁ is a stretch of 9 residues as present in the Als3 protein and Z₁ is absent. Note that this can equivalently be stated as X₁ is RNG, X2 is R, and Y₁ is a contiguous heptapeptide sequence present in the Als3 protein. However, since NG are the neighbouring residues in the Als3 sequence (see SEQ ID NO: 2), and the nonapeptide fulfills the requirements of an Yᵢ stretch, the former notation is preferred. For E2, X₁ is R and X₂ is K, Y₁ is a stretch of 16 residues as present in the Als3 protein and Z₁ is absent.

F9 is a molecule where n=2. X₁ is RK and X₂ is G, Y₁ is a synthetic sequence, Z1 is S, X₃ and X₄ are each an arginine residue, Y₂ is a stretch of 9 residues as present in the Als3 protein and Z₂ is absent (i.e., the second part of the molecule is identical to E1). Note that in this molecule, X₁ and Y₁ together form an artificial sequence derived from sigma factor 32 (see McCarty J. et al (1996) J. Mol. Bio. 256, 829-837) with high aggregation propensity.

The next 20 peptides all have a similar design where n=2, all X moieties are a single residue, both Y₁ and Y₂ are a sequence occurring in the Als3 protein, Z₁ is a GS linker and Z₂ is absent.

**Table 1: Sequences of the 23 interferor peptides which were tested. All sequences were designed by TANGO.**

| **Label** | **Sequence of the peptide** | **Concentration tested** |
|---|---|---|
| E1 | RNGIVIVATTR (SEQ ID NO: 7) | 50 µM; 10 µM; 2.5 µM |
| E2 | RLQQYTLLLIYLSVATAK (SEQ ID NO: 8) | 50 µM; 10 µM; 2.5 µM |
| F9 | RKLLFNLGSRNGIVIVATTR (SEQ ID NO: 9) | 50 µM; 10 µM; 2.5 µM |
| p_1 | RNGIVIVATRGSRNGIVIVATR (SEQ ID NO: 10) | 50 µM; 10 µM; 2.5 µM |
| p_2 | RVIQHSTWLRGSRVIQHSTWLR (SEQ ID NO: 11) | 50 µM; 10 µM; 2.5 µM |
| p_3 | RLITLLSLFRGSRLITLLSLFR (SEQ ID NO: 12) | 50 µM; 10 µM; 2.5 µM |
| p_4 | RQYTLLLIYRGSRQYTLLLIYR (SEQ ID NO: 13) | 50 µM; 10 µM; 2.5 µM |
| p_5 | KNGIVIVATKGSKNGIVIVATK (SEQ ID NO: 14) | 50 µM; 10 µM; 2.5 µM |
| p_6 | KVIQHSTWLKGSKVIQHSTWLK (SEQ ID NO: 15) | 50 µM; 10 µM; 2.5 µM |
| p_7 | KLITLLSLFKGSKLITLLSLFK (SEQ ID NO: 16) | 50 µM; 10 µM; 2.5 µM |
| p_8 | KQYTLLLIYKGSKQYTLLLIYK (SEQ ID NO: 17) | 50 µM; 10 µM; 2.5 µM |
| p_9 | DNGIVIVATDGSDNGIVIVATD (SEQ ID NO: 18) | 50 µM; 10 µM; 2.5 µM |
| p_10 | DVIQHSTWLDGSDVIQHSTWLD (SEQ ID NO: 19) | 50 µM; 10 µM; 2.5 µM |
| p_11 | DLITLLSLFDGSDLITLLSLFD (SEQ ID NO: 20) | 50 µM; 10 µM; 2.5 µM |
| p_12 | DQYTLLLIYDGSDQYTLLLIYD (SEQ ID NO: 21) | 50 µM; 10 µM; 2.5 µM |
| p_13 | ENGIVIVATEGSENGIVIVATE (SEQ ID NO: 22) | 50 µM; 10 µM; 2.5 µM |
| p_14 | EVIQHSTWLEGSEVIQHSTWLE (SEQ ID NO: 23) | 50 µM; 10 µM; 2.5 µM |
| p_15 | ELITLLSLFEGSELITLLSLFE (SEQ ID NO: 24) | 50 µM; 10 µM; 2.5 µM |
| p_16 | EQYTLLLIYEGSEQYTLLLIYE (SEQ ID NO: 25) | 50 µM; 10 µM; 2.5 µM |
| p_17 | PNGIVIVATPGSPNGIVIVATP (SEQ ID NO: 26) | 50 µM; 10 µM; 2.5 µM |
| p_18 | PVIQHSTWLPGSPVIQHSTWLP (SEQ ID NO: 27) | 50 µM; 10 µM; 2.5 µM |
| p_19 | PLITLLSLFPGSPLITLLSLFP (SEQ ID NO: 28) | 50 µM; 10 µM; 2.5 µM |
| p_20 | PQYTLLLIYPGSPQYTLLLYP (SEQ ID NO: 29) | 50 µM; 10 µM; 2.5 µM |

The initial screening and search for optimal Als3 candidate interferors was based on the *C. albicans* SC5314 adhesion assay on 96-well polystyrene plates upon administration of all the peptides at three different concentrations (250 µM, 50 µM and 10 µM). Based on the results obtained with this adhesion assay, we also tested the effect of peptides on mature biofilm development. The final election of the most optimal candidate interferor peptides was based on the results showing reduction in adhesion and mature biofilm formation caused by the lowest concentration of the peptide tested (10 µM or 2.5 µM). An example of three suitable interferor peptides is shown in figure 5A and 5B.

Among the 23 different peptide interferors tested, peptide F9 showed the best activity against mature biofilm development *in vitro,* even when using very low concentrations (p < 0.001) (see Figure 5). Based on the data depicted in Figure 5, peptide F9 (RKLLFNLGSRNGIVIVATTR (SEQ ID NO: 9)) was selected for further experiments. The main aggregator sequence of peptide F9 is NGIVIVATT (SEQ ID NO: 71) with a high-aggregation potential. Apart from peptide F9, also peptide E1 (RNGIVIVATTR (SEQ ID NO: 7)) manifested significant activity against mature biofilm development *in vitro,* when using higher concentrations (250 µM and 50 µM).

### 1.3.1 Determination of the optimum concentration of interferor F9 on adhesion and biofilm development

The initial results (see Figure 5) showed promising activity of interferor peptide F9 against *in vitro* mature *C. albicans* biofilm when use at initial screening conditions (250 µM, 50 µM, 10 µM). In a next step, lower concentrations of peptide F9 (50 µM, 10 µM and 2.5 µM) were also tested. First, we elucidated the effect of peptide F9 during the period of adhesion (90 min at 37 °C). Then, we tested its effect on further biofilm development, by changing the medium with fresh RPMI1640-MOPS, in the absence of peptide for 48 h. Both assays were performed in 96-well polystyrene plates and quantified by the XTT reduction assay (see material and methods section). The results are shown in figure 6A and B.

As shown in figure 6A, even the lowest concentration (2.5 µM) of interferor peptide F9 already dramatically decreased adhesion properties of *Candida* cells (*p* < 0.001). And higher concentrations of interferor peptide F9 (50 µM and 10 µM) almost completely abolished *Candida* attachment. *C. albicans als3Δ*/*als3Δ was* used as a control. It was surprisingly found that the *ALS3* knockout strain still manifested better adherence properties than F9 interferor peptide-treated wild type cells. Without being limited to a possible mechanism, one plausible explanation may be that the interferor peptide F9 causes beta-aggregation not only of Als3 but additionally also of the homologous Als1 and Als5 proteins of *Candida albicans.*

As it was shown before in figure 5B, continuous administration of peptide F9 during the complete *C. albicans* biofilm development (i.e. during a period of 48 h), including adhesion, resulted in strong reduction of mature biofilm formation. Whereas, here depicted in Figure 6B, it is shown that *C. albicans* cells were treated with peptide F9 only during the adhesion period. Mature biofilms were allowed to form in fresh RPMI1640-MOPS without peptide. Importantly, peptide interferor F9 decreased the ability of *Candida* cells to form a biofilm similarly to the *C*. *albicans als3Δ*/*als3Δ* strain. Higher concentrations tested (50 µM), caused almost a 90% biofilm inhibition, whereas lower concentrations (10 µM and 2.5 µM), caused reduction of biofilm development from 40% to 50% (*p <* 0.001). We conclude that the interferor peptide F9 was able to efficiently interfere with two different experimental conditions of *C. albicans* biofilm development.

In a next step we also investigated a potential inhibitory role of interferor peptide F9 during *in vivo C. albicans* biofilm development. We used the *in vivo* subcutaneous *C. albicans* biofilm model in rats (i.e. *in vivo* biofilms formed inside polyurethane triple lumen catheter pieces) as further outlined in the materials and methods section. Only one concentration of peptide F9 (i.e. 50 µM) was tested in the experimental conditions. The peptide was administrated *ex vivo,* during initial attachment of *Candida* to the substrate (90 min, 37 °C). Afterwards, the catheters were washed and implanted subcutaneously in rats. Biofilms were studied six days post implantation and quantified by CFUs. The results of these analyses are shown in figure 7.

The mean CFUs ± SD obtained per catheter fragment of peptide-treated catheters (2.25 ± 1.08 log₁₀ CFU/catheter fragment) was significantly different from the amount of *Candida* cells gained from catheters of the control (3.69 ± 0.42 log₁₀ CFU/catheter fragment) (p < 0.001). Importantly, four peptide-treated catheters (20%) out of 20 contained less than 2.0 log₁₀ CFU/catheter fragment, which is the threshold for determination of *Candida* infection in clinical practice (Mermel et al (2001) Clin. Infect. Dis. 32:1249-1272. *C*. *albicans als3Δ*/*als3Δ* manifested similar biofilm capacity (3.30 ± 0.55 log₁₀ CFU/catheter fragment) as non-treated wild type cells but *C*. *albicans als1Δ*/*als1Δ als3Δ*/*als3Δ* significantly failed to form biofilm in the *in vivo* subcutaneous biofilm model, as witnessed by the low CFU values (1.46 ± 1.24 log₁₀ CFU/catheter fragment) (*p* < 0.001). The potent activity of peptide F9 might come from the effect of the peptide during the period of adhesion. Therefore, we also tested the activity of peptide F9 during *C. albicans* adhesion on polyurethane substrate (figure 8).

The treatment of *Candida* cells with peptide F9 significantly decreased attachment to polyurethane devices (2.18 ± 0.12 log₁₀ CFU/catheter fragment) compared to the control (3.06 ± 0.39 log₁₀ CFU/catheter fragment) (*p* < 0.001). These results are in line with the data obtained during *Candida* adhesion properties on polystyrene. This suggests that the protein aggregation of Als3 appeared during early stages of biofilm development. As expected, *C. albicans als3Δ*/*als3Δ* and *C*. *albicans als1Δ*/*als1Δ als3Δ*/*als3Δ* showed significantly less adherence capabilities on polyurethane compared to the wild type strain (*p* < 0.001).

### 1.3.2 Specificity of interferor F9 for Candida albicans

The specificity of interferor peptide F9 was investigated for *C. albicans* as compared to a distant *Candida* spp., i.e. *Candida glabrata* ATCC2001, for biofilm formation inhibition *in vitro.* The beta-aggregation sequence of the F9 peptide (NGIVIVATT (SEQ ID NO: 71)) is not present in *C*. *glabrata* - the most closely related sequence in C. glabrata is NGVVIVAAT (SEQ ID NO: 73). *Candida glabrata* was shown to form an efficient biofilm *in vitro* on polystyrene and polyurethane. We tested the effect of F9 against *C*. *glabrata* adhesion and biofilm formation on 96-well polystyrene plates *in vitro.* Biofilms were quantified by XTT reduction assay and the results pointed out that peptide F9 did not interfere with *C. glabrata* adhesion and biofilm development. These data support the fact that the efficiency of peptide F9 against adhesion and mature biofilm development is sequence-dependent and highly specific.

### 1.3.3 Application of interferor peptide F9 also decreases the ability of C. albicans to adhere and to invade epithelial cells

As discussed herein before, *C. albicans* Als3 also plays a role in adherence and invasion to epithelial and endothelial cells. We showed (in example 1.2.2) that recombinant strains expressing an Als3 specific interferor significantly decreased the ability to adhere and to invade epithelial cell lines under interferor induced conditions. Here we investigated the effect of external application of interferor peptide F9 on *C. albicans* SC5314 adherence and invasion to epithelial cells. Thereto, *Candida* cells were incubated together with the human cells (epithelial cell line TR-146) in the presence of peptide F9 during adhesion (1 h) or invasion (3 h). The amount of adhered and internalized cells was determined by counting using epifluorescence. The results are displayed in figure 9.

As expected, *C*. *albicans als3Δ*/*als3Δ* showed significantly reduced (60%) capacity to adhere and to invade epithelial cells (*p* < 0.001). The effect of peptide F9 on *C. albicans* SC5314 adherence and invasion showed to be concentration dependent (figure 9). The highest concentration (50 µM) decreased *Candida* adhesion for 40% compared to the control (without peptide). Lower concentrations (10 µM and 2.5 µM) caused an effect of approximately 20% - 15%, respectively. Besides adhesion, we also determined the effect of peptide F9 on invasion. Higher concentrations (50 µM) of peptide F9 decreased invasion for 34%, whereas intermediate concentrations (10 µM) reduced invasion for 15%. The lowest concentration (2.5 µM) did not have any effect on invasion (100%). Whereas adhesion was clearly affected by the addition of F9, the effect on invasion was less clear. Without being bound to a particular theory, it is known that invasion may occur by different and additional mechanisms, of which some may be less dependent on Als3.

### 1.3.4 Specificity of the F9 peptide interferor for Als3

In what has been described before we have shown that peptide F9 causes a significant effect on *C. albicans* adhesion to biomaterials (silicone square disks, polystyrene and polyurethane), also during *in vitro* and *in vivo* biofilm formation, and also on adhesion and invasion to epithelial cells. Although, these results point to the fact that Als3 is targeted it remained to be demonstrated whether Als3 is specifically targeted or not. To demonstrate specificity, we decided to detect the presence of Als3 on the surface of *C. albicans* cells upon peptide F9 administration by fluorescence microscopy in competition with available Als3 specific antibodies. Als3 is mainly expressed on hyphal cells and therefore hyphae formation was first induced for 20 min. Different concentrations of peptide F9 (50 µM, 10 µM and 2.5 µM) were administered to hyphal cells for 45 min. The cells were subsequently washed and incubated in the presence of *ALS3* antiserum for 60 min at 30 °C and counterstained with secondary anti-rabbit IgG conjugated with Alexa Fluor 488. *Candida* cells treated with 1% DMSO were used as a control. Stained cells were visualized with epi-fluorescence using a filter set to detect Alexa Fluor 488. It was found that the interferor peptide-treated *Candida* cells were capable of efficiently competing with the anti-*ALS3* antibody binding in a concentration dependent manner. Hence, *Candida* cells treated with the highest concentration (50 µM) of peptide F9 demonstrated about 50 % reduction in *ALS3* antiserum binding as compared to the control (without peptide, only 1% DMSO was added). In addition, also intermediate concentrations (10 µM) decreased the ability of *Candida* cells to bind the Als3 antibody. These data were also quantified by determining the capability of interferor peptide-treated *C. albicans* SC5314 cells to bind anti-*ALS3* antibody in a Fluorescence-Activated Cell Sorting (FACS) approach. In comparison to fluorescence microscopy, this method allowed us to detect the fluorescence signal from only 10 000 *Candida* cells. The FACS data clearly supported the observation obtained from microscopy images. *C. albicans* hyphal cells treated with peptide F9 (50 µM), displayed a significantly reduced amount of fluorescent signal (54%) compared to the control (*p* < 0.001). In addition, the intermediate concentration (10 µM) also caused a striking reduction in *ALS3* antiserum binding (45%), whereas the lowest concentration (2.5 µM) also caused a 10% reduction. Thus the data show that peptide F9 specifically targets Als3 and induces its aggregation which results in a significantly decreased amount of Als3 on hyphal cells, which was characterized by a diminished anti-*ALS3* antibody binding in an F9 concentration dependent manner.

### 1.4 Interferor-coated medical devices prevent C. albicans biofilm formation

*Candida albicans* is commonly diagnosed in biofilm-infected catheters. *Candida sp.* can form biofilms on a wide variety of medical devices from urinary and vascular catheters, to dental, hip, knee and voice prostheses and even pacemakers. This wide range of implanted biomaterials that can be infected with Candida reflects on the ability to adhere to many types of substrate, including plastic materials such as silicone, polystyrene and polyurethane. Infection of these medical devices often leads to the loss of their function, and can constitute a source of systemic infection. Since *C. albicans* biofilms are often extremely resistant to common antifungal therapy, the infected devices often need to be removed. The mechanisms of resistance are not well established but limitation of access of the drug to the fungal cells is one possible explanation. In the previous examples we have shown that an alternative approach is to interfere with the adhesion of *C. albicans* to the substrate (e.g. silicone (example 1.2.1), polystyrene (example 1.3.2) and polyurethane (example 1.3.2), i.e. preventing the biofilm to be formed instead of combatting a mature biofilm. In our current approach we are preparing catheter material which is covalently linked with the F9 peptide interferor. Methods for covalently linking peptides to a solid support are described in the art. The performance of these interferor-coupled catheters are evaluated in an *in vivo* rat model as described in example 1.3.2 in accordance with the materials and methods section 1.6.7.2.

### 1.5 Downregulation of calcineurin using transgenic molecules

In Candida albicans, Hsp90 is an essential, highly conserved chaperone involved in pathogenicity and in resistance to antifungals. The serine/threonine specific phosphatase calcineurin (CNB1) is a target of Hsp90, and a homozygous deletion mutant for cnb1 is sensitive to stress. Thus, it was decided to target the calcineurin protein using the molecules presented herein.

### Design of Cnb1 aggregation-inducing molecules

TANGO analysis of the regulatory subunit of Calcineurin revealed two regions prone to aggregation. The predicted protein sequences FAFNIY (SEQ ID NO: 104) and LFIVM (SEQ ID NO: 105) show a β-aggregation propensity of respectively 40% and 96%. These two short sequences were used to design several different constructs for downregulating CNB1 using a transgenic approach. The constructs had the following structure:
Promoter - yEGFP - HA tag (sequence YPYDVPDYA (SEQ ID NO: 108)) - amino acid linker (sequence MAQW (SEQ ID NO: 109)) - molecule with structure (X₂ᵢ₋₁-Yᵢ-X₂ᵢ-Zᵢ)ₙ, with n=5 and
X₁ = QN
Y₁ = STLIVL (SEQ ID NO: 110)
X₂ = Q
Z₁ = 0
X₃ = N
Y₂ = STVIF (SEQ ID NO: 111)
X₄ = E
Z₂ = Q
X₅ = N
Y₃ = STVIF (SEQ ID NO: 111)
X₆ = EQN
Z₃ = KPAGAAKPGAAG (SEQ ID NO: 112)
X₇, Y₄, X₈, Z₄, X₉, Y₅, X₁₀ are varied according to what is shown in table 2; and Z₅ is nothing.

**Table 2. Structure of constructs used for downregulation of calcineurin.**

| construct | X₇ | Y₄ | X₈ | Z₄ | X₉ | Y₅ | X₁₀ |
|---|---|---|---|---|---|---|---|
| #1 | R | FAFNIY (SEQ ID NO: 104) | R | GS | R | LFIVM (SEQ ID NO: 105) | R |
| #2 | R | FAFNIY (SEQ ID NO: 104) | D | GS | E | LFIVM (SEQ ID NO: 105) | R |
| #3 | R | FAFNIY (SEQ ID NO: 104) | D | PPP | E | LFIVM (SEQ ID NO: 105) | R |
| #4 | R | FAFNIY (SEQ ID NO: 104) | R | PPP | R | LFIVM (SEQ ID NO: 105) | R |

Written contiguously, the sequence for which e.g. construct number 1 encodes thus looks as follows:
Promoter - yEGFP - YPYDVPDYA MAQW QNSTLIVLQNSTVIFEQNSTVIFEQNKPAGAAKPGAAGRFAFNIYRGSRLFIVMR (SEQ ID NO: 113)

The molecules used in these constructs contain essentially two kinds of aggregating-inducing regions: Y1, Y2 and Y3 serve as synthetic (i.e. not occurring in Candida/not part of CNB1) sequences that boost aggregation, while Y4 and Y5 are the two aggregation-prone regions identified in the CNB1 protein that ensure specificity.

### Properties of transformants

The initial characterization of transformants carrying these constructs was done by means of in vivo test for resistance to various kinds of stress (to which *cnb1Δ* mutants are sensitive). Constructs were placed under the control of a PCK1 promoter which is repressed in glucose containing medium (Synthetic Dextrose (SD) medium) and induced in gluconeogenetic conditions (Synthetic Casamino Acids (SCAA) medium). As shown in Fig.10, transformants are indeed more sensitive to the presence of SDS in the medium, and this only when the aggregator protein is induced. No effect on growth is observed when no stress is present, indicating that the observed phenotype is not due to aspecific or toxic effects linked to expression of the constructs.

The four different transformants differ, and differ only, in the nature (and charge) of the X₈ and X₉ gatekeepers and the Z₄ linker. However, they all share the same phenotype typical for calcineurin downregulation. So, in this experimental setup, the charge of the gatekeepers and the nature of the linker is only of secondary importance - the system is robust enough to achieve downregulation of the target protein irrespective of these variants.

As the four constructs all share the same CNB1 aggregation-inducing regions in the same order (i.e. FAFNIY (SEQ ID NO: 104) and LFIVM (SEQ ID NO: 105)), which is also the same N- to C-terminal order these regions occur in the calcineurin protein, it was decided to test whether the order of these aggregation determining regions would be important.

This has been evaluated by creating constructs were only the Y4 and Y5 regions were swapped, as shown in table 3.

**Table 3. Structure of constructs used for downregulation of calcineurin, with swapped aggregating regions.**

| construct | X₇ | Y₄ | X₈ | Z₄ | X₉ | Y₅ | X₁₀ |
|---|---|---|---|---|---|---|---|
| #5 | R | LFIVM (SEQ ID NO: 105) | R | GS | R | FAFNIY (SEQ ID NO: 104) | R |
| #6 | R | LFIVM (SEQ ID NO: 105) | D | GS | E | FAFNIY (SEQ ID NO: 104) | R |
| #7 | R | LFIVM (SEQ ID NO: 105) | D | PPP | E | FAFNIY (SEQ ID NO: 104) | R |
| #8 | R | LFIVM (SEQ ID NO: 105) | R | PPP | R | FAFNIY (SEQ ID NO: 104) | R |

Transformants 5 to 8 were also tested for their sensitivity to SDS using the same assay. All of the transformants behaved similarly to those with constructs 1 to 4, i.e. the phenotype resembles that of wildtype yeast in promoter-repressing conditions and resembles that of the homozygous cnb1 deletion mutant in promoter-inducing conditions. This allows us to conclude that, at least in this experimental setup, the order of the aggregating-inducing regions is not vital to achieve specific downregulation, and needs not be identical to the order in which the regions occur in the target protein.

To check whether both regions contribute to specific aggregation, constructs where Y4 and Y5 are identical (and thus correspond to either LFIVM (SEQ ID NO: 105) or FAFNIY (SEQ ID NO: 104)) were also tested. These transformants showed the same phenotype (i.e., the presence of either region alone is enough to achieve calcineurin downregulation), but to a lesser extent (i.e., not in all transformants tested the phenotype was observed, possibly because of higher threshold expression levels required to induce specific aggregation). The combination of two different aggregation-inducing regions in the same protein to target the protein accordingly may prove beneficial.

### 1.6 Materials and methods to the anti-fungal applications

### 1.6.1. Animals and immunosuppression

The animals used for the *in vivo* experiments were 200g specific pathogen free female Sprague Dawley rats (Janvier, France). All animals were given standard ad libitum diet and were immunosuppressed with 1mg/L of dexamethasone (Organon, Netherlands) in their drinking water up to 24 hours before and during the whole experimental procedure. Tetracycline (1g/L) or Ampicillin (0.5g/L) was added to the water to minimize bacterial infections. All animal experiments were maintained in accordance with European regulations regarding the protection and the well-being of laboratory animals and were approved by the animal ethical committee of the Katholieke Universiteit Leuven.

### 1.6.2. Peptide-based therapeutics with the high propensity to aggregate the protein of interest - Als3

*C. albicans* specific peptide-based therapeutics with the high propensity to cause cross beta-aggregation of Als3p were synthesized by JPT Peptide Technologies GmbH (Berlin, Germany). Highly purified peptides (>90%, purity detected by HPLC) were delivered in lyophilized form and kept at -20 °C before use. Stock solution of each peptide was prepared in 50% DMSO in ultra pure water (Invitrogen) to a final concentration 10 mM - 20 mM. Three different concentrations (50µM, 10µM and 2.5µM) of positive peptide F9 were tested in every assay. Experiments including peptides were carried out in low adhesion plastic material (Bioplastics, Netherlands) to lower self-aggregation.

### 1.6.3. Yeast culture conditions

In general, yeast cells were cultured overnight under continuous shaking at 30 °C in rich YP medium containing 1% (w/v) yeast extract, 2% (w/v) Bactopeptone and 2% (w/v) *D*-glucose (YPD) unless stated otherwise. Prior to *in vitro* and *in vivo C. albicans* biofilm experiments, the cells were grown on solid YPD medium (containing 1.5% (w/v) agar) overnight at 37 °C. Strains carrying aggregator constructs were grown in Yeast Nitrogen Base (YNB) with amino acids and ammonium sulfate, pH 6.5, (Difco, USA) supplemented with succinate, Casamino acids (promoter inducing conditions) or supplemented with 4% *D*-glucose (promoter repressing conditions). To study the potential effect of aggregator peptides on fungal growth, the cells were incubated in the presence of 50 µM concentration of each peptide at 30 °C. Final concentration of DMSO in the control sample did not exceed 1%. Samples were collected every hour and the cell density was measured using a spectrophotometer (BioPhotometer, Eppendorf) at 600 nm.

### 1.6.4. Yeast-to-hyphae transition

*Candida* cells were grown overnight in YPD medium, washed twice and further incubated in sterile water for an additional 2 h at 30 °C (starvation period). Next, cell concentration was adjusted to 1 x 10⁶ cells/ml, and cells were incubated in YP medium containing 10% Fetal bovine serum (FBS) (F7524, Sigma, USA) or in RPMI1640-MOPS, pH 7.0 with/without peptides (50µM). Cultures were incubated for 1.5 h - 2 h at 37 °C. The proportion of true hyphae vs. budding yeasts was determined by light microscopy (Axiostar plus, Carl Zeiss, Germany) at magnification 40x.

### 1.6.5. Determination of Minimal Inhibitory Concentration (MIC)

The Minimal inhibitory concentration (MIC) of planktonic cells to antifungals was determined according to NCCLS M27-A3 (2008). Briefly, *C. albicans* strains were grown overnight at 30 °C on YPD plate. *Candida* cell suspension (1-5 x 10⁶ cells/ml) was prepared in RPMI1640-MOPS medium. The cells were further diluted 1:50 and from such suspension, 100 µl of *Candida* cells was applied into each well of a 96-well polystyrene plate. Subsequently, 100 µl, containing different concentrations of antifungals to be tested, was added. Control wells included *Candida* cells where only RPMI1640-MOPS was added. The cells were allowed to grow for two days and effectiveness of the antifungals was determined by measuring the optical density (OD) of the cells using a spectrophotometer (Spectra max Plus 384) at 490 nm. The data were determined as MIC₅₀ and MIC₉₅, which represents minimal inhibitory concentration of the drug which inhibits fungal growth for 50% or 95%, respectively.

### 1.6.6. Determination of Minimal Fungicidal Activity (MFC)

The Minimal fungicidal activity (MFC) was determined as described by Cantón et al. (2009) Antimicrob Agents Chemother. Jul;53(7):3108-11. The MFC was defined only for anidulafungin, as this is the only drug with fungicidal activity in our study. Briefly, 100 µl of *Candida* cell suspension pretreated with different concentrations of antifungal drug was plated on YPD plates and further incubated at 37 °C for 24 h. The MFC was determined as a result of 99% growth inhibition.

### 1.6.7. C. albicans biofilm models

### 1.6.7.1 In vitro C. albicans biofilm system

*In vitro C. albicans* biofilm was studied on three different types of biomaterials, namely flat bottom 96-well polystyrene plate (Greiner Bio-One, Germany), silicone (CS Hyde, USA) and polyurethane triple lumen intravenous catheters (2.4mm diameter) (Arrow International Reading, USA). Prior the biofilm set up, silicone was cut into small square pieces (1cm x 1cm) and polyurethane into 1 cm pieces.

Silicone or polyurethane devices were incubated in 99% FBS (F7524, Sigma) overnight at 37 °C. Cells were washed and resuspended in 1x phosphate buffered saline (PBS), pH 7.4. *Candida* suspension of 1 x 10⁷ cells/ml or 5.10⁴ cells/ml was prepared in RPMI1640-MOPS medium, Ssuccinate, pH 6.5 or in YNB medium, 4% D-glucose. In total, 1 ml of the cell suspension was added to the silicone discs or polyurethane catheters placed in 24 well plate and 100 µl of cell suspension was inoculated into 96-well polystyrene plate. The attachment of the cells to a substrate was achieved by incubation at 37 °C, for 90 min under static conditions (period of adhesion). Afterwards, non-attached *Candida* cells were removed by two rounds of washing steps with 1 x PBS and submerged in fresh medium for 48 h to 144 h at 37 °C (mature biofilm). After that, the biofilms were washed twice with 1 x PBS and quantified.

### 1.6.7.2 In vivo subcutaneous C. albicans biofilm system

The experimental time line of *in vivo C. albicans* biofilm development in a new subcutaneous model is illustrated in scheme 1.

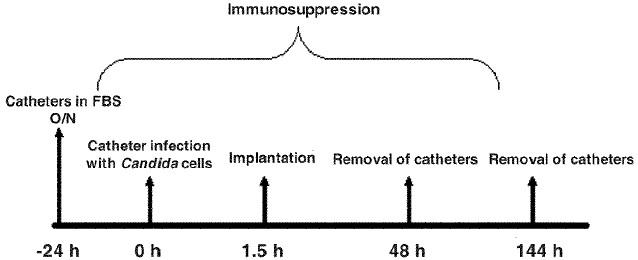

Scheme 1: Experimental time line of animal *Candida albicans* biofilm model. Experimental time line of *in vivo Candida albicans* biofilm development in a new subcutaneous model.

*C. albicans* cells were grown overnight at 37 °C on YPD plates, washed and resuspended in 1 x PBS. *Candida* cells suspension (5.10⁴ cells/ml) was prepared in RPMI1640-MOPS medium by counting. Polyurethane triple lumen intravenous catheters (2.4 mm diameter) cut into segments of 1 cm (Arrow International Reading, USA) were incubated overnight in FBS at 37 °C. Serum-coated catheters were incubated for 90 min at 37 °C in 1 ml of *Candida* cell suspension (period of adhesion). After the period of adhesion, catheters were washed twice with 1 x PBS before being implanted under the skin of rats as described (Van Wijngaerden et al. (1999) J Antimicrob Chemoth 44:669-674). Anaesthesia was performed by a short inhalation period of enflurane gas (Alyrane™, Pharmacia). Rats were kept asleep during the implant procedure by a gaseous mix of enflurane (20 %) and oxygen (80 %). The lower back of the rat was shaved and disinfected with chlorhexidine 0.5 % in alcohol 70 %. A 10 mm incision was made longitudinally and the subcutis was carefully dissected to create 3 subcutaneous tunnels. Up to ten catheter fragments were implanted. The incision was closed with surgical staplers (Precise™, USA), and disinfected with chlorhexidine 0.5% in 70% alcohol. Biofilms were formed for 48 h and 144 h. For catheter explant, rats were euthanized by CO₂ inhalation. The skin was disinfected and catheter fragments were removed from under the subcutaneous tissue, washed twice with 1 x PBS and quantified or visualized.The effect of peptides promoting the aggregation of Als3p during *Candida* biofilm development *in vivo* was characterized, as well. Serum-coated polyurethane fragments were incubated with *Candida* cells (5 x 10⁴ cells/ml) in the presence of 50 µM concentration of positive peptide F9 and negative peptide F9_negat, p13 and p20 during adhesion period (90 min, 37 °C). Afterwards, non-adhered cells were removed by two rounds of washing steps. Catheters were implanted subcutaneously to the back side of the immunosuppressed rats as described above. Biofilms were studied after six days post implant by CFU counting.

### 1.6.8. Biofilm quantification methods

### 1.6.8.1 XTT reduction assay

The metabolic activity of *Candida* cells within *in vitro* biofilms was studied using the XTT reduction assay. This method is based on colorimetric change of a specific substrate - XTT (2,3-bis(2-methoxy-4-nitro-5-sulpho-phenyl)-2H-tetrazolium-5-carboxanilide) (Sigma, USA) which is reduced to XTT formazan by mitochondrial dehydrogenases of metabolic active cells measured by spectrophotometer at 490 nm. The XTT (Sigma, USA) working solution was prepared in sterile 1 x PBS with the final concentration of 1 mg/ml. Before use, menadione was added to the XTT solution at a final concentration of 1 µM. This solution was vortexed and 100 µl was applied into each well containing biofilm and incubated for 3-5 h at 37 °C in the dark. The intensity of colorimetric change was measured by spectrophotometer (Spectra max Plus 384) at 490 nm. The XTT-menadione solution without *Candida* cells was used as a blank.

### 1.6.8.2 Quantification of fungal biofilm biomass

The amount of fungal biofilm biomass formed inside the catheter lumen of *in vitro* and *in vivo* explanted catheters was determined by colony forming units (CFU). Briefly, *in vitro* substrates and catheters from *in vivo* biofilms were sonicated for 10min at 40000Hz in a water bath sonicator (Branson 2210) and further vortexed for 30s in 1 x PBS. Original samples and a 1:10 dilution were plated on YPD plates always in duplicate. CFUs were counted after two days at 37 °C.

### 1.6.9. Visualization of Candida biofilms by microscopy

### 1.6.9.1 Fluorescence microscopy

Prior the fluorescence microscopy *in vitro* and *in vivo* catheters with attached biofilms were longitudinally cut and incubated in 1 x PBS buffer with 50 µg/ml calcofluor white (Sigma, USA) for 20 min. These devices were observed with a Zeiss Axioplan 2 fluorescence microscope. Images were acquired by a Zeiss Axiocam HRm camera using Axiovision 3.0 software (Carl Zeiss, Thornwood, NY).

### 1.6.9.2 Scanning electron microscopy

Catheters (longitudinally cut) were fixed in 3% glutaraldehyde in 0,1 M sodium cacodylate buffer (pH 7.4) prior the microscopy. Catheters were removed from the fixation solution and dried overnight. Mounted samples were sputter-coated with gold and viewed in a XL30 ESEM FEG scanning electron microscope (Philips).

### 1.6.9.3 Confocal scanning laser microscopy

Longitudinally cut and fixed catheters were incubated with 50 µg/ml concanavalin A for one hour at 37 °C (concanavalin A, Alexa Fluor®488 conjugate, Invitrogen). Confocal images were acquired and analysed with a LSM510/ConfoCor2 system (Carl Zeiss, Jena, Germany). The Argon laser (6 A, acousto-optical tunable filter adjusted to 50%) provided the excitation light of 488 nm (for ConcanavalinA-Alexa488 fluorescence). The excitation light was reflected by a dichroic mirror (HFT 488) and focused through a Plan-NeoFluar 20x NA0.5 objective. The fluorescence emission light passed through a 505-nm longpass filter and a 1-Airy unit pinhole. Before, the biofilm thickness was assessed, first, the 3-dimensional (3-D) pictures of mature biofilms were captured. Approximately 200 sections were made through the whole biofilm architecture. Then, the biofilm thickness was estimated from the outer edges of the area where fluorescent signal gain intensity above half of its maximum until the area where the fluorescent signal could no longer be determined.

### 1.6.10. Green Fluorescent Protein (GFP) fluorescence microscopy

Cells were used directly without fixing and viewed using a Zeiss Axioplan 2 fluorescence microscope. GFP was visualized with a UV light source and a long pass GFP filter. Images were taken by Quantix charge-coupled device camera using Axiovision 3.0 software (Carl Zeiss, Thornwood, NY).

### 1.6.11. Absorption of the rabbit ALS3 antiserum with C. albicans als3Δ/als3Δ germ tubes

The lyophilized form of *ALS3* antibody was reconstituted in 500 µl of sterile water. The antiserum was absorbed with *C. albicans als3Δ*/*als3Δ* germ tubes before using it for fluorescence microscopy and FACS. Three flasks containing 1 x 10⁶ cells/ml were germinated in RPMI1640 medium with L-glutamine and with HEPES (PAA, Austria) for 90 min at 37 °C. Germ tubes were divided into micro-centrifuge tubes, mixed with *ALS3* antiserum and incubated for 1 h on ice with gentle shaking. Afterwards, the cells were centrifuged and the supernatant was transferred to another flask containing fresh *C. albicans als3Δ*/*als3Δ* germ tubes. This procedure was repeated in total three times. *ALS3* antiserum was aliquoted into smaller volume (20 µl) and stored at -80 °C. The specificity of the antibody was confirmed by fluorescence microscopy (Leica DFC350 FX, Mannheim, Germany).

### 1.6.12. Growth and differentiation of epithelial cells

Originally, the squamous carcinoma of buccal mucosa derived epithelial cell line TR-146 was obtained from Cancer Research Technology, London. TR-146 cells are capable of forming stratified layers of cells showing many similarities compared with normal human buccal mucosa (Rupniak et al., (1985) J Natl Cancer Inst 75:621-35. TR-146 cells were routinely grown (passages 4 - 20) in Dulbecco's modified Eagle's medium (DMEM) containing NaHCO₃, *D*-glucose, Na-pyruvate and stable glutamine with 10% fetal calf serum (FCS) (Sigma, USA), without antibiotics or antifungal agents. Cells were maintained in a humidified incubator at 37 °C in 5% CO₂. For adherence and invasion experiments, 1 x 10⁵ of TR-146 cells were seeded onto 12 mm diameter glass coverslips previously placed in 24 well plates.

### 1.6.13. Adherence assay

*C. albicans* strains were grown in liquid YPD, succinate, pH 6.5 or YNB (4% D-glucose) at 30 °C in a shaking incubator overnight. *Candida* cells were washed three times with 1 x PBS and determined to a final concentration 1 x 10⁵ cells/ml in DMEM containing NaHCO₃, D-glucose, Na-pyruvate and stable glutamine without FCS. The TR-146 were grown on 12 mm glass slides and inoculated with *Candida* cells. Different concentrations of positive peptide F9 (50µM, 10µM and 2.5 µM) and negative peptides F9_negat, 13 and 20 (50µM) were tested on *Candida* adhesion to epithelial cells. Adhesion assay was allowed for 1 h at 37 °C, 5% CO₂. After adhesion, the cells were washed three times with 1 x PBS to remove non-adherent cells and then fixed with 4% paraformaldehyde for 30 min at room temperature. After extensive rinsing with 1 x PBS, the cells were permeabilized in 0.5% Triton X-100 in water for 5 min, and subsequently stained with calcofluor white (dilution 1:100) for 15 min and further washed with water, three times for 10 min, at 30 °C, 180 rpm. Coverslips were mounted inverted on a microscope slide and quantified under epifluorescence using a filter set to detect calcofluor white (filter for DAPI) (Leica DFC350 FX, Mannheim, Germany). The percentage of adhered cells was calculated as an average of attached *Candida* cells on one hundred areas spread over the entire surface of the coverslip.

### 1.6.14. Invasion assay

The monolayers of TR-146 cell lines were infected with *Candida* cells as previously described in 6.2.15. After 3 h incubation period of monolayers with *Candida* cells at 37 °C, 5% CO₂, the medium above the epithelial cells was aspirated and the monolayers were rinsed three times with 1 x PBS to remove fungal cells which were not associated with epithelial cells. Next, the epithelial cells were fixed with 4% Histofix (Roth) for 20 min at 37 °C. All fungal cells remaining adherent to the surface were stained for 1 h with a rabbit anti-*C*. *albicans* polyclonal antibody (Acris Antibodies, Germany) (dilution 1:2000) and counterstained with a secondary anti-rabbit IgG conjugated with Alexa Fluor 488 (Invitrogen) (dilution 1:5000). After extensive rinsing with 1 x PBS, the cells were permeabilized in 0.5% Triton X-100 in water for 5 min. Further, adherent and invading parts of fungal cells within epithelial cells were stained with calcofluor white (dilution 1:100) for 15 min and washed three times with water, for 10 min at 30 °C, 180 rpm. Coverslips were mounted inverted onto glass slides and the stained cells were visualized with epifluorescence using a filter set to detect calcofluor white (filter for DAPI) and Alexa Fluor 488 (Leica DFC350 FX, Mannheim, Germany). The percentage of invading *C. albicans* cells was determined by dividing the number of internalized cells by the total number of adherent cells. At least 100 fungal cells were counted on each coverslip.

### 1.6.15. Detection of C. albicans ALS3 antibody binding by fluorescence microscopy and by Fluorescence-Activated Cell Sorting (FACS)

Overnight cultures of *C. albicans* cells were collected and washed three times with 1 x PBS. *Candida* cells (2.5 x 10⁶ cells/ml) were prepared in RPMI1640 medium with L-glutamine and with HEPES (PAA, Austria) with/without peptide F9 (50µM, 10µM and 2.5 µM) or negative peptides F9_negat, 13 and 20 (50µM). *Candida* adhesion and hyphae induction was performed on 12 mm glass slides or glass Petri dishes (Ø 5 cm) for 45, 60 or 90 min at 37 °C, 5% CO₂. Non-adhered cells were washed with 1 x PBS and immediately fixed with 4% Histofix (Roth) for 20 min at 37 °C. After extensive rinsing with 1 x PBS, the cells were permeabilized in 0.5% Triton X-100 for 5 min, washed, and incubated in 1% Bovine Serum Albumin (BSA) for 20 min at RT. Further, adhered cells were incubated in the presence of *ALS3* antiserum (dilution 1:500) for 60 min at 30 °C and counterstained with secondary anti-rabbit IgG conjugated with Alexa Fluor 488 (Invitrogen) (dilution 1:2000). After the final washing step, the coverslips were mounted inverted onto glass slides and the stained cells were visualized with epifluorescence using a filter set to detect Alexa Fluor 488 (Leica DFC350 FX, Mannheim, Germany). Finally, the cells were detached from the cover-slips using a cell scraper and resuspended in 0.5 ml 1 x PBS. The fluorescent intensity of the hyphae was measured using a LSRII flow cytometer (Becton Dickinson, http://www.bd.com). Fluorescence data for 10,000 cells of each strain were collected.

### 1.6.16. Isolation of spheroplasts and Coomassie staining of poly-acrylamide gels

*Candida* cells (1 x 10⁷ cells/ml) were incubated in YPD medium in the presence of peptide F9, F9_negat, 13 and 20 (50 µM) at 37 °C. Cells were collected 5 min, 30 min and 60 min upon addition of peptides, centrifuged at 4500 rpm, 5 min at room temperature. Pellets were washed twice with one isovolume of digestion buffer (2 M sorbitol, 1 M KH₂PO₄, pH 7.5, 0.5 M EDTA) without zymolyase, weighed and further dissolved in digestion buffer containing 10mg zymolyase 20T (MP Biomedicals, USA) (5ml buffer/1g of cells). The reaction was supplemented with 10 µl of *β*-mercaptoethanol per 1 ml of digestion buffer. Cells were incubated for 30 min - 45 min at 37 °C. Spheroplasts vs. intact cells were determined in a small volume of 0.5% SDS and observed under the microscope. Intact cells were not influenced by the presence of 0.5% SDS whereas spheroplasts leave only *"ghosts".* Spheroplasts were collected by centrifugation at 2000 rpm, 2 min, RT and washed twice with 1.2 M cold sorbitol. Importantly, all solutions used for isolation of spheroplasts contained protease inhibitor mix (complete EDTA-free, Roche). Finally, spheroplasts were dissolved in NuPAGE® LDS Sample buffer (Invitrogen) supplemented with 4% *β*-mercaptoethanol, boiled for 5 min at 65 °C. Before loading on the gel, samples were briefly centrifuged. Proteins were separated via SDS-PAGE (NuPAGE® 4-12% Bis-Tris gel, Invitrogen) in NuPAGE® MES SDS running buffer (Invitrogen) at a constant voltage of 120 V. After electrophoresis, gels were transferred to a plastic tray and the proteins were stained in 0.25% Coomassie Brilliant Blue in 30% (v/v) methanol and 10% (v/v) acetic acid overnight with gentle shaking. Gels were destained in 30% (v/v) methanol and 10% acetic acid until protein bands became clearly visible.

### 1.6.17. Statistical Analysis

For the statistical analyses, the student *t*-test was used. Results were considered to be statistically significant when *p* < 0.001. The statistical significance of the antifungal treatment was analysed with a Mann-Whitney test (Analyse-it Software).

All experiments were repeated at least three times in duplicate. Each i*n vitro C. albicans* adhesion or biofilm assay was repeated five times in triplicate. *In vivo C. albicans* biofilms were repeated four times, always including two animals per strain. During *C. albicans* biofilm susceptibility determination, each concentration of the drug was tested in quadruplicate. All experimental procedures including TR-146 epithelial cells were performed in duplicate on five times separate occasions. FACS analyses and the determination of *ALS3* antibody binding were repeated five times.

### 2. Anti-bacterial applications

### 2.1 Introduction

The emerging antibiotic resistance is an inevitable evolutionary process. Up to now about 90% of bacteria which cause serious infections are resistant to most of the available antibiotics. The majority of new antibiotics are derivatives of the previous compounds or compounds that belong to the well-known classes. Therefore there is a need for antibacterials with novel mechanisms of action.

In the present example we have designed a library of interferor peptides based on aggregation-prone sequences present in target proteins of the publicly available genomes of *Staphylococcus epidermidis* and *Staphylococcus aureus* (MRSA strain). These genomes were selected based on the clinical relevance of these bacterial strains; especially in a context of hospital acquired diseases. In the present example we have shown that the designed interferors have strong *in vitro* and *in vivo* antibacterial activity against a broad range of Gram positive bacteria, including methicilin-resistant *Staphylococcus aureus* (MRSA) and vancomycin-resistant enterococci (VRE) and also gram negative bacteria. Our results convincingly show that aggregation can serve as a successful antimicrobial strategy. This is an important breakthrough for the treatment of nosocomial diseases, especially in the light of emerging multi-drug and pan-resistant strains.

### 2.2 Primary screening of interferor peptide library, determination of minimum inhibitory concentration (MIC)

A total of 50 different interferor peptides were designed based on the occurrence of aggregation prone regions present in multiple proteins encoded by the genomes of *Staphylococcus epidermidis* and *Staphylococcus aureus.* Because of the design of these interferor peptides it is likely that some of these regions are also present in proteins of other (distant) bacterial species. In the screening effort a two-step procedure was followed: 1) initial screening of all peptides at higher concentrations (75-300 µg/ml) followed by 2) a screening at lower concentrations (<75µg/ml) for a selected set of compounds with a demonstrated activity in step 1). Bacteria were grown in a shaking incubator at 37°C and 160rpm in 50 ml BHI, using individual colonies retrieved from a fresh overnight TSA-sheep blood plate. The cultures were grown to a density of approximately 1x10⁸ cells/ml and then diluted to 5x10⁵ cells/ml in CAMHB (Mc Farland 0.5). Each well contained 100µl (50µl of peptide containing MHB plus 50 µl inoculum). The final cell density was 1x10⁵ to 5x10⁵/ml. After addition of the cell suspension, plates were incubated at 37°C for 18 to 24 hours. The optical density at 590nm (OD₅₉₀) of each well was measured after 5 seconds of shaking the plate using Perkin Elmer spectrophotometer (1420 Multilabel Counter Vicotr 3). The MIC value was read as the minimum concentration that was needed to fully inhibit the growth of bacteria in a well. Each well, where no growth was observed, was also plated on TSA-sheep blood agar plates, incubated at 37°C overnight and visually inspected.

This yielded several molecules with high activity against Staphylococcus and other bacterial species (see Table 4). In addition to the compounds shown in the table, which were primarily designed to be active against *S. aureus,* two molecules worth mentioning and which were designed to be active against *S. epidermidis* are C29 (sequence: RLFNFLKRGSRLFNFLKR (SEQ ID NO: 32), i.e. identical to Hit11 in Table 4) and C30 (RILLGLIRRGSRILLGLIRR (SEQ ID NO: 115)).

**Table 4: Summary of the in vitro potency of peptides designed against S.aureus. Results shown are for peptides synthesized on microscale format.**

| **.Peptide name** | **Sequence** | **MIC values (in µg/ml) against different species** | | |
|---|---|---|---|---|
| | | ***S.aureus* ATCC 29213** | ***S.epidermidis* ATCC 12228** | ***B.cereus* ATCC** |
| Hit50 | RFFIALSRRGSRVQAYLYRR (SEQ ID NO: 30) | 50 | 25 | >100 |
| Hit1 | RWVSMLLRRGSRWVSMLLRR (SEQ ID NO: 31) | 100 | 50 | 6 |
| C29 / Hit11 | RLFNFLKRGSRLFNFLKR (SEQ ID NO: 32) | 12.5-25 | 12.5 | 6-12.5 |
| Hit14 | RRWVSMLLRRGSRWVSMLLRR (SEQ ID NO: 33) | 25 | 50 | 100 |
| Hit57A | RFFIGLSRRGSRLFNFLKR (SEQ ID NO: 34) | 100 | 25 | Not tested |
| Hit50A | RFFIGLSRRGSRIQAYLYRR (SEQ ID NO: 78) | 100 | 12.5 | 50 |
| Hit37 | RWVSMLLRRGSRVGYVIARR (SEQ ID NO: 114) | 100 | 50 | >100 |

### 2.2.1. Spectrum of the antibacterial compounds

In Table 4 the MIC activity (in µg/ml) is shown of six different interferor peptides which have been selected after the first initial screen. The structure of these molecules corresponds to the formula outlined in the application where n is two, X₁ is 1 amino acid (Hit50, Hit1, Hit11, Hit57A, Hit50A) or 2 amino acids (Hit14), Y₁ is 5 (Hit11) or 6 amino acids (the others), X₂ is two amino acids, Z₁ is a two amino acid linker, X₃ is 1 amino acid, Y₂ is 5 (Hit11 and Hit 57A) or 6 amino acids, X₄ is two amino acids and Z₂ is absent. Note that Y1 and Y2 can be identical or different. Of note, in the Y moieties with a sequence of 5 amino acids, the flanking K residue is also present in *S. aureus* proteins, so the sequences are identical over 6 amino acids rather than 5.
These interferor peptides were originally designed to target specifically the *Staphylococcus aureus* MRSA strain. Due to the presence of the same aggregation prone sequence (i.e. TANGO sequence) in genomes of other Gram-positive bacteria there was also an activity of the interferor peptides towards other bacterial species. Peptide interferor Hit1 shows to be a broad-spectrum interferor. Hit14 seems more specific for the bacterial strain it was designed for (i.e. *Staphylococcus aureus* MRSA). Hit11, although effective against both Staphylococcus species, is most active against S. epidermidis, which is in agreement with the fact that the exact sequence of the Y regions is more often present in the S. epidermidis genome. The MIC value (or Minimal Inhibitory Concentration) is the lowest concentration of an antibacterial that will inhibit the visible growth of the bacterium after incubation. Minimum bactericidal concentration (MBC) was considered as the lowest concentration of peptide which prevented growth and reduced the inoculum by a 99.90% within 24 h. MBCs were established by plating the content of each well on a blood agar plate for viability check. Additionally the content of the well was also used as an inoculum for a new microwell plate (in which no peptide was added) and progressive change of turbidity was noted for another 24hrs. MBC values were either the same or 2-fold higher than the MIC values which suggests that these are bactericidal agents.

In a next step peptides that maintained a high activity (low MIC) over repeated screens were reordered in highly purified, scaled-up form and subjected to further testing on several Gram-positive and Gram-negative bacteria. Table 5 depicts the MIC-values of compound 30 (C30) for a variety of bacterial species. As can be seen in table 5 compound 30 is not only very effective against *Staphylococcus epidermidis,* in which genome the aggregation-inducing sequence is encoded, which sequence was used to design compound 30, but also against other clinically significant pathogens such as methicillin resistant (MRSA) *S. aureus* and vancomycin resistant enterococci (VRE), nosocomial pathogen *Enterococcus faecalis,* a group of foodborne pathogens: *Bacillus subtilis, B.cereus, Listeria monocytogenes.* It is very promising to see that the activity against antibiotic-resistant strains is just as high as against antibiotic-sensitive strains. This is indicative of the complete novel mechanism of action of these antimicrobial compounds. As antibiotic resistance is a major problem in hospitals, completely different molecules offer great perspective in clinical applications.

We recently found that C30, like some of the compounds shown in Table 4, is also active against the genus *Corynebacterium* (not shown in Table 5). The activity against *Corynebacteriae* is promising in the light of new anti-tuberculosis peptide-design, since the cell wall composition of *Corynebacterium* mimics the one of *Mycobacteriae.* The cell wall structure of *Mycobacterium tuberculosis* deserves special attention because it is unique among prokaryotes, and it is a major determinant of virulence for the bacterium. The cell wall complex contains peptidoglycan, but otherwise it is composed of complex lipids. Over 60% of the mycobacterial cell wall is lipid. The lipid fraction of MTB's cell wall consists of three major components, mycolic acids, cord factor, and wax-D. The low permeability of the mycobacterial cell wall, with its unusual structure, is now known to be a major factor in this resistance. Thus hydrophilic agents cross the cell wall slowly because the myobacterial porin is inefficient in allowing the permeation of solutes and exists in low concentration. Lipophilic agents are presumably slowed down by the lipid bilayer which is of unusually low fluidity and abnormal thickness.

The higher MIC value of C30 for gram-negative bacteria may reflect the fact that the structure of C30 is a tandem interferor peptide (i.e. a peptide with two identical aggregation-inducing stretches (2 identical Y moieties)). Recent evidence has shown that single interferor peptides also display low MIC values for Gram-negative bacteria. Without limiting the invention to a particular mechanism of action this may point to the fact that Gram-negative bacteria have smaller pores in their bacterial cell wall which would limit the entrance of tandem interferors as compared to single interferor peptides. Note however also that there are several proteins containing the aggregation-inducing stretches, and these groups of proteins are not identical in Gram-positive and Gram-negative bacteria. Thus, it may be that one or more different target proteins is/are aggregated in Gram-positive bacteria than in Gram-negative ones.

### Results planktonic bacteria : MIC values for compound 30

**Table 5: MIC values for compound 30 (C30) to a wide range of Gram-positive and Gram-negative species. In addition, the table also includes 7 MRSA and 7 VRE strains. Read-out for MIC values was done after 18 hours of growth.**

| • Gram-negatives | • Gram-positives |
|---|---|
| • *E. coli* ATCC25922 : 100 µg/ml | |
| • *Ps. aeruginosa* ATCC27853 : > 100 µg/ml | • *S. epidermidis* ATCC 13228: 1,5 µg/ml |
| | • S101: 1,5 µg/ml |
| • Gram-positives | • S103: 1,5 µg/ml |
| • *S. aureus* ATCC29213 : 3 µg/ml | • S104: 1,5 µg/ml |
| • MRSA 204: 6 µg/ml | • S109: 3 µg/ml |
| • MRSA 418: 6 µg/ml | • *S. capitis :* 1,5µg/ml |
| • MRSA 274: 3 µg/ml | • *S.hominis:*3 µg/ml |
| • MRSA 165: 3 µg/ml | • *S.haemolyticus:*1,5 µg/ml |
| • MRSA 351: 3 µg/ml | • *Nocardiaasteroides* ATCC 3308: 1,5 µg/ml |
| • MRSA 115: 6 µg/ml | • *Micrococcusluteus* ATCC 9341: 3 µg/ml |
| • MRSA 651: 3 µg/ml | • *Listeriamonocytogenes* ATCC 11994: 1,5 µg/ml |
| • *E. faecalis* ATCC 19433: 6 µg/m l | |
| • VRE 8 : 3 µg/ml | • *Bacillussubtilis* ATCC 6051:1,5 µg/ml |
| • VRE 11: 1,5 µg/ml | • *Bacillussubtilis* IP 5832: 6 µg/ml |
| • VRE 12: 12,5 µg/ml | • *Bacilluscereus* 1: 6 µg/ml |
| • VRE 40: 3 µg/ml | • *Bacilluscereus* 2: 6 µg/ml |
| • VRE 60: 3 µg/ml | |
| • VRE 70: 1,5 µg/ml | |
| • VRE 54: 3 µg/ml | |

### 2.2.2. Effect of purity and modifications on peptide activity

We have observed a significant (up to 5-fold) improvement of the interferor peptides activity when synthesized in a highly pure form. The result of this improved activity is shown in Table 6. Importantly this improvement of activity does not increase the toxicity towards mammalian cells (data not shown). In addition, we have found that interferor peptides tagged with Biotin were as active as non-tagged interferor peptides. The latter means that different detection methods can be used.

**Table 6: Summary of the in vitro minimum inhibitory concentration (MIC) against different bacteria of peptides with purity of >95% (HPLC-220nm-C18-linear gradient) designed against S.aureus MRSA.**

| **Peptide name** | **Sequence** | **MIC values (µg/ml) against different species** | | | |
|---|---|---|---|---|---|
| | | ***S.*aureus ATCC 29213** | **MRSA 326** | **S.epidermidis ATCC 12228** | **Corynebacterium** |
| Hit50 | RFFIALSRRGSRVQAYLYRR (SEQ ID NO: 30) | 25 | 50 | 3 | 12.5 |
| Hit1 | RWVSMLLRRGSRWVSMLLRR (SEQ ID NO: 31) | 3 | 6 | 1.5 | 3 |
| C29/ Hit11 | RLFNFLKRGSRLFNFLKR (SEQ ID NO: 32) | 12.5-25 | 12.5 | 0.6-3 | 6 |
| Hit14 | RRWVSMLLRRGSRWVSMLLRR (SEQ ID NO: 33) | 12.5 | Not tested | 50 | Not tested |
| Hit57A | RFFIGLSRRGSRLFNFLKR (SEQ ID NO: 34) | 50 | 6 | 3-6 | Not active |
| Hit50A | RFFIGLSRRGSRIQAYLYRR (SEQ ID NO: 78) | 100 | 50 | 6 | Not tested |
| Hit37 | RWVSMLLRRGSRVGYVIARR (SEQ ID NO: 114) | 100 | 100 | 6 | Not tested |

Not shown in the table is Hit24. The sequence of Hit24 (i.e. RRLFNFLKRGSRLFNFLKR (SEQ ID NO: 74)) is a modified sequence of Hit 11 (see Table 4) wherein Hit24 has a double gatekeeper in front. Although it was not tested against all bacteria, it has considerable activity against S.aureus ATCC 29213 (MIC value 12.5 µg/ml), S.epidermidis ATCC 12228 (MIC 1.5 µg/ml) and the Gram-negative E. cloacae (25 µg/ml).

Apart from biotinylation, several other modifications of the interferors were tested. These were based on C30 and include a D-amino acid version of C30.

Another modification that was tested is PEGylation of peptide C30. The covalent attachment of PEG in different position of the peptide C30 was tested. It was used both internally (i.e. as the Zi linker moiety) and as a N-terminal moiety. The aim of PEG introduction mainly was to provide better compound stability in vivo as well as better solubility.

The following compounds, based on the C30 sequence (SEQ ID NO: 115), have been synthesized:
P2170: Ac-RILLGLIRRGSRILLGLIRR-CONH2, an acetylated and amidated version of C30.
P2175: NH2-RILLGLIRR(Peg)₂RILLGLIRR-CONH2, an amidated version of C30 wherein the Z₁ linker between the aggregating regions consists of two PEG units (i.e. two ethylene oxide units linked by an ether bond) instead of the GS amino acid sequence.
P2151: NH2-RILLGLIRRGSRILLGLIRR-OH, normal C30 but prepared using different chemical peptide synthesis.
P2153: NH2-(Peg)₂RILLGLIRRGSRILLGLIRR-OH, C30 which is N-terminally fused to two PEG units. This can also be phrased as C30 with an additional Z₀ moiety that consists of two PEG units.
P2154: NH2-Peg3RILLGLIRRGSRILLGLIRR-OH, C30 which is N-terminally fused to three PEG units. This can also be phrased as C30 with an additional Z₀ moiety that consists of three PEG units.
DAA: a D-amino acid version of C30 wherein all amino acids are D amino acids.

These peptides were tested the same way as for the original screens. A microbroth dilution method was used with twofold serial dilutions in CAMHB (according to EMEA guidelines) was used for screening of peptide activity, with concentrations ranging from 03µg/ml to 200µg/ml.

Results are shown in Table 7. For P2170 and P2175, two fractions were tested corresponding to two peaks obtained during peptide synthesis. These are indicated as P1 and P2, respectively.

**Table 7. MIC values for selected bacterial strains for modified C30 peptides.**

| **Bacteria type** | **Minimum inhibitory concentrations MIC₉₀(µg/ml)** | | | | | | | |
|---|---|---|---|---|---|---|---|---|
| | **P2170/P1** | **P2170/P2** | **P2175/P1** | **P2175/P2** | **DAA** | **P2151** | **P2153** | **P2154** |
| S.aureus ATCC | 12.5 | 12.5 | 100 | 100 | 25 | 50 | 25 | 25 |
| S.epidermidis ATCC12228 | 6 | 12.5 | 12.5 | 6 | 1.5 | 12.5 | 12.5 | 12.5 |
| B.cereus | 6 | 6 | 12.5 | 12.5 | 6 | 12.5 | 6 | 12.5 |
| MRSA 326 | 12.5 | 12.5 | 25 | 25 | 6 | 12.5 | 12.5 | 12.5 |
| E.coli ATCC | 25 | 25 | 25 | 50 | 25 | 25 | 25 | 50 |
| Corynebacterium | >200 | 100 | >200 | >200 | >200 | | | |
| E.faecalis ATCC 1943 | 12.5 | 12.5 | 12.5 | 6 | 6 | 25 | 25 | 12.5 |
| Klebsiella pneumoniae | >200 | >200 | 50 | 100 | 25 | 25 | 50 | 100 |
| Nocardia asteroides 3908 | 6 | 3 | 3 | 6 | >200 | 12.5 | 6 | 6 |
| S.hominis DSM20328 | 3 | 3 | 3 | 1.5 | 1.5 | 3 | 3 | 3 |
| Salmonella typhi ATCC14028 | 100 | 100 | 50 | 25 | 100 | 100 | >200 | >200 |

As can be seen from the table, all modified peptides retain antibacterial activity that is comparable to the non-modified C30. Thus, modifications such as D-amino acids and PEGylation have no negative effect on activity of the molecules presented herein. It is particularly encouraging that MIC values (and thus antibacterial activity) are not affected by PEGylation.

### 2.2.3 Non-aggregating control peptides

To validate the design principle of aggregators we've constructed control peptides, here so called 'scrambled' peptides. These are composed of the same amino acids as the original aggregators (and thus have the same charge), but the order is scrambled so that they no longer comply with the design principles presented herein. These peptides do not result in inhibition of bacterial growth or survival.

### 2.2.4. Targeting more than one bacterial protein

As mentioned, a first screen was based on the identification of aggregation prone regions present in proteins encoded by the genomes of *Staphylococcus epidermidis* and *Staphylococcus aureus.* As a very promising compound, C30, contains aggregation-inducing sequences that are present in more than one protein in these genomes, it was hypothesized that this could be used in the design of new compounds. Thus, in a next step we designed interferor peptides for which more than one predicted target was available in the bacterial cells of *S. epidermidis* and *S*. *aureus* (i.e., containing beta-aggregating sequences that are present in more than one protein encoded by either of these bacteria). This so called 'mtop' interferor peptide screen showed a very high hit rate indicating that the design of anti-bacterial interferor peptides can be based on a number of targets available within the bacterial cell (i.e. the more target proteins that contain a particular TANGO region, the higher the possibility of obtaining bactericidal effect with the interferor. This is logical, since the more proteins are targeted, the higher the chance that inhibiting one or more of them inhibits an essential function. Table 8 shows the MIC-activity of bispecific interferor peptides that were designed on the basis of encountering more than one target protein in the bacteria. It is important to mention that the interferors used in Table 5 were synthesized in microscale format; meaning that we expect considerable improvement of interferor activity for the high purity interferor peptides.

The molecules listed in Table 5 correspond to the formula as outlined in the application where n is 2, X₁ and X₄ are two amino acids, Y₁ and Y₂ are 6 amino acids, X₂ and X₃ are 1 amino acid, Z₁ is a two amino acid linker and Z₂ is absent.

**Table 8: MIC values of several multi-target interferor peptides**

| **Name** | **Sequence** | **Bacterial strain** | **MIC value (µg/ml)** |
|---|---|---|---|
| mtop_1 | RRIILFILRPPRLILFLGRR (SEQ ID NO: 35) | *S.epidermidis* | 50 |
| | | *S.aureus* | 200 |
| mtop_4 | RRIILSLIRPPRLLGVVLRR (SEQ ID NO: 36) | *S.epidermidis* | 3 |
| | | *S.aureus* | 100 |
| mtop_5 | RRVLSLILRPPRIALLGLRR (SEQ ID NO: 37) | *S.epidermidis* | 25 |
| | | *S.aureus* | 100 |
| mtop_6 | RRIALLLIRPPRLLAIAVRR (SEQ ID NO: 38) | *S.epidermidis* | 50 |
| | | *S.aureus* | >200 |
| mtop_11 | RRILLGLIRPPRTIIGLVRR (SEQ ID NO: 39) | *S.epidermidis* | 12.5 |
| | | *S.aureus* | >200 |
| mtop_12 | RRILLLIARPPRILLGAIRR (SEQ ID NO: 40) | *S.epidermidis* | 12.5 |
| | | *S.aureus* | >200 |
| mtop_17 | RRLLGLIIRPPRAIALTLRR (SEQ ID NO: 41) | *S.epidermidis* | 100 |
| | | *S.aureus* | 50 |
| mtop_18 | RRILGLIARPPRIAFVILRR (SEQ ID NO: 42) | *S.epidermidis* | 50 |
| | | *S.aureus* | >200 |
| mtop_22 | RRIIGIIARPPRVLVTLLRR (SEQ ID NO: 43) | *S.epidermidis* | 50 |
| | | *S.aureus* | >200 |

### 2.3 Antimicrobial killing kinetics of the interferor molecules

Time-kill curves were obtained to study the kinetics of bactericidal activity. Compounds C30, Hit50 and C29 (for sequences see above) were tested against *S. epidermidis* strain ATCC 12228 and *S. aureus* ATCC at the compound's MIC and two times MIC value. It was shown that compound C30 decreased the viable counts by 100% within 5 min, while Hit50 reduced the viable counts by ^{∼}3 log units after the same incubation period, both at a concentration that equaled its MIC value (see Figure 11). All peptides showed a concentration-dependent activity, since doubling the concentration to twice the MIC value resulted in faster killing. For example the time needed to decrease the number of CFU/ml one-fold for C29 was 60 minutes (at the minimum inhibitory concentration), and only 10 minutes for 2xMIC. This is an important effect, as antibiotics that have a faster bactericidal effect are generally believed to be more efficient and resistance to such compounds develops slower.

### 2.4 Monitoring of bacterial resistance development against anti-bacterial interferors

The ability of the *S. aureus* clinical strain MRSA 204 to develop resistance to interferor C30 was evaluated by repeated passaging and MIC determination. *S. aureus* cells growing in the presence of C30 at half the MIC on one day were used the day after in a MIC assay of that same compound. In this way, bacterial cells were continually exposed to a single compound (i.e. C30) at half the MIC-value while being passaged over 15 days. It could be demonstrated that continuous exposure to interferor peptide C30 only resulted in a minor tendency to develop resistance since ten passages on one half of the initial MIC were needed to elevate the MIC two-fold. Nine passages at sub-MIC were needed before MIC were 2-fold elevated, however this resistance did not succeed to be maintained over time and after 12 passages the MIC went back to its original value. See Fig. 15. Similar results were obtained with other of the above listed peptides. When resistance was monitored over a longer period of time (31 instead of 15 days), no further increase in the MIC value was observed (see further).

In contrast the MIC of rifampin gets significantly increased over the same period of time (as much as 512-fold over 15 days)-rifampin is an agent to which resistance is known to arise quite easily by spontaneous chromosomal point mutations. Similarly to the tested peptides, the MIC of vancomycin under these conditions only increased two-fold; vancomycin is generally regarded as an antibiotic to which spontaneous resistance development is unlikely to occur under conditions where horizontal genetic transfer between species is excluded. It is worth mentioning the compounds target hydrophobic regions buried inside proteins. It is believed that these regions are generally well conserved and cannot easily be mutated, as this would give rise to misfolded (non-functional) proteins. Without being bound to a particular mechanism, this may contribute to the slow resistance development observed.

Because resistance development is a stochastic process, we've repeated the previous experiment using the same broth microdilution method (always 4 duplicates), and also adding common antibiotics like Ampicillin and Gentamycin. It can be seen that both C30 and C29 show a much slower onset of resistance than Ampicillin, and that MIC values increase considerably less than those of gentamycin and ampicillin (Fig. 12). Note that, as the initial MIC values of gentamycin is lower than that of the compounds, the fold increase is even much more pronounced than that shown in the figure.

A second series of experiments consisted of inoculating 2ml BHI broth supplemented with defined concentrations of the peptide C30 with an M RSA strain 326. Each day the MIC was tested as previously described. The concentration of the peptide in medium was adjusted according to the MIC from the previous day, so that the growth media supported selection of resistance. This was repeated 3 times for 31 days. The MIC for C30 showed similar fluctuations as during the previous experiment, and never increased more than 4-fold. On the last day of the experiment the MIC was 12,5µg/ml. In comparison, the MIC level for Ampicilin treated cells fluctuated highly, increasing up to 7 fold (MIC at the end of experiment= 100µg/ml). Increasing Ampicillin concentrations during overnight incubations led to selection of resistant bacteria and a further increase in MIC level, whilst increase of C30 concentration did not speed up the resistance development. Results are shown in Fig. 13.

Interestingly withdrawal of the C30 from the culture caused either 50% reduction of the MIC level or did not have any effect on strain susceptibility.

### 2.5 Membrane permeability caused by antibacterial interferors

The effect of peptide interferors on the membranes of living microbial cells was studied with the membrane impermeant DNA-binding dye Sytox Green (Invitrogen). Membrane permeabilization allows entry of the dye which is monitored by an increase in fluorescence. Sytox Green nucleic acid stain was used to monitor bacterial membrane permeability in time (at the constant interferor peptide concentration of 25µg/ml (see Figure 18) and at various concentrations of peptides (see Figure 19) at a constant time of 15 minutes). We observed a sharp increase in Sytox Green binding in the first 5 minutes which was stabilized after this time point, with only a minor further increase after 5 minutes. This further minor increase could be attributed to the rapid insertion of the amphiphilic interferor peptides into the bacterial lipid bilayer, which induces local defects in lipid packing and causes further enhanced permeability. Compared to the lytic control agent (lysostaphin), which lyses *Staphylococcus* very efficiently, the Sytox Green signal was slightly lower for the bacteria treated with antibacterial interferors. Figure 11 shows that the killing curve of *S. aureus* treated with compound 30 at 3µg/ml (which is approximately its MIC-value) was able to destruct these bacteria very rapidly (since killing was concentration dependent); therefore an equally fast rise in Sytox Green binding would be expected to occur if membrane-lysis is the mechanism of action. This is not the case, however. To support the hypothesis that the membrane lysis is not a primary cause of cell death, we've progressed with proteomic approaches to verify the intracellular target that is involved in primary peptide-target interaction, as well as with electron microscopy analysis to show intact cells.

In a next step we studied the membrane potential (MP) change for peptide interferor C30. Thereto the BacLight kit (Invitrogen) was used. Figure 20 shows that in bacterial cells treated with compound C30 there was a significant decrease in red fluorescence after only 5 minutes, displaying an equal red to green ratio which indicates a full depolarization of the MP. In comparison, the lytic control (lysostaphin) showed a much slower membrane depolarization. The dot plot pattern of C30-treated cells is very similar to the depolarized control. When combining these data we can assume that after 5 minutes of C30 treatment there is a partial collapse in the MP leading to an influx of the Sytox Green nucleic acid stain, which indicates to some extent a membrane permeabilization. Depolarization caused by these interferors showed to be rather time-dependent than concentration-dependent (data not shown). These results further prove the rapid bactericidal mechanism of action of the antibacterial interferors.

### 2.6 Detection of aggregates in bacteria

Aggregates were visualized in antibacterial interferor treated bacteria with two different amyloid diagnostic dyes (thioflavin-T and Congo red). Thioflavin-T fluorescence has been described as a specific marker for the extended sheet conformation of beta-aggregated structures. When this dye binds to amyloid fibrils there is a large enhancement in the fluorescence of Th-T relative to free dye. Th-T was used in the bacterial assays to investigate whether peptide interferors induce aggregation within the bacterial cells. As a control we used two-fold dilutions of the peptide interferor alone in physiological water, to be sure that external-self aggregating interferor peptides do not give a false signal. We clearly observed a significant concentration-dependent increase in Th-T dye binding in bacterial cells treated with interferor peptides (see Figure 21), indicating the presence of more β-sheet structures in bacterial cells treated with interferor peptides. In addition, we also showed that Congo red (CR) binds to aggregated beta structures (see Figure 22) and this binding induces a characteristic shift in CR maximal optical absorbance from 490 nm to 540 nm. This experiment confirms the Th-T concentration dependent binding experiment, independently confirming the increase in β-structure formation during the peptide treatment.

### 2.7 Morphological changes induced by interferors in bacteria

Scanning electron microscopy (SEM) and transmission electron microscopy (TEM) were used to examine the ultra structural changes in bacteria induced by interferor peptides. Both Staphylococci and Bacilli were used for a comparison. It could be observed that both *Bacillus cereus* and *Staphylococcus aureus* displayed a smooth and intact surface after 5 minutes of treatment with antibacterial interferor C30, showing no obvious craters, holes nor pores in their envelope. However after about 20 minutes the Bacilli surface started to wrinkle and shrink after the treatment which wasn't apparent in Staphylococci probably due to its small, round size. In both bacterial species cell content started to be released, in Bacillus already after 20 minutes (see Figure 23, right panel, bottom) of treatment and in *S. aureus* as long as after 1 hour (see Figure 24). During healthy cell division *Bacillus cereus* forms very long chains, which are non-separated from mother cells. However it was recorded that in bacterial cell populations of *Bacillus cereus* treated with peptide C30 these long chains are less common indicating a hampered cell division. A similar conclusion could be drawn from the fact that significantly less treated Staphylococci contained division septa as compared to untreated, healthy bacterial cells. Finally we can conclude that the SEM data depict a lack of rapid lysis. Furthermore, knowing that compound 30 needs as short as 5-10 minutes to kill Staphylococci we would expect a very fast rupture of bacterial cells which was clearly not the case even after one hour of treatment with the antibacterial interferor. To further support our hypothesis, we also looked at ultrathin sections of C30 treated staphylococci. Transmission electron microscopy of these treated cells also confirms a lack of lysis, although there is an obvious shrinkage of cytoplasm (see figure 25 and 26). Additionally there is an increase in electron density in a region of nucleic acid, suggesting its condensation. DNA condensation is one of the later stages of apoptotic responses. The apoptotic cell death could be supported by our two-dimensional gel analysis, which revealed 3-fold increase in expression of MarR family transcriptional regulator, involved in autolytic activity. Transmission electron microscopic analysis of the ulthrathin sections of *Staphylococcus aureus* treated with compound 30 showed membranous structures (see the arrow in figure 25) which were previously described as mesosomes.

### Immunoelectron microscopy analysis

The localization of immunolabelled aggregator was studied using transmission electron microscopy. For this purpose peptide C30 was labeled with 5(6)Carboxyfluorescein on its N-terminus (FITC tag).

Bacteria of the exponential growth phase were treated with FITC-tagged peptide (2xMICs value) for 30 minutes followed by fixing with double strenght fixative (4% paraformaldehyde+ 0.4% gluteraldehyde in 0.1M P-buffer,pH=7.4)for 10 minutes and single strenght fixative (half of above) for 1 hour. After several washing steps (P-buffer and P-buffer/glycin)pellet was suspended in 12%gelatin/p-buffer, incubated on ice, cut up in small cubes and set to incubate overnight in 2.3M Sucrose. Samples were mounted on specimen holders, frozen in liquid nitrogen, and sectioned with a diamond knife at-100°C with an ultracut S/FCScryoultyramicrotome (Leica). Ultrathin thawed sections were placed on Formavar-carbon-coated copper grids(400mesh),floated sections six times for 10minutes each time on drops with glycine-PBS. Grids were then washed in 10mM PBS buffer for 5 minutes, blocked with PBS/BSA (0.1%) and incubated for 30 minutes in a drop of anti-FITC goat primary antibody (Abcam) (diluted 1:1000 in PBS buffer), washed 5 times in PBS buffer, incubated for 30mins with rabbit anti-goat protein-A-gold conjugate (5nm;BBInternational EM Rag5) diluted 1:50in PBS buffer. Section was then washed 6 times for 5 minutes in PBS and 3 times in ddH20. Grids were stained for 5 minutes with uranyl acetate -Methyl cellulose (1%) on ice, dried carefully and observed using JEOL JEM 2100 Transmission electron microscope, operating at accelerating voltage of 80kV.No major non-specific binding of antibodies was detected in the different control procedures, background labeling was a minimal problem and most of the times linked to insufficient washing steps after protein-A-gold incubation, which has been optimized.

After 30 minutes of exposure, FITC-C30 peptides were predominantly present in the bacterial cytoplasm, clustered together in a form of aggregates (Figure 27, red arrows). Because no non-specific binding was observed, immunogold-labelled particles represent the presence of peptides. These results confirm intracellular activity of peptides. Moreover, cells treated with peptide have a clearly disturbed division process. Division of peptide-treated cells is asymmetrical when compared with untreated staphylococci; septa also are much thicker and lost their margin. Interestingly, aggregate clusters could be seen only in one part of the cell, which suggest evolutionary pressure against heritance of aggregates , a process described previously in *E.coli* (Rokney, A., M. Shagan, et al. (2009). "E. coli Transports Aggregated Proteins to the Poles by a Specific and Energy-Dependent Process." Journal of Molecular Biology 392(3): 589-601; Lindner, A. B., R. Madden, et al. (2008). "Asymmetric segregation of protein aggregates is associated with cellular aging and rejuvenation." Proceedings of the National Academy of Sciences 105(8): 3076-3081.)

The morphological analysis and membrane permeability studies of treated bacteria seems to suggest that interferor peptides insert themselves into the bacterial cytoplasmic membrane, leading to a rapid membrane depolarization and loss of its integrity. Without being bound to a particular mechanism, this seems not to be their bactericidal mode of action, but rather a "side effect". The interferor peptides are only active on bacteria that encode in their genome the β-aggregating region also present in the interferor molecule. Interferors are able to induce aggregation of their targets, leading to cell death. Therefore, it is hypothesized that the combination of the intracellular target aggregation and the membrane effect leads to the rapid cell death of the bacteria.

### 2.8 Toxicity of anti-bacterial interferors on mammalian cells

### 2.8.1 Hemolysis assay

To distinguish between selective antimicrobial activity from non-selective lytic activity on eukaryotic cells we measured the lytic abilities of the most active interferor peptides using human erythrocytes. As shown in Figure 14, compounds: C29, Hit57A, Hit 50 and Hit24 exhibited no significant hemolytic activity at clinically relevant concentrations, while peptides C30 and Hit1 had HD₅₀s (concentration at which 50% of red blood cells are lysed) ranging between 25 µg/ml and 50 µg/ml. The detergent Tween was used as a positive control, causing 100% lysis of RBCs. It could be demonstrated that interferor peptides do not show major hemolysis at their MIC values (cf. Table 4) and some interferor peptides are even non-hemolytic at concentrations as high as 100µg/ml.

### 2.8.2 Alamar Blue assay and Lactate Dehydrogenase (LDH)-release assay on mammalian cells

Human embryonic kidney (HEK293T) cells treated with different concentrations of antimicrobial interferor peptide C30 resulted in higher levels of LDH release when compared with the interferor peptide Hit50. Concentration-dependent increase in extracellular LDH was observed, indicating that these peptides caused some loss of plasma membrane integrity. Figure 16 shows that peptide interferor C30 causes an LDH release higher than 50% when used at 50µg/ml or beyond while peptide interferor hit 50 caused only 5% LDH-release at the same concentration (see Table 4).

Alamar blue assay allowed monitoring of the total percentage of cell growth recovery in time in the presence of peptides. For most of peptide interferors (at concentration as high as 100µg/ml) we found that 80-100% of the mammalian cells showed a total growth recovery within 3-24 hours. Figure 17 shows the alamar blue cytotoxicity on human embryonic kidney cells (HEK293T cell line) for two different peptide interferors. Despite the LDH release caused by high concentrations of C30, it can be seen that with concentrations lower than 100 µg/ml, viability of mammalian cells is not significantly affected by administration of this compound. The data indicate the specificity of the antibacterial peptide interferors for bacteria with only a minimal effect on mammalian cells.

### 2.8.3 Invasion assay

In this assay HCT116 cell monolayers (a human colon tumor cell line) were cultured at the bottom of a microwell plate. The next day fresh *S.aureus* ATCC 27853 bacteria were added (approximately 10⁶ CFU/ml) and a negative uninfected control included. After 90 minutes of infection different dilution of peptides were added and the cultures were incubated for another hour. As a positive control Gentamycin was used, given that it has a good intracellular activity. Each well was washed with prewarmed physiological water to get rid of any extra-cellular bacteria, followed by 1% Triton treatment to release all entrapped bacteria from mammalian cells. The content of the well was serially diluted and plated on TSA agar plates for CFU count. The results of this assay are shown in Fig. 28.

Of note, peptide concentration can be further decreased to as low as 12.5 µg/ml while maintaining similar antibacterial activity. This is interesting to avoid toxic side effects that possibly arise with high concentrations of the peptides. Either way, these results show that aggregator peptides are tolerated by mammalian cells at concentrations relevant to the bactericidal effect and can target *S. aureus* residing in cultured Human Colon Tumor (HCT-116) cells (i.e. they are taken up by the cells and show a bactericidal effect).

### 2.9 Proteomic analysis of targeted proteins

The aggregation technology is based on the assumption that short amino acid stretches, with a high propensity of aggregation (e.g. assessed by the TANGO score) and that are derived from a target protein, can induce aggregation of that protein. This hypothesis has been confirmed by a shotgun proteomic analysis of the insoluble fraction. If we assume that our aggregator peptides work through specific interaction with its target and causes its aggregation, it is expected that the target enters the insoluble fraction. For this reason we've decided to split the lysate into 2 fractions: insoluble and soluble and compare these with untreated cell fractions. Proteins present in both treated (TC30) and untreated (NT) insoluble fractions were assumed to present a background of non-specific naturally non-soluble proteins. Additionally we've examined soluble fractions of both treated and untreated bacteria and as expected the protein of interest was present only in the untreated fraction. This means that the following criteria for target search were used:
1) potential aggregation targets are proteins obtained from the list of proteins in the Insoluble fraction of C30 treated cells minus proteins in the Insoluble fraction of non-treated cells
2) the proteins in the insoluble fraction of C30 treated cells minus proteins in the soluble fraction of C30 treated cells are potential targets only if absent in the insoluble NT fraction, AND if present in the soluble NT fraction, and only when it contains the tango sequence or part thereof within its amino acid sequence.

By the SOSPA (sawn-off shotgun proteomic analysis) method we can confirm the target protein for each of the aggregators. This way we've confirmed so far the target for compound 30 in two different bacterial strains: S.aureus and B.cereus.The insoluble fractions of both treated species contained the in silico predicted protein; i.e. the negative regulator of genetic competence ClpC/mecB which in fact contains the Tango stretch within its FASTA sequence (Uniprot accession number: Q63HB8). As expected, the target protein of C30 shifted from soluble into insoluble fraction upon peptide treatment.

Additionally we've blotted different fractions and used these blots for target detection with specific recognition peptides. Again we could confirm the presence of the target protein, ClpC/MecB (90kDa) in the insoluble fraction of C30-treated Bacillus cereus (fig 29).

Altogether these results prove our hypothesis that aggregators possess the ability to interact with bacterial membranes (interfacial activity), they penetrate the cytoplasm of bacteria and if the target has the accessible tango region, the aggregator binds to it, aggregating it and thus seeding a further reaction cascade. As already mentioned in the literature, aggregates cause lipid rearrangements and membrane permeablization. Extensive electron microscopy analysis proves that aggregators act on the membrane from inside the cell and not from the outside. We conclude that aggregators act on both cytoplasmic target protein and in the later stage, the membrane itself, leading to a rapid cell death.

The data obtained by SOSPA could also be confirmed using two-dimension gel electrophoresis (data not shown).

### 2.10 Serum stability of antibacterial interferors and detection in serum

To assess interferor activity in the presence of serum, MIC values for *S. aureus* were established (as described above) for several antibacterial peptide interferors in a medium containing 50% fetal bovine serum. As expected Table 9 shows that the MIC values were somewhat higher in the presence of serum but importantly the interferor peptides retained their antimicrobial activity indicating a possible *in vivo* use of the antibacterial interferor peptides.

**Table 9: MIC values for several antibacterial interferors with and without 50% serum.**

| **Peptide interferor name** | **MIC** | **MIC in presence of 50% FBS** |
|---|---|---|
| **C30** | 6µg/ml | 50µg/ml |
| **Hit1** | 3µg/ml | 25µg/ml |
| **Hit50** | 25µg/ml | 100µg/ml |
| **Hit57A** | 50µg/ml | 100µg/ml |

To detect the peptides in serum, a dot-blot assay was used. The peptides were also diluted in PBS, blotted and used as a quantification guide. Starting from 100µg/ml dots were spotted on a nitrocellulose membrane following 2-fold dilutions of peptide.

Since peptide was biotin labeled, Streptavidin-HRP was used for probing. There was a linear response suggesting that this method is successful for serum detection in a range between 300µg/ml and 6µg/ml (Figure 30). For more information on detection of peptides, see Example 4.

### 2.11 Preliminary assessment of in vivo toxicity of antibacterial interferors in rats and mice

In order to assess the toxicity of the antibacterial peptide interferors *in vivo,* eight germ-free fisher male and female rats, weighing approximately ±350 grams were injected with peptide interferor C30 in buffer A: histidine-acetate(pH 6.5) and buffer B phosphate buffer (pH 6.5). Two rats were injected with a concentration of 1.5mg/kg and two with a concentration of 3mg/kg. These were single non repeatable injections into a tail vein. Rats were observed over several days but no mortality nor any other side effects were apparently visible.

Further, three groups of 6-weeks old female Swiss mice were injected with increasing C30 doses over a period of 1 week. C30 was dissolved in physiological water and 1 injection per day was given. In the first 3 days injections in the tail vein were given (150µl per injection) and during the remaining 2 days IP injections (300µl per injection). The primary dose started from 3mg/kg and each day the dose was increased gradually. The control group received the same volume of saline. The highest dose analyzed for IV injections was 50mg/kg and for the IP injections 100mg/kg. IV injected mice showed no signs of mortality nor any other visible side effects. IP injected mouse however developed a subcutaneous lesion bump after the highest dose of 100mg/kg, suggesting that the peptide solution was too dense and formed insoluble aggregates. Organs of treated and control mice were subjected to further histological analysis. After all 5 injections were given, blood was taken by retro-orbital puncture and analyzed. No abnormalities were found in the blood.

### 2.12 In vivo efficacy of compound C30

### Animals:

Six-week-old, specific-pathogen-free, NIH Swiss female mice (Harlan Sprague-Dawley, Indianapolis, IN) weighing 21 to 24 g were used for all studies. All experimental procedures were approved by the local Ethical Committee of Animal Experiments.

### Neutropenic-mouse thigh model of S.aureus MRSA 326 strain.

Mice were rendered neutropenic by injecting cyclophosphamide (Sigma) intraperitoneally 4 days (150 mg/kg of body weight) and 1 day (100 mg/kg) before experimental infection. Previous studies have shown that this regimen produces neutropenia in this model for 5 days. Broth cultures of freshly plated S.aureus MRSA 326 were grown overnight in Mueller-Hinton broth (MHB). After a 1:4,000 dilution into PBS, bacterial counts of the inoculum ranged between 10⁶ and 2 x 10⁶ CFU/ml. Mice were anesthetized briefly with approximately 4% isoflurane just prior to inoculation. The bacterial suspension (0.05 ml) was injected intramuscularly into each thigh (approximately 5x10⁴ CFU). Treatment was initiated 2 h following bacterial inoculation through an i.v. (intravenous) or i.p. (intraperitoneal) route. Four groups of 3 animals per group were used in this study:
- group A received buffer only and was sacrificed 4hrs post infection,
- group B has been treated i.v. with 15mg/kg of C30 (total volume of 150ul) and was sacrificed 4 hours post-infection,
- group C received two i.p. injections of 30mg/kg after 2 hours and 4 hours post-infection and was sacrificed 6 hours post-infection.
- group D was injected i.v with 15mg/kg of vancomycin and sacrificed 4 hours post-infection.

At various time points following treatment, groups of three mice were humanely sacrificed by CO₂ asphyxiation. The thigh muscle mass was homogenized and decimally diluted in iced PBS, and 10 µl aliquots of five serial dilutions were plated on blood agar (in 3 independent dilution repeats per thigh homogenate). Following overnight incubation at 37°C, CFU were enumerated for each thigh and expressed as the log₁₀ CFU/thigh.

As shown in Fig. 31, compound C30 showed to reduce expansion of initial inoculum by 6.9 Log₁₀ CFU/ml when injected as a bolus i.v. dose of 15mg/kg. In comparison, the same dose of vancomycin reduced growth by 8.7 Log₁₀ CFU/ml. Intraperitoneal, double dose of C30 showed no rapid effect on CFU reduction in thigh (which in fact is to be expected given the administration route). Further time-dependent evaluation of this route of delivery is needed. Also the experiment lacks a control group of animals sacrificed after 6 hours post-infection. Nevertheless, taking into account that bacteria expanded 4.7 Log₁₀ CFU/ml within 4 hours of inoculation in untreated mice (1.7 times) one could argue that after 6 hours post-infection the amount of CFU should further increase logarithmically. For this reason i.p. delivery should not yet be dismissed.

### 3. Antiviral applications

The disease burden caused by seasonal influenza, the 2009 pandemic H1N1 outbreak and the increased spreading of drug (adamantanes and neuraminidase inhibitors) resistant influenza viruses demonstrate that there is a medical need for new drugs which can inhibit many types or variants of Influenza viruses and are less subject to seasonal virus variability. Currently, Tamiflu® by Roche and Relenza® by GSK are the most commonly prescribed drugs in the treatment of Influenza. Both drugs target the neuraminidase protein. Circulating influenza viruses are rapidly developing resistance against Tamiflu® and there are only a very limited number of anti-influenza drugs under clinical development.

Therefore, it was decided to design interferors against conserved regions of influenza proteins, to ensure a broad target specificity. Importantly, the mechanism of action of the interferor technology is fundamentally different from vaccines directed against infectious diseases and all other currently used anti-viral intervention strategies, i.e. it targets the elemental process of protein folding, and therefore, represents an entirely novel molecular intervention and selection pressure on the viral fitness. Also, it allows the identification of new viral targets that were so far not amenable to inhibition. In addition, provided the anti-viral interferors show high stability behavior, delivery of the interferors through intranasal or intratracheal sprays or aerosols might be possible for local administration at the site of infection and may therefore reveal yet an additional advantage of interferors compared to the currently prescribed drugs.

For the design of the interferors, there was particular focus on the viral polymerase subunits (PA, PB1, PB2) and on nucleoprotein (NP). These proteins are largely conserved, and the latter viral protein was recently identified as a target for a small-molecule compound that exerts its anti-viral effect by triggering NP aggregation, although influenza viruses exist that are naturally resistant against this experimental drug (Kao et al., Nat Biotechnol. 2010; 28(6):600-5). In summary, interferors targeting these conserved proteins would provide an answer to the increasing resistance of influenza viruses against currently available antivirals.

A panel of 36 peptides directed against different influenza A virus proteins was evaluated in a preliminary experiment. Madin Darby canine kidney (MDCK) cells were grown to confluence and preincubated for 1 hour at 37°C with interferors in serum-free medium. The cells were subsequently infected for 16 hours with NIBRG-14 (an H5N1 strain) or PR8 (an H1N1 strain) virus. The most promising peptides for each of the selected proteins were chosen for further evaluation. These peptides are shown in Table 10.

**Table 10. Sequences of antiviral interferors selected for further evaluation**

| **Interferor #** | Sequence of interferor peptide | Target protein | Identical sequence in target protein |
|---|---|---|---|
| **1** | O Z RLIQLIVSRGSRLIQLIVSR (SEQ ID NO: 116) | PB2 | R LIQLIVS (SEQ ID NO: 120) |
| **2** | O Z RTTIMAAFRGSRTTIMAAFR (SEQ ID NO: 117) | NP | TTIMAAF (SEQ ID NO: 121) |
| **3** | O Z RTMAWTVVRGSRTMAWTVVR (SEQ ID NO: 118) | PA | TMAWTVV (SEQ ID NO: 122) |
| **4** | O Z RGVSILNLRGSRGVSILNLR (SEQ ID NO: 119) | PB1 | GVSILNL (SEQ ID NO: 123) |

The O and Z in front of the sequence refer to an optical label and a linker respectively. O stands for 5(6)-Carboxyfluorescein. The linker used is N-(3-{2-[2-(3-amino-propoxy)-ethoxy]-ethoxy}-propyl)-succinamic acid], sometimes also referred to as 4, 7, 10-trioxatridecan-succin(am)ic acid or Ttds. The structure of the molecules thus corresponds to the formula:
Label - Z1-X1-Y1-X2-Z2-X3-Y2-X4, i.e. n=2, the label and Z1 are as defined above, all 4 X moieties equal a single R residue, Z2 is a GS linker, Y1=Y2 and equals the sequence listed in the last column of the table, except for interferor 1, where the Y moiety is LIQLIVS (SEQ ID NO: 124). For this aggregating region, the N-terminal gatekeeper residue equals the flanking residue in the protein. All the aggregating regions are unique to the influenza A protein they target.

As a follow-up experiment, MDCK cells were grown into confluent monolayers in a 24-well format. The interferors (200mg) were dissolved in 45microl DMSO to get a 2mM stock solution. 7 microliters of interferor stock was added to 400microl PBS to get a 35microM solution. Cells were washed with serum-free medium, after which 250 microliter fresh serum-free medium and 100 microliter interferor were added. Interferors were diluted so that they were applied to the cells in concentrations of 1 or 10 µM. Subsequently, cells were incubated for 4 hours at 37°C and 5% CO₂. After 4 hours pre-incubation with the interferor peptides, cells were inoculated with 10 plaque-forming units (pfu) of PR8 virus. The inoculum was removed and trypsin-containing medium was added. Samples were taken at 0, 8, 12, 16, 24 and 36 hours for virus titration. As positive control, cells were treated with 1µM or 10 nM of Tamiflu. Results of duplicate experiments are shown in Fig. 32.

As can be seen from the figure, at 10 µM all peptides inhibit virus replication. At 1 µM, interferors 1 and 4 still reproducibly inhibit virus replication, while the effect of interferors 2 and 3 is less strong. Note however that Tamiflu also did not completely inhibit viral replication in one setup.

We also used a more sensitive influenza A minireplicon assay to assess the potential of the candidate interferors. This system requires the presence of functional PA, PB1, PB2 and NP proteins. As reporter, an antisense firefly luciferase construct was used, which was transfected into mammalian cells together with expression plasmids for PB1, PB2, PA and NP. Normalization for transfection was performed using a Renilla luciferase reporter. A control experiment confirmed that the firefly luciferase minireplicon yielded luciferase activity when all 4 components were present, but not when one of them was missing or when an expression vector encoding mouse Mx1 protein, which confers selective resistance to influenza virus by inhibiting viral mRNA synthesis in the nucleus of influenza virus-infected cells, is cotransfected (fig. 33)

Using this system as a read-out, we could confirm antiviral activity of the interferor constructs. Variations of these interferors with different gatekeepers (R or D) and/or different Z₂ linker moieties (GS, PP or PS) were also tested, as were interferors with other aggregating sequences (see Fig. 34). Sequences of these constructs are shown in Table 11.

**Table 11. Sequences of further interferors directed against viral proteins.**

| **Interferor** | Sequence of interferor peptide | Target protein | Identical sequence in target protein |
|---|---|---|---|
| **PA-1** | O Z RTMAWTVVRGSRTMAWTVVR (=#3 in Table 10) (SEQ ID NO: 118) | PA | TMAWTVV (SEQ ID NO: 122) |
| | O Z RTMAWTVVRPPRTMAWTVVR (SEQ ID NO: 125) | | |
| | O Z DTMAWTVVDPPDTMAWTVVD (SEQ ID NO: 126) | | |
| | O Z RTMAWTVVRPSRTMAWTVVR (SEQ ID NO: 127) | | |
| **PA-2** | O Z DVHIYYLDPPDVHIYYLD (SEQ ID NO: 128) | PA | VHIYYL (SEQ ID NO: 164) |
| | O Z RVHIYYLRPSRVHIYYLR (SEQ ID NO: 129) | | |
| **PA-3** | O Z DNLYGFIIDPPDNLYGFIID (SEQ ID NO: 130) | PA | NLYGFII (SEQ ID NO: 165) |
| | O Z RNLYGFIIRPSRNLYGFIIR (SEQ ID NO: 131) | | |
| **PB1-1** | O Z RGVSILNLRPPRGVSILNLR (SEQ ID NO: 132) | PB1 | GVSILNL (SEQ ID NO: 123) |
| | O Z DGVSILNLDPPDGVSILNLD (SEQ ID NO: 133) | | |
| | O Z RGVSILNLRPSRGVSILNLR (SEQ ID NO: 134) | | |
| **PB1-2** | O Z RGFVYFVRPPRGFVYFVR (SEQ ID NO: 135) | PB1 | R GFVYFV (SEQ ID NO: 166) |
| | O Z DGFVYFVDPPDGFVYFVD (SEQ ID NO: 136) | | |
| **PB1-3** | O Z RMALQLFIRPPRMALQLFIR (SEQ ID NO: 137) | PB1 | MALQLFI (SEQ ID NO: 167) |
| | O Z DMALQLFIDPPDMALQLFID (SEQ ID NO: 138) | | |
| **PB2-1** | O Z RLIQLIVSRGSRLIQLIVSR (=#1 in Table 10) (SEQ ID NO: 116) | PB2 | R LIQLIVS (SEQ ID NO: 120) |
| | O Z RLIQLIVSRPPRLIQLIVSR (SEQ ID NO: 139) | | |
| | O Z DLIQLIVSDPPDLIQLIVSD (SEQ ID NO: 140) | | |
| | O Z RLIQLIVSRPSRLIQLIVSR (SEQ ID NO: 141) | | |
| **PB2-2** | O Z RLAVTWWNRPPRLAVTWWNR (SEQ ID NO: 142) | PB2 | LAVTWWN R (SEQ ID NO: 168) |
| | O Z DLAVTWWNDPPDLAVTWWND (SEQ ID NO: 143) | | |
| **PB2-3** | O Z RQSLIIAARPPRQSLIIAAR (SEQ ID NO: 144) | PB2 | R QSLIIAA R (SEQ ID NO: 169) |
| | O Z DQSLIIAADPPDQSLIIAAD (SEQ ID NO: 145) | | |
| **PB2-4** | O Z RGFLILGRPPRGFLILGR (SEQ ID NO: 146) | PB2 | GFLILG R (SEQ ID NO: 170) |
| | O Z DGFLILGDPPDGFLILGD (SEQ ID NO: 147) | | |
| | O Z RGFLILGRPSRGFLILGR (SEQ ID NO: 148) | | |
| **PB2-5** | O Z DLMVAYMLDPPDLMVAYMLD (SEQ ID NO: 149) | PB2 | LMVAYML (SEQ ID NO: 171) |
| **NP-1** | O Z RTTIMAAFRGSRTTIMAAFR (=#2 in Table 10) (SEQ ID NO: 117) | NP | TTIMAAF (SEQ ID NO: 121) |
| | O Z RTTIMAAFRPPRTTIMAAFR (SEQ ID NO: 150) | | |
| | O Z DTTIMAAFDPPDTTIMAAFD (SEQ ID NO: 151) | | |
| | O Z RTTIMAAFRPSRTTIMAAFR (SEQ ID NO: 152) | | |
| **NP-2** | O Z RLVWMACHRPPRLVWMACHR (SEQ ID NO: 153) | NP | LVWMACH (SEQ ID NO: 172) |
| | O Z DLVWMACHDPPDLVWMACHD (SEQ ID NO: 154) | | |
| | O Z RLVWMACHRPSRLVWMACHR (SEQ ID NO: 155) | | |
| **M1-A** | O Z RVAFGLVCRPPRVAFGLVCR (SEQ ID NO: 156) | M1 | VAFGLVC (SEQ ID NO: 173) |
| | O Z DVAFGLVCDPPDVAFGLVCD (SEQ ID NO: 157) | | |
| | O Z RVAFGLVCRPSRVAFGLVCR (SEQ ID NO: 158) | | |
| **M1-B** | O Z RLGFVFTLRPPRLGFVFTLR (SEQ ID NO: 159) | M1 | LGFVFTL (SEQ ID NO: 174) |
| | O Z DLGFVFTLDPPDLGFVFTLD (SEQ ID NO: 160) | | |
| | O Z RLGFVFTLRPSRLGFVFTLR (SEQ ID NO: 161) | | |
| **NS1-C** | O Z RAVGVLIGRPPRAVGVLIGR (SEQ ID NO: 162) | NS1 | AVGVLIG (SEQ ID NO: 67) |
| | O Z DAVGVLIGDPPDAVGVLIGD (SEQ ID NO: 66) | | |
| | O Z RAVGVLIGRPSRAVGVLIGR (SEQ ID NO: 163) | | |

| | | | |
|---|---|---|---|
| O and Z are identical labels and linkers as specified for Table 10 above. | | | |

By way of control, interferors designed against internal viral proteins (M1 and NS1) that are not involved in the minireplicon assay were also tested, and these indeed showed no reduction in luciferase activity (Fig.35). The design of these irrelevant interferors is identical to that of the 4 interferors against the polymerase proteins and against NP, with gatekeepers and Z2 linkers (i.e. internal linkers, formula here is Z1-X1-Y1-X2-Z2-X3-Y2-X4) as indicated in the figure. The aggregating sequence and peptide sequences for peptides against matrix protein 1 and nonstructural protein 1 are also shown in Table 11.

Thus, interferor peptides can be designed against each of the polymerase proteins of influenza virus, as well as against nucleoprotein. These interferors succeeded in decreasing influenza RNA replication, an effect that is specific and dependent on target downregulation, as interferors against non-relevant targets did not result in achieving downregulation.

### 4. Detection and diagnosis

### 4.1 Detection of β-galactosidase (β-gal) with specific interferors

### 4.1.1 Specific interferor peptide design and synthesis

The aggregation nucleating segments of the target to be detected, i.e. β-galactosidase, were identified using the Tango algorithm, a statistical mechanical algorithm for predicting β-aggregation prone regions in proteins based on three physiochemical parameters viz. low net charge, high hydrophobicity and β-sheet propensity. These were subsequently checked to the requirements for the Yᵢ moieties outlined in the application, to ensure these sequences possess high aggregation propensity. Tango compares the propensity of a given amino acid sequence against a set of similar sequence to form various secondary structural elements and assigns a score (0-100) proportional to its ability to form β-sheet aggregates. A stretch of sequence of total score <5 is considered to have low propensity to aggregate and those >50 are strongly aggregating. From the β-gal protein sequence (depicted in SEQ ID NO: 3, without the M initiator residue), two stretches of amino acid sequences with a total tango score >50 *viz.* residues 7 to 12 in SEQ ID NO: 3 (LAVVLQ (SEQ ID NO: 75, Tango 1) and residues 453 to 460 in SEQ ID NO: 3 (VIIWSLGN (SEQ ID NO: 76), Tango 2) were selected and a collection of high purity (>95%) peptides comprising the wild type sequence flanked with one or more gatekeeper residues (Arg, Lys, Asp, Glu and Pro) were synthesized by solid phase synthesis. A sequence stretch comprising residues 106 to 113 from SEQ ID NO: 3 showing a total Tango score <5 was selected as a negative control to establish the poor aggregation propensity of this segment as predicted by the Tango algorithm. In addition three mutant interferor versions comprising the VIIWSLGN (SEQ ID NO: 76)(Tango 2-region) were synthesized. The sequences of the 6 interferor peptides (comprising the identified Tango-regions, comprising the negative control sequence or comprising the variant (or mutated) Tango 2-region) are depicted in Table 12. The C-terminus of these 6 peptides were acetylated and labeled with biotin or poly-histidine (His)₆ tag at the N-terminus for western blotting (WB) detection and *in vitro* screening, respectively (see example 2).

SEQ ID NO: 3, amino acid sequence of β-galactosidase. The identified Tango regions are depicted in bold

**Table 12: list of the 6 different interferor peptides used for detection of β-Gal in complete E. coli BL21 cell lysate.**

| **Interferor peptides** | **Interferor sequence#** | **Wild type sequence^{℘}** |
|---|---|---|
| Tango 1 | *b*∼RLAVVLQR (SEQ ID NO: 44) | DS**LAVVLQ**RR (SEQ ID NO: 50) |
| Tango 2 | *b*∼RVIIWSLGNR (SEQ ID NO: 45) | PS**VIIWSLGN**ES (SEQ ID NO: 51) |
| Off-Target | *b*∼RPITVNPPFR (SEQ ID NO: 46) | TY**PITVNPPF**VP (SEQ ID NO: 52) |
| Tango 2 Mut_1 | *b*∼RV**P**IWSLGNR (SEQ ID NO: 47) | PS**VIIWSLGN**ES (SEQ ID NO: 51) |
| Tango 2 Mut_2 | *b*∼RVI**P**WSLGNR (SEQ ID NO: 48) | PS**VIIWSLGN**ES (SEQ ID NO: 51) |
| Tango 2 Mut_3 | *b*∼RVI**PE**SLGNR (SEQ ID NO: 49) | PS**VIIWSLGN**ES (SEQ ID NO: 51) |

| | | |
|---|---|---|
| # The annotation *b*^{∼} as used herein indicates that the peptide is N-terminally biotinylated and fused with a linker. ^{℘} The wild type sequences are shown with their naturally flanking residues. | | |

The names of the interferors, as used in the outline of the following examples, are depicted in column 1, the sequences are depicted in column 2. In these interferor molecules, n is 1, X₁ and X₂ are R residues, Y₁ is a stretch of 6 (Tango 1') or 8 ('Tango 2') residues of the target protein, Z₁ is an N-terminal amino acid linker APAA (SEQ ID NO: 77), and the molecules are fused to a detectable label - in this case biotin (*b*). For further experiments, other linkers such as Ttds and PEG have also been used, as well as different labels such as HA-tag (YPYDVPDYA (SEQ ID NO: 108)), Flag-tag (DYKDDDDK (SEQ ID NO: 106)), and His-tag (polyhistidine) - representative experiments are shown. The mutant peptides comprise an Y1 region with 1 or 2 non-conservative substitutions relative to the sequence of the target protein β-gal.

A schematic overview of the general detection method of proteins by use of interferors, which we hereafter refer to as PepBlot, is depicted in Figure 36.

### 4.1.2. Detection of β-galactosidase via Western Blot and PepBlot analysis with interferors specific for β-galactosidase

The β-galactosidase was first expressed in *E. coli.* Thereto, *E. coli* BL21 cells with the expression construct pBad_βgal_WT (pBad vector obtained from Invitrogen) were cultured in LB medium at 37°C. When the culture reached OD_{600 nm} ^{∼}0.6, the cells were induced with 0.2% arabinose and allowed to grow overnight at 37°C. In parallel competent BL21 cells that do not express β-Gal were grown for control and titration experiments.

A 0.4 ml suspension of BL21 cells (OD_{600 nm} ^{∼}1.2) expressing β-Gal (grown under induced conditions) was centrifuged at 5000 g for 10 min at room temperature. The supernatant was discarded and 0.8 ml of bacterial protein extraction reagent (B-PER, Thermo scientific) containing protease inhibitor was added to the bacterial pellet and vortex mixed for 30 s in order to lyse the cells. To 21 µl of the complete BL21 cell lysate 5 µl of 5x SDS sample loading buffer (Fermentas) was added and heated at 99°C for 3 min. This mixture (26 µl per well) was loaded in a 10-well NuPage 4-12% Bis-Tris gel and the proteins were separated under denaturing conditions. The separated proteins were then transferred to a nitrocellulose membrane and blocked overnight with 1% BSA in phosphate buffer saline, 0.05% Tween 20, pH 7.4 (PBS-T) at 4°C. Each lane of the membrane was cut out and separately incubated with either rabbit anti-β-Gal antibody either biotinylated Tango 1, either biotinylated Tango 2, or the biotinylated off-target peptide or either a biotinylated Tango 2 mutant peptide.

The procedure followed for western blot (WB) detection of β-gal with anti-β-Gal antibody was as follows. The first lane of the membrane was cut out and was incubated with rabbit polyclonal anti-β-Gal antibody (1:1000 dilution) for 1 h under gentle agitation in PBS-T. This was followed by 3x 10 min wash with PBS-T. After the final wash the membrane was incubated with goat polyclonal anti-rabbit antibody conjugated to horseradish peroxidase (HRP, 1:5000 dilution) for 1 h in PBS-T. Then this lane of the membrane was washed with PBS-T 3x 10 min and finally rinsed in deionised water for 10 min. The lane of the membrane was exposed to chemiluminescence HRP substrate reagent (SuperSignal West Femto Maximum Sensitivity Substrate, Thermo Fisher Scientific) and target protein was visualized using Bio-Rad ChemiDoc XRS imaging system (see Figure 37, lane 1).

The procedure for PepBlot detection (i.e. a protocol similar to Western blot but with interferors instead of antibody as detection agent) of β-gal with biotinylated interferor peptides was as follows. A stock solution (10 µM) of each of the biotinylated peptides (Tango 1, Tango 2, off-target and mutants of tango 2 (see table 12) was prepared in 100% DMSO. The peptide stock was diluted (1/40) in 10 mM MES buffer, 100 mM Trehalose, 0.02% Tween 20, pH 5.5 to obtain a final concentration of the peptide of 250 nM. The freshly prepared peptide solution was immediately added to the membrane strip and gently agitated at room temperature. After 1 h the peptide solution was decanted and the membrane strip was washed 4x 10 min with 10 mM MES buffer, 0.05% Tween 20, pH 5.5. Then the membrane strip was incubated with biotin affinity reagent (1:100,000 dilution) streptavidin (SRP) conjugated to HRP in PBS-T for 1 h at room temperature. This was followed by 3x 10 min wash with PBS-T and finally rinsed in deionised water for 10 min. The membrane strip was exposed to chemiluminescence HRP substrate reagent (SuperSignal West Femto Maximum Sensitivity Substrate, Thermo Fisher Scientific) and target protein visualized using Bio-Rad ChemiDoc XRS imaging system.

### 4.1.2.1 Selectivity of the different interferors used

In Figure 37 a comparison is shown of WB detection of β-gal from complete bacterial cell lysates using rabbit polyclonal anti-β-Gal antibody and also the detection using PepBlot with three different interferors: Tango 1 peptide (*b*^{∼}RLAVVLQR (SEQ ID NO:44)) and Tango 2 peptide (*b*^{∼}RVIIWSLGNR (SEQ ID NO: 45)) and an off-target peptide (*b*^{∼}RPITVNPPFR (SEQ ID NO: 46)). Here also, biotin was used as label and APAA (SEQ ID NO: 77) as an amino acid linker. It was observed (see Figure 37) that the Tango 1 and Tango 2 interferors bind to β-Gal with high specificity. Although the Tango 1 peptide is predicted to show the strongest propensity to aggregate with β-gal, it exhibits a weaker binding (see lane 2) than the Tango 2 interferor peptide. Without being bound to a particular mechanism of action, one explanation is that the Tango 1 peptide does interact less efficiently to the N-terminus of the β-gal because this N-terminus is less available in the immobilized state of β-gal on the membrane strip. This non-limiting explanation is further supported by the fact that addition of the same interferor peptide (i.e. Tango 1 peptide) to free β-gal *in vitro* results in faster aggregation kinetics as compared to the Tango 2 peptide (see further). Figure 37 clearly shows that the Tango 2 peptide displays strong affinity to the β-Gal with a signal comparable to the detection with a specific antibody for β-gal (compare lane 1 and lane 3). Although the off-target peptide interferor is populated with hydrophobic amino acids, the presence of more than one β-sheet breaker P residues in the centre of the sequence disrupts β-aggregation effectively. The band in lane 4 at the position ^{∼}20 kDa is not an effect of peptide cross-reactivity but due to non-specific binding of streptavidine (SRP) in the absence (or low availability) of biotin. The experiments described in the following sections will be primarily based on the Tango 2 sequence.

### 4.1.2.2 Specificity of binding of interferor probes

In order to evaluate the specificity of interaction of Tango 2 interferor peptide with β-Gal, detection experiments using additional peptides were performed. First, we probed β-Gal with a non-aggregating but hydrophobic β-Gal peptide (P₁₀₆ITVNPPF₁₁₃), and found no interaction of the corresponding probe peptide *b*^{∼}RPITVNPPFR (SEQ ID NO: 46) with β-Gal (see above, Fig. 37, lane 4). Second, we employed four probe peptides whose sequences correspond to aggregating regions identified using TANGO in unrelated bacterial and human proteins. This was done using biotin labeled peptides derived from human cyclin-dependent kinase 4 inhibitor B (sequence *b*^{∼}FLDTLVVLHRA (SEQ ID NO: 175)), human prostate specific antigen (PSA, sequence *b*^{∼}RQWVLTAAR (SEQ ID NO: 85)) and proline dehydrogenase (PD, *b*^{∼}RFFIALSR (SEQ ID NO: 176)) and ClpB ATPase (*b*^{∼}RILLGLIR (SEQ ID NO: 177)), both taken from Staphylococcus epidermidis. The design of the latter three probes corresponds to molecules with n=1, X₁=X₂= one Arg residue, with the Y₁ sequence in between. In the first probe, FLD and RA are naturally flanking sequences of the TLVVLH (SEQ ID NO: 178) moiety that corresponds to Y₁. In these flanking sequences, D corresponds to the X₁ gatekeeper, R to the X₂ gatekeeper.

All of these peptides failed to yield specific staining of the band corresponding to β-Gal (data not shown) showing that aggregation propensity is necessary but not sufficient for specific interaction. Finally, to test whether high-scoring Tango regions tolerate substitution with other residues and maintain their properties, mutants were generated. In the first case "I"-residue was replaced with β-sheet breaker "P" residue and 2 different mutant peptides with an "I" to "P" change were generated (see table 12 for the specific sequences). In addition the effect of a double mutant peptide replacing "IW" with "PE" was used to study the influence of charged gatekeeper residue on the β-aggregation propensity. It is expected that the aggregation is suppressed by the introduction of a repulsive charge. PepBlot analysis of the two single point mutant peptides (*b*^{∼}RVPIWSLGNR (SEQ ID NO: 47) and *b*^{∼}RVIPWSLGNR (SEQ ID NO: 48)) illustrated in Figure 38 shows that these mutant peptides still bind efficiently to β-Gal. However, it is clear that by introducing substitutions off-target binding is observed. This is not illogical, since the remaining short aggregation-inducing stretch is not unique to the β-Gal protein in *E. coli.* Furthermore, additional introduction of a charged residue leads to an almost complete loss of interaction. This demonstrates the high sequence specificity of probe binding as a single point mutant is sufficient to suppress binding specificity whereas suppression of the aggregation propensity is sufficient to abolish binding altogether despite 80% sequence identity. These data confirm our hypothesis that aggregation propensity and sequence matching are a prerequisite for the specificity of peptide mediated interaction and are in line with a recent study (Sabate, R., et al. J. Mol. Biol. 404: 337-352, 2010) showing that scrambled or reversed versions of the islet amyloid polypeptide do not cross-seed with each other or the wild type sequence, confirming position dependence beyond mere sequence composition.

### 4.1.2.3 Kinetics of the interferors on the binding

In order to study the Tango 2 peptide interferor binding kinetics to β-Gal (i.e. the contact time between interferor and target) in complete BL21 cell lysate, the peptide was incubated with the membrane from 30 s to 1 h. The membrane was removed from incubation buffer at selective time intervals and further processed as described above.

Figure 39 illustrates that detection of β-Gal from complete bacterial BL21 cell lysate can be achieved in a short time frame (as low as 30 seconds) incubation with the peptide.

### 4.1.2.4 Sensitivity of the PepBlot detection method based on the use of interferors

The sensitivity of the interferor peptide based PepBlot detection was determined. Thereto, β-Gal in the concentration range 0.1 pmol (11.6 ng) to 10 pmol (1160 ng) was spiked to a complete non-induced BL21 cell lysate (OD_{600 nm} ^{∼}0.6). To 21 µl of the complete BL21 cell lysate 5 µl of 5x SDS loading buffer (Fermentas) was added and heated at 99°C for 3 min. 26 µl of this mixture containing different concentrations of β-Gal were loaded to a 10-well NuPage 4-12% Bis-Tris gel and the proteins were separated under denaturing conditions. The separated proteins were subsequently transferred to a nitrocellulose membrane and blocked overnight with 1% BSA in PBS-T at 4°C. Further incubations and handling with the interferors were as described herein before. Figure 40 shows the result of this experiment. From the data it is apparent that subpicomolar amounts of β-Gal (as little as 58 ng) can be detected (see the detection of β-Gal in lane 2 of Figure 40A), which is comparable to the detection limit of an antibody. Moreover, the signal obtained is also comparable to the anti-β-Gal antibody detection (Figure 41).

### 4.1.2.5 Specificity of PepBlot detection

To determine the influence of gatekeeper residues on the specificity of PepBlot detection, we generated interferor peptides against the target protein β-gal with core Tango sequence "VIIWSLGN" (SEQ ID NO: 76). Thereto, 3 interferor peptides were generated: 1) No gatekeeper residue, with the sequence VIIWSLGN (SEQ ID NO: 76) linked to Flag-tag (DYKDDDDK (SEQ ID NO: 106)) in the C-terminus via GSGS amino acid linker (VIIWSLGNGSGSDYKDDDDK (SEQ ID NO: 179)). 2) Tango sequence flanked by natural gatekeeper residues (RDRNHPSVIIWSLGNESGHG (SEQ ID NO: 180)). 3) Complementary charged gatekeeper residues with the residue 'D' and 'R' positioned to complement polar 'S' and 'E' residues (DVIIWSLGNR (SEQ ID NO: 181)). The first two peptides confirm to definitions of interferor peptides provided in WO2007/071789, whereas the third peptide is an interferor molecule according to the structural definition provided herein. All the peptides listed in Table 13 were double biotinylated (on both the N-terminus and C-terminus) and linked to the interferor peptides by Ttds- linker.

**Table 13: List of the 3 different interferor peptides used for studying the influence of gatekeepers on the specificity of β-Gal detection in a competitive PepBlot platform.**

| **Interferor peptides** | **Interferor sequences^{℘}** | **Natural sequence** |
|---|---|---|
| No gatekeeper | *b*∼VIIWSLGNGSGSDYKDDDDK∼*b* (SEQ ID NO: 179) | RDRNHPSVIIWSLGNESGHG |
| Natural flanks | *b*∼RDRNHPSVIIWSLGNESGHG∼*b* (SEQ ID NO: 180) | |
| Complimentary charge | *b*∼DVIIWSLGNR∼*b* (SEQ ID NO: 181) | |

| | | |
|---|---|---|
| ^{℘}The interferor peptides were linked to biotin on both the C-terminus (indicated by ^{∼}b) and N-terminus (b^{∼}) with Ttds linker. Note the Tango sequence is underlined in each peptide. | | |

We tested the performance of the peptides to selectively bind to β-gal in a competitive PepBlot platform where 7% clinical serum was taken in one lane and β-gal in complete *E. coli* lysate in other lane. A stock solution (10 µM) of each of the biotinylated peptides was prepared in 100% DMSO. The peptide stock was diluted (1/400) in 10 mM MES buffer, 100 mM Trehalose, 0.05% Tween 20, pH 5.5 to obtain a final concentration of the peptide of 25 nM. The freshly prepared peptide solution was immediately added to the membrane strip and gently agitated at room temperature. After 15 min the peptide solution was decanted and the membrane strip was washed 3x 10 min with 10 mM MES buffer, 0.05% Tween 20, pH 5.5 and further processed as described herein before.

Figure 42 shows the influence of gatekeeper residues on the specificity of interferor peptide based PepBlot detection. All the three peptides bind to β-gal but by far the best probe was the interferor peptide with complimentary charged gatekeepers. This peptide shows strong interaction to β-gal compared to other two peptides and does not cross react with proteins in the serum. The charge complementation along with β-sheet propensity of the Tango sequence contributes to favorable intermolecular association that appears to be highly specific. On the other hand, the peptide with natural flanks displays low specificity that is evident from aspecific bands in clinical serum. The data suggests gatekeepers are not necessarily required for interferor peptide interaction with the target protein - as was also shown in WO2007/071789. However, detection can be fine-tuned to achieve high specificity by designing peptides with complimentary flank residues which may include polar amino acids such R, D, E, K, H and β-sheet breaker P.

### 4.2. Interferor-target interactions studied with Surface Plasmon Resonance

In this *in vitro* experiment the affinity of different interferor peptides was measured for binding to β-gal by means of surface plasmon resonance (SPR). SPR experiments were performed at 25° C using a Biacore T100 equipped with CM5 sensor chip (GE Healthcare.). Coupling reagents (N-ethyl-N'-(3-dimethylaminopropyl)carbodiimide (EDC), N-hydroxysuccinimide (NHS) and ethanolamine-HCI) were purchased from GE Healthcare. BSA and β-gal were purchased from Sigma-Aldrich and Roche Diagnostics, respectively. Peptides were synthesized at JPT, GmbH and were of >95% purity. The proteins BSA and β-Gal were immobilized respectively, in the reference and sample channels on a CM5 sensor chip by standard amine coupling chemistry at a flow of 10 µl min⁻¹. The carboxymethyl dextran surface was activated by the injection of a 1:1 ratio of EDC and NHS for 7 minutes. The proteins were diluted in 10 mM sodium-acetate buffer, pH 4.5 to a final concentration of 0.2 mg ml⁻¹ and injected in short pulses over the activated surface until the immobilization levels reached 4000 RU or 8000 RU, for BSA and β-Gal, respectively. The remaining reactive groups were blocked with 1 M ethanolamine, pH 8. After completion of coupling, the surface was regenerated with short pulses of 50 mM NaOH and 8 M urea to remove non-covalently attached proteins. The immobilization levels after the regeneration was typically between 3500 RU to 4000 RU in both reference and sample channels. The affinity of several interferors (see Table 14 for the sequences of the interferors): Tango zone 1, Tango zone 2, off-target, Tango zone 2 mutants and tandem repeat of Tango zone 2 peptide to bind β-Gal was investigated by injecting the hexahistidine-tagged peptides over the reference (BSA) and sample (β-Gal) surfaces at 25°C. The peptides, dissolved in the running buffer (10 mM sodium phosphate, pH 6.8, 150 mM NaCl, 3 mM EDTA and 0.015% Tween 20) at a concentration of 1 µM, were injected for 60 s at a flow rate of 30 µl min⁻¹ and then allowed to dissociate for 600 s. The injection was repeated on the same surface as well as on independently immobilized surfaces. The surface was regenerated between each cycle by 30 s pulses of i) 50 mM NaOH, and ii) 8 M urea, and was allowed to stabilize for 400 s before the next cycle.

**Table 14: Sequences of the different interferor peptides used in the SPR analysis and in vitro aggregation kinetics.**

| **Interferor peptides** | **Sequences^{#}** | **Wild Type Sequence^{℘}** |
|---|---|---|
| Tango 1 | (His)₆∼RLAVVLQR (SEQ ID NO: 53) | DS**LAVVLQ**RR (SEQ ID NO: 50) |
| Tango 2 | (His)₆∼RVIIWSLGNR (SEQ ID NO: 54) | PS**VIIWSLGN**ES (SEQ ID NO: 51) |
| Off-Target | (His)₆∼RPITVNPPFR (SEQ ID NO: 55) | TY**PITVNPPF**VP (SEQ ID NO: 52) |
| Tango 2 Mut_1 | (His)₆~RV**P**IWSLGNR (SEQ ID NO: 56) | PS**VIIWSLGN**ES (SEQ ID NO: 51) |
| Tango 2 Mut_2 | (His)₆∼RVI**P**WSLGNR (SEQ ID NO: 57) | PS**VIIWSLGN**ES (SEQ ID NO: 51) |
| Tango 2 Mut_3 | (His)₆∼RVI**PE**SLGNR (SEQ ID NO: 58) | PS**VIIWSLGN**ES (SEQ ID NO: 51) |
| Tandem of Tango 2 | (His)₆∼RVIIWSLGNRGSGSAPAARVIIWSLGNR (SEQ ID NO: 59) | PS**VIIWSLGN**ES (SEQ ID NO: 51) |

| | | |
|---|---|---|
| ^{#}The peptides are linked to His-tag with an additional amino acid 'APAA' (SEQ ID NO: 77) linker in the N-terminus. ^{℘} The wild type sequences are shown with their naturally flanking residues. | | |

The kinetics of the co-aggregation of β-gal and the Tango 2 peptide (HHHHHHAPAARVIIWSLGNR (SEQ ID NO: 54)) was investigated at 25°C. The peptide dissolved in the running buffer was injected for 60 s at a flow-rate of 30 µl min⁻¹ over the reference (BSA) and sample (β-Gal) surfaces in a range of concentrations (0 - 4 µM, with one internal replicate) and were then allowed to dissociate for 600 s. The concentration series were repeated on the same surface (three times) as well as on independently immobilized surfaces. The surface was regenerated between each cycle by 30 s pulses of i) 50 mM NaOH, and ii) 8 M urea, and was allowed to stabilize for 400 s before the next cycle.

All sensorgrams were double reference subtracted (Myszka, D.G., et al. J. Mol. Recognit. 12: 279-284, 1999) by i) subtraction of the response observed on the reference surface and ii) subtraction of the responses observed for buffer injections, the latter in order to remove systematic artifacts. The data were globally fitted to a three-state model, using the Biacore T100 software, assuming an initial encounter between the peptide in solution and the protein on the chip followed by a two-step rearrangement before an identical binding site becomes available at the growing β-sheet.

A compilation of the data obtained from the SPR analysis is shown in the figures 43 to 46.

The β-Gal protein was immobilized on the sensor chip by amine coupling, which may hinder the accessibility of Tango zone 1 (residues 7 to 12) near the N-terminus. It is likely the weak binding affinity of Tango peptide 1 with β-Gal observed in SPR is due to restriction imposed by the protein in the immobilized state to interact with the peptide. However, *in vitro* the Tango 1 peptide exhibits higher affinity to free β-Gal in solution compared Tango 2 peptide that is in accordance to the Tango algorithm prediction.

The sensorgram depicting the change in the instrument response units (RU) as a function of analyte (Tango 2 peptide) concentration is shown in Figure 45. The calculated RUₘₐₓ for a 1:1 stoichiometry is ^{∼}80 RU, however, our data exceeds this value and stability is not achieved even at higher concentration and prolonged contact time (>10 min). This is because after the peptide binds to β-Gal a new binding site is created allowing aggregate growth on the sensor chip surface. To model the aggregate growth a simple 1-state or 2-state kinetics was found to be inadequate. The kinetic data is best represented using a multistep process involving 3-state model. According to this model the peptide reversibly docks to the target followed by conformation switch it locks in position to receive subsequent peptide, which binds to the first peptide and blocks the binding site. This model has been previously reported to describe the amyloid fibril elongation on SPR chip. The calculated kₐ and k_{d} for the 3-steps mechanism were: kₐ₁ = 2.744E+4, kₐ₂ = 0.02359, kₐ₃ = 0.005491 and k_{d1} = 0.4123, k_{d2} = 0.02509 k_{d3} = 0.002654. Due to complexity of the model we were unable to calculate the equilibrium association constant Kₐ and equilibrium dissociation constant K_{d}. The kinetics gets even more complex if a tandem repeat peptide (n=2 and both Y moieties are identical) was used as an analyte (see Figure 46). In this molecule, all X moieties are a single R residue, and the Z1 linker is GSGSAPAA (SEQ ID NO: 90) (cf. Table 14).

### 4.3 Determination of the in vitro aggregation kinetics

The co-aggregation of Tango 1 peptide, Tango 2 peptide and a tandem repeat of Tango 2 peptide with β-Gal *in vitro* were monitored via light scattering from the apparent change in the optical density (OD) at 340 nm due to growth of aggregate particulates at room temperature. The interferor-β-Gal co-aggregation was initiated by adding equimolar molar concentration (10 µM) of either Tango 1 peptide, either Tango 2 peptide, either tandem repeat of Tango 2 peptide and β-Gal in 20 mM sodium phosphate buffer, pH 6.8 followed by gently stirring the sample at 50 rpm. The sequence of the interferors is depicted in Table 14.

To characterise the size of the aggregates the hydrodynamic radius (RH) of β-Gal at time zero and after co-incubation with the either Tango 1 peptide, either Tango 2 peptide, either tandem repeat of Tango 2 peptide (Table 14) for 2 h was measured using dynamic light scattering (DLS), DynaPro DLS (Wyatt Technology Europe, Germany).

Figure 47 shows the Tango 1 peptide with higher score exhibits faster co-aggregation kinetics with free β-Gal in solution compared to the Tango 2 peptide *in vitro.* When a tandem repeat of Tango 2 peptide was added to β-Gal, fast co-aggregation kinetics were observed and the size of the aggregates formed after 2 h were larger compared to those of single Tango 2 peptide (Figure 48).

To evaluate the limit of single Tango 2 peptide to initiate co-aggregation of β-Gal a series of samples with β-Gal to peptide molar ratio of 1:0, 1:0.2, 1:0.5 and 1:1 was prepared in 20 mM phosphate buffer, pH 6.8. The concentration of β-Gal was kept constant at 10 µM while the peptide amount was 2 µM, 5 µM and 10 µM. The interferor -β-Gal co-aggregation was initiated by gently stirring the sample at 50 rpm.

Light scattering and DLS experiments showed that sub-stoichiometric concentrations of the peptide were sufficient to induce aggregation of β-Gal (see Figure 49). The visible aggregates thus formed were not soluble in 8 M urea or 6 M GdHCl.

### 4.4. β-gal enzyme functional knockout

In order to study the effect of the Tango 2 peptide interferor on the enzyme function of β-Gal the catalytic activity was assayed before and after incubation with this peptide. Enzyme functional readout was performed using the substrate fluorescein-di-β-D-galactopyranoside (FDG), which is non-fluorescent but on enzymatic cleavage by β-Gal fluorescent fluorescein is liberated with intensity proportional to the catalytic activity. Figure 50 shows the effect of increasing concentrations of the Tango 2 peptide interferor on the enzymatic activity of β-galactosidase. It is apparent that equimolar concentrations of enzyme and interferor lead to a complete inhibition of enzymatic activity (or in other words to a functional knockout by complete co-aggregation).

### 4.5. Structure of isolated β-gal-Tango 2 peptide interferor co-aggregate

The insoluble β-gal-Tango 2 peptide interferor co-aggregate was isolated after co-incubation for 2 h. The isolated aggregates were repeatedly washed with 20 mM sodium phosphate buffer, pH 6.8 until the supernatant showed negligible absorbance at 280 nm and suspended in buffer for structural characterization using Fourier transform infra-red (FTIR) spectroscopy, circular dichroism (CD) and electron microscope (EM).

FTIR spectra of native β-gal shows an amide I peak at 1638 cm⁻¹ characteristic of secondary structure elements rich in β-sheets, which shifts to 1628 cm⁻¹ due to intermolecular β-sheet formation upon co-aggregation with the Tango 2 peptide interferor (Figure 51). The CD spectra of the native β-gal solution displays features of a α+β structure class whereas the co-aggregate suspension spectra with a negative band ^{∼}218 nm suggests complete loss of native protein structure and a shift to aggregated β-sheets (Figure 52). Finally, EM of the isolated β-gal-Tango 2 peptide interferor co-aggregate illustrates that the formed aggregates are amorphous in nature (and thus not amylogenic) (Figure 53).

### 4.6. Diagnostic applications of interferors: detection of three different protein biomarkers in serum

In the following example we demonstrate the feasibility of the use of interferors for diagnostic applications. Thereto, three medically relevant biomarkers were chosen as examples for detection in human serum: 1) prostate specific antigen (PSA) for which the amino acid sequence is depicted in SEQ ID NO: 4, 2) C-reactive Protein (CRP) for which the amino acid sequence is depicted in SEQ ID NO: 5 and 3) β-2-microglobulin (β-2M) for which the amino acid sequence is depicted in SEQ ID NO: 6. Sequences are shown without their signal peptide. Note that the Tango regions used for the design of the specific interferors are underlined in the respective amino acid sequences. The sequences of the biomarker-specific interferors are depicted in Table 15.
SEQ ID NO: 4: amino acid sequence of Prostate specific antigen (PSA)
SEQ ID NO: 5: amino acid sequence of C-Reactive Protein (CRP)
SEQ ID NO: 6: amino acid sequence of β-2-Microglobulin (β-2M)

**Table 15: List of specific interferor sequences used for PepBlot detection of the three different protein biomarkers in serum. The tandem repeat PSA peptide is linked to biotin by Ttds-APAA (SEQ ID NO: 77) linker.**

| Interferor peptide | Sequence | Wild Type Sequence^{℘} |
|---|---|---|
| PSA | *b*∼RWQVLVASD (SEQ ID NO: 182) | QPWQVLVASRG (SEQ ID NO: 183) |
| | *b*∼RQWVLTAAR (SEQ ID NO: 60) | HPQWVLTAAHC (SEQ ID NO: 63) |
| | *b*∼RQWVLTAARGSGSAPAARQWVLTAAR (SEQ ID NO: 89) | |
| CRP | *b*∼RILIFWSKR (SEQ ID NO: 61) | NEILIFWSKD (SEQ ID NO: 64) |
| β-2M | *b*∼RWSFYLLYYTR (SEQ ID NO: 62) | KDWSFYLLYYTEF (SEQ ID NO: 65) |

| | | |
|---|---|---|
| ^{℘} The wild type sequences are shown with their naturally flanking residues. | | |

The membrane for the detection of protein biomarkers was prepared as follows. To 21 µl of 5% human serum (Lonza) 50 pmol of the target protein biomarkers (respectively 1.55 ng PSA, 1.25 ng CRP and 0.6 ng β-2M) was added and this was mixed with 5 µl of 5x SDS loading buffer with and without DTT (Fermentas) and heated at 82°C for 3 min. 26 µl of this mixture containing PSA or CRP or β-2M was loaded to a 10-well NuPage 4-12% Bis-Tris gel and the proteins were separated under denaturing or reduced denaturing condition. The separated proteins are then transferred to a nitrocellulose membrane and blocked overnight with 1% BSA in PBS-T at 4°C.

### 4.6.1 PepBlot and WB detection of PSA spiked in human serum

A stock solution (10 µM) of the PSA-specific biotinylated peptide (for sequence see Table 15) was prepared in 100% DMSO. The peptide stock was diluted (1/40) in 25 mM MES buffer, 100 mM Trehalose, 0.02% Tween 20, pH 5.0 so the final concentration of the peptide was 250 nM. The freshly prepared peptide solution was immediately added to the membrane strip and gently agitated at room temperature. After 1 h the peptide solution was decanted and the membrane was washed 4x 10 min with 25 mM MES buffer, 0.05% Tween 20, pH 5.0. Then the membrane was incubated with biotin affinity reagent (1:100,000 dilution) SRP-HRP conjugate in PBS-T for 1 h at room temperature. This was followed by 3x 10 min wash with PBS-T and finally rinsed in deionised water for 10 min. For WB detection of PSA spiked in human serum, the membrane was incubated with (1:5000) rabbit monoclonal anti-PSA antibody (EP1588Y, Abcam) specific to the c-terminal peptide of PSA. This was followed by staining with (1:30,000) goat polyclonal anti-rabbit antibody conjugated to HRP. The membranes were exposed to chemiluminescence HRP substrate reagent (SuperSignal West Femto Maximum Sensitivity Substrate, Thermo Fisher Scientific) and target protein visualized using Bio-Rad ChemiDoc XRS imaging system.

Figure 54 shows the comparison between interferor peptide, *b*^{∼}RQWVLTAAR (SEQ ID NO: 85) detection (panel A) and the antibody detection for PSA (panel B). WB analysis of PSA has been shown to display multiple bands depending on the gel running condition. In non-reduced denaturing condition two bands were observed and in reduced denaturing condition (not shown) multiple fragments appear due to internal cleavage (Wang, T.J., et al, Tumor Biol. 20: 79-85, 1999).

### 4.6.2 PepBlot and WB detection of CRP spiked in human serum

A stock solution (10 µM) of biotinylated peptide was prepared in 100% DMSO. The peptide stock was diluted (1/40) in 25 mM MES buffer, 100 mM Trehalose, 0.02% Tween 20, pH 5.0 so the final concentration of the peptide was 250 nM. The freshly prepared peptide solution was immediately added to the membrane strip and gently agitated at room temperature. After 60 min the peptide solution was decanted and the membrane was washed 4x 10 min with 25 mM MES buffer, 0.05% Tween 20, pH 5.0. Then the membrane was incubated with biotin affinity reagent (1:30,000 dilution) Neutravidin conjugated to HRP in PBS-T for 1 h at room temperature. This was followed by 3x 10 min wash with PBS-T and finally rinsed in deionised water for 10 min. WB detection of CRP spiked in human serum was performed by incubating (1:2000) rabbit monoclonal anti-CRP antibody (Abcam) for 1 h. This was followed by staining with goat (1:30,000) polyclonal anti-rabbit antibody conjugated to HRP. The membranes were exposed to chemiluminescence HRP substrate reagent (SuperSignal West Femto Maximum Sensitivity Substrate, Thermo Fisher Scientific) and target protein visualized using Bio-Rad ChemiDoc XRS imaging system. The blots are shown in Figure 55.

### 4.6.3 PepBlot and WB detection of β-2M spiked in human serum

A stock solution (10 µM) of biotinylated peptide interferor specific voor β-2M (see Table 15 for the sequence) was prepared in 100% DMSO. The peptide stock was diluted (1/40) in 25 mM MES buffer, 100 mM Trehalose, 0.02% Tween 20, pH 5.0 to obtain a final concentration of the peptide interferor of 250 nM. The freshly prepared peptide interferor solution was immediately added to the membrane strip and gently agitated at room temperature. After 30 min the peptide interferor solution was decanted and the membrane was washed 4x 10 min with 25 mM MES buffer, 0.05% Tween 20, pH 5.0. Then the membrane was incubated with biotin affinity reagent (1:100,000 dilution) SRP-HRP conjugate in PBS-T for 1 h at room temperature. This was followed by 3x 10 min wash with PBS-T and finally rinsed in deionized water for 10 min.

To detect β-2M spiked in human serum by WB, the membrane was incubated with (1:5000) rabbit monoclonal anti-β-2M antibody (Abcam) for 1 h and stained with (1:30,000) goat anti-rabbit secondary antibody conjugated to HRP. The membrane strips were then exposed to chemiluminescence HRP substrate reagent (SuperSignal West Femto Maximum Sensitivity Substrate, Thermo Fisher Scientific) and target protein visualized using Bio-Rad ChemiDoc XRS imaging system. The result of this analysis is shown in Figure 56.

### 4.6.4 Detection of CRP and PSA in non-spiked complex human samples

To explore whether the present approach could also be applied for quantitative detection in clinical settings to detect naturally secreted or leaked proteins, with proteomes of other origin, composition and complexity than *E. coli* lysate, we designed assays to detect human protein biomarkers in the relevant complex samples, i.e. CRP in human blood serum and PSA in seminal plasma. CRP is a widely used biomarker for inflammation, which gave us an abundant source of clinical serum samples for which independent immunoturbidimetric assay based CRP determinations were available.

For CRP detection in serum the probe peptide *b*^{∼}RILIFWSR (SEQ ID NO: 86), which is unique to CRP, was selected. CRP detection in serum was performed using clinical samples of two patients containing low (1 µg ml⁻¹) and high (317 µg ml⁻¹) CRP. The serum sample was diluted to 7% in MilliQ water and mixed with 5x SDS loading buffer, heated at 82°C for 5 min. Denatured proteins were electrophoresed on 4-12% Bis-Tris gels, transferred to polyvinylidene fluoride (PVDF) membranes and blocked with 1% BSA in phosphate buffer saline, pH 7.4 and 0.05% tween 20 (PBS-T). The membranes were agitated with 250 nM *b*^{∼}RILIFWSR (SEQ ID NO: 86) peptide formulated in 10 mM MES buffer pH 5.1, 100 mM trehalose, 0.05% Tween 20 and 2.5% DMSO at 25°C for 1 h. After rinsing with 10 mM MES buffer pH 5.1, 0.05% Tween 20 the membranes were stained with SRP-HRP conjugate and visualized using Bio-Rad ChemiDoc XRS imaging system.

Immunoblot detection of CRP was achieved using rabbit monoclonal anti-CRP antibody (1:2000) under gentle agitation. After 1 h the membrane was rinsed with PBS-T, stained with goat polyclonal anti-rabbit antibody conjugated to HRP (Promega) for 1 h, and then exposed to chemiluminescence reagent (SuperSignal West femto maximum sensitivity substrate, Thermo Fisher Scientific) and protein visualized using Bio-Rad ChemiDoc XRS imaging system. Results are shown in Figure 57.

We then proceeded to evaluate the diagnostic utility of our probe peptide to detect CRP in clinical serum samples. Blood samples of 20 patients (Universiteit Ziekenhuis, Leuven) were processed according to standard protocol and serum was separated to analyse CRP with standard laboratory diagnostics employing an immunoturbidimetry assay and PepBlot. The concentration of CRP was measured by an immunoturbidimetry-based method using latex particles coupled to monoclonal mouse anti-CRP antibody. The test was performed on a Hitachi/Roche Modular P system (Roche Diagnostic).

PepBlot analysis of the 20 clinical samples shown in Figure 58 revealed specific staining to a well-defined band corresponding to the molecular weight of CRP. The quantification of the intensity of these bands compares well with the data obtained from the standard clinical immunoturbidimetric assay as performed independently on the same samples.

Next, we designed a PepBlot assay for detection of PSA, a member of the tissue kallikrein family of proteases that is synthesized in prostate gland, secreted in seminal fluid and is used as a biomarker for prostate cancer. We synthesized peptides targeting the sequence W₁₄QVLVAS₂₀ and the sequence Q₃₄WVLTAA₄₀ that were predicted by Tango (cf. Table 15).

Semen from a male volunteer was collected and allowed to liquefy at room temperature. After 2 h the seminal fluid was centrifuged at 10,000 xg for 15 min to separate the plasma from sperm cells. A sample of 10% seminal plasma was fractionated under non-reducing condition using denaturing gel electrophoresis then transferred to PVDF membrane and blocked using protocol described for serum electrophoresis. Lanes of membrane were agitated separately with 250 nM of peptide *b*^{∼}RWQVLASD (SEQ ID NO: 88) or peptide *b*^{∼}RQWVLTAAR (SEQ ID NO: 85) or 250 pM tandem repeat peptide *b*^{∼}RQWVLTAARGSGSAPAARQWVLTAAR (SEQ ID NO: 89) formulated in 10 mM MES buffer pH 5.1, 100 mM trehalose, 0.05% Tween 20 and 2.5% DMSO at 25°C for 1 h. The membranes were rinsed in 10 mM MES buffer pH 5.1, 0.05% tween 20 and stained with HRP-conjugated SRP and visualized with ECL system. In parallel WB detection of PSA in seminal plasma was carried out by incubating the membrane with (1:5000) rabbit monoclonal anti-PSA antibody (EP1588Y, Abcam) specific to the c-terminal peptide of PSA. This was followed by staining with goat polyclonal anti-rabbit antibody conjugated to HRP and visualised using ECL system.

As can be seen in Figure 59A, the peptides (*b*^{∼}RWQVLASD (SEQ ID NO: 88) and *b*^{∼}RQWVLTAAR (SEQ ID NO: 85)) accumulated on a protein band at an apparent molecular weight of approximate 30 kDa that was identified by the monoclonal antibody to be specific to the C-terminal sequence of PSA. Both peptides can detect PSA to comparable levels as the antibody. Note that the D gatekeeper of the WQVLAS peptide is selected to provide a complementary charge to the flanking residue in the protein sequence. Interestingly, a strong band signal can be achieved using a sufficiently low concentration (250 pM) of the tandem repeat (n=2 and both Y moieties are identical) interferor peptide *b*^{∼}RQWVLTAARGSGSAPAARQWVLTAAR (SEQ ID NO: 89) as the probe (Figure 59 B). In this molecule, all X moieties are a single R residue, and the Z1 linker is GSGSAPAA (SEQ ID NO: 90). This result suggests biomarker detection can be boosted using repeat aggregating sequences (i.e. those with n at least 2, and of which at least two Yi are identical to each other and to a region in a protein), and the probes fan be further diluted in solution. As further confirmation we performed trypsin digestion and mass spectrometry analysis as N-terminal sequencing, which confirm PSA as the main component of the band.

### 4.6.5 Detection of cytokines interleukin 1-β (IL1β) and tumor necrosis factor α (TNFα) in human serum

5 µg each of cytokines interleukin 1-β (IL1β) and tumor necrosis factor α (TNFα) were spiked separately in 5% human serum and separated by electrophoresis using a 4-12% Bis-Tris gel, transferred to PVDF membrane and blocked with 1% BSA in PBS-T. As a control one lane adjacent to the serum sample contained either 5 µg IL1β or 5 µg TNFα alone.

IL1β was detected using the peptides (***b***^{∼}RQQVVFSMSFVQD (SEQ ID NO: 184) and ***b***^{∼}KQQVVFSMSFVQD (SEQ ID NO: 185)). A 10 µM stock solution of the peptides were prepared separately in DMSO and diluted in the formulation buffer (10 mM MES, 100 mM trehalose, 0.05% Tween 20, pH 7.4). The membranes were gently agitated with the peptide formulation at 25°C for 1 h. After four rinses in 10 mM MES, 0.05% Tween 20, pH 7.4 buffer, the membranes were stained with the biotin affinity reagent SRP conjugated to HRP. The results of this PepBlot detection are presented in Figure 60.

For TNFα detection the 10 µM peptides (***b***^{∼}RGLYUYSQVLFP (SEQ ID NO: 186), ***b***^{∼}RGLYLIYSQVLFH (SEQ ID NO: 187)) were diluted (1/40) separately in formulation buffer (10 mM MES, 100 mM trehalose, 0.05% Tween 20, pH 6.5) and gently agitated with the membranes at 25°C for 1 h. After four rinses in 10 mM MES, 0.05% Tween 20, pH 6.5 buffer, the membranes were stained with the biotin affinity reagent SRP conjugated to HRP. The PepBlot data of TNFα detection using interferor peptides were shown in Figure 61.

### 4.7 Quantitative detection of proteins using ELISA

To explore the applications of the present technology in further diagnostics technology, we incorporated the targeted aggregation of selective proteins markers in quantitative platform like ELISA. In this approach the selectivity and sensitivity of the peptide probes to detect protein markers of interest was investigated in complex media in microtitre plates. First, we titrated β-gal in noninduced *E. coli* lysates and captured the whole cell lysates on a 96-well microtitre plate. The captured protein was then probed with a tandem repeat peptide, *b*^{∼}RVIIWSLGNRGSGSAPAARVIIWSLGNR (SEQ ID NO: 91) generated against β-Gal. (Tandem repeat means that n=2 and both Y moieties are identical (here VIIWSLGN (SEQ ID NO: 76)). In this molecule, all X moieties are a single R residue, and the Z1 linker is GSGSAPAA (SEQ ID NO: 90)). The SRP conjugated HRP acted as the secondary regent and the signal detected by calorimetric assay at 450 nm. Our results show good correlation to the concentration of β-Gal in the whole cell lysates, as shown in Fig.62. (Remark that this data are similar to detection of ClpC protein in a dot blot assay, shown in Example 2 (fig. 30))

### 4.8 Quantitative detection of proteins using ForteBio Octet sensor

As an alternative approach, a label free assay set-up was tried, using the Bio-Layer Interferometry (BLI) technology (Octet, ForteBio) in which the tandem repeat probe peptides generated against β-Gal and CRP were immobilized on the sensor probes. The recombinant protein biomarkers (β-gal and CRP) solubilized in phosphate buffered saline, pH 6.8 containing 0.015% Tween 20 and 3 mM EDTA were titrated into the microwell plates and the interaction with the target peptide was then directly read out using an Octet instrument (ForteBio). The high sensitivity of the sensor means picomolar concentration of the analytes can be detected. These results (shown in Fig. 63 and 64 for β-Gal and CRP respectively) suggest the peptide-based assays have the potential to perform quantitative detection of biomarkers, including human biomarkers, in clinical diagnostics that can be readily incorporated into an existing technology platform.

### 4.9 Peptide microarray for detection of proteins

Similar to the coating of the interferors on the sensor probes in Example 4.8, initial experiments showed that the interferor peptides described herein can be covalently immobilized onto a cellulose membrane and used for protein detection (assay based on the PepSpot™ platform (JPT, GmbH). This allows detection of multiple proteins simultaneously (upwards of 50 probes were used together; data not shown).

To assess whether these peptides can also be used in other microarray formats, a preliminary experiment was performed to design a peptide microarray on glass slides by using PepStar™ peptide microarrays (JPT, GmbH). Peptides were designed against ten different targets; these peptides include Hit1, Hit57 (see Example 2), peptides against β-Gal, p53, p16, CS (citrate synthase), CRP, PSA, SEGN (Secretagogin), and A2MG (α-2-Macroglobulin). The peptides were synthesized and spotted (500 µmol) on the glass side with 5 different pin sizes: 62.5 µm, 165 µm, 265 µm 335 µm and 400 µm. Triplicate spots (62.5 µm) of biotin were included in the array along with each pin size as marker.

Different variations of peptides were tested: peptides flanked with natural gatekeeper residues (i.e. short protein fragments containing the Yi moiety), the Yi moiety flanked by an Arg gatekeeper at both sides (i.e. n=1, X₁=X₂=R), and peptides having a tandem repeat with Arg gatekeepers (i.e. n=2, all X moieties equal R, Y₁=Y₂). Moreover, the 'single' peptides - those with only one Y moiety - were linked to the glass slide using two different linker lengths. Each glass slide has 3 repeats of the peptides.

An overview of all the peptides is given in Table 16. For the natural flanked peptides, the short linker consisted of the sequence GS (followed by one or two residues and then the Yi moiety). The long linker is GSPGSPGS (SEQ ID NO: 188). For the single interferor peptides, the short linker consists of the sequence GSA (followed by one R residue, the first gatekeeper) and the long linker is GSPGSPGSA (SEQ ID NO: 189). For the tandem repeat peptides, GA or GAS was used as linker.

Serum sample of a patient containing 317 µg ml⁻¹ CRP was diluted to 10% in PBS-T prior to incubation. A 300 µl of 10% serum was pipetted onto the slide then covered with a glass slip and incubated for 1 h at 37°C. The slides were washed 3x 10 min in PBS-T and incubated with 1:1000 dilution rabbit monoclonal anti-CRP antibody (Abcam) for 1 h followed by 1:1000 Goat anti-rabbit antibody labeled with DyLight 488 (Thermo). To visualize the biotin markers an additional step was included where the slides were incubated with SRP conjugated to DyLight 594. The slides were scanned using a GenePix 4400 (Molecular devices) scanner at 488 nm and 594 nm channels.

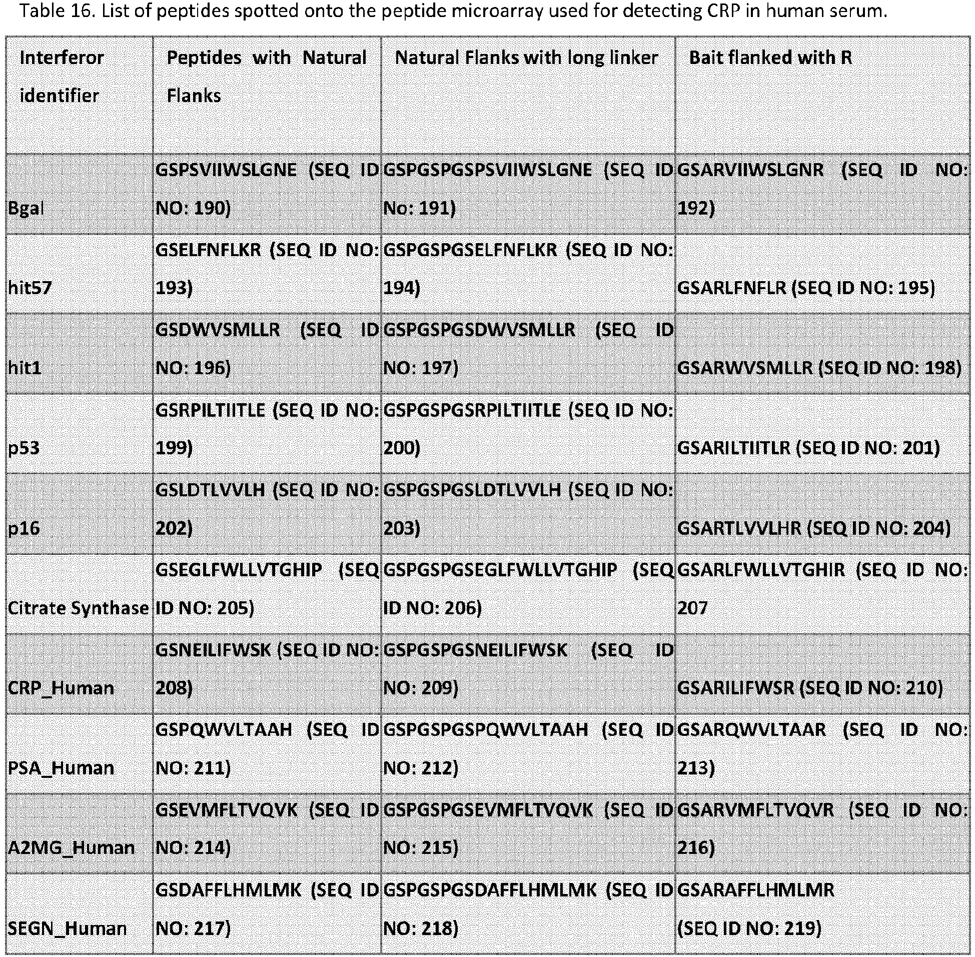

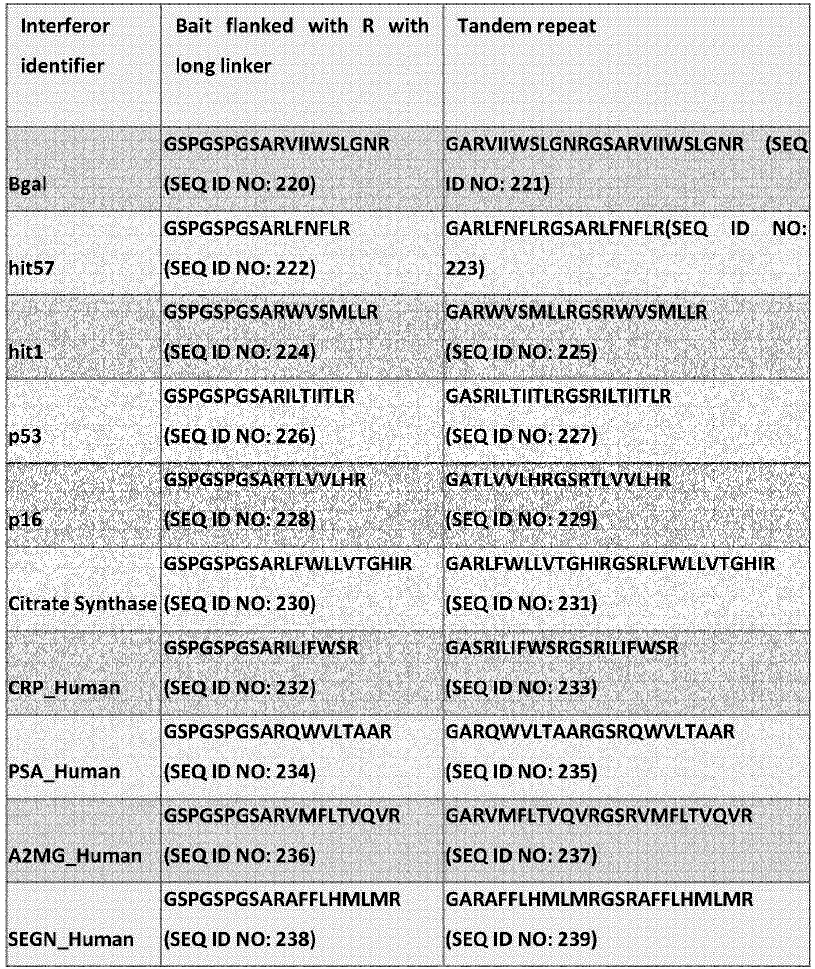

Results for the 'R' flanked peptides with long linkers and the 'R' flanked tandem peptides are shown in Fig. 65.

Even though this is only a preliminary experiment, and it needs further optimization, results are encouraging. As was to be expected, the results for the lowest concentration of CRP are inconclusive (data not shown). For the higher concentration of CRP, however, the peptides that relatively show the highest signal to noise ratio are the CRP single interferor peptides and the CRP tandem repeat peptides. Moreover, these are also the highest signals for each of the microarray slides, and both signals have a signal to noise ratio of over 2.5 .
Using the native sequence does not appear to result in efficient CRP detection. Without being bound to a particular mechanism, this might at least in part be due to the fact that the natural sequences flanking the CRP aggregating moiety contain an opposite charge (negative E residue N-terminal, positive K residue C-terminal). In such instances, it might be better to provide complementation of charges in the gatekeepers, to avoid repulsion of the identical charges and attraction by the opposite ones (in other words, to avoid that the peptides will not align properly due to the charges pushing them in the reverse orientation).

It is believed that further optimization (e.g. other linkers, variation with gatekeeper residues, formulation, pretreatment of slide, kinetics, temperature and other protocol variables) will yield even better results, indicating that the interferor peptides can be used in microarray format to detect multiple analytes in complex samples such as clinical serum.

### 5. Non-infectious medical applications

### 5.1 Targeted aggregation of growth hormone receptors

### 5.1.1 Introduction

The receptor tyrosine kinase (RTK) superfamily is a key target for cancer drug development (Zhang et al., Nat Rev Cancer 2009; 9(1):28). Current anti-cancer therapies target mostly kinases, such as members of the epidermal growth factor receptors, which usually also harbor anti-angiogenic effects as well. However, it usually remains a daunting task to identify inhibitors for each of these molecules that are specific.

Therefore, we aimed to target RTKs using the molecules described herein. By screening a set of peptides designed to specifically induce aggregation, several novel peptides were identified directed against EGFR and VEGFR2 that are capable of inhibiting functional signaling through these receptors.

Vascular endothelial growth factor (VEGF) is an important signaling protein involved, amongst others in angiogenesis. There are three main subtypes of VEGF receptors, numbered 1, 2 and 3. Of these, VEGFR-2 (also known as KDR or Flk-1) appears to mediate most of the known cellular responses to VEGF. One of the pathways activated upon binding of VEGF to VEGFR-2 is the ERK pathway, leading to phosphorylation of Erk1/2. Considering the importance of angiogenesis in cancers, different inhibitors of VEGFR-2 are currently being tested for their potential as anti-cancer drug (e.g. Guo et al., Biochim Biophys Acta. 2010; 1806(1):108-21; Subramanian et al., Clin Lung Cancer. 2010; 11(5):311-9; ramucirumab: Spratlin, Curr Oncol Rep. 2011; 13(2):97-102; vandetanib: Morabito et al., Drugs Today (Barc). 2010; 46(9):683-98). Moreover, anti-VEGFR2 compounds also show promise in treatment of age-related macular degeneration by countering choroidal neovascularization (Miao et al., Biochem Biophys Res Commun. 2006; 345(1):438-45; Takahashi et al., Curr Eye Res. 2008; 33(11):1002-10; Chappelow and Kaiser, Drugs. 2008; 68(8):1029-36).

The epidermal growth factor receptor (EGFR) is a receptor tyrosine kinase of the ErbB family. Four members of the ErbB family have been identified; EGFR (aka ErbB1, HER1), EGFR2 (ErbB2 or HER2), ErbB3 (HER3) and ErbB4 (HER4). EGFR signaling is initiated by ligand binding to the extracellular ligand binding domain. Binding of the protein to a ligand induces receptor dimerization and tyrosine autophosphorylation and leads to cell proliferation. Mutations in this gene are associated with lung cancer, and amplification or over-expression of this gene has been shown to play an important role in the pathogenesis and progression of certain aggressive types of breast cancer. In recent years it has evolved to become an important biomarker and target of therapy for the disease. For instance, EGFR2 is the target of the monoclonal antibody trastuzumab (marketed as Herceptin).

Aggregating peptides against these receptors were designed as already elaborated herein. They consist of two core aggregating sequences (the Yi moieties taken from the aggregating core of the protein sequence), flanked by either D- or R-gatekeeper residues (the numbered X moieties). In most instances, the Yᵢ moieties are identical ('tandem repeat' peptides), but in some, the Yᵢ moieties correspond to two different aggregating domains in the receptor. For these molecules where n=2, the Z₁ moiety is a proline-rich linker that assures both core domains are available for aggregation.

### 5.1.2 Targeting of VEGFR2 with interferor peptides

Different interferor peptides were designed based on the occurrence of aggregation prone regions in the murine VEGFR2 protein (in signal peptide, extracellular region, transmembrane region or cytoplasmic region). To test the ability of peptides to interfere with VEGFR signaling upon ligand binding, the following assay was performed. First, the mVEGFR2 was overexpressed in HEK293 cells via FuGENE transfection. HEK293 cells are devoid of endogenous VEGFR2. The cells were grown for one day and subsequently starved overnight in presence of 10 or 20µM peptide (or carrier control) in DMSO. Next, the cells were stimulated with 25ng/ml VEGF for 5 min in starvation/peptide medium and downstream signaling via the MAP kinase phosphorylation cascade was analysed by studying both ERK1/2 phosphorylation and total ERK1/2 levels. This was assessed by using either western blot or a more quantitative ELISA.

An array of peptides was designed, and synthesized by JPT GmbH in nanomole scale. The peptides targeted several aggregating zones in mouse VEGFR2. The exact sequences of the peptides are shown in table 17. Each peptide was dissolved in DMSO at a concentration of 5 mM, and, after use, kept frozen at -20°C.

**Table 17. Peptide sequences targeting mouse VEGFR2.**

| **R-gatekeeper Peptide** | **D-gatekeeper Peptide** | **Location in the receptor** |
|---|---|---|
| RLAVALWFRPPRLAVALWFR (SEQ ID NO: 240) | DLAVALWFDPPDLAVALWFD (SEQ ID NO: 241) | Predicted signal peptide |
| RIASTVYVRPPRIASTVYVR (SEQ ID NO: 242) | DIASTVYVDPPDIASTVYVD (SEQ ID NO: 243) | Extracellular |
| RILTILANRPPRILTILANR (SEQ ID NO: 244) | DILTILANDPPDILTILAND (SEQ ID NO: 245) | Extracellular |
| RVIILVGTRPPRVIILVGTR (SEQ ID NO: 246) | DVIILVGTDPPDVIILVGTD (SEQ ID NO: 247) | Transmembrane |
| RMISYAGMRPPRMISYAGMR (SEQ ID NO: 248) | DMISYAGMDPPDMISYAGMD (SEQ ID NO: 249) | Extracellular |
| RLMVIVEFRPPRLMVIVEFR (SEQ ID NO: 250) | DLMVIVEFDPPDLMVIVEFD (SEQ ID NO: 251) | Cytoplasmic |
| RTVSTLVIRPPRTVSTLVIR (SEQ ID NO: 252) | DTVSTLVIDPPDTVSTLVID (SEQ ID NO: 253) | Extracellular |
| RLICYSFQRPPRLICYSFQR (SEQ ID NO: 254) | DLICYSFQDPPDLICYSFQD (SEQ ID NO: 255) | Cytoplasmic |
| RVISFHVIRPPRVISFHVIR (SEQ ID NO: 256) | DVISFHVIDPPDVISFHVID (SEQ ID NO: 257) | Extracellular |
| RGYLSIVMRPPRGYLSIVMR (SEQ ID NO: 258) | DGYLSIVMDPPDGYLSIVMD (SEQ ID NO: 259) | Cytoplasmic |
| RLAVALWFRPPRIASTVYVR (SEQ ID NO: 260) | DLAVALWFDPPDIASTVYVD (SEQ ID NO: 261) | Predicted signal peptide + extracellular |
| RIASTVYVRPPRILTILANR (SEQ ID NO: 262) | DIASTVYVDPPDILTILAND (SEQ ID NO: 263) | Extracellular |
| RILTILANRPPRVIILVGTR (SEQ ID NO: 264) | DILTILANDPPDVIILVGTD (SEQ ID NO: 265) | Extracellular + transmembrane |
| RVIILVGTRPPRMISYAGMR (SEQ ID NO: 266) | DVIILVGTDPPDMISYAGMD (SEQ ID NO: 267) | transmembrane + extracellular |
| RMISYAGMRPPRLMVIVEFR (SEQ ID NO: 268) | DMISYAGMDPPDLMVIVEFD (SEQ ID NO: 269) | Extracellular + cytoplasmic |
| RLMVIVEFRPPRTVSTLVIR (SEQ ID NO: 270) | DLMVIVEFDPPDTVSTLVID (SEQ ID NO: 271) | cytoplasmic + extracellular |
| RTVSTLVIRPPRLICYSFQR (SEQ ID NO: 272) | DTVSTLVIDPPDLICYSFQD (SEQ ID NO: 273) | Extracellular + cytoplasmic |
| RLICYSFQRPPRVISFHVIR (SEQ ID NO: 274) | DLICYSFQDPPDVISFHVID (SEQ ID NO: 275) | cytoplasmic + extracellular |
| RVISFHVIRPPRGYLSIVMR (SEQ ID NO: 276) | DVISFHVIDPPDGYLSIVMD (SEQ ID NO: 277) | Extracellular + cytoplasmic |

Tests were first performed with peptides synthesized on microscale, which yielded two peptides that significantly (i.e. more than 75%) reduced ERK1/2 phosphorylation after treatment (results of 3 independent experiments, data not shown). These peptides repeatedly showing most effect on ERK phosphorylation were reordered as high purity peptides and retested in the same assay. To compare different upscaling protocols, the peptides were obtained from two sources: JPT GmbH, Berlin and the lab of Professor Kris Gevaert (University Gent). Results of 2 of these high purity peptides are shown in Fig. 66.

Each of the peptide productions turned out to be equally potent. Peptide B8 has sequence DLAVALWFDPPDLAVALWFD (SEQ ID NO: 241), peptide B12 DMISYAGMDPPDMISYAGMD (SEQ ID NO: 249). The structure of these molecules corresponds to the formula outlined in the application where n is two, X₁ to X₄ is 1 amino acid (i.e., D) Y₁ is 7 amino acids and is identical to Y₂, Z₁ is a two amino acid linker (i.e., PP), and Z₂ is absent. The Y moieties of B8 (LAVALWF (SEQ ID NO: 278)) correspond to part of the sequence of the predicted signal peptide of VEGFR2, the Y moieties of B12 (MISYAGM (SEQ ID NO: 279)) are identical to part of the extracellular sequence of VEGFR2. Note that both these 7 amino acid sequences are uniquely encoded in the mouse genome, i.e. these 7 contiguous amino acid stretches are only found in the mVEGFR2 and not in other mouse proteins. Without being bound to a particular mechanism, as the B8 peptide targets the signal peptide, it is most likely that inhibition occurs upon translation of the VEGFR2 protein (i.e. before the signal peptide is cleaved), indicating that this peptide is internalized by the cell.

As shown in Fig.66A, both peptides almost completely abolish ERK1/2 phosphorylation, while not interfering with total ERK1/2 levels. This effect is already observed with 5 µM of peptide. If the ratio of phosphorylated ERK is plotted versus the total ERK present, it can be seen that adding the interferor molecules almost completely abolishes ligand-induced signaling of VEGFR-2: the ratio is in the range of the non-stimulated cells and much lower than those that were stimulated with the same amount of VEGF but without interferor peptides (Fig. 66B).

To assess the specificity of the identified peptides, a set of peptides was designed based on the sequence of the B8 or B12 peptides, but containing additional mutations with proline residues in the core aggregating regions. Using these 'inactivated' peptides showed no significant reduction in phosphorylation of ERK1/2 (data not shown).

As a next step, to test the specificity of the VEGFR2 peptides, we tested their cross reactivity towards the EGFR family. Therefore, HeLa cells were treated with peptide B8 and B12 and subsequently stimulated with EGF (25ng/ml). In this set-up, B8 and B12 do not inhibit ERK1/2 phosphorylation, indicating that these peptides (i) are specific for VEGFR2 and (ii) don't affect general cellular activity or the ERK1/2 cascade directly as EGFR2 and VEGFR2 use nearly identical signaling cascades (Figure 67)

To better understand the cellular mechanism of peptide inhibition, a preliminary experiment was performed in which cells were stained with an anti-VEGFR2 antibody to track the cellular localization. As can be seen in Fig. 68, in control treated cells, VEGFR2 was mainly present on the cell membrane of HEK293 cells. However, after overnight treatment of cells with 10 µM of peptide B8, the VEGFR2 molecules were no longer present on the cell surface, but present in intracellular vesicles most probably containing aggregated VEGFR2.

Thus, as these experiments show, interferor peptides can be used to specifically target and inhibit the function of a single protein (in this regard, note that the LAVALWF (SEQ ID NO: 278) sequence of B8 is not encoded in the human genome (and thus not present in HEK293 cells), and the MISYAGM (SEQ ID NO: 279) sequence of B12 is unique to the VEGFR2 in humans (but normally not expressed in HEK293 cells)). Given the established role of VEGFR-2 in cancer and AMD pathology and the clinical trials focused on VEGFR-2 inhibition, peptides like those described here have high potential in treatment of these diseases. As the (mouse) sequence used for the B12 peptide is also present in the human VEGFR-2, it is expected that this peptide will show cross-reactivity and can be used for human therapy.

### 5.1.2.1 Inhibition of VEGFR2 in a choroidal neovascularization (CNV) model

Choroidal neovascularization (CNV) is one of the severe pathological consequences of the end-stage of age-related macular degeneration (AMD). Several lines of evidence implicate increased levels of VEGF signaling in the retinas of AMD patients, and inhibition of VEGFR2 has been shown to inhibit neovascularization in a CNV model. To test whether the interferor peptides directed against VEGFR2 can inhibit the function of VEGFR2 in vivo, they were evaluated in a murine model of neovascularization (i.e. laser-induced CNV). This model has been described before (Lambert et al., FASEB Journal; 15:1021-1027, 2001).

Briefly, the experimental set-up was as follows:
3 laser burns were administered on day 1 in the right eye of C57/BI6 mice. On day 1 and day 3, 1µl of either DMSO (negative control), B8-FITC or B12-FITC (the interferor peptides labeled with fluorescein isothiocyanate) or DC101 (an anti-VEGFR-2 mAb, Fischer et al., Cell, 131: 463-475, 2007). The interferor peptides were provided as a 5 mM solution in DMSO, sonicated prior to injection. On day 5, eyes were perfused with TRITC-dextran, mice were humanely killed and dissected, followed by retinal flat mount. Pictures were taken and the % vessels over the total area of the lesion was determined.

Results are shown in Fig. 69
As can be seen from the figure, there is a clear decrease of neovascularization upon treatment with interferors. The effect is not yet as large as that of DC101, but this is a very well characterized antibody, while the peptides have not been optimized yet. The experiment will be repeated over a time course of 14 days, possibly with repeated administration, as this will allow better discrimination between actual neovascularization and inflammation response.

### 5.1.3 Targeting of EGFR2 with interferor peptides

The EGFR2 peptides are designed to target the human EGFR2 receptor. Thus, for these assays, HeLa cells were initially used, as these cells endogenously express EGFR1 and EGFR2. After stimulation with EGF (25 ng/ml), phosphorylation of ERK1/2 is also induced, so that the same assays can be used as described for VEGFR2.

As for VEGFR2, an array of peptides was designed and synhesized by JPT in nanomole scale, each targeting several aggregating zones in human EGFR2 (ErbB2 or HER2). The exact sequences of the peptides are shown in table 18. Each peptide was dissolved in DMSO at a concentration of 5 mM, and, after use, kept frozen at -20°C.

**Table 18. Peptide sequences targeting human EGFR2**

| **R-gatekeeper Peptide** | **D-gatekeeper Peptide** | **Location in the receptor** |
|---|---|---|
| RSLTSTVQRPPRSLTSTVQR (SEQ ID NO: 280) | DSLTSTVQDPPDSLTSTVQD (SEQ ID NO: 281) | Intracellular domain |
| RVWSYGVTRPPRVWSYGVTR (SEQ ID NO: 282) | DVWSYGVTDPPDVWSYGVTD (SEQ ID NO: 96) | Intracellular domain |
| RITGYLYIRPPRITGYLYIR (SEQ ID NO: 283) | DITGYLYIDPPDITGYLYID (SEQ ID NO: 97) | Extracellular domain |
| RLLGISLTRPPRLLGISLTR (SEQ ID NO: 284) | DLLGISLTDPPDLLGISLTD (SEQ ID NO: 98) | Intracellular domain |
| RWGLLLALRPPRWGLLLALR (SEQ ID NO: 93) | DWGLLLALDPPDWGLLLALD (SEQ ID NO: 99) | Signaling peptide |
| RSTVQLVTRPPRSTVQLVTR (SEQ ID NO: 285) | DSTVQLVTDPPDSTVQLVTD (SEQ ID NO: 286) | Intracellular domain |
| RLGVVFGIRPPRLGVVFGIR (SEQ ID NO: 287) | DLGVVFGIDPPDLGVVFGID (SEQ ID NO: 288) | Transmembrane domain |
| RSYGVTVWRPPRSYGVTVWR (SEQ ID NO: 289) | DSYGVTVWDPPDSYGVTVWD (SEQ ID NO: 290) | Intracellular domain |
| RSAVVGILRPPRSAVVGILR (SEQ ID NO: 291) | DSAVVGILDPPDSAVVGILD (SEQ ID NO: 292) | Transmembrane domain |
| RGYLYISARPPRGYLYISAR (SEQ ID NO: 293) | DGYLYISADPPDGYLYISAD (SEQ ID NO: 294) | Extracellular domain |
| RTGYLYISRPPRTGYLYISR (SEQ ID NO: 94) | DTGYLYISDPPDTGYLYISD (SEQ ID NO: 295) | Extracellular domain |
| RIISAVVGRPPRIISAVVGR (SEQ ID NO: 95) | DIISAVVGDPPDIISAVVGD (SEQ ID NO: 296) | Transmembrane domain |
| RVYMIMVRPPRVYMIMVR (SEQ ID NO: 297) | DVYMIMVDPPDVYMIMVD (SEQ ID NO: 298) | Intracellular domain |
| RVVGILLVRPPRVVGILLVR (SEQ ID NO: 299) | DVVGILLVDPPDVVGILLVD (SEQ ID NO: 300) | Transmembrane domain |
| RAVVGILLRPPRAVVGILLR (SEQ ID NO: 301) | DAVVGILLDPPDAVVGILLD (SEQ ID NO: 302) | Transmembrane domain |
| RVLGVVFGRPPRVLGVVFGR (SEQ ID NO: 303) | DVLGVVFGDPPDVLGVVFGD (SEQ ID NO: 304) | Transmembrane domain |
| RVVFGILIRPPRVVFGILIR (SEQ ID NO: 305) | DVVFGILIDPPDVVFGILID (SEQ ID NO: 306) | Transmembrane domain |
| RGVVFGILRPPRGVVFGILR (SEQ ID NO: 307) | DGVVFGILDPPDGVVFGILD (SEQ ID NO: 308) | Transmembrane domain |

For EGFR2, a similar screen was performed as for VEGFR2. Instead of using the time-consuming Western Blot method, however, an ELISA protocol was used that can detect both phospho- and total ERK1/2. 3 peptides that significantly and repeatedly reduced EGF signaling were identified: RWGLLLALRPPRWGLLLALR (SEQ ID NO: 93) (designated A5, targeting the signaling peptide), RTGYLYISRPPRTGYLYISR (SEQ ID NO: 94) (designated A11, targeting the extracellular domain) and RIISAVVGRPPRIISAVVGR (SEQ ID NO: 95) (designated A12, targeting the transmembrane domain). Results are shown in Fig.70

The inhibitory effect is significant, even though the inhibition is incomplete. This is likely due to expression of other EGFRs in HeLa cells (Masui et al., Cancer Res. 1984; 44(3):1002-7), which also signal through ERK phosphorylation and are not targeted by the present peptides.

### 5.2 Anti-inflammatory applications

The nuclear factor (NF)-kappaB pathway has an important role in immunity and inappropriate NF-kappaB pathway activity has been linked with many autoimmune and inflammatory diseases. Multiple mechanisms normally ensure the proper termination of NF-κB pathway activation. In this context, the intracellular ubiquitin-editing protein A20 (also known as Tumor Necrosis Factor Alpha-Induced Protein 3 or TNFAIP3) is a key player in the negative feedback regulation of NF-kappaB signaling in response to multiple stimuli.

To evaluate the potential of interferors in modulation of immune responses, it was decided to inhibit proteins involved in the NF-κB pathway (e.g. A20) using interferor peptides.

Since A20 regulates tumor necrosis factor (TNF)-induced apoptosis and recent genetic studies demonstrate a clear association between several mutations in the human A20 locus and immunopathologies such as Crohn's disease, rheumatoid arthritis, systemic lupus erythematosus, psoriasis and type 1 diabetes (Vereecke et al., Trends Immunol.; 30(8):383-9, 2009), this protein was chosen for a first proof of principle in modulating the NF-κB pathway.

To this end, the kinetics of A20 induction in response to TNF-α were determined. A549 cells (an adenocarcinomic human alveolar basal epithelial cell line) were stimulated with 1000 IU/ml human TNF. At 0, 1, 3, 6 and 8 hours after stimulation, the supernatant was checked for presence of IL-8 using an ELISA, and for presence of IL-6 using a bio-assay (IL-8 and IL-6 expression are NF-κB induced responses upon TNF stimulation). At the same time, cells were lysed and A20 was detected in the lysates using Western blot.

IL-6 was assayed on the basis of the proliferative response of 7TD1 cells (Beyaert, Schulze-Osthoff, Van Roy and Fiers, Cytokine 1991). Briefly, A549 cells are treated for the indicated times with TNF, supernatant is taken and used to treat 7TD1 cells, which are dependent on IL-6 for proliferation. As a standard, a known quantity of recombinant IL-6 is used; by comparing proliferation one can extrapolate the quantities of IL-6.

Results are shown in Fig. 71 .

IL-8 induction reaches a peak 6 hours after stimulation, at which time IL-6 is also clearly induced. Thus, to test activity of A20 interferors on A20 expression and NF-κB signaling, A549 cells were preincubated for 20h with 20 µM A20 interferors (dissolved in DMSO, diluted in serumfree medium to contain only 2% DMSO). After 6h of stimulation with 1000 IU/ml TNF, the same IL-8 ELISA, IL-6 bio-assay and A20 WB were performed.

All tested peptides had the same general formula X1-Y-X2-Z1-X3-Y2-X4 (i.e., n=2 with no external linker), wherein the X moieties are single R residues. Peptides are shown in table 19.

**Table 19. Sequences of A20 interferors.**

| Interferor # | X1 | Y1 | X2 | Z1 | X3 | Y2 | X4 |
|---|---|---|---|---|---|---|---|
| 1 | R | IHIFVLS (SEQ ID NO: 309) | R | PP | R | IHIFVLS (SEQ ID NO: 309) | R |
| 2 | R | HIFVLSN (SEQ ID NO: 310) | R | PP | R | HIFVLSN (SEQ ID NO: 310) | R |
| 3 | R | IFVLSNI (SEQ ID NO: 311) | R | PP | R | IFVLSNI (SEQ ID NO: 311) | R |
| 4 | R | FVLSNIL (SEQ ID NO: 312) | R | PP | R | FVLSNIL (SEQ ID NO: 312) | R |
| 5 | R | IIVIS (SEQ ID NO: 313) | R | PP | R | IIVIS (SEQ ID NO: 313) | R |
| 6 | R | YLMVI (SEQ ID NO: 314) | R | PP | R | YLMVI (SEQ ID NO: 314) | R |
| 7 | R | FSTLSFI (SEQ ID NO: 315) | R | PP | R | FSTLSFI (SEQ ID NO: 315) | R |
| 8 | R | IHIFVLS (SEQ ID NO: 309) | R | PP | R | IIVIS (SEQ IDNO: 313) | R |
| 9 | R | HIFVLSN (SEQ ID NO: 310) | R | PP | R | YLMVI (SEQ ID NO: 314) | R |
| 10 | R | IFVLSNI (SEQ ID NO: 311) | R | PP | R | FSTLSFI (SEQ ID NO: 315) | R |
| 11 | R | IIVIS (SEQ ID NO: 313) | R | | R | IHIFVLSNIL (SEQ ID NO: 316) | R |
| 12 | R | YLMVI (SEQ ID NO: 314) | R | | R | IHIFVLSNIL (SEQ ID NO; 316) | R |

Results of the ELISA assay (2 independent experiments) are shown in Fig. 72. As can be seen from the figure, almost all interferors succeed in significantly increasing IL-8 levels upon TNF stimulation, a measure for NF-κB signaling activity. As A20 is a cytoplasmic protein, the interferor peptides need to enter the cells to be able to inhibit the pathway. Similar, but less pronounced, results were obtained when testing the same interferors where the X moieties were all a single D residue (not shown). Western blots show a decrease of A20 protein levels, but this was harder to quantify and needs further optimization (data not shown). Results of a representative IL-6 bio-assay are shown in Fig.73, and these are very similar to the results obtained with the IL-8 assay.

These results show the feasibility of influencing the immune response regulated by the NF-κB pathway. In a next step, peptides designed against TNF will be used to evaluate inhibition of TNF-induced signals. Examples of interferor peptides that will be tested are RGLYLIYSQVLFDPPRGLYLIYSQVLFD (SEQ ID NO: 317), RGLYLIYSQVLFRPPRGLYLIYSQVLFR (SEQ ID NO: 318), RGLYLIYSQVLFPPPRGLYLIYSQVLFP (SEQ ID NO: 319) and RGLYLIYSQVLFHPPRGLYLIYSQVLFH (SEQ ID NO: 320).

### 6. Application in plants

### 6.1 Introduction

In order to test the protein interference technology *in planta,* a cytosolic player of the brassinosteroids (BR) signalling pathway has been selected. BR are steroidal hormones affecting many cellular processes involved in organ growth and plant development including vascular differentiation, senescence, male fertility, flowering, photomorphogenesis, tolerance to biotic and abiotic stresses (Bajguz and Hayat (2009) Plant Physiol. Biochem 47(1): 1-8). They are also acting on agronomically interesting traits in crop plants as tiller number, leaf size, and leaf angle (Morinake Y et al (2006) Plant Physiol 141(3): 924-31.. As an example, the tissue-specific expression of the sterol C-22 hydroxylases, an enzyme controlling BR hormone levels, can enhance the grain filling in rice (Wu CY et al (2008) Plant Cell 20(8): 2130-45).

In *Arabidopsis* BR are perceived by the plasma membrane leucine-rich repeat (LRR) receptor-like kinases (RLK) BRASSINOSTEROID INSENSITIVE 1 (BRI1) which gets activated upon BR binding and associates with BRIL-ASSOCIATED RECEPTOR KINASE 1 (BAK1) inducing sequential transphosphorylation events by which the fully activated BRI1 can further phosphorylate BR-SIGNALING KINASES (BSKs). Then the phosphorylated BSKs are released from the receptor complex and bind to the BRI1 SUPPRESSOR 1 (BSU1) phopsphates, presumably enhancing its activity. Activated BSU1 inhibits Brassinosteroid Insensitive 2 (BIN2) and other kinases belonging to the glycogen synthase kinase-3 (GSK3) family by dephosphorylating its phospho-tyrosine residue. Unphopsphorylated BIN2 allows accumulation of active unphosphorylated BRASSINAZOLE RESISTANT1 (BZR1) and BZP2/bri1-EMS-SUPPPESSOR1 (BZR2/BES1) transcription factors in the nucleus. Active BZR1 and BZR2/BES1 bind to genomic DNA to regulate BR-target gene expression, thereby modulating growth and development of plants.

In this pathway a good putative target has been identified in the BR negative regulator BIN2 as we expected that interfering with it by using the protein interference technology would result in agronomicallly interesting phenotypic changes. To this end the protein aggregation knock out efficiency was correlated with the phenotypic alterations observed by scoring growth parameters as leaf shape and plant height. These parameters have important agronomic effects on crop yield as it has been shown in rice knock-out brassinosteroid signalling mutants which have higher yields in dense planting conditions.

In the present study two main biological questions are addressed; at first whether it is possible to visualize and evaluate the β-aggregation phenomenom in plants, and then if the targeting of a specific protein of interest by aggregating baits is achievable by proving its functional knock-out.

### 6.2. Design of BIN2 interferor expressing constructs

The GSK3-like kinase BIN2 was selected as a suitable plant target because of its cytosolic and nuclear localization that could permit a targeting by the expressed aggregating peptides (i.e. interferor peptides). We identified two short amino acidic stretches in the BIN2 primary amino acid sequence (see Fig. 74a) with the TANGO algorithm. The algorithm predicted that these two beta-aggregation prone amino acid sequences have a propensity to aggregate higher than 50%. These interferor peptides, which are also further designated herein as baits, cover the BIN2 regions from 44-55aa (bait44) and in the kinase domain from 249-257aa (bait249). For experimental reasons we decided to focus our study on bait249. To induce BIN2 aggregation, several constructs wherein bait249 was C-terminally fused to eGFP fluorescent protein were engineered in plant binary expression vectors. These bait249 expressing constructs have been engineered with and without positively charged amino acids (herein designated as gatekeepers); the construct comprising gatekeepers was designated as "bait249R" and the construct without these gatekeepers was designated as "bait249". The latter construct was made to evaluate the role of gatekeeper residues in enhancing the cytosolic localization of the expressed bait. In addition, a synthetic sequence known to boost the aggregation process (herein designated as booster) has been inserted between the bait and the eGFP through a linker sequence (the specific sequences are depicted in Fig. 74b). Thus, the "bait249R" construct corresponds to an interferor molecule wherein n=1, i.e. of the formula X₁-Y₁-X₂-Z₁, fused to further moieties. Both X₁ and X₂ are R, Y₁ is the sequence QLVEIIKVL (SEQ ID NO: 321), Z₁ is an amino acid linker with the sequence KPAGAAKPGAAG (SEQ ID NO: 112), and the further moieties are an aggregation booster sequence and a GFP moiety for detection.

To evaluate which biochemical features of the aggregating peptides were more optimal in achieving aggregates formation and specific targeting, different variants of the bait249 have also been further engineered. Two constructs expressing the bait249, flanked by 5-7aa naturally flanking the bait sequence in the BIN2 protein, inserted either in single copy (designated as bait249NF) or in tandem repeat (designated as bait249NF_Tand) have been generated. In this second set of vectors no booster of aggregation was inserted (see Fig. 74c). In these cases, part of the natural flanking residues act as gatekeepers. Thus, bait249NF corresponds to an interferor molecule wherein n=1, i.e. of the formula X₁-Y₁-X₂-Z₁, wherein X₁ is D (the last amino acid of the flanking ENAVD (SEQ ID NO: 322) sequence), X₂ is G (the first amino acid of the flanking GTPTREE (SEQ ID NO: 323) sequence), Y₁ is the sequence QLVEIIKVL (SEQ ID NO: 321), Z₁ is an amino acid linker with the sequence AGSPKGAPAAKGSGA (SEQ ID NO: 324), and the molecule is fused to a GFP moiety for detection through the Z₁ linker. Bait249NF_Tand is built from 2 units of bait249NF (i.e. n=2, or X₁-Y₁-X₂-Z₁-X₃-Y₂-X₄-Z₂ with X₁=X₃, X₂=X₄, Y₁=Y₂, Z₁=Z₂ and fusion to the GFP moiety through the Z₂ linker).

### 6.3. Visualization of bait249 aggregation by transient expression in N. benthamiana leaves

The ability of the different baits to induce the formation of aggregates in plants has been initially checked with the Confocal Laser Scanning Microscope (CLSM) in a transient expression system by overexpressing the baits through *Agrobacterium tumefaciens*-mediated infiltration in *Nicotiana benthamiana* leaves. It was observed that the absence of gatekeepers in the bait249 vector strongly induced the formation of insoluble inclusion bodies rather than cytosolic expression (as witnessed in Fig.75a). Conversely the bait249R induced a very strong cytosolic perinuclear aggregation as clearly shown by comparing the effect with the free GFP localization pattern (see Fig.75b,e). The signal detected was more uniform than for bait249 and no inclusion bodies were identified, indicating the importance of the gatekeeper residues. The second set of constructs used in this study showed a clear presence of perinuclear aggregates in both versions, wherein the construct expressing the bait in tandem repeats (bait249NF_Tand) was the strongest aggregating construct as observed with the CLSM (see Fig.75c,d). In these constructs it appears that the extra flanking amino acids (including gatekeepers), added to the bait sequences, play an important role in achieving cytosolic aggregation.

### 6.4. Bait259 co-localizes and physically interacts with the BIN2 target protein in N. benthamiana cells

A transient co-localization assay in *N. benthamiana* leaves of BIN2 and bait249 has been performed to have a fast indication of the bait targeting and co-aggregation tendency towards its target. Thereto, leaves were co-injected with *A. tumefaciens* strains expressing the bait249 strongest aggregating version as observed at the CLSM level, i.e. bait249NF_Tand fused to a RFP fluorescent protein and BIN2 protein fused to eGFP. The expression of the bait249NF_Tand was induced 24 hours before the subsequent fluorescence microscopy evaluation. CLSM analysis of leaves 4-5 days after injection showed a clear co-localization between the bait and the target evidenced both by overlapping expression patterns than by the calculated co-localization Mander's coefficients with values higher than 0.8. The formation of co-localizing cytosolic aggregates was also assessed in fluorescence microscopy (see Fig. 76).

In a next step, after CLSM confirmation of co-localization and protein aggregation between the bait-GFP proteins and the BIN2 target, their stable physical interaction in a transient expression system was also assessed by co-immunoprecipitation (co-IP) experiments. To circumvent the lack of a specific BIN2 antibody the target protein was N-terminally fused to a haemaglutinin (HA) immunological tag. *N*. *benthamiana* leaves were then co-injected with *Agrobacterium* strains transformed with different bait249-GFP expressing vectors and the BIN2-HA was expressed under control of the 35S promoter. Both the bait249 with and without gatekeepers (and with booster of aggregation) and the bait249 in single and in tandem repeats (without booster) were tested. The co-IP experiment was performed by pull down of the GFP tagged baits by anti-GFP agarose coupled beads and subsequent Western blot detection was achieved with an anti-HA monoclonal antibody. Different negative controls were used: i) to test for unspecific binding of GFP to the beads a freeGFP encoding vector was co-injected with BIN2HA; ii) to test for unspecific binding of the synthetic booster to the beads a vector encoding only the booster and the linker sequence fused to GFP has been engineered and co-injected with BIN2-HA, iii)to test for unspecific binding either of the BIN2 protein or of the HA tag to the beads the BIN2-HA construct was injected alone; and iv) also a wild type plant extract has been used as additional negative control. The co-IP experiment indicated a positive interaction for any version of the bait249 tested with BIN2 thereby strongly demonstrating that the bait and the target can interact via the formation of a specific biochemical interaction, i.e. a cross-β-sheet-mediated aggregation (see Fig. 77). This result confirmed what was observed in the co-localization assays thereby generating proof of concept that a physical interaction between the two partners occurs in an *in vivo* system.

### 6.5. Assessing the efficiency of protein aggregation in Arabidopsis transgenic plants

After transformation of the several GFP tagged bait249 expressing constructs the efficiency of aggregation was also monitored in transgenic *Arabidopsis* plants.

The evaluation of the induced aggregator complexes was assessed by imaging the GFP fluorescent protein at the CLSM microscope in each homozygous line.

It was observed that the 35S::bait249R-GFP and the 35S::bait249NF_Tand-GFP expressing lines showed the strongest subcellular GFP expression pattern with a clear perinuclear aggregation in different seedlings tissues (cotyledons, petioles, hypocotyls, and root) (see Fig. 78a-d, e-h).

In contrast, for the *Arabidopsis* plants comprising the 35S::bait249-GFP construct, the absence of gatekeepers impaired the expression of any cytosolically localized aggregates in *Arabidopsis* cells leading to a weaker expression of the reporter protein and the formation of round-shaped insoluble bodies in the cells, as was also observed in transiently transformed leaves of *Nicotiana.* The 35S::bait249NF-GFP expression pattern was weaker than for the bait in tandem (see Fig. 79a-h). For the abovementioned reasons the constructs 35S::bait249-GFP and 35S::bait249NF-GFP have not been considered for further functional analyses.

To investigate at the subcellular level the 35S::bait249R-GFP and 35S::bait249NF_Tand-GFP localization pattern in *Arabidopsis* cells, Transmission Electron Microscopy (TEM) was performed on seedlings 8 days after sowing (D.A.S.) stably expressing these constructs. The cytosolic bait-GFP localization pattern of the lines selected for further analyses have been confirmed by immunogold labeling experiments. In this approach labeling of hypocotyls and root cells in 35S::bait249R-GFP line with an anti-GFP antibody resulted in specific subcellular localization of the bait mainly in the cytosol of cells belonging to the root elongation area. The aggregating proteins appeared to be arranged both in fibrillar structures than in clustered agglomerations indicating that the aggregates can acquire different shapes in the cells (Fig. 80a-c). Golgi stacks were free from gold particles (see Fig. 80c) that instead appear to be more abundant in membrane-like structures (i.e. the ER) (Fig. 80b). The presence of free cytosolic bait249RGFP protein was also rarely found. For 35S::bait249NF_Tand-GFP a massive cytosolic and perinuclear localization was noticed in palisade cells in cotyledons and in root elongation area cells and no peculiar aggregator complexes shapes were detected (Fig. 80d-e).

Biochemical confirmation of aggregator proteins levels has been assessed for each *Arabidopsis* transformed line by Native-PAGE electrophoresis and subsequent Western blot analysis with an anti-GFP monoclonal antibody (see Fig. 81a).

The biochemical nature of the aggregates has been then further analyzed by Fourier Transform-Infra Red (FT-IR) Spectroscopy after their immunoprecipitation (IP) with anti-GFP antibody. FT-IR spectra clearly showed two peaks in absorbance at 1616 and 1680 λ values indicating a high content of β-sheets aggregates in 35S::bait249R-GFP and 35S::bait249-GFP lines, besides their different subcellular localization pattern (see Fig. 81b). For lines 35S::bait249NF-GFP and 35S::bait249NF_Tand-GFP a slighter increase in 1616 and 1680 absorption values was detected indicating a β-sheet content in the immunoprecipitated material, although at a lesser extent than for the previously analyzed lines (Fig. 81c).

### 6.5.1 Phenotype of transgenic Arabidopsis plants

The homozygotic bait249 expressing lines were then further analyzed both *in vitro* and in soil for the appearance of phenotypes showing that a knock-down in BIN2 was occurring. The 35S::bait249R-GFP and 35S::bait249NF_Tand-GFP transgenic seedlings, vertically grown for 8 days *in vitro,* had longer roots and hypocotyls than the untransformed line Col-0; this observation was also confirmed by quantification with the ImageJ software (Fig. 82a). The statistical evaluation performed indicated a statistical significance between Col-0 and transgenic lines. One month old transgenic plants grown in soil also resulted in bigger individuals with respect to Col-0 (Fig. 82a). The 35S::bait249-GFP and 35S::bait249NF-GFP transgenic seedlings did not show any phenotypical difference with Col-0 neither *in vitro* nor in soil conditions and were not included in the further analysis.

In a next step, to provide further evidence that the BIN2 function is affected by its specific aggregation, 35S::bait249R-GFP and 35S::bait249NF-GFP lines were examined for resistance to the brassinosteroid biosynthesis inhibitor, brassinazole (BRZ). As a positive control the triple mutant knock-out in BIN2 and its two close homologues (atsk22 and atsk23) was used (Vert G and Chory J (2006) Nature 441 (7089): 96-100). We expected that if the function of BIN2 was affected it would result in plants being at least partially resistant to brassinazole (please note that the triple mutant (Vert G and Chory J (2006) Nature 441 (7089): 96-100) is resistant to brassinazole. We could indeed show that the transgenic lines showed a partial resistance to the inhibitor brassinazole, as quantified in terms of hypocotyl length (see Fig. 82b).

### 6.5.2 Gene expression changes in transgenic Arabidopsis plants

In a quantitative real-time PCR (qRT-PCR) analysis on BRs-related *DWF4* and *CPD* gene expression we demonstrated a decreased expression level of *DWF4* in the two aggregator lines. In the case of the *CPD* gene expression, an effect was only observed for 35S:bait249NF_Tand:GFP, indicating a feedback inhibition, and thus an activated BRs signaling (see Fig.83a). Accordingly, the analysis of the relative expression levels of a BR-responsive transcription factor from the NAC family showed a slightly increased expression for the 35S:bait249NF_Tand:GFP construct (see Fig. 83a).

Besides BR-related genes, the effect of 35S::bait249R-GFP and 35S::bait249NF_Tand-GFP expression in transgenic *Arabidopsis* lines in the induction of the expression of chaperone proteins was also monitored. Interestingly, the two aggregator lines, but in particular the transgenic plant expressing the bait249 in tandem repeats (35S::bait249NF_Tand-GFP) showed higher (induced) expression levels of *HSP70, HSP90-1, HSP101, HSC70-1, HSC70-2* and *HSC70-3* genes (see Fig. 83b).

### 6.5.3 Morphological evaluation of transgenic Arabidopsis plants

In addition a morphological evaluation at the transmission electron microscopy (TEM) level of the transgenic lines was performed to monitor a possible cytotoxic effect of the aggregator constructs at the subcellular level. With this approach no peculiar alteration in size and shapes of cells and subcellular organelles could be observed in different tissues of the 35S::bait249R-GFP line (see Fig. 84). Occasionally a larger amount of plastoglobuli was found in chloroplasts of the transgenic line. The latter phenomenon is usually an indication of stress which in the present case could also be caused by the *in vitro* growth conditions on nylon meshes. TEM evaluation is also performed on the homozygotic line expressing the bait249 in tandem repeats without booster of aggregation.

To assess the co-localization of the target protein and the bait in stably transformed *Arabidopsis* lines, we aimed to visualize the co-localization between 35S::BIN2-GFP with the strongly expressed aggregator variant bait249NF_Tand fused to tagRFP fluorescent protein expressed under an inducible promoter (pMDC::bait249NF_Tand-RFP). To this end the best 35S::BIN2-GFP expressing line was super-transformed with the estradiol inducible pMDC::bait249NF_Tand-RFP construct. The co-localization assays were performed on the primary transformants after 24 hours of bait249NF_Tand induction and then analyzed at the CLSM. The confocal analysis of 8 D.A.S. transformed seedlings showed a clear overlapping localization pattern of the bait and the target (see Fig. 85). The intensity of the co-localization observed was also quantified with an *ad hoc* software (ImageJ MBF) which released Mander's coefficients close to 1 for all the pictures processed, meaning that a high co-localization between the bait and the target protein occurred.

As for morphological changes, interestingly, a preliminary experiment showed that expression of the bait249 tandem construct rescues the phenotype of the bri1-5 mutant. Reduction of BIN2 expression is expected to rescue the bri1-5 mutant phenotype (Li and Nam, Science 295:1299-1301, 2002).

### 6.6 Materials and Methods for the plant examples

### 6.6.1. Cloning of bait259 aggregator in plant compatible Gateway vectors

By using the TANGO prediction tool (http://tango.switchlab.org/), two aggregator peptides that target two different bait regions (44-55aa: RVVGTGSFGIVFK (SEQ ID NO: 325); 249-257aa: QLVEIIKVL (SEQ ID NO: 321)) in the BIN2 protein were initially selected. For BIN2 region 249-257aa, the aggregator constructs were designed both with (bait249R: RQLVEIIKVLR (SEQ ID NO: 326)) and without (bait249: QLVEIIKVL (SEQ ID NO: 321)) flanking gatekeepers, represented by positively charged arginine residues.

The respective bait sequences were C-terminally fused to a synthetic sequence booster of aggregation (QWQNSTLIVLQNSTVIFEQNSTVIFEQN (SEQ ID NO: 327)) by PCR analysis, introducing a flexible linker sequence KPAGAAKPGAAG (SEQ ID NO: 112).

By using the rationale of checking which bait amino-acidic modifications could lead to better targeting of the BIN2 protein, two other vectors expressing the bait249 were then generated. The bait249 was modified by adding 5-7aa naturally flanking the 249-257aa region in BIN2 (bait249NF: ENAVDQLVEIIKVLGTPTREE (SEQ ID NO: 328)), as well as 6 amino acids (MADDKE (SEQ ID NO: 329)) corresponding to the beginning of the BIN2 protein sequence. The flexible linker sequence has been changed to AGSPKGAPAAKGSGA (SEQ ID NO: 324) and the booster sequence removed. In one construct the bait has been inserted in tandem repeat (bait249NF_Tand: ENAVDQLVEIIKVLGTPTREEENAVDQLVEIIKVLGTPTREE (SEQ ID NO: 330)). The resulting DNA sequences were Gateway cloned in pDONR221 entry vectors. After sequence confirmation, the inserts were transferred to the pK7WG2,0 (Karimi et al. 2007) destination vector to generate plant binary vectors containing the 35S promoter and the heterologous sequence C-terminally fused to eGFP fluorescent tag. The bait249NF_Tand was also cloned in a Gateway pMDC-m13GW vector containing an estradiol inducible promoter (Curtis and Grossniklaus 2003) and the heterologous sequence has been inserted C-terminally fused to tagRFP fluorescent protein (pMDC::bait249NF_Tand-tagRFP). The 35S::BIN2-HA vector has been engineered by using pKWG2,0 destination vector to generate plant binary vectors containing the 35S promoter and the heterologous sequence that was C-terminally fused by homologous recombination to an HA fluorescent tag.

### 6.6.2. Plant materials and growth conditions

*N. benthamiana* plants were grown directly in soil under a 16L /8D photoperiod at 21°C for 45 days and infiltrated before flowering.

*Arabidopsis thaliana* L. (Heyhn.) (Columbia ecotype, Col-0) seedlings were stratified for 2 days at 4°C and germinated in square plates on vertical half-strength Murashige & Skoog (MS) medium (Duchefa) containing 1% sucrose and 0.8% agar, pH 5.9, at 22ºC in a 16-h/8-h light-dark cycle with a light intensity of 80 to 100 mE m⁻² s⁻¹ supplied by cool-white fluorescent tubes (Spectralux Plus 36W/840; Radium) except when indicated. Seedlings grown *in vitro* for 21 days were transferred to soil in growth room with similar light and temperature conditions. The following mutant line was used in this study: bin2-3/atsk22/atsk23 triple mutant (Vert and Chory 2006). Previously described transgenic lines used in the study are: *pBIN2::BIN2*-GFP and *35S::BIN2-*GFP (Vert and Chory, 2006). Brassinazole (BRZ) was purchased from TCI EUROPE N.V. (Belgium), 24-Epibrassinolide (BL) from Fuji Chemical Industries (Japan). The expression of pMDC::bait249NF_Tand-RFP was induced by adding or infiltrating 20µM Estradiol (Sigma) for 24h.

### 6.6.3. Plants transformation

To generate stable *A. thaliana* transgenic plants, the engineered constructs were transformed in A. *tumefaciens* C58C1 strain. Suspensions of the transformed bacterial strains were then used to dip A. *thaliana* Col-0 wild-type floral buds. Primary transformants were selected by germinating the seeds of the transformed flowers on antibiotic-selective medium. Trough 3:1 segregation analysis of the next generation homozygotic transgenic lines were further isolated.

For agroinfiltrations *N. benthamiana* leaves were injected with *A. tumefaciens* strains C58C1(pCH32) transformed with vectors 35S::bait249R-GFP, 35S::bait249-GFP, 35S::bait249NF-GFP, 35S::bait249NF_Tand-GFP, pMDC:bait249NF_Tand-RFP together with 35S::P19, encoding the silencing inhibitor protein p19 derived from the *Tomato Bushy Stunt Virus* (Voinnet et al. 2003).

The strains were used to co-infiltrate, with a syringe without needle, the abaxial side of *N*. *benthamiana* leaves following a previously published protocol with minor modifications (English et al. 1996). Briefly, bacteria were grown overnight at 28°C in YEB medium containing 10 mM 2-(*N-*morpholino)ethanesulfonic acid pH 5.5 and 20 µM acetosyringone. At the optical density (O.D.) of 0.8 bacteria were pelletted, resuspended in 10 mM MES, 10 mM MgCl₂, 100 µM acetosyringone and kept at room temperature for 3 hours before infiltration.

### 6.6.4. Imaging and image analysis

Seedlings were imaged on a laser scanning confocal microscope (Olympus FluoView 1000) with a 20X or a 60X water immersion lens, NA1.2. Image analysis was done with Olympus FluoView FV10-ASW software. For co-localization experiments, Mander's overlap coefficient calculations were done with ImageJ MBF software.

### 6.6.5. Tissue fixation and Immunological labeling for electron microscopy

For morphological studies, fragments (1-2 mm²) of cotyledons, hypocotyls and roots of 35S::bait249R-GFP and 35S::bait249NF_Tand-GFP 8 days after sowing (D.A.S.) seedlings were immersed in a fixative solution of 3 % paraformaldehyde and 2.5 % glutaraldehyde and postfixed in 1% OsO₄ with 1.5% K₃Fe(CN)₆ in 0.1 M NaCacodylate buffer, pH 7.2. Samples were dehydrated through a graded ethanol series, including a bulk staining with 2% uranyl acetate at the 50% ethanol step followed by embedding in Spurr's resin. Ultrathin sections were made using an ultramicrotome (Leica EM UC6) and post-stained in a Leica EM AC20 for 40 min in uranyl acetate at 20 °C and for 10 min in lead stain at 20 °C.

Grids were viewed with a JEM 1010 transmission electron microscope (JEOL, Tokyo, Japan) operating at 80 kV.

For immunocytochemical detection, fragments (1-2 mm²) of cotyledons, hypocotyls and roots of 35S::bait249R-GFP and 35S::bait249NF_Tand-GFP 8 D.A.S. seedlings were immersed in a fixative solution of 2,5 % paraformaldehyde and 0,3% glutaraldehyde in 0.1 M NaCacodylate buffer, pH 7.2. Samples were dehydrated through a graded ethanol series and infiltrated stepwise over 3 d at 4°C in LR-White, hard grade (London Resin), followed by embedding in capsules. Polymerization was done by UV illumination for 24 h at 4°C followed by 16 h at 60°C. Ultrathin sections of gold interference colour were cut with an ultramicrotome (Leica EM UC6) and collected on formvar-coated copper slot grids. All steps of immunolabeling were performed in a humid chamber at room temperature. Grids were floated upside down on 25 µl of blocking solution (5% (w/v) bovine serum albumin (BSA),for 30 min followed by washing five times for 5 min each times with 1% BSA in PBS. Incubation in a 1:50 dilution (1%BSA in PBS) of primary antibodies anti-GFP rabbit (AbCam) for 60 min was followed by washing five times for 5 min each time with 0.1% BSA in PBS. The grids were incubated with PAG 10 nm 1:60 dilution (1% BSA in PBS) (Cell Biology, Utrecht University, The Netherlands) and washed twice for 5 min each time with 0.1% BSA in PBS, PBS, and double-distilled water. Sections were post-stained in a Leica EM AC20 for 40 min in uranyl acetate at 20°C and for 10 min in lead citrate at 20°C. Grids were viewed with a JEM 1010 transmission electron microscope (Jeol Ltd., Tokyo, Japan) operating at 80 kV.

### 6.6.6. Electrophoresis and western blot

Total soluble proteins (TSP) were extracted from *Arabidopsis* 8 D.A.S. seedlings or fragments of *N*. *benthamiana* agroinfiltrated leaves were grinded in a mortar and rinsed with ice-cold 0.01 M phosphate buffered saline (PBS; 10 mM Na₂HPO₄, 10 mM NaH₂PO₄, 150 mM NaCl, pH 7.2), added of proteases inhibitors (Complete® EDTA free, Roche, Germany). The homogenates were then centrifuged at 20,000 x g for 20' at 4°C. Supernatants were quantified for protein content with the Bradford assay (Micro Assay kit, Bio-Rad Laboratories Inc., Hercules, CA, U.S.A.).

The co-immunoprecipitation experiment has been carried out by using agarose-coupled anti-GFP beads (Chromotek) according to the manufacturer instructions.

Whole protein extracts or IP products were fractionated either by SDS-PAGE (Biorad) or BN-PAGE (NativePAGE system, Invitrogen) following the product manuals. For SDS-PAGE, approximately 60 µg of TSP were denatured at 95 °C for 10 min in the presence of 1% SDS and 0.1M DTT and then fractionated by 10%,12% or 15% (w/v) SDS-PAGE gels in TGS running buffer (25 mM Tris, 192 mM glycine and 0.1% SDS). Membranes were blocked overnight with 5% (w/v) nonfat milk in PBS at 4 °C before immunoblotting.

For BN-PAGE, protein extract was added with 20% glycerol and 5 mM Coomassie G-250 before loading onto 3-12% Novex Bis-Tris gradient gels. The electrophoresis was performed in a running buffer containing 50 mM BisTris and 50 mM Tricine (plus 0.004% Coomassie G-250 in cathode buffer) under fixed voltage (100 V) at 21 °C for 120 min. Proteins were transferred onto polyvinylidene fluoride membranes and stained with Coomassie G-250 to show molecular-weight markers (NativeMark, Invitrogen). After fixation with 8% acetic acid for 20 min, the polyvinylidene fluoride membranes were air dried and destained with 100% methanol. Membranes were blocked overnight with 4% BSA in TBS at 4 °C before immunoblotting.

To detected HA tagged BIN2 or GFP tagged bait249, bait249R, bait249NF, bait249NF_Tand on the membrane, the primary antibodies (rat anti-HA, Roche; mouse anti-GFP, Living Colors) have been diluted to 1:1,000 or 1:5,000 respectively in PBS containing 0.1% Tween-20 (PBS-T) and 3% (w/v) nonfat milk and incubated for 1 h at RT. After four rinses with PBS-T, the membrane was stained with horseradish peroxidase (HRP)-conjugated goat anti-rat IgG (GE-Healthcare) or sheep anti-mouse IgG (GE-Healthcare) or and visualized with electrochemical luminescence system.

### 6.6.7. qRT-PCR

RNA was extracted from whole 8 D.A.S. seedlings treated as indicated in 0.5MS medium and RNA was extracted using the RNeasy kit (Qiagen) according to the supplier's instructions and quantified on a NanoDrop® ND-100 Spectrophotometer. Poly(dT) cDNA was prepared from 1 µg of total RNA with iScript reverse transcriptase (Biorad). PCR was performed on 384-well reaction plates, which were heated for 10 min to 95°C, followed by 45 cycles of denaturation for 10 s at 95°C and annealing and extension for 15 s at 60°C and 72°C, respectively. Target quantifications were performed with specific primer pairs listed in Table 20. All PCRs were done in three technical repeats, and at least two biological repeats were used for each sample. For chaperones expressions analysis Taqman primer triplets were purchased from Integrated DNA Technologies (IDT). qRT-PCR was performed using the Applied Biosystems Fast Realtime PCR mixture in a Biorad iQ5 machine with detection of the Fam fluorophore. Relative expression levels were normalized to CDKA and EF expression levels.

**Table 20: Primers used for qRT-PCR analysis**

| **Primer** | **SEQ ID NO** | **Sequence** |
|---|---|---|
| DWF4FOR | 331 | GTGATCTCAGCCGTACATTTGGA |
| DWF4*REV* | 332 | CACGTCGAAAAACTACCACTTCCT |
| CPD*FOR* | 333 | GAATGGAGTGATTACAAGTC |
| CPD*REV* | 334 | GTGAACACATTAGAAGGGCCTG |
| NAC*FOR* | 335 | CTCATTTGCCAATCCTGTATC |
| NAC*REV* | 336 | GCACTGAGATGCGACATCTTG |
| HSP70*FOR* | 337 | TGACTCTTATCCGCTTGAACAG |
| HSP70*REV* | 338 | TCCTACGTTGCTTTCACTGAC |
| HSP90-1*FOR* | 339 | GTGGTTCCTTCACTGTCACTAG |
| HSP90-1*REV* | 340 | TTCACCAAGTCTTTGAGTCTCC |
| HSP101*FOR* | 341 | TGAAAGGAAGAGGATGCAGC |
| HSP101*REV* | 342 | TGTATTTCATCGTGAGAGGCTG |
| HSC70-1*FOR* | 343 | GCTATTCTCAGCGGTGAAGG |
| HSC70-1*REV* | 344 | TTCTCGTCTTGGATGGTGTTC |
| HSC70-2*FOR* | 345 | GAAACAGAACCACTCCCTCG |
| HSC70-2*REV* | 346 | CCAATCAACCTCTTTGCATCG |
| HSC70-3*FOR* | 347 | AACAGAACCACACCGTCTTAC |
| HSC70-3*REV* | 348 | ACCAATCAACCTCTTCGCATC |
| CDKA*FOR* | 349 | ATTGCGTATTGCCACTCTCATAGG |
| CDKA*REV* | 350 | TCCTGACAGGGATACCGAATGC |
| EF*FOR* | 351 | CTGGAGGTTTTGAGGCTGGTAT |
| EF*REV* | 352 | CCAAGGCTGAAAGCAAGAAGA |

### 6.6.8. FT-IR spectroscopy

Fourier Transform Infrared Spectroscopy has been performed on a Tensor 37 FT-IR spectrometer equipped with a BioATR II cell (Bruker) as previously reported (Xu et al.). Briefly, the detector was cooled with liquid nitrogen, and the Bio-ATR II cell was purged by a continuous flow of dried air to minimize water vapour that may interfere with the results. Before and after each measurement, the crystal of the ATR cell was washed with ethanol and water. Samples were measured against background composed of buffer-covered crystal.

## Claims

1. A method for inducing aggregation of a protein comprising contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids occurring in said protein; and
- each Zᵢ is a linker and Zₙ is independently selected from a linker or nothing;
wherein the molecule is capable of inducing aggregation of said protein;
and wherein the method is not practiced on the human or animal body.

2. A method for down-regulating the biological function of a protein comprising contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in said protein; and
- each Zᵢ is a linker and Zₙ is independently selected from a linker or nothing;
wherein the molecule is capable of down-regulating the biological function of said protein;
and wherein the method is not practiced on the human or animal body.

3. A molecule of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein; and
- each Zᵢ is a linker and Zₙ is independently selected from a linker or nothing;
wherein the molecule is capable of binding to, inducing aggregation of or down-regulating the biological function of said protein and wherein binding of the molecule to said protein, aggregation of said protein or downregulation of the biological function of said protein in a subject is therapeutically useful;
for use as a medicament.

4. The molecule of claim 3, wherein the expression or overexpression of said protein is associated with or causative of a disease, for use in treatment or prevention of said disease.

5. The molecule of claim 3, wherein binding of the molecule to said protein, aggregation of said protein or downregulation of the biological function of said protein in a subject is therapeutically useful in cancer, such as wherein the expression or overexpression of said protein is associated with or causative of cancer, for use in treatment of cancer.

6. The molecule of claim 3, wherein binding of the molecule to said protein, aggregation of said protein or downregulation of the biological function of said protein in a subject is therapeutically useful in AMD, such as wherein the expression or overexpression of said protein is associated with or causative of AMD, for use in treatment of AMD.

7. The molecule of claim 3, wherein binding of the molecule to said protein, aggregation of said protein or downregulation of the biological function of said protein in a subject is therapeutically useful in inflammatory disease, such as wherein the expression or overexpression of said protein is associated with or causative of inflammatory disease, for use in treatment of inflammatory disease.

8. A molecule of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of a pathogenic organism; and
- each Zᵢ is a linker and Zₙ is independently selected from a linker or nothing;
wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism; for use as an antipathogenic compound in treatment of an infectious disease caused by the pathogenic organism.

9. A method for killing a pathogenic organism or inhibiting the growth and/or reproduction of a pathogenic organism comprising contacting the pathogenic organism with a molecule of the following structure: (X₂ᵢ₋₁-Yᵢ-X₂ᵢ₋Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of the pathogenic organism; and
- each Zᵢ is a linker and Zₙ is independently selected from a linker or nothing;
wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism; and wherein the method is not practiced on the human or animal body.

10. The molecule for use of claim 8 or the method of claim 9, wherein the pathogen is a viral organism.

11. The molecule for use of claim 8 or the method of claim 9, wherein the pathogen is a microbial organism selected from Gram-positive bacteria, Gram-negative bacteria, mycobacteria, fungi, yeasts and moulds.

12. The molecule for use of any one of claims 3-8 or the method of claim 9, wherein the molecule is provided on an implantable device at least partly coated with the molecule.

13. An implantable device at least partly coated with molecules of the structure (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in a protein of a pathogenic organism; and
- each Zᵢ is a linker and Zₙ is independently selected from a linker or nothing;
wherein the molecule is capable of binding to, inducing aggregation of or down-regulating the biological function of said protein and wherein binding of the molecule to said protein, aggregation of said protein or downregulation of the biological function of said protein in the pathogenic organism kills the pathogenic organism or inhibits the growth and/or reproduction of the pathogenic organism.

14. A method to screen for new inhibitory and/or detection compounds, comprising the steps of:
a) identifying in at least one protein at least one region of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present;
b) synthesizing a molecule of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is a beta-aggregating sequence independently selected from 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein identified in step a); and
- each Zᵢ is a linker and Zₙ is independently selected from a linker or nothing;
c) bringing the molecule made in step b) in contact with the protein of step a); and
d) assessing the function and/or aggregation of the protein.

15. A method to identify new targets for inhibitory compounds, comprising the method of claim 14, wherein the protein in step a) is not a known target for inhibitory compounds.

16. A method to detect a protein in a sample, comprising the steps of:
a) contacting a sample suspected of containing the protein with a molecule of the following structure: (X₂ᵢ₋₁-Yᵢ-X₂ᵢ-Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein to be detected; and
- each Zᵢ is a linker and Zₙ is independently selected from a linker or nothing;
wherein the molecule is capable of binding to, inducing aggregation of or down-regulating the biological function of said protein;
b) detecting the presence of the molecules reacted with the protein.

17. The method of claim 16, wherein the at least one Yᵢ which is identical to or differs with no more than one amino acid substitution from the contiguous stretch of amino acids naturally occurring in the protein to be detected is unique to said protein in said sample.

18. The method of claim 16 or 17, wherein the molecule comprises a detectable label, and the detecting in step b) is through detection of the detectable label.

19. The method of any one of claims 16 to 18, wherein the molecule is provided on a solid support.

20. The method of claim 19, wherein at least two different molecules capable of binding to, inducing aggregation of or down-regulating the biological function of two different proteins to be detected are provided on the solid support.

21. An *in vitro* method for diagnosis of a disease status comprising detecting a protein which is indicative of the disease status in a sample from a subject, wherein the protein is detected by a method comprising the steps of:
a) contacting a sample suspected of containing the protein with a molecule of the following structure: (X₂ᵢ₋₁-Yᵢ-X₂ᵢ₋Zᵢ)ₙ, wherein:
- n is 1 to 5 and i increases from 1 to n with each repeat;
- each X₂ᵢ₋₁ and X₂ᵢ are independently selected from 1 to 4 contiguous amino acids selected from: R, K, E, D, P, N, S, A, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in the protein to be detected; and
- each Zᵢ is a linker and Zₙ is independently selected from a linker or nothing;
wherein the molecule is capable of binding to, inducing aggregation of or down-regulating the biological function of the protein to be detected;
b) detecting the presence of the molecules reacted with the protein.

22. A method for down-regulating the biological function of a protein in a plant or plant cell or plant seed, comprising contacting said protein with a molecule of the following structure: (X₂ᵢ₋₁-Yᵢ-X₂ᵢ-Zᵢ)ₙ, wherein:
- n is an integer from 1 to 5 and i increases from 1 to n with each repeat;
- each X₂ᵢ₋₁ and X₂ᵢ are independently selected from 1 to 4 contiguous amino acids selected from: R, K, E, D, P, N, S, H, G and Q; more particularly 1 to 4 amino acids selected from R, K, E, D and P;
- each Yᵢ is a beta-aggregating sequence independently selected from a stretch of 6 to 16 contiguous amino acids, at least 50% of which are hydrophobic amino acids, and in which at least one aliphatic residue or F is present, and if only one aliphatic residue or F is present, at least one, and preferably at least two, other residues are selected from Y, W, A, M and T; and in which no more than 1, and preferably none, P, R, K, D or E residue is present, and wherein at least one Yᵢ is a stretch identical to or differing with no more than one amino acid substitution from a contiguous stretch of amino acids naturally occurring in said protein in said plant, plant cell or plant seed; and
- each Zᵢ is a linker and Zₙ is independently selected from a linker or nothing;
wherein the molecule is capable of down-regulating the biological function of said protein.

23. The method according to claim 22, wherein the molecule is a polypeptide encoded by a nucleotide sequence present on a recombinant vector and which, upon introduction into the plant cell, plant seed or plant, produces said polypeptide in said plant cell, plant seed or plant.

24. The method of any one of claims 1-2, 9-12, 14-20, or 21-23, the molecule for use of any one of claims 3-8, or 10-12, or the implantable device of claim 13, wherein each X₂ᵢ₋₁ and X₂ᵢ are 1 or 2 amino acids.

25. The method of any one of claims 1-2, 9-12, 14-20, 21-24, the molecule for use of any one of claims 3-8, 10-12, or 24, or the implantable device of claim 13 or 24, wherein at least one, and most particularly all, Yᵢ is a stretch of 6 to 13 amino acids, particularly of 6 to 9 amino acids, more particularly 6 or 7 amino acids.

26. The method of any one of claims 1-2, 9-12, 14-20, 21-25, the molecule for use of any one of claims 3-8, 10-12, or 24-25, or the implantable device of claim 13 or 24-25, wherein at least two, and particularly every, Yᵢ are each independently identical to or differ with no more than one amino acid substitution from a stretch of 6 to 16 contiguous amino acids naturally occurring in a protein.

27. The method of any one of claims 1-2, 9-12, 14-20, 21-26, the molecule for use of any one of claims 3-8, 10-12, or 24-26, or the implantable device of claim 13 or 24-26, wherein each Zᵢ is independently selected from stretch of between 0 and 20 units, wherein a unit is an amino acid, a monosaccharide, a nucleotide, trioxatridecan-succinic acid (Ttds) or PEG.

28. The method of any one of claims 1-2, 9-12, 14-20, 21-26, the molecule for use of any one of claims 3-8, 10-12, or 24-26, or the implantable device of claim 13 or 24-26, wherein each Zᵢ is independently selected from stretch of between 0 and 20 units, wherein a unit is an amino acid.

29. The method of any one of claims 1-2, 9-12, 14-20, 21-26, the molecule for use of any one of claims 3-8, 10-12, or 24-26, or the implantable device of claim 13 or 24-26, wherein each Zᵢ is independently selected from stretch of between 0 and 10 units, wherein a unit is an amino acid or PEG.

30. The method of any one of claims 1-2, 9-12, 14-20, 21-29, the molecule for use of any one of claims 3-8, 10-12, or 24-29, or the implantable device of claim 13 or 24-29, wherein n is 1, X₁ and X₂ are in total no more than 5 amino acids, Y₁ is a stretch of between 6 and 10 amino acids and Z₁ is a stretch of 0 units.

31. The method of any one of claims 1-2, 9-12, 14-20, 21-29, the molecule for use of any one of claims 3-8, 10-12, or 24-29, or the implantable device of claim 13 or 24-29, wherein n is 2, Z₁ is a linker and Z₂ is nothing.

32. The method of any one of claims 1-2, 9-12, 14-20, 21-31, the molecule for use of any one of claims 3-8, 10-12, or 24-31, or the implantable device of claim 13 or 24-31, further comprising a moiety that increases solubility of the molecule.

## Patentansprüche

1. Verfahren zum Induzieren der Aggregation eines Proteins, umfassend das Inkontaktbringen des Proteins mit einem Molekül der folgenden Struktur: (X₂ᵢ₋₁-Yᵢ-X₂ᵢ-Zᵢ)ₙ, worin:
- n eine ganze Zahl von 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die in dem Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, die Aggregation des Proteins zu induzieren,
und wobei das Verfahren nicht am menschlichen oder tierischen Körper durchgeführt wird.

2. Verfahren zum Nach-unten-Regulieren der biologischen Funktion eines Proteins, umfassend das Inkontaktbringen des Proteins mit einem Molekül der folgenden Struktur: (X₂ᵢ₋₁-Yᵢ-X₂ᵢ-Zᵢ)_{η}, worin:
- n eine ganze Zahl von 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in dem Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, die biologische Funktion des Proteins nach unten zu regulieren,
und wobei das Verfahren nicht am menschlichen oder tierischen Körper durchgeführt wird.

3. Molekül der folgenden Struktur: (X₂ᵢ₋₁-Yᵢ-X₂ᵢ-Zᵢ)ₙ, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in dem Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das Protein zu binden, dessen Aggregation zu induzieren oder dessen biologische Funktion nach unten zu regulieren und wobei die Bindung des Moleküls an das Protein, die Aggregation des Proteins oder die Nach-unten-Regulation der biologischen Funktion des Proteins in einem Individuum therapeutisch nützlich ist,
für die Verwendung als Medikament.

4. Molekül nach Anspruch 3, wobei die Expression oder Überexpression des Proteins mit einer Krankheit in Zusammenhang steht oder deren Ursache ist, zur Verwendung bei der Behandlung oder Vorbeugung der Krankheit.

5. Molekül nach Anspruch 3, wobei die Bindung des Moleküls an das Protein, die Aggregation des Proteins oder die Nach-unten-Regulation der biologischen Funktion des Proteins in einem Individuum therapeutisch nützlich bei Krebs ist, wie wenn die Expression oder Überexpression des Proteins mit Krebs in Zusammenhang steht oder dessen Ursache ist, zur Verwendung bei der Behandlung von Krebs.

6. Molekül nach Anspruch 3, wobei die Bindung des Moleküls an das Protein, die Aggregation des Proteins oder die Nach-unten-Regulation der biologischen Funktion des Proteins in einem Individuum therapeutisch nützlich bei AMD ist, wie wenn die Expression oder Überexpression des Proteins mit AMD in Zusammenhang steht oder deren Ursache ist, zur Verwendung bei der Behandlung von AMD.

7. Molekül nach Anspruch 3, wobei die Bindung des Moleküls an das Protein, die Aggregation des Proteins oder die Nach-unten-Regulation der biologischen Funktion des Proteins in einem Individuum therapeutisch nützlich bei entzündlicher Erkrankung ist, wie wenn die Expression oder Überexpression des Proteins mit entzündlicher Erkrankung in Zusammenhang steht oder deren Ursache ist, zur Verwendung bei der Behandlung von entzündlicher Erkrankung.

8. Molekül der folgenden Struktur: (X₂ᵢ₋₁-Yᵢ-X₂ᵢ-Zᵢ)ₙ, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, A, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in einem Protein eines pathogenen Organismus vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das Protein zu binden, dessen Aggregation zu induzieren oder dessen biologische Funktion nach unten zu regulieren, und wobei die Bindung des Moleküls an das Protein, die Aggregation des Proteins oder die Nach-unten-Regulation der biologischen Funktion des Proteins in dem pathogenen Organismus den pathogenen Organismus abtötet oder das Wachstum und/oder die Reproduktion des pathogenen Organismus hemmt;
für die Verwendung als Anti-Pathogen-Verbindung bei der Behandlung einer durch den pathogenen Organismus verursachten Infektionskrankheit.

9. Verfahren zum Abtöten eines pathogenen Organismus oder zur Hemmung des Wachstums und/oder der Reproduktion des pathogenen Organismus, umfassend das Inkontaktbringen des pathogenen Organismus mit einem Molekül der folgenden Struktur: (X₂ᵢ₋₁-Yᵢ-X₂ᵢ-Zᵢ)_{η}, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, A, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die in einem Protein des pathogenen Organismus vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das Protein zu binden, dessen Aggregation zu induzieren oder dessen biologische Funktion nach unten zu regulieren, und wobei die Bindung des Moleküls an das Protein, die Aggregation des Proteins oder die Nach-unten-Regulation der biologischen Funktion des Proteins in dem pathogenen Organismus den pathogenen Organismus abtötet oder das Wachstum und/oder die Reproduktion des pathogenen Organismus hemmt;
und wobei das Verfahren nicht am menschlichen oder tierischen Körper durchgeführt wird.

10. Molekül für die Verwendung nach Anspruch 8 oder Verfahren nach Anspruch 9, wobei das Pathogen ein viraler Organismus ist.

11. Molekül für die Verwendung nach Anspruch 8 oder Verfahren nach Anspruch 9, wobei das Pathogen ein mikrobieller Organismus ist, ausgewählt aus Grampositiven Bakterien, Gram-negativen Bakterien, Mykobakterien, Pilzen, Hefen und Schimmelpilzen.

12. Molekül für die Verwendung nach einem der Ansprüche 3-8 oder Verfahren nach Anspruch 9, wobei das Molekül auf einer implantierbaren Vorrichtung, die zumindest zum Teil mit dem Molekül beschichtet ist, bereitgestellt wird.

13. Implantierbare Vorrichtung, die zumindest zum Teil mit Molekülen der Struktur (X₂ᵢ₋₁-Yᵢ-X₂ᵢ-Zᵢ)ₙ beschichtet ist, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in einem Protein eines pathogenen Organismus vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das Protein zu binden, dessen Aggregation zu induzieren oder dessen biologische Funktion nach unten zu regulieren, und wobei die Bindung des Moleküls an das Protein, die Aggregation des Proteins oder die Nach-unten-Regulation der biologischen Funktion des Proteins in dem pathogenen Organismus den pathogenen Organismus abtötet oder das Wachstum und/oder die Reproduktion des pathogenen Organismus hemmt.

14. Verfahren für das Screening nach neuen inhibitorischen und/oder Nachweis-Verbindungen, das die folgenden Schritte umfasst:
a) Identifizieren, in mindestens einem Protein, mindestens einer Region von 6 bis 16 zusammenhängenden Aminosäuren, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und in der nicht mehr als 1 und vorzugsweise kein Rest P, R, K, D oder E vorhanden ist;
b) Synthetisieren eines Moleküls der folgenden Struktur: (X₂ᵢ₋₁-Yᵢ-X₂ᵢ-Zᵢ)ₙ, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, A, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus 6 bis 16 zusammenhängenden Aminosäuren ausgewählt ist, von denen mindestens 50% hydrophobe Aminosäuren sind, und in denen mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer in Schritt a) identifizierten zusammenhängenden Abfolge von Aminosäuren ist, die natürlicherweise in dem Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
c) Inkontaktbringen des in Schritt b) hergestellten Moleküls mit dem Protein von Schritt a) und
d) Untersuchen der Funktion und/oder Aggregation des Proteins.

15. Verfahren zum Identifizieren neuer Ziele für inhibitorische Verbindungen, umfassend das Verfahren nach Anspruch 14, wobei das Protein in Schritt a) kein bekanntes Ziel für inhibitorische Verbindungen ist.

16. Verfahren zum Nachweisen eines Proteins in einer Probe, das die folgenden Schritte umfasst:
a) Inkontaktbringen einer Probe, von der vermutet wird, dass sie das Protein enthält, mit einem Molekül der folgenden Struktur: (X₂ᵢ₋₁-Yᵢ-X₂ᵢ-Zᵢ)ₙ, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, A, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in dem nachzuweisenden Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das Protein zu binden, dessen Aggregation zu induzieren oder dessen biologische Funktion nach unten zu regulieren;
b) Nachweisen des Vorhandenseins der mit dem Protein umgesetzten Moleküle.

17. Verfahren nach Anspruch 16, wobei das mindestens eine Yᵢ, das identisch mit der natürlicherweise in dem nachzuweisenden Protein vorkommenden zusammenhängenden Abfolge von Aminosäuren ist oder sich durch nicht mehr als eine Aminosäuresubstitution von dieser unterscheidet, für das Protein in der Probe einzigartig ist.

18. Verfahren nach Anspruch 16 oder 17, wobei das Molekül eine nachweisbare Markierung umfasst und das Nachweisen in Schritt b) durch Nachweis der nachweisbaren Markierung erfolgt.

19. Verfahren nach einem der Ansprüche 16 bis 18, wobei das Molekül auf einem festen Träger bereitgestellt wird.

20. Verfahren nach Anspruch 19, wobei mindestens zwei verschiedene Moleküle, die in der Lage sind, an zwei verschiedene nachzuweisende Proteine zu binden, deren Aggregation zu induzieren oder deren biologische Funktion nach unten zu regulieren, auf dem festen Träger bereitgestellt werden.

21. *In vitro*-Verfahren zur Diagnose eines Krankheitsstatus, umfassend das Nachweisen eines Proteins, das für den Krankheitsstatus indikativ ist, in einer Probe von einem Individuum, wobei das Protein durch ein Verfahren nachgewiesen wird, das die folgenden Schritte umfasst:
a) Inkontaktbringen einer Probe, von der vermutet wird, dass sie das Protein enthält, mit einem Molekül der folgenden Struktur: (X₂ᵢ₋₁-Yᵢ-X₂ᵢ-Zᵢ)ₙ, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, A, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in dem nachzuweisenden Protein vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, an das nachzuweisende Protein zu binden, dessen Aggregation zu induzieren oder dessen biologische Funktion nach unten zu regulieren;
b) Nachweisen des Vorhandenseins der mit dem Protein umgesetzten Moleküle.

22. Verfahren zum Nach-unten-Regulieren der biologischen Funktion eines Proteins in einer Pflanze oder einer Pflanzenzelle oder einem Pflanzensamen, umfassend das Inkontaktbringen des Proteins mit einem Molekül der folgenden Struktur: (X₂ᵢ₋₁-Yᵢ-X₂ᵢ-Zᵢ)_{η}, worin:
- n 1 bis 5 ist und i mit jeder Wiederholung von 1 bis n zunimmt;
- jedes X₂ᵢ₋₁ und X₂ᵢ unabhängig aus 1 bis 4 zusammenhängenden Aminosäuren, ausgewählt aus: R, K, E, D, P, N, S, H, G und Q; insbesondere 1 bis 4 Aminosäuren, ausgewählt aus R, K, E, D und P, ausgewählt sind;
- jedes Yᵢ eine beta-Aggregationssequenz ist, die unabhängig aus einer Abfolge von 6 bis 16 zusammenhängenden Aminosäuren ausgewählt ist, von denen mindestens 50% hydrophobe Aminosäuren sind, und in der mindestens ein aliphatischer Rest oder F vorhanden ist und, wenn nur ein aliphatischer Rest oder F vorhanden ist, mindestens ein und vorzugsweise mindestens zwei andere Reste aus Y, W, A, M und T ausgewählt sind; und worin nicht mehr als 1 und vorzugsweise kein Rest P, R, K, D oder E vorhanden ist; und wobei mindestens ein Yᵢ eine Abfolge ist, die identisch mit einer zusammenhängenden Abfolge von Aminosäuren ist, die natürlicherweise in dem Protein in der Pflanze, der Pflanzenzelle oder dem Pflanzensamen vorkommt, oder sich durch nicht mehr als eine Aminosäuresubstitution davon unterscheidet; und
- jedes Zᵢ ein Linker ist und Zₙ unabhängig aus einem Linker oder nichts ausgewählt ist;
wobei das Molekül in der Lage ist, die biologische Funktion des Proteins nach unten zu regulieren.

23. Verfahren nach Anspruch 22, wobei das Molekül ein Polypeptid ist, das von einer Nukleotidsequenz codiert wird, die auf einem rekombinanten Vektor vorhanden ist und die nach Einführung in die Pflanzenzelle, den Pflanzensamen oder die Pflanze das Polypeptid in der Pflanzenzelle, dem Pflanzensamen oder der Pflanze produziert.

24. Verfahren nach einem der Ansprüche 1-2, 9-12, 14-20 oder 21-23, Molekül für die Verwendung nach einem der Ansprüche 3-8 oder 10-12 oder implantierbare Vorrichtung nach Anspruch 13, wobei jedes X₂ᵢ₋₁ und X₂ᵢ 1 oder 2 Aminosäuren sind.

25. Verfahren nach einem der Ansprüche 1-2, 9-12, 14-20, 21-24, Molekül für die Verwendung nach einem der Ansprüche 3-8, 10-12 oder 24 oder implantierbare Vorrichtung nach Anspruch 13 oder 24, wobei mindestens ein und am stärksten bevorzugt alle Yᵢ eine Abfolge von 6 bis 13 Aminosäuren, insbesondere von 6 bis 9 Aminosäuren, genauer gesagt 6 oder 7 Aminosäuren ist/sind.

26. Verfahren nach einem der Ansprüche 1-2, 9-12, 14-20, 21-25, Molekül für die Verwendung nach einem der Ansprüche 3-8, 10-12 oder 24-25 oder implantierbare Vorrichtung nach Anspruch 13 oder 24-25, wobei mindestens zwei und insbesondere jedes Yᵢ jeweils unabhängig identisch mit einer natürlicherweise in einem Protein vorkommenden Abfolge von 6 bis 16 zusammenhängenden Aminosäuren sind oder sich durch nicht mehr als eine Aminosäuresubstitution davon unterscheiden.

27. Verfahren nach einem der Ansprüche 1-2, 9-12, 14-20, 21-26, Molekül für die Verwendung nach einem der Ansprüche 3-8, 10-12 oder 24-26 oder implantierbare Vorrichtung nach Anspruch 13 oder 24-26, wobei jedes Zᵢ unabhängig aus Abfolge von zwischen 0 und 20 Einheiten ausgewählt ist, wobei eine Einheit eine Aminosäure, ein Monosaccharid, ein Nukleotid, Trioxatridecanbernsteinsäure (Ttds) oder PEG ist.

28. Verfahren nach einem der Ansprüche 1-2, 9-12, 14-20, 21-26, Molekül für die Verwendung nach einem der Ansprüche 3-8, 10-12 oder 24-26 oder implantierbare Vorrichtung nach Anspruch 13 oder 24-26, wobei jedes Zᵢ unabhängig aus Abfolge von zwischen 0 und 20 Einheiten ausgewählt ist, wobei eine Einheit eine Aminosäure ist.

29. Verfahren nach einem der Ansprüche 1-2, 9-12, 14-20, 21-26, Molekül für die Verwendung nach einem der Ansprüche 3-8, 10-12 oder 24-26 oder implantierbare Vorrichtung nach Anspruch 13 oder 24-26, wobei jedes Zᵢ unabhängig aus Abfolge von zwischen 0 und 10 Einheiten ausgewählt ist, wobei eine Einheit eine Aminosäure oder PEG ist.

30. Verfahren nach einem der Ansprüche 1-2, 9-12, 14-20, 21-29, Molekül für die Verwendung nach einem der Ansprüche 3-8, 10-12 oder 24-29 oder implantierbare Vorrichtung nach Anspruch 13 oder 24-29, wobei n 1 ist, X₁ und X₂ insgesamt nicht mehr als 5 Aminosäuren sind, Yᵢ eine Abfolge von zwischen 6 und 10 Aminosäuren ist und Z₁ eine Abfolge von 0 Einheiten ist.

31. Verfahren nach einem der Ansprüche 1-2, 9-12, 14-20, 21-29, Molekül für die Verwendung nach einem der Ansprüche 3-8, 10-12 oder 24-29 oder implantierbare Vorrichtung nach Anspruch 13 oder 24-29, wobei n 2 ist, Z₁ ein Linker ist und Z₂ nichts ist.

32. Verfahren nach einem der Ansprüche 1-2, 9-12, 14-20, 21-31, Molekül für die Verwendung nach einem der Ansprüche 3-8, 10-12 oder 24-31 oder implantierbare Vorrichtung nach Anspruch 13 oder 24-31, weiterhin umfassend eine Einheit, die die Löslichkeit des Moleküls erhöht.

## Revendications

1. Méthode d'induction de l'agrégation d'une protéine, comprenant la mise en contact de ladite protéine avec une molécule de structure suivante : (X₂ᵢ₋₁-Yₗ-X₂ᵢ-Zᵢ)ₙ, où :
- n est un nombre entier allant de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent dans ladite protéine ; et
- chaque Zᵢ est un bras de liaison et Zₙ est choisi indépendamment parmi un bras de liaison ou rien ;
où la molécule est capable d'induire l'agrégation de ladite protéine ;
et où la méthode n'est pas pratiquée sur le corps humain ou animal.

2. Méthode de régulation à la baisse de la fonction biologique d'une protéine, comprenant la mise en contact de ladite protéine avec une molécule de structure suivante : (X₂ᵢ₋₁-Yᵢ-X₂ᵢ-Zᵢ)ₙ, où :
- n est un nombre entier allant de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans ladite protéine ; et
- chaque Zᵢ est un bras de liaison et Zₙ est choisi indépendamment parmi un bras de liaison ou rien ;
où la molécule est capable de réguler à la baisse la fonction biologique de ladite protéine ;
et où la méthode n'est pas pratiquée sur le corps humain ou animal.

3. Molécule de structure suivante : (X₂ᵢ₋₁-Yᵢ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans une protéine ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de ladite protéine, et où la fixation de la molécule à ladite protéine, l'agrégation de ladite protéine ou la régulation à la baisse de la fonction biologique de ladite protéine chez un sujet est thérapeutiquement utile ;
pour une utilisation comme médicament.

4. Molécule selon la revendication 3, où l'expression ou la surexpression de ladite protéine est associée à ou causale d'une maladie, pour une utilisation dans le traitement ou la prévention de ladite maladie.

5. Molécule selon la revendication 3, où la fixation de la molécule à ladite protéine, l'agrégation de ladite protéine ou la régulation à la baisse de la fonction biologique de ladite protéine chez un sujet est thérapeutiquement utile dans le cas du cancer, tel qu'où l'expression ou la surexpression de ladite protéine est associée à ou causale d'un cancer, pour une utilisation dans le traitement du cancer.

6. Molécule selon la revendication 3, où la fixation de la molécule à ladite protéine, l'agrégation de ladite protéine ou la régulation à la baisse de la fonction biologique de ladite protéine chez un sujet est thérapeutiquement utile dans le cas de la DMA, tel qu'où l'expression ou la surexpression de ladite protéine est associée à ou causale d'une DMA, pour une utilisation dans le traitement de la DMA.

7. Molécule selon la revendication 3, où la fixation de la molécule à ladite protéine, l'agrégation de ladite protéine ou la régulation à la baisse de la fonction biologique de ladite protéine chez un sujet est thérapeutiquement utile dans le cas d'une maladie inflammatoire, tel qu'où l'expression ou la surexpression de ladite protéine est associée à ou causale d'une maladie inflammatoire, pour une utilisation dans le traitement d'une maladie inflammatoire.

8. Molécule de structure suivante : (X₂ᵢ₋₁-Yᵢ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, A, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans une protéine d'un organisme pathogène ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de ladite protéine, et où la fixation de la molécule à ladite protéine, l'agrégation de ladite protéine ou la régulation à la baisse de la fonction biologique de ladite protéine chez l'organisme pathogène tue l'organisme pathogène ou inhibe la croissance et/ou la reproduction de l'organisme pathogène ;
pour une utilisation comme composé anti-pathogène dans le traitement d'une maladie infectieuse provoquée par l'organisme pathogène.

9. Méthode destinée à tuer un organisme pathogène ou à inhiber la croissance et/ou la reproduction d'un organisme pathogène, comprenant la mise en contact de l'organisme pathogène avec une molécule de structure suivante : (X₂ᵢ₋₁-Yᵢ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, A, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans une protéine de l'organisme pathogène ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de ladite protéine, et où la fixation de la molécule à ladite protéine, l'agrégation de ladite protéine ou la régulation à la baisse de la fonction biologique de ladite protéine chez l'organisme pathogène tue l'organisme pathogène ou inhibe la croissance et/ou la reproduction de l'organisme pathogène ;
et où la méthode n'est pas pratiquée sur le corps humain ou animal.

10. Molécule pour une utilisation selon la revendication 8, ou méthode selon la revendication 9, où le pathogène est un organisme viral.

11. Molécule pour une utilisation selon la revendication 8, ou méthode selon la revendication 9, où le pathogène est un organisme microbien choisi parmi les bactéries à Gram positif, les bactéries à Gram négatif, les mycobactéries, les champignons, les levures et les moisissures.

12. Molécule pour une utilisation selon l'une quelconque des revendications 3-8, ou méthode selon la revendication 9, où la molécule est fournie sur un dispositif implantable au moins partiellement revêtu par la molécule.

13. Dispositif implantable au moins partiellement revêtu par des molécules de structure (X₂ᵢ₋₁-Yᵢ-X₂ᵢ-Zᵢ)ₙ, où :
- n est un nombre entier allant de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans une protéine de l'organisme pathogène ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de ladite protéine, et où la fixation de la molécule à ladite protéine, l'agrégation de ladite protéine ou la régulation à la baisse de la fonction biologique de ladite protéine chez l'organisme pathogène tue l'organisme pathogène ou inhibe la croissance et/ou la reproduction de l'organisme pathogène.

14. Méthode de criblage de nouveaux composés inhibiteurs et/ou de détection, comprenant les étapes :
a) d'identification, dans au moins une protéine, d'au moins une région de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent ;
b) de synthèse d'une molécule de structure suivante : (X₂ᵢ₋₁-Yₗ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, A, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans la protéine identifiée dans l'étape a) ; et
- chaque Zᵢ est un bras de liaison et Zₙ est choisi indépendamment parmi un bras de liaison ou rien ;
c) de mise en contact de la molécule préparée dans l'étape b) avec la protéine de l'étape a) ; et
d) d'évaluation de la fonction et/ou de l'agrégation de la protéine.

15. Méthode d'identification de nouvelles cibles pour des composés inhibiteurs, comprenant la méthode selon la revendication 14, dans laquelle la protéine dans l'étape a) n'est pas une cible connue pour des composés inhibiteurs.

16. Méthode de détection d'une protéine dans un échantillon, comprenant les étapes :
a) de mise en contact d'un échantillon, suspecté comme contenant la protéine, avec une molécule de structure suivante : (X₂ᵢ₋₁-Yᵢ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, A, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans la protéine à détecter ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de ladite protéine ;
b) de détection de la présence des molécules ayant réagi avec la protéine.

17. Méthode selon la revendication 16, dans laquelle le au moins un Yₗ qui est identique ou différent d'au plus une substitution d'acide aminé par rapport au segment contigu d'acides aminés étant présent naturellement dans la protéine à détecter est unique vis-à-vis de ladite protéine dans ledit échantillon.

18. Méthode selon la revendication 16 ou 17, dans laquelle la molécule comprend un marqueur détectable, et la détection dans l'étape b) est faite via la détection du marqueur détectable.

19. Méthode selon l'une quelconque des revendications 16 à 18, dans laquelle la molécule est fournie sur un support solide.

20. Méthode selon la revendication 19, dans laquelle au moins deux molécules différentes capables de se fixer à, d'induire l'agrégation de, ou de réguler à la baisse la fonction biologique de deux protéines différentes à détecter, sont fournies sur le support solide.

21. Méthode *in vitro* de diagnostic d'un état pathologique, comprenant la détection d'une protéine qui constitue une indication de l'état pathologique dans un échantillon issu d'un sujet, dans laquelle la protéine est détectée par une méthode comprenant les étapes :
a) de mise en contact d'un échantillon, suspecté comme contenant la protéine, avec une molécule de structure suivante : (X₂ᵢ₋₁-Yᵢ-X₂ᵢ-Zᵢ)ₙ, où :
- n va de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, A, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans la protéine à détecter ; et
- chaque Zᵢ est un bras de liaison et Zₙ est choisi indépendamment parmi un bras de liaison ou rien ;
où la molécule est capable de se fixer à, d'induire l'agrégation de, ou de réguler à la baisse la fonction biologique de la protéine à détecter ;
b) de détection de la présence des molécules ayant réagi avec la protéine.

22. Méthode de régulation à la baisse de la fonction biologique d'une protéine dans une plante ou une cellule végétale ou une semence végétale, comprenant la mise en contact de ladite protéine avec une molécule de structure suivante : (X₂ᵢ₋₁-Yᵢ-X₂ᵢ-Zᵢ)ₙ, où :
- n est un nombre entier allant de 1 à 5 et i augmente de 1 à n avec chaque répétition ;
- chaque X₂ᵢ₋₁ et X₂ᵢ sont choisis indépendamment parmi de 1 à 4 acides aminés contigus choisis parmi R, K, E, D, P, N, S, H, G et Q ; plus particulièrement de 1 à 4 acides aminés choisis parmi R, K, E, D et P ;
- chaque Yₗ est une séquence de bêta-agrégation choisie indépendamment parmi un segment de 6 à 16 acides aminés contigus, au moins 50% d'entre eux étant des acides aminés hydrophobes, et où au moins un résidu aliphatique ou F est présent, et si seulement un résidu aliphatique ou F est présent, au moins un, et préférablement au moins deux, autres résidus sont choisis parmi Y, W, A, M et T ; et où pas plus d'un, et préférablement aucun, résidu P, R, K, D ou E n'est présent, et où au moins un Yₗ est un segment identique ou différent d'au plus une substitution d'acide aminé par rapport à un segment contigu d'acides aminés étant présent naturellement dans ladite protéine dans ladite plante, cellule végétale ou semence végétale ; et
- chaque Zᵢ est un bras de liaison et Zₙ est choisi indépendamment parmi un bras de liaison ou rien ;
où la molécule est capable de réguler à la baisse la fonction biologique de ladite protéine.

23. Méthode selon la revendication 22, dans laquelle la molécule est un polypeptide codé par une séquence nucléotidique présente sur un vecteur recombiné et qui, lors de son introduction dans la cellule végétale, semence végétale ou plante, produit ledit polypeptide dans ladite cellule végétale, semence végétale ou plante.

24. Méthode selon l'une quelconque des revendications 1-2, 9-12, 14-20, ou 21-23, molécule pour une utilisation selon l'une quelconque des revendications 3-8, ou 10-12, ou dispositif implantable selon la revendication 13, où chaque X₂ᵢ₋₁ et X₂ᵢ sont 1 ou 2 acides aminés.

25. Méthode selon l'une quelconque des revendications 1-2, 9-12, 14-20, 21-24, molécule pour une utilisation selon l'une quelconque des revendications 3-8, 10-12, ou 24, ou dispositif implantable selon la revendication 13 ou 24, où au moins un, et tout particulièrement tous, parmi les Yₗ sont un segment de 6 à 13 acides aminés, particulièrement de 6 à 9 acides aminés, plus particulièrement de 6 ou 7 acides aminés.

26. Méthode selon l'une quelconque des revendications 1-2, 9-12, 14-20, 21-25, molécule pour une utilisation selon l'une quelconque des revendications 3-8, 10-12, ou 24-25, ou dispositif implantable selon la revendication 13 ou 24-25, où au moins deux, et tout particulièrement tous, parmi les Yₗ sont chacun indépendamment identiques ou diffèrent d'au plus une substitution d'acide aminé par rapport à un segment contigu de 6 à 16 acides aminés contigus étant présent naturellement dans une protéine.

27. Méthode selon l'une quelconque des revendications 1-2, 9-12, 14-20, 21-26, molécule pour une utilisation selon l'une quelconque des revendications 3-8, 10-12, ou 24-26, ou dispositif implantable selon la revendication 13 ou 24-26, où chaque Zᵢ est choisi indépendamment parmi un segment compris entre 0 et 20 unités, où une unité est un acide aminé, un monosaccharide, un nucléotide, un acide trioxatridécan-succinique (Ttds) ou du PEG.

28. Méthode selon l'une quelconque des revendications 1-2, 9-12, 14-20, 21-26, molécule pour une utilisation selon l'une quelconque des revendications 3-8, 10-12, ou 24-26, ou dispositif implantable selon la revendication 13 ou 24-26, où chaque Zᵢ est choisi indépendamment parmi un segment compris entre 0 et 20 unités, où une unité est un acide aminé.

29. Méthode selon l'une quelconque des revendications 1-2, 9-12, 14-20, 21-26, molécule pour une utilisation selon l'une quelconque des revendications 3-8, 10-12, ou 24-26, ou dispositif implantable selon la revendication 13 ou 24-26, où chaque Zᵢ est choisi indépendamment parmi un segment compris entre 0 et 10 unités, où une unité est un acide aminé ou du PEG.

30. Méthode selon l'une quelconque des revendications 1-2, 9-12, 14-20, 21-29, molécule pour une utilisation selon l'une quelconque des revendications 3-8, 10-12, ou 24-29, ou dispositif implantable selon la revendication 13 ou 24-29, où n vaut 1, X₁ et X₂ sont au total au plus 5 acides aminés, Yₗ est un segment compris entre 6 et 10 acides aminés et Z₁ est un segment de 0 unité.

31. Méthode selon l'une quelconque des revendications 1-2, 9-12, 14-20, 21-29, molécule pour une utilisation selon l'une quelconque des revendications 3-8, 10-12, ou 24-29, ou dispositif implantable selon la revendication 13 ou 24-29, où n vaut 2, Z₁ est un bras de liaison et Z₂ n'est rien.

32. Méthode selon l'une quelconque des revendications 1-2, 9-12, 14-20, 21-31, molécule pour une utilisation selon l'une quelconque des revendications 3-8, 10-12, ou 24-31, ou dispositif implantable selon la revendication 13 ou 24-31, comprenant en outre un motif qui augmente la solubilité de la molécule.


### SEQUENCE LISTING

<110> VIB VZW
   VRIJE UNIVERSITEIT BRUSSEL
   KATHOLIEKE UNIVERSITEIT LEUVEN, K.U.LEUVEN R&D
   UNIVERSITEIT GENT
<120> Molecules and methods for inhibition and detection of proteins
<130> JSC/PRO2/369
<150> EP 11157842.3
   <151> 2011-03-11
<150> US 61/451,855
   <151> 2011-03-11
<150> US 61/487,595
   <151> 2011-05-18
<150> EP 11176725.7
   <151> 2011-08-05
<160> 352
<170> PatentIn version 3.5
<210> 1
   <211> 17
   <212> PRT
   <213> artificial
<220>
   <223> Peptide sequence
<400> 1
   Leu Gln Gln Tyr Thr Leu Leu Leu Ile Tyr Leu Ser Val Ala Thr Ala
   1               5                   10                  15
   Lys
<210> 2
   <211> 883
   <212> PRT
   <213> Candida albicans
<400> 2
   Met Leu Gln Gln Tyr Thr Leu Leu Leu Ile Tyr Leu Ser Val Ala Thr
   1               5                   10                  15
   Ala Lys Thr Ile Thr Gly Val Phe Asn Ser Phe Asn Ser Leu Thr Trp
               20                  25                  30
   Ser Asn Ala Ala Thr Tyr Asn Tyr Lys Gly Pro Gly Thr Pro Thr Trp
           35                  40                  45
   Asn Ala Val Leu Gly Trp Ser Leu Asp Gly Thr Ser Ala Ser Pro Gly
       50                  55                  60
   Asp Thr Phe Thr Leu Asn Met Pro Cys Val Phe Lys Phe Thr Thr Ser
   65                  70                  75                  80
   Gln Thr Ser Val Asp Leu Thr Ala His Gly Val Lys Tyr Ala Thr Cys
                   85                  90                  95
   Gln Phe Gln Ala Gly Glu Glu Phe Met Thr Phe Ser Thr Leu Thr Cys
               100                 105                 110
   Thr Val Ser Asn Thr Leu Thr Pro Ser Ile Lys Ala Leu Gly Thr Val
           115                 120                 125
   Thr Leu Pro Leu Ala Phe Asn Val Gly Gly Thr Gly Ser Ser Val Asp
       130                 135                 140
   Leu Glu Asp Ser Lys Cys Phe Thr Ala Gly Thr Asn Thr Val Thr Phe
   145                 150                 155                 160
   Asn Asp Gly Gly Lys Lys Ile Ser Ile Asn Val Asp Phe Glu Arg Ser
                   165                 170                 175
   Asn Val Asp Pro Lys Gly Tyr Leu Thr Asp Ser Arg Val Ile Pro Ser
               180                 185                 190
   Leu Asn Lys Val Ser Thr Leu Phe Val Ala Pro Gln Cys Ala Asn Gly
           195                 200                 205
   Tyr Thr Ser Gly Thr Met Gly Phe Ala Asn Thr Tyr Gly Asp Val Gln
       210                 215                 220
   Ile Asp Cys Ser Asn Ile His Val Gly Ile Thr Lys Gly Leu Asn Asp
   225                 230                 235                 240
   Trp Asn Tyr Pro Val Ser Ser Glu Ser Phe Ser Tyr Thr Lys Thr Cys
                   245                 250                 255
   Ser Ser Asn Gly Ile Phe Ile Thr Tyr Lys Asn Val Pro Ala Gly Tyr
               260                 265                 270
   Arg Pro Phe Val Asp Ala Tyr Ile Ser Ala Thr Asp Val Asn Ser Tyr
           275                 280                 285
   Thr Leu Ser Tyr Ala Asn Glu Tyr Thr Cys Ala Gly Gly Tyr Trp Gln
       290                 295                 300
   Arg Ala Pro Phe Thr Leu Arg Trp Thr Gly Tyr Arg Asn Ser Asp Ala
   305 310 315 320
   Gly Ser Asn Gly Ile Val Ile Val Ala Thr Thr Arg Thr Val Thr Asp
   325 330 335
   Ser Thr Thr Ala Val Thr Thr Leu Pro Phe Asp Pro Asn Arg Asp Lys
   340 345 350
   Thr Lys Thr Ile Glu Ile Leu Lys Pro Ile Pro Thr Thr Thr Ile Thr
   355 360 365
   Thr Ser Tyr Val Gly Val Thr Thr Ser Tyr Ser Thr Lys Thr Ala Pro
   370 375 380
   Ile Gly Glu Thr Ala Thr Val Ile Val Asp Ile Pro Tyr His Thr Thr
   385 390 395 400
   Thr Thr Val Thr Ser Lys Trp Thr Gly Thr Ile Thr Ser Thr Thr Thr
   405 410 415
   His Thr Asn Pro Thr Asp Ser Ile Asp Thr Val Ile Val Gln Val Pro
   420 425 430
   Ser Pro Asn Pro Thr Val Thr Thr Thr Glu Tyr Trp Ser Gln Ser Phe
   435 440 445
   Ala Thr Thr Thr Thr Ile Thr Gly Pro Pro Gly Asn Thr Asp Thr Val
   450 455 460
   Leu Ile Arg Glu Pro Pro Asn His Thr Val Thr Thr Thr Glu Tyr Trp
   465 470 475 480
   Ser Glu Ser Tyr Thr Thr Thr Ser Thr Phe Thr Ala Pro Pro Gly Gly
   485 490 495
   Thr Asp Ser Val Ile Ile Lys Glu Pro Pro Asn Pro Thr Val Thr Thr
   500 505 510
   Thr Glu Tyr Trp Ser Glu Ser Tyr Thr Thr Thr Thr Thr Val Thr Ala
   515 520 525
   Pro Pro Gly Gly Thr Asp Thr Val Ile Ile Arg Glu Pro Pro Asn His
   530 535 540
   Thr Val Thr Thr Thr Glu Tyr Trp Ser Gln Ser Tyr Thr Thr Thr Thr
   545 550 555 560
   Thr Val Ile Ala Pro Pro Gly Gly Thr Asp Ser Val Ile Ile Arg Glu
   565 570 575
   Pro Pro Asn Pro Thr Val Thr Thr Thr Glu Tyr Trp Ser Gln Ser Tyr
   580 585 590
   Ala Thr Thr Thr Thr Ile Thr Ala Pro Pro Gly Glu Thr Asp Thr Val
   595 600 605
   Leu Ile Arg Glu Pro Pro Asn His Thr Val Thr Thr Thr Glu Tyr Trp
   610 615 620
   Ser Gln Ser Tyr Ala Thr Thr Thr Thr Ile Thr Ala Pro Pro Gly Glu
   625 630 635 640
   Thr Asp Thr Val Leu Ile Arg Glu Pro Pro Asn His Thr Val Thr Thr
   645 650 655
   Thr Glu Tyr Trp Ser Gln Ser Tyr Thr Thr Thr Thr Thr Val Ile Ala
   660 665 670
   Pro Pro Gly Gly Thr Asp Ser Val Ile Ile Lys Glu Pro Pro Asn Pro
   675 680 685
   Thr Val Thr Thr Thr Glu Tyr Trp Ser Gln Ser Tyr Ala Thr Thr Thr
   690 695 700
   Thr Ile Thr Ala Pro Pro Gly Glu Thr Asp Thr Val Leu Ile Arg Glu
   705 710 715 720
   Pro Pro Asn His Thr Val Thr Thr Thr Glu Tyr Trp Ser Gln Ser Tyr
   725 730 735
   Ala Thr Thr Thr Thr Ile Thr Ala Pro Pro Gly Glu Thr Asp Thr Val
   740 745 750
   Leu Ile Arg Glu Pro Pro Asn His Thr Val Thr Thr Thr Glu Tyr Trp
   755 760 765
   Ser Gln Ser Phe Ala Thr Thr Thr Thr Val Thr Ala Pro Pro Gly Gly
   770 775 780
   Thr Asp Thr Val Ile Ile Arg Glu Pro Pro Asn His Thr Val Thr Thr
   785 790 795 800
   Thr Glu Tyr Trp Ser Gln Ser Phe Ala Thr Thr Thr Thr Val Thr Ala
   805 810 815
   Pro Pro Gly Gly Thr Asp Thr Val Leu Ile Arg Glu Pro Pro Asn Pro
   820 825 830
   Thr Val Thr Thr Thr Glu Tyr Trp Ser Gln Pro Tyr Thr Thr Thr Thr
   835 840 845
   Thr Val Ile Ala Pro Pro Gly Gly Thr Asp Thr Val Ile Ile Tyr Asp
   850 855 860
   Thr Met Ser Ser Ser Glu Ile Ser Ser Phe Ser Arg Pro His Tyr Thr
   865 870 875 880
   Asn His Thr
<210> 3
   <211> 1023
   <212> PRT
   <213> Escherichia coli
<400> 3
   Thr Met Ile Thr Asp Ser Leu Ala Val Val Leu Gln Arg Arg Asp Trp
   1 5 10 15
   Glu Asn Pro Gly Val Thr Gln Leu Asn Arg Leu Ala Ala His Pro Pro
   20 25 30
   Phe Ala Ser Trp Arg Asn Ser Glu Glu Ala Arg Thr Asp Arg Pro Ser
   35 40 45
   Gln Gln Leu Arg Ser Leu Asn Gly Glu Trp Arg Phe Ala Trp Phe Pro
   50 55 60
   Ala Pro Glu Ala Val Pro Glu Ser Trp Leu Glu Cys Asp Leu Pro Asp
   65 70 75 80
   Ala Asp Thr Val Val Val Pro Ser Asn Trp Gln Met His Gly Tyr Asp
   85 90 95
   Ala Pro Ile Tyr Thr Asn Val Thr Tyr Pro Ile Thr Val Asn Pro Pro
   100 105 110
   Phe Val Pro Ala Glu Asn Pro Thr Gly Cys Tyr Ser Leu Thr Phe Asn
   115 120 125
   Ile Asp Glu Ser Trp Leu Gln Glu Gly Gln Thr Arg Ile Ile Phe Asp
       130                 135                 140
   Gly Val Asn Ser Ala Phe His Leu Trp Cys Asn Gly Arg Trp Val Gly
   145                 150                 155                 160
   Tyr Gly Gln Asp Ser Arg Leu Pro Ser Glu Phe Asp Leu Ser Ala Phe
                   165                 170                 175
   Leu Arg Ala Gly Glu Asn Arg Leu Ala Val Met Val Leu Arg Trp Ser
               180                 185                 190
   Asp Gly Ser Tyr Leu Glu Asp Gln Asp Met Trp Arg Met Ser Gly Ile
           195                 200                 205
   Phe Arg Asp Val Ser Leu Leu His Lys Pro Thr Thr Gln Ile Ser Asp
       210                 215                 220
   Phe Gln Val Thr Thr Leu Phe Asn Asp Asp Phe Ser Arg Ala Val Leu
   225                 230                 235                 240
   Glu Ala Glu Val Gln Met Tyr Gly Glu Leu Arg Asp Glu Leu Arg Val
                   245                 250                 255
   Thr Val Ser Leu Trp Gln Gly Glu Thr Gln Val Ala Ser Gly Thr Ala
               260                 265                 270
   Pro Phe Gly Gly Glu Ile Ile Asp Glu Arg Gly Gly Tyr Ala Asp Arg
           275                 280                 285
   Val Thr Leu Arg Leu Asn Val Glu Asn Pro Glu Leu Trp Ser Ala Glu
       290                 295                 300
   Ile Pro Asn Leu Tyr Arg Ala Val Val Glu Leu His Thr Ala Asp Gly
   305                 310                 315                 320
   Thr Leu Ile Glu Ala Glu Ala Cys Asp Val Gly Phe Arg Glu Val Arg
                   325                 330                 335
   Ile Glu Asn Gly Leu Leu Leu Leu Asn Gly Lys Pro Leu Leu Ile Arg
               340                 345                 350
   Gly Val Asn Arg His Glu His His Pro Leu His Gly Gln Val Met Asp
           355                 360                 365
   Glu Gln Thr Met Val Gln Asp Ile Leu Leu Met Lys Gln Asn Asn Phe
       370                 375                 380
   Asn Ala Val Arg Cys Ser His Tyr Pro Asn His Pro Leu Trp Tyr Thr
   385 390 395 400
   Leu Cys Asp Arg Tyr Gly Leu Tyr Val Val Asp Glu Ala Asn Ile Glu
   405 410 415
   Thr His Gly Met Val Pro Met Asn Arg Leu Thr Asp Asp Pro Arg Trp
   420 425 430
   Leu Pro Ala Met Ser Glu Arg Val Thr Arg Met Val Gln Arg Asp Arg
   435 440 445
   Asn His Pro Ser Val Ile Ile Trp Ser Leu Gly Asn Glu Ser Gly His
   450 455 460
   Gly Ala Asn His Asp Ala Leu Tyr Arg Trp Ile Lys Ser Val Asp Pro
   465 470 475 480
   Ser Arg Pro Val Gln Tyr Glu Gly Gly Gly Ala Asp Thr Thr Ala Thr
   485 490 495
   Asp Ile Ile Cys Pro Met Tyr Ala Arg Val Asp Glu Asp Gln Pro Phe
   500 505 510
   Pro Ala Val Pro Lys Trp Ser Ile Lys Lys Trp Leu Ser Leu Pro Gly
   515 520 525
   Glu Met Arg Pro Leu Ile Leu Cys Glu Tyr Ala His Ala Met Gly Asn
   530 535 540
   Ser Leu Gly Gly Phe Ala Lys Tyr Trp Gln Ala Phe Arg Gln Tyr Pro
   545 550 555 560
   Arg Leu Gln Gly Gly Phe Val Trp Asp Trp Val Asp Gln Ser Leu Ile
   565 570 575
   Lys Tyr Asp Glu Asn Gly Asn Pro Trp Ser Ala Tyr Gly Gly Asp Phe
   580 585 590
   Gly Asp Thr Pro Asn Asp Arg Gln Phe Cys Met Asn Gly Leu Val Phe
   595 600 605
   Ala Asp Arg Thr Pro His Pro Ala Leu Thr Glu Ala Lys His Gln Gln
   610 615 620
   Gln Tyr Phe Gln Phe Arg Leu Ser Gly Arg Thr Ile Glu Val Thr Ser
   625 630 635 640
   Glu Tyr Leu Phe Arg His Ser Asp Asn Glu Phe Leu His Trp Met Val
                   645                 650                 655
   Ala Leu Asp Gly Lys Pro Leu Ala Ser Gly Glu Val Pro Leu Asp Val
               660                 665                 670
   Gly Pro Gln Gly Lys Gln Leu Ile Glu Leu Pro Glu Leu Pro Gln Pro
           675                 680                 685
   Glu Ser Ala Gly Gln Leu Trp Leu Thr Val Arg Val Val Gln Pro Asn
       690                 695                 700
   Ala Thr Ala Trp Ser Glu Ala Gly His Ile Ser Ala Trp Gln Gln Trp
   705                 710                 715                 720
   Arg Leu Ala Glu Asn Leu Ser Val Thr Leu Pro Ser Ala Ser His Ala
                   725                 730                 735
   Ile Pro Gln Leu Thr Thr Ser Gly Thr Asp Phe Cys Ile Glu Leu Gly
               740                 745                 750
   Asn Lys Arg Trp Gln Phe Asn Arg Gln Ser Gly Phe Leu Ser Gln Met
           755                 760                 765
   Trp Ile Gly Asp Glu Lys Gln Leu Leu Thr Pro Leu Arg Asp Gln Phe
       770                 775                 780
   Thr Arg Ala Pro Leu Asp Asn Asp Ile Gly Val Ser Glu Ala Thr Arg
   785                 790                 795                 800
   Ile Asp Pro Asn Ala Trp Val Glu Arg Trp Lys Ala Ala Gly His Tyr
                   805                 810                 815
   Gln Ala Glu Ala Ala Leu Leu Gln Cys Thr Ala Asp Thr Leu Ala Asp
               820                 825                 830
   Ala Val Leu Ile Thr Thr Ala His Ala Trp Gln His Gln Gly Lys Thr
           835                 840                 845
   Leu Phe Ile Ser Arg Lys Thr Tyr Arg Ile Asp Gly His Gly Glu Met
       850                 855                 860
   Val Ile Asn Val Asp Val Ala Val Ala Ser Asp Thr Pro His Pro Ala
   865                 870                 875                 880
   Arg Ile Gly Leu Thr Cys Gln Leu Ala Gln Val Ser Glu Arg Val Asn
   885 890 895
   Trp Leu Gly Leu Gly Pro Gln Glu Asn Tyr Pro Asp Arg Leu Thr Ala
   900 905 910
   Ala Cys Phe Asp Arg Trp Asp Leu Pro Leu Ser Asp Met Tyr Thr Pro
   915 920 925
   Tyr Val Phe Pro Ser Glu Asn Gly Leu Arg Cys Gly Thr Arg Glu Leu
   930 935 940
   Asn Tyr Gly Pro His Gln Trp Arg Gly Asp Phe Gln Phe Asn Ile Ser
   945 950 955 960
   Arg Tyr Ser Gln Gln Gln Leu Met Glu Thr Ser His Arg His Leu Leu
   965 970 975
   His Ala Glu Glu Gly Thr Trp Leu Asn Ile Asp Gly Phe His Met Gly
   980 985 990
   Ile Gly Gly Asp Asp Ser Trp Ser Pro Ser Val Ser Ala Glu Phe Gln
   995 1000 1005
   Leu Ser Ala Gly Arg Tyr His Tyr Gln Leu Val Trp Cys Gln Lys
   1010 1015 1020
<210> 4
   <211> 237
   <212> PRT
   <213> Homo sapiens
<400> 4
   Ile Val Gly Gly Trp Glu Cys Glu Lys His Ser Gln Pro Trp Gln Val
   1               5                   10                  15
   Leu Val Ala Ser Arg Gly Arg Ala Val Cys Gly Gly Val Leu Val His
               20                  25                  30
   Pro Gln Trp Val Leu Thr Ala Ala His Cys Ile Arg Asn Lys Ser Val
           35                  40                  45
   Ile Leu Leu Gly Arg His Ser Leu Phe His Pro Glu Asp Thr Gly Gln
       50                  55                  60
   Val Phe Gln Val Ser His Ser Phe Pro His Pro Leu Tyr Asp Met Ser
   65                  70                  75                  80
   Leu Leu Lys Asn Arg Phe Leu Arg Pro Gly Asp Asp Ser Ser His Asp
                   85                  90                  95
   Leu Met Leu Leu Arg Leu Ser Glu Pro Ala Glu Leu Thr Asp Ala Val
               100                 105                 110
   Lys Val Met Asp Leu Pro Thr Gln Glu Pro Ala Leu Gly Thr Thr Cys
           115                 120                 125
   Tyr Ala Ser Gly Trp Gly Ser Ile Glu Pro Glu Glu Phe Leu Thr Pro
       130                 135                 140
   Lys Lys Leu Gln Cys Val Asp Leu His Val Ile Ser Asn Asp Val Cys
   145                 150                 155                 160
   Ala Gln Val His Pro Gln Lys Val Thr Lys Phe Met Leu Cys Ala Gly
                   165                 170                 175
   Arg Trp Thr Gly Gly Lys Ser Thr Cys Ser Gly Asp Ser Gly Gly Pro
               180                 185                 190
   Leu Val Cys Asn Gly Val Leu Gln Gly Ile Thr Ser Trp Gly Ser Glu
           195                 200                 205
   Pro Cys Ala Leu Pro Glu Arg Pro Ser Leu Tyr Thr Lys Val Val His
       210                 215                 220
   Tyr Arg Lys Trp Ile Lys Asp Thr Ile Val Ala Asn Pro
   225                 230                 235
<210> 5
   <211> 206
   <212> PRT
   <213> Homo sapiens
<400> 5
   Gln Thr Asp Met Ser Arg Lys Ala Phe Val Phe Pro Lys Glu Ser Asp
   1               5                   10                  15
   Thr Ser Tyr Val Ser Leu Lys Ala Pro Leu Thr Lys Pro Leu Lys Ala
               20                  25                  30
   Phe Thr Val Cys Leu His Phe Tyr Thr Glu Leu Ser Ser Thr Arg Gly
           35                  40                  45
   Tyr Ser Ile Phe Ser Tyr Ala Thr Lys Arg Gln Asp Asn Glu Ile Leu
       50                  55                  60
   Ile Phe Trp Ser Lys Asp Ile Gly Tyr Ser Phe Thr Val Gly Gly Ser
   65                  70                  75                  80
   Glu Ile Leu Phe Glu Val Pro Glu Val Thr Val Ala Pro Val His Ile
                   85                  90                  95
   Cys Thr Ser Trp Glu Ser Ala Ser Gly Ile Val Glu Phe Trp Val Asp
               100                 105                 110
   Gly Lys Pro Arg Val Arg Lys Ser Leu Lys Lys Gly Tyr Thr Val Gly
           115                 120                 125
   Ala Glu Ala Ser Ile Ile Leu Gly Gln Glu Gln Asp Ser Phe Gly Gly
       130                 135                 140
   Asn Phe Glu Gly Ser Gln Ser Leu Val Gly Asp Ile Gly Asn Val Asn
   145                 150                 155                 160
   Met Trp Asp Phe Val Leu Ser Pro Asp Glu Ile Asn Thr Ile Tyr Leu
                   165                 170                 175
   Gly Gly Pro Phe Ser Pro Asn Val Leu Asn Trp Arg Ala Leu Lys Tyr
               180                 185                 190
   Glu Val Gln Gly Glu Val Phe Thr Lys Pro Gln Leu Trp Pro
           195                 200                 205
<210> 6
   <211> 98
   <212> PRT
   <213> Homo sapiens
<400> 6
   Ile Gln Arg Thr Pro Lys Ile Gln Val Tyr Ser Arg His Pro Ala Glu
   1               5                   10                  15
   Asn Gly Lys Ser Asn Phe Leu Asn Cys Tyr Val Ser Gly Phe His Pro
               20                  25                  30
   Ser Asp Ile Glu Val Asp Leu Leu Lys Asn Gly Glu Arg Ile Glu Lys
           35                  40                  45
   Val Glu His Ser Asp Leu Ser Phe Ser Lys Asp Trp Ser Phe Tyr Leu
       50                  55                  60
   Leu Tyr Tyr Thr Glu Phe Thr Pro Thr Glu Lys Asp Glu Tyr Ala Cys
   65                  70                  75                  80
   Arg Val Asn His Val Thr Leu Ser Gln Pro Lys Ile Val Lys Trp Asp
                       85                  90                  95
   Arg Met
<210> 7
   <211> 11
   <212> PRT
   <213> artificial
<220>
   <223> Peptide sequence
<400> 7
   Arg Asn Gly Ile Val Ile Val Ala Thr Thr Arg
   1               5                   10
<210> 8
   <211> 18
   <212> PRT
   <213> artificial
<220>
   <223> Peptide sequence
<400> 8
   Arg Leu Gln Gln Tyr Thr Leu Leu Leu Ile Tyr Leu Ser Val Ala Thr
   1               5                   10                  15
   Ala Lys
<210> 9
   <211> 20
   <212> PRT
   <213> artificial
<220>
   <223> Peptide sequence
<400> 9
   Arg Lys Leu Leu Phe Asn Leu Gly Ser Arg Asn Gly Ile Val Ile Val
   1               5                   10                  15
   Ala Thr Thr Arg
   20
<210> 10
   <211> 22
   <212> PRT
   <213> artificial
<220>
   <223> Peptide sequence
<400> 10
   Arg Asn Gly Ile Val Ile Val Ala Thr Arg Gly Ser Arg Asn Gly Ile
   1               5                   10                  15
   Val Ile Val Ala Thr Arg
   20
<210> 11
   <211> 22
   <212> PRT
   <213> artificial
<220>
   <223> Peptide sequence
<400> 11
   Arg Val Ile Gln His Ser Thr Trp Leu Arg Gly Ser Arg Val Ile Gln
   1               5                   10                  15
   His Ser Thr Trp Leu Arg
   20
<210> 12
   <211> 22
   <212> PRT
   <213> artificial
<220>
   <223> Peptide sequence
<400> 12
   Arg Leu Ile Thr Leu Leu Ser Leu Phe Arg Gly Ser Arg Leu Ile Thr
   1               5                   10                  15
   Leu Leu Ser Leu Phe Arg
               20
<210> 13
   <211> 22
   <212> PRT
   <213> artificial
<220>
   <223> Peptide sequence
<400> 13
   Arg Gln Tyr Thr Leu Leu Leu Ile Tyr Arg Gly Ser Arg Gln Tyr Thr
   1               5                   10                  15
   Leu Leu Leu Ile Tyr Arg
            20
<210> 14
   <211> 22
   <212> PRT
   <213> artificial
<220>
   <223> Peptide sequence
<400> 14
   Lys Asn Gly Ile Val Ile Val Ala Thr Lys Gly Ser Lys Asn Gly Ile
   1               5                   10                  15
   Val Ile Val Ala Thr Lys
               20
<210> 15
   <211> 22
   <212> PRT
   <213> artificial
<220>
   <223> Peptide sequence
<400> 15
   Lys Val Ile Gln His Ser Thr Trp Leu Lys Gly Ser Lys Val Ile Gln
   1               5                   10                  15
   His Ser Thr Trp Leu Lys
               20
<210> 16
   <211> 22
   <212> PRT
   <213> artificial
<220>
   <223> Peptide sequence
<400> 16
   Lys Leu Ile Thr Leu Leu Ser Leu Phe Lys Gly Ser Lys Leu Ile Thr
   1               5                   10                  15
   Leu Leu Ser Leu Phe Lys
               20
<210> 17
   <211> 22
   <212> PRT
   <213> artificial
<220>
   <223> Peptide sequence
<400> 17
   Lys Gln Tyr Thr Leu Leu Leu Ile Tyr Lys Gly Ser Lys Gln Tyr Thr
   1               5                   10                  15
   Leu Leu Leu Ile Tyr Lys
               20
<210> 18
   <211> 22
   <212> PRT
   <213> artificial
<220>
   <223> Peptide sequence
<400> 18
   Asp Asn Gly Ile Val Ile Val Ala Thr Asp Gly Ser Asp Asn Gly Ile
   1               5                   10                  15
   Val Ile Val Ala Thr Asp
               20
<210> 19
   <211> 22
   <212> PRT
   <213> artificial
<220>
   <223> Peptide sequence
<400> 19
   Asp Val Ile Gln His Ser Thr Trp Leu Asp Gly Ser Asp Val Ile Gln
   1               5                   10                  15
   His Ser Thr Trp Leu Asp
               20
<210> 20
   <211> 22
   <212> PRT
   <213> artificial
<220>
   <223> Peptide sequence
<400> 20
   Asp Leu Ile Thr Leu Leu Ser Leu Phe Asp Gly Ser Asp Leu Ile Thr
   1               5                   10                  15
   Leu Leu Ser Leu Phe Asp
               20
<210> 21
   <211> 22
   <212> PRT
   <213> artificial
<220>
   <223> Peptide sequence
<400> 21
   Asp Gln Tyr Thr Leu Leu Leu Ile Tyr Asp Gly Ser Asp Gln Tyr Thr
   1               5                   10                  15
   Leu Leu Leu Ile Tyr Asp
               20
<210> 22
   <211> 22
   <212> PRT
   <213> artificial
<220>
   <223> Peptide sequence
<400> 22
   Glu Asn Gly Ile Val Ile Val Ala Thr Glu Gly Ser Glu Asn Gly Ile
   1               5                   10                  15
   Val Ile Val Ala Thr Glu
               20
<210> 23
   <211> 22
   <212> PRT
   <213> artificial
<220>
   <223> Peptide sequence
<400> 23
   Glu Val Ile Gln His Ser Thr Trp Leu Glu Gly Ser Glu Val Ile Gln
   1               5                   10                  15
   His Ser Thr Trp Leu Glu
               20
<210> 24
   <211> 22
   <212> PRT
   <213> artificial
<220>
   <223> Peptide sequence
<400> 24
   Glu Leu Ile Thr Leu Leu Ser Leu Phe Glu Gly Ser Glu Leu Ile Thr
   1               5                   10                  15
   Leu Leu Ser Leu Phe Glu
               20
<210> 25
   <211> 22
   <212> PRT
   <213> artificial
<220>
   <223> Peptide sequence
<400> 25
   Glu Gln Tyr Thr Leu Leu Leu Ile Tyr Glu Gly Ser Glu Gln Tyr Thr
   1               5                   10                  15
   Leu Leu Leu Ile Tyr Glu
               20
<210> 26
   <211> 22
   <212> PRT
   <213> artificial
<220>
   <223> Peptide sequence
<400> 26
   Pro Asn Gly Ile Val Ile Val Ala Thr Pro Gly Ser Pro Asn Gly Ile
   1               5                   10                  15
   Val Ile Val Ala Thr Pro
               20
<210> 27
   <211> 22
   <212> PRT
   <213> artificial
<220>
   <223> Peptide sequence
<400> 27
   Pro Val Ile Gln His Ser Thr Trp Leu Pro Gly Ser Pro Val Ile Gln
   1               5                   10                  15
   His Ser Thr Trp Leu Pro
                        20
<210> 28
   <211> 22
   <212> PRT
   <213> artificial
<220>
   <223> Peptide sequence
<400> 28
   Pro Leu Ile Thr Leu Leu Ser Leu Phe Pro Gly Ser Pro Leu Ile Thr
   1               5                   10                  15
   Leu Leu Ser Leu Phe Pro
               20
<210> 29
   <211> 21
   <212> PRT
   <213> artificial
<220>
   <223> Peptide sequence
<400> 29
   Pro Gln Tyr Thr Leu Leu Leu Ile Tyr Pro Gly Ser Pro Gln Tyr Thr
   1               5                   10                  15
   Leu Leu Leu Tyr Pro
               20
<210> 30
   <211> 20
   <212> PRT
   <213> artificial
<220>
   <223> Peptide sequence
<400> 30
   Arg Phe Phe Ile Ala Leu Ser Arg Arg Gly Ser Arg Val Gln Ala Tyr
   1               5                   10                  15
   Leu Tyr Arg Arg
               20
<210> 31
   <211> 20
   <212> PRT
   <213> artificial
<220>
   <223> Peptide sequence
<400> 31
   Arg Trp Val Ser Met Leu Leu Arg Arg Gly Ser Arg Trp Val Ser Met
   1 5 10 15
   Leu Leu Arg Arg
   20
<210> 32
   <211> 18
   <212> PRT
   <213> artificial
<220>
   <223> Peptide sequence
<400> 32
   Arg Leu Phe Asn Phe Leu Lys Arg Gly Ser Arg Leu Phe Asn Phe Leu
   1 5 10 15
   Lys Arg
<210> 33
   <211> 21
   <212> PRT
   <213> artificial
<220>
   <223> Peptide sequence
<400> 33
   Arg Arg Trp Val Ser Met Leu Leu Arg Arg Gly Ser Arg Trp Val Ser
   1 5 10 15
   Met Leu Leu Arg Arg
   20
<210> 34
   <211> 19
   <212> PRT
   <213> artificial
<220>
   <223> Peptide sequence
<400> 34
   Arg Phe Phe Ile Gly Leu Ser Arg Arg Gly Ser Arg Leu Phe Asn Phe
   1 5 10 15
   Leu Lys Arg
<210> 35
   <211> 20
   <212> PRT
   <213> artificial
<220>
   <223> Peptide sequence
<400> 35
   Arg Arg Ile Ile Leu Phe Ile Leu Arg Pro Pro Arg Leu Ile Leu Phe
   1               5                   10                  15
   Leu Gly Arg Arg
               20
<210> 36
   <211> 20
   <212> PRT
   <213> artificial
<220>
   <223> Peptide sequence
<400> 36
   Arg Arg Ile Ile Leu Ser Leu Ile Arg Pro Pro Arg Leu Leu Gly Val
   1               5                   10                  15
   Val Leu Arg Arg
               20
<210> 37
   <211> 20
   <212> PRT
   <213> artificial
<220>
   <223> Peptide sequence
<400> 37
   Arg Arg Val Leu Ser Leu Ile Leu Arg Pro Pro Arg Ile Ala Leu Leu
   1               5                   10                  15
   Gly Leu Arg Arg
               20
<210> 38
   <211> 20
   <212> PRT
   <213> artificial
<220>
   <223> Peptide sequence
<400> 38
   Arg Arg Ile Ala Leu Leu Leu Ile Arg Pro Pro Arg Leu Leu Ala Ile
   1 5 10 15
   Ala Val Arg Arg
   20
<210> 39
   <211> 20
   <212> PRT
   <213> artificial
<220>
   <223> Peptide sequence
<400> 39
   Arg Arg Ile Leu Leu Gly Leu Ile Arg Pro Pro Arg Thr Ile Ile Gly
   1 5 10 15
   Leu Val Arg Arg
   20
<210> 40
   <211> 20
   <212> PRT
   <213> artificial
<220>
   <223> Peptide sequence
<400> 40
   Arg Arg Ile Leu Leu Leu Ile Ala Arg Pro Pro Arg Ile Leu Leu Gly
   1 5 10 15
   Ala Ile Arg Arg
   20
<210> 41
   <211> 20
   <212> PRT
   <213> artificial
<220>
   <223> Peptide sequence
<400> 41
   Arg Arg Leu Leu Gly Leu Ile Ile Arg Pro Pro Arg Ala Ile Ala Leu
   1 5 10 15
   Thr Leu Arg Arg
   20
<210> 42
   <211> 20
   <212> PRT
   <213> artificial
<220>
   <223> Peptide sequence
<400> 42
   Arg Arg Ile Leu Gly Leu Ile Ala Arg Pro Pro Arg Ile Ala Phe Val
   1               5                   10                  15
   Ile Leu Arg Arg
               20
<210> 43
   <211> 20
   <212> PRT
   <213> artificial
<220>
   <223> Peptide sequence
<400> 43
   Arg Arg Ile Ile Gly Ile Ile Ala Arg Pro Pro Arg Val Leu Val Thr
   1               5                   10                  15
   Leu Leu Arg Arg
               20
<210> 44
   <211> 8
   <212> PRT
   <213> artificial
<220>
   <223> Peptide sequence
<400> 44
   Arg Leu Ala Val Val Leu Gln Arg
   1               5
<210> 45
   <211> 10
   <212> PRT
   <213> artificial
<220>
   <223> Peptide sequence
<400> 45
   Arg Val Ile Ile Trp Ser Leu Gly Asn Arg
   1 5 10
<210> 46
   <211> 10
   <212> PRT
   <213> artificial
<220>
   <223> Peptide sequence
<400> 46
   Arg Pro Ile Thr Val Asn Pro Pro Phe Arg
   1 5 10
<210> 47
   <211> 10
   <212> PRT
   <213> artificial
<220>
   <223> Peptide sequence
<400> 47
   Arg Val Pro Ile Trp Ser Leu Gly Asn Arg
   1 5 10
<210> 48
   <211> 10
   <212> PRT
   <213> artificial
<220>
   <223> Peptide sequence
<400> 48
   Arg Val Ile Pro Trp Ser Leu Gly Asn Arg
   1 5 10
<210> 49
   <211> 10
   <212> PRT
   <213> artificial
<220>
   <223> Peptide sequence
<400> 49
   Arg Val Ile Pro Glu Ser Leu Gly Asn Arg
   1 5 10
<210> 50
   <211> 10
   <212> PRT
   <213> artificial
<220>
   <223> Peptide sequence
<400> 50
   Asp Ser Leu Ala Val Val Leu Gln Arg Arg
   1               5                   10
<210> 51
   <211> 12
   <212> PRT
   <213> artificial
<220>
   <223> Peptide sequence
<400> 51
   Pro Ser Val Ile Ile Trp Ser Leu Gly Asn Glu Ser
   1               5                   10
<210> 52
   <211> 12
   <212> PRT
   <213> artificial
<220>
   <223> Peptide sequence
<400> 52
   Thr Tyr Pro Ile Thr Val Asn Pro Pro Phe Val Pro
   1               5                   10
<210> 53
   <211> 8
   <212> PRT
   <213> artificial
<220>
   <223> Peptide sequence
<400> 53
   Arg Leu Ala Val Val Leu Gln Arg
   1               5
<210> 54
   <211> 10
   <212> PRT
   <213> artificial
<220>
   <223> Peptide sequence
<400> 54
   Arg Val Ile Ile Trp Ser Leu Gly Asn Arg
   1 5 10
<210> 55
   <211> 10
   <212> PRT
   <213> artificial
<220>
   <223> Peptide sequence
<400> 55
   Arg Pro Ile Thr Val Asn Pro Pro Phe Arg
   1 5 10
<210> 56
   <211> 10
   <212> PRT
   <213> artificial
<220>
   <223> Peptide sequence
<400> 56
   Arg Val Pro Ile Trp Ser Leu Gly Asn Arg
   1 5 10
<210> 57
   <211> 10
   <212> PRT
   <213> artificial
<220>
   <223> Peptide sequence
<400> 57
   Arg Val Ile Pro Trp Ser Leu Gly Asn Arg
   1 5 10
<210> 58
   <211> 10
   <212> PRT
   <213> artificial
<220>
   <223> Peptide sequence
<400> 58
   Arg Val Ile Pro Glu Ser Leu Gly Asn Arg
   1 5 10
<210> 59
   <211> 28
   <212> PRT
   <213> artificial
<220>
   <223> Peptide sequence
<400> 59
   Arg Val Ile Ile Trp Ser Leu Gly Asn Arg Gly Ser Gly Ser Ala Pro
   1               5                   10                  15
   Ala Ala Arg Val Ile Ile Trp Ser Leu Gly Asn Arg
               20                  25
<210> 60
   <211> 9
   <212> PRT
   <213> artificial
<220>
   <223> Peptide sequence
<400> 60
   Arg Gln Trp Val Leu Thr Ala Ala Arg
   1               5
<210> 61
   <211> 9
   <212> PRT
   <213> artificial
<220>
   <223> Peptide sequence
<400> 61
   Arg Ile Leu Ile Phe Trp Ser Lys Arg
   1               5
<210> 62
   <211> 11
   <212> PRT
   <213> artificial
<220>
   <223> Peptide sequence
<400> 62
   Arg Trp Ser Phe Tyr Leu Leu Tyr Tyr Thr Arg
   1               5                   10
<210> 63
   <211> 11
   <212> PRT
   <213> artificial
<220>
   <223> Peptide sequence
<400> 63
   His Pro Gln Trp Val Leu Thr Ala Ala His Cys
   1 5 10
<210> 64
   <211> 10
   <212> PRT
   <213> artificial
<220>
   <223> Peptide sequence
<400> 64
   Asn Glu Ile Leu Ile Phe Trp Ser Lys Asp
   1 5 10
<210> 65
   <211> 13
   <212> PRT
   <213> artificial
<220>
   <223> Peptide sequence
<400> 65
   Lys Asp Trp Ser Phe Tyr Leu Leu Tyr Tyr Thr Glu Phe
   1 5 10
<210> 66
   <211> 20
   <212> PRT
   <213> artificial
<220>
   <223> Peptide sequence
<400> 66
   Asp Ala Val Gly Val Leu Ile Gly Asp Pro Pro Asp Ala Val Gly Val
   1 5 10 15
   Leu Ile Gly Asp
   20
<210> 67
   <211> 7
   <212> PRT
   <213> artificial
<220>
   <223> Peptide sequence
<400> 67
   Ala Val Gly Val Leu Ile Gly
   1 5
<210> 68
   <211> 7
   <212> PRT
   <213> artificial
<220>
   <223> Peptide sequence
<400> 68
   Arg Lys Leu Leu Phe Asn Leu
   1 5
<210> 69
   <211> 7
   <212> PRT
   <213> artificial
<220>
   <223> Peptide sequence
<400> 69
   Arg Lys Leu Phe Phe Asn Leu
   1 5
<210> 70
   <211> 10
   <212> PRT
   <213> artificial
<220>
   <223> Peptide sequence
<400> 70
   Tyr Thr Leu Leu Leu Ile Tyr Leu Ser Val
   1 5 10
<210> 71
   <211> 9
   <212> PRT
   <213> artificial
<220>
   <223> Peptide sequence
<400> 71
   Asn Gly Ile Val Ile Val Ala Thr Thr
   1 5
<210> 72
   <211> 13
   <212> PRT
   <213> artificial
<220>
   <223> Peptide sequence
<400> 72
   Thr Trp Leu Cys Gly Leu Ile Thr Leu Leu Ser Leu Phe
   1 5 10
<210> 73
   <211> 9
   <212> PRT
   <213> artificial
<220>
   <223> Peptide sequence
<400> 73
   Asn Gly Val Val Ile Val Ala Ala Thr
   1 5
<210> 74
   <211> 19
   <212> PRT
   <213> artificial
<220>
   <223> Peptide sequence
<400> 74
   Arg Arg Leu Phe Asn Phe Leu Lys Arg Gly Ser Arg Leu Phe Asn Phe
   1 5 10 15
   Leu Lys Arg
<210> 75
   <211> 6
   <212> PRT
   <213> artificial
<220>
   <223> Peptide sequence
<400> 75
   Leu Ala Val Val Leu Gln
   1 5
<210> 76
   <211> 8
   <212> PRT
   <213> artificial
<220>
   <223> Peptide sequence
<400> 76
   Val Ile Ile Trp Ser Leu Gly Asn
   1 5
<210> 77
   <211> 4
   <212> PRT
   <213> artificial
<220>
   <223> Peptide sequence
<400> 77
   Ala Pro Ala Ala
   1
<210> 78
   <211> 20
   <212> PRT
   <213> artificial
<220>
   <223> Peptide sequence
<400> 78
   Arg Phe Phe Ile Gly Leu Ser Arg Arg Gly Ser Arg Ile Gln Ala Tyr
   1 5 10 15
   Leu Tyr Arg Arg
   20
<210> 79
   <211> 8
   <212> PRT
   <213> artificial
<220>
   <223> Peptide sequence
<400> 79
   Arg Leu Ala Val Val Leu Gln Arg
   1 5
<210> 80
   <211> 10
   <212> PRT
   <213> artificial
<220>
   <223> Peptide sequence
<400> 80
   Arg Val Ile Ile Trp Ser Leu Gly Asn Arg
   1 5 10
<210> 81
   <211> 10
   <212> PRT
   <213> artificial
<220>
   <223> Peptide sequence
<400> 81
   Arg Pro Ile Thr Val Asn Pro Pro Phe Arg
   1 5 10
<210> 82
   <211> 10
   <212> PRT
   <213> artificial
<220>
   <223> Peptide sequence
<400> 82
   Arg Val Pro Ile Trp Ser Leu Gly Asn Arg
   1 5 10
<210> 83
   <211> 10
   <212> PRT
   <213> artificial
<220>
   <223> Peptide sequence
<400> 83
   Arg Val Ile Pro Trp Ser Leu Gly Asn Arg
   1 5 10
<210> 84
   <211> 10
   <212> PRT
   <213> artificial
<220>
   <223> Peptide sequence
<400> 84
   Arg Val Ile Pro Glu Ser Leu Gly Asn Arg
   1 5 10
<210> 85
   <211> 9
   <212> PRT
   <213> artificial
<220>
   <223> Peptide sequence
<400> 85
   Arg Gln Trp Val Leu Thr Ala Ala Arg
   1 5
<210> 86
   <211> 8
   <212> PRT
   <213> artificial
<220>
   <223> Peptide sequence
<400> 86
   Arg Ile Leu Ile Phe Trp Ser Arg
   1 5
<210> 87
   <211> 11
   <212> PRT
   <213> artificial
<220>
   <223> Peptide sequence
<400> 87
   Arg Trp Ser Phe Tyr Leu Leu Tyr Tyr Thr Arg
   1 5 10
<210> 88
   <211> 8
   <212> PRT
   <213> artificial
<220>
   <223> Peptide sequence
<400> 88
   Arg Trp Gln Val Leu Ala Ser Asp
   1 5
<210> 89
   <211> 26
   <212> PRT
   <213> artificial
<220>
   <223> Peptide sequence
<400> 89
   Arg Gln Trp Val Leu Thr Ala Ala Arg Gly Ser Gly Ser Ala Pro Ala
   1               5                   10                  15
   Ala Arg Gln Trp Val Leu Thr Ala Ala Arg
               20                  25
<210> 90
   <211> 8
   <212> PRT
   <213> artificial
<220>
   <223> Peptide sequence
<400> 90
   Gly Ser Gly Ser Ala Pro Ala Ala
   1               5
<210> 91
   <211> 28
   <212> PRT
   <213> artificial
<220>
   <223> Peptide sequence
<400> 91
   Arg Val Ile Ile Trp Ser Leu Gly Asn Arg Gly Ser Gly Ser Ala Pro
   1               5                   10                  15
   Ala Ala Arg Val Ile Ile Trp Ser Leu Gly Asn Arg
               20                  25
<210> 92
   <211> 24
   <212> PRT
   <213> artificial
<220>
   <223> Peptide sequence
<400> 92
   Arg Ile Leu Ile Phe Trp Ser Arg Gly Ser Gly Ser Ala Pro Ala Ala
   1               5                   10                  15
   Arg Ile Leu Ile Phe Trp Ser Arg
               20
<210> 93
   <211> 20
   <212> PRT
   <213> artificial
<220>
   <223> Peptide sequence
<400> 93
   Arg Trp Gly Leu Leu Leu Ala Leu Arg Pro Pro Arg Trp Gly Leu Leu
   1               5                   10                  15
   Leu Ala Leu Arg
               20
<210> 94
   <211> 20
   <212> PRT
   <213> artificial
<220>
   <223> Peptide sequence
<400> 94
   Arg Thr Gly Tyr Leu Tyr Ile Ser Arg Pro Pro Arg Thr Gly Tyr Leu
   1               5                   10                  15
   Tyr Ile Ser Arg
               20
<210> 95
   <211> 20
   <212> PRT
   <213> artificial
<220>
   <223> Peptide sequence
<400> 95
   Arg Ile Ile Ser Ala Val Val Gly Arg Pro Pro Arg Ile Ile Ser Ala
   1               5                   10                  15
   Val Val Gly Arg
               20
<210> 96
   <211> 20
   <212> PRT
   <213> artificial
<220>
   <223> Peptide sequence
<400> 96
   Asp Val Trp Ser Tyr Gly Val Thr Asp Pro Pro Asp Val Trp Ser Tyr
   1               5                   10                  15
   Gly Val Thr Asp
               20
<210> 97
   <211> 20
   <212> PRT
   <213> artificial
<220>
   <223> Peptide sequence
<400> 97
   Asp Ile Thr Gly Tyr Leu Tyr Ile Asp Pro Pro Asp Ile Thr Gly Tyr
   1               5                   10                  15
   Leu Tyr Ile Asp
               20
<210> 98
   <211> 20
   <212> PRT
   <213> artificial
<220>
   <223> Peptide sequence
<400> 98
   Asp Leu Leu Gly Ile Ser Leu Thr Asp Pro Pro Asp Leu Leu Gly Ile
   1               5                   10                  15
   Ser Leu Thr Asp
               20
<210> 99
   <211> 20
   <212> PRT
   <213> artificial
<220>
   <223> Peptide sequence
<400> 99
   Asp Trp Gly Leu Leu Leu Ala Leu Asp Pro Pro Asp Trp Gly Leu Leu
   1               5                   10                  15
   Leu Ala Leu Asp
               20
<210> 100
   <211> 15
   <212> PRT
   <213> artificial
<220>
   <223> Peptide sequence
<400> 100
   Asn Lys Leu Arg Phe Ala Phe Asn Ile Tyr Asp Ile Asp Arg Asp
   1               5                   10                  15
<210> 101
   <211> 13
   <212> PRT
   <213> artificial
<220>
   <223> Peptide sequence
<400> 101
   Gly Asn Gly Glu Leu Phe Ile Val Met Lys Met Met Val
   1               5                   10
<210> 102
   <211> 4
   <212> PRT
   <213> artificial
<220>
   <223> Peptide sequence
<400> 102
   Asn Lys Leu Arg
   1
<210> 103
   <211> 5
   <212> PRT
   <213> artificial
<220>
   <223> Peptide sequence
<400> 103
   Asp Ile Asp Arg Asp
   1 5
<210> 104
   <211> 6
   <212> PRT
   <213> artificial
<220>
   <223> Peptide sequence
<400> 104
   Phe Ala Phe Asn Ile Tyr
   1 5
<210> 105
   <211> 5
   <212> PRT
   <213> artificial
<220>
   <223> Peptide sequence
<400> 105
   Leu Phe Ile Val Met
   1 5
<210> 106
   <211> 8
   <212> PRT
   <213> artificial
<220>
   <223> Peptide sequence
<400> 106
   Asp Tyr Lys Asp Asp Asp Asp Lys
   1 5
<210> 107
   <211> 13
   <212> PRT
   <213> artificial
<220>
   <223> Peptide sequence
<400> 107
   Tyr Thr Leu Leu Leu Ile Tyr Leu Ser Val Ala Thr Ala
   1 5 10
<210> 108
   <211> 9
   <212> PRT
   <213> artificial
<220>
   <223> Peptide sequence
<400> 108
   Tyr Pro Tyr Asp Val Pro Asp Tyr Ala
   1               5
<210> 109
   <211> 4
   <212> PRT
   <213> artificial
<220>
   <223> Peptide sequence
<400> 109
   Met Ala Gln Trp
   1
<210> 110
   <211> 6
   <212> PRT
   <213> artificial
<220>
   <223> Peptide sequence
<400> 110
   Ser Thr Leu Ile Val Leu
   1               5
<210> 111
   <211> 5
   <212> PRT
   <213> artificial
<220>
   <223> Peptide sequence
<400> 111
   Ser Thr Val Ile Phe
   1               5
<210> 112
   <211> 12
   <212> PRT
   <213> artificial
<220>
   <223> Peptide sequence
<400> 112
   Lys Pro Ala Gly Ala Ala Lys Pro Gly Ala Ala Gly
   1 5 10
<210> 113
   <211> 68
   <212> PRT
   <213> artificial
<220>
   <223> Peptide sequence
<400> 113
   Tyr Pro Tyr Asp Val Pro Asp Tyr Ala Met Ala Gln Trp Gln Asn Ser
   1 5 10 15
   Thr Leu Ile Val Leu Gln Asn Ser Thr Val Ile Phe Glu Gln Asn Ser
   20 25 30
   Thr Val Ile Phe Glu Gln Asn Lys Pro Ala Gly Ala Ala Lys Pro Gly
   35 40 45
   Ala Ala Gly Arg Phe Ala Phe Asn Ile Tyr Arg Gly Ser Arg Leu Phe
   50 55 60
   Ile Val Met Arg
   65
<210> 114
   <211> 20
   <212> PRT
   <213> artificial
<220>
   <223> Peptide sequence
<400> 114
   Arg Trp Val Ser Met Leu Leu Arg Arg Gly Ser Arg Val Gly Tyr Val
   1 5 10 15
   Ile Ala Arg Arg
   20
<210> 115
   <211> 20
   <212> PRT
   <213> artificial
<220>
   <223> Peptide sequence
<400> 115
   Arg Ile Leu Leu Gly Leu Ile Arg Arg Gly Ser Arg Ile Leu Leu Gly
   1               5                   10                  15
   Leu Ile Arg Arg
               20
<210> 116
   <211> 20
   <212> PRT
   <213> artificial
<220>
   <223> Peptide sequence
<400> 116
   Arg Leu Ile Gln Leu Ile Val Ser Arg Gly Ser Arg Leu Ile Gln Leu
   1               5                   10                  15
   Ile Val Ser Arg
               20
<210> 117
   <211> 20
   <212> PRT
   <213> artificial
<220>
   <223> Peptide sequence
<400> 117
   Arg Thr Thr Ile Met Ala Ala Phe Arg Gly Ser Arg Thr Thr Ile Met
   1               5                   10                  15
   Ala Ala Phe Arg
               20
<210> 118
   <211> 20
   <212> PRT
   <213> artificial
<220>
   <223> Peptide sequence
<400> 118
   Arg Thr Met Ala Trp Thr Val Val Arg Gly Ser Arg Thr Met Ala Trp
   1               5                   10                  15
   Thr Val Val Arg
               20
<210> 119
   <211> 20
   <212> PRT
   <213> artificial
<220>
   <223> Peptide sequence
<400> 119
   Arg Gly Val Ser Ile Leu Asn Leu Arg Gly Ser Arg Gly Val Ser Ile
   1               5                   10                  15
   Leu Asn Leu Arg
               20
<210> 120
   <211> 8
   <212> PRT
   <213> artificial
<220>
   <223> Peptide sequence
<400> 120
   Arg Leu Ile Gln Leu Ile Val Ser
   1               5
<210> 121
   <211> 7
   <212> PRT
   <213> artificial
<220>
   <223> Peptide sequence
<400> 121
   Thr Thr Ile Met Ala Ala Phe
   1               5
<210> 122
   <211> 7
   <212> PRT
   <213> artificial
<220>
   <223> Peptide sequence
<400> 122
   Thr Met Ala Trp Thr Val Val
   1               5
<210> 123
   <211> 7
   <212> PRT
   <213> artificial
<220>
   <223> Peptide sequence
<400> 123
   Gly Val Ser Ile Leu Asn Leu
   1               5
<210> 124
   <211> 7
   <212> PRT
   <213> artificial
<220>
   <223> Peptide sequence
<400> 124
   Leu Ile Gln Leu Ile Val Ser
   1               5
<210> 125
   <211> 20
   <212> PRT
   <213> artificial
<220>
   <223> Peptide sequence
<400> 125
   Arg Thr Met Ala Trp Thr Val Val Arg Pro Pro Arg Thr Met Ala Trp
   1               5                   10                  15
   Thr Val Val Arg
               20
<210> 126
   <211> 20
   <212> PRT
   <213> artificial
<220>
   <223> Peptide sequence
<400> 126
   Asp Thr Met Ala Trp Thr Val Val Asp Pro Pro Asp Thr Met Ala Trp
   1               5                   10                  15
   Thr Val Val Asp
               20
<210> 127
   <211> 20
   <212> PRT
   <213> artificial
<220>
   <223> Peptide sequence
<400> 127
   Arg Thr Met Ala Trp Thr Val Val Arg Pro Ser Arg Thr Met Ala Trp
   1               5                   10                  15
   Thr Val Val Arg
               20
<210> 128
   <211> 18
   <212> PRT
   <213> artificial
<220>
   <223> Peptide sequence
<400> 128
   Asp Val His Ile Tyr Tyr Leu Asp Pro Pro Asp Val His Ile Tyr Tyr
   1               5                   10                  15
   Leu Asp
<210> 129
   <211> 18
   <212> PRT
   <213> artificial
<220>
   <223> Peptide sequence
<400> 129
   Arg Val His Ile Tyr Tyr Leu Arg Pro Ser Arg Val His Ile Tyr Tyr
   1               5                   10                  15
   Leu Arg
<210> 130
   <211> 20
   <212> PRT
   <213> artificial
<220>
   <223> Peptide sequence
<400> 130
   Asp Asn Leu Tyr Gly Phe Ile Ile Asp Pro Pro Asp Asn Leu Tyr Gly
   1               5                   10                  15
   Phe Ile Ile Asp
               20
<210> 131
   <211> 20
   <212> PRT
   <213> artificial
<220>
   <223> Peptide sequence
<400> 131
   Arg Asn Leu Tyr Gly Phe Ile Ile Arg Pro Ser Arg Asn Leu Tyr Gly
   1               5                   10                  15
   Phe Ile Ile Arg
               20
<210> 132
   <211> 20
   <212> PRT
   <213> artificial
<220>
   <223> Peptide sequence
<400> 132
   Arg Gly Val Ser Ile Leu Asn Leu Arg Pro Pro Arg Gly Val Ser Ile
   1               5                   10                  15
   Leu Asn Leu Arg
               20
<210> 133
   <211> 20
   <212> PRT
   <213> artificial
<220>
   <223> Peptide sequence
<400> 133
   Asp Gly Val Ser Ile Leu Asn Leu Asp Pro Pro Asp Gly Val Ser Ile
   1               5                   10                  15
   Leu Asn Leu Asp
               20
<210> 134
   <211> 20
   <212> PRT
   <213> artificial
<220>
   <223> Peptide sequence
<400> 134
   Arg Gly Val Ser Ile Leu Asn Leu Arg Pro Ser Arg Gly Val Ser Ile
   1               5                   10                  15
   Leu Asn Leu Arg
               20
<210> 135
   <211> 18
   <212> PRT
   <213> artificial
<220>
   <223> Peptide sequence
<400> 135
   Arg Gly Phe Val Tyr Phe Val Arg Pro Pro Arg Gly Phe Val Tyr Phe
   1               5                   10                  15
   Val Arg
<210> 136
   <211> 18
   <212> PRT
   <213> artificial
<220>
   <223> Peptide sequence
<400> 136
   Asp Gly Phe Val Tyr Phe Val Asp Pro Pro Asp Gly Phe Val Tyr Phe
   1               5                   10                  15
   Val Asp
<210> 137
   <211> 20
   <212> PRT
   <213> artificial
<220>
   <223> Peptide sequence
<400> 137
   Arg Met Ala Leu Gln Leu Phe Ile Arg Pro Pro Arg Met Ala Leu Gln
   1               5                   10                  15
   Leu Phe Ile Arg
               20
<210> 138
   <211> 20
   <212> PRT
   <213> artificial
<220>
   <223> Peptide sequence
<400> 138
   Asp Met Ala Leu Gln Leu Phe Ile Asp Pro Pro Asp Met Ala Leu Gln
   1               5                   10                  15
   Leu Phe Ile Asp
               20
<210> 139
   <211> 20
   <212> PRT
   <213> artificial
<220>
   <223> Peptide sequence
<400> 139
   Arg Leu Ile Gln Leu Ile Val Ser Arg Pro Pro Arg Leu Ile Gln Leu
   1               5                   10                  15
   Ile Val Ser Arg
               20
<210> 140
   <211> 20
   <212> PRT
   <213> artificial
<220>
   <223> Peptide sequence
<400> 140
   Asp Leu Ile Gln Leu Ile Val Ser Asp Pro Pro Asp Leu Ile Gln Leu
   1               5                   10                  15
   Ile Val Ser Asp
               20
<210> 141
   <211> 20
   <212> PRT
   <213> artificial
<220>
   <223> Peptide sequence
<400> 141
   Arg Leu Ile Gln Leu Ile Val Ser Arg Pro Ser Arg Leu Ile Gln Leu
   1               5                   10                  15
   Ile Val Ser Arg
               20
<210> 142
   <211> 20
   <212> PRT
   <213> artificial
<220>
   <223> Peptide sequence
<400> 142
   Arg Leu Ala Val Thr Trp Trp Asn Arg Pro Pro Arg Leu Ala Val Thr
   1               5                   10                  15
   Trp Trp Asn Arg
               20
<210> 143
   <211> 20
   <212> PRT
   <213> artificial
<220>
   <223> Peptide sequence
<400> 143
   Asp Leu Ala Val Thr Trp Trp Asn Asp Pro Pro Asp Leu Ala Val Thr
   1               5                   10                  15
   Trp Trp Asn Asp
               20
<210> 144
   <211> 20
   <212> PRT
   <213> artificial
<220>
   <223> Peptide sequence
<400> 144
   Arg Gln Ser Leu Ile Ile Ala Ala Arg Pro Pro Arg Gln Ser Leu Ile
   1               5                   10                  15
   Ile Ala Ala Arg
               20
<210> 145
   <211> 20
   <212> PRT
   <213> artificial
<220>
   <223> Peptide sequence
<400> 145
   Asp Gln Ser Leu Ile Ile Ala Ala Asp Pro Pro Asp Gln Ser Leu Ile
   1               5                   10                  15
   Ile Ala Ala Asp
               20
<210> 146
   <211> 18
   <212> PRT
   <213> artificial
<220>
   <223> Peptide sequence
<400> 146
   Arg Gly Phe Leu Ile Leu Gly Arg Pro Pro Arg Gly Phe Leu Ile Leu
   1               5                   10                  15
   Gly Arg
<210> 147
   <211> 18
   <212> PRT
   <213> artificial
<220>
   <223> Peptide sequence
<400> 147
   Asp Gly Phe Leu Ile Leu Gly Asp Pro Pro Asp Gly Phe Leu Ile Leu
   1               5                   10                  15
   Gly Asp
<210> 148
   <211> 18
   <212> PRT
   <213> artificial
<220>
   <223> Peptide sequence
<400> 148
   Arg Gly Phe Leu Ile Leu Gly Arg Pro Ser Arg Gly Phe Leu Ile Leu
   1               5                   10                  15
   Gly Arg
<210> 149
   <211> 20
   <212> PRT
   <213> artificial
<220>
   <223> Peptide sequence
<400> 149
   Asp Leu Met Val Ala Tyr Met Leu Asp Pro Pro Asp Leu Met Val Ala
   1               5                   10                  15
   Tyr Met Leu Asp
               20
<210> 150
   <211> 20
   <212> PRT
   <213> artificial
<220>
   <223> Peptide sequence
<400> 150
   Arg Thr Thr Ile Met Ala Ala Phe Arg Pro Pro Arg Thr Thr Ile Met
   1               5                   10                  15
   Ala Ala Phe Arg
               20
<210> 151
   <211> 20
   <212> PRT
   <213> artificial
<220>
   <223> Peptide sequence
<400> 151
   Asp Thr Thr Ile Met Ala Ala Phe Asp Pro Pro Asp Thr Thr Ile Met
   1 5 10 15
   Ala Ala Phe Asp
   20
<210> 152
   <211> 20
   <212> PRT
   <213> artificial
<220>
   <223> Peptide sequence
<400> 152
   Arg Thr Thr Ile Met Ala Ala Phe Arg Pro Ser Arg Thr Thr Ile Met
   1 5 10 15
   Ala Ala Phe Arg
   20
<210> 153
   <211> 20
   <212> PRT
   <213> artificial
<220>
   <223> Peptide sequence
<400> 153
   Arg Leu Val Trp Met Ala Cys His Arg Pro Pro Arg Leu Val Trp Met
   1 5 10 15
   Ala Cys His Arg
   20
<210> 154
   <211> 20
   <212> PRT
   <213> artificial
<220>
   <223> Peptide sequence
<400> 154
   Asp Leu Val Trp Met Ala Cys His Asp Pro Pro Asp Leu Val Trp Met
   1 5 10 15
   Ala Cys His Asp
   20
<210> 155
   <211> 20
   <212> PRT
   <213> artificial
<220>
   <223> Peptide sequence
<400> 155
   Arg Leu Val Trp Met Ala Cys His Arg Pro Ser Arg Leu Val Trp Met
   1 5 10 15
   Ala Cys His Arg
   20
<210> 156
   <211> 20
   <212> PRT
   <213> artificial
<220>
   <223> Peptide sequence
<400> 156
   Arg Val Ala Phe Gly Leu Val Cys Arg Pro Pro Arg Val Ala Phe Gly
   1 5 10 15
   Leu Val Cys Arg
   20
<210> 157
   <211> 20
   <212> PRT
   <213> artificial
<220>
   <223> Peptide sequence
<400> 157
   Asp Val Ala Phe Gly Leu Val Cys Asp Pro Pro Asp Val Ala Phe Gly
   1 5 10 15
   Leu Val Cys Asp
   20
<210> 158
   <211> 20
   <212> PRT
   <213> artificial
<220>
   <223> Peptide sequence
<400> 158
   Arg Val Ala Phe Gly Leu Val Cys Arg Pro Ser Arg Val Ala Phe Gly
   1               5                   10                  15
   Leu Val Cys Arg
               20
<210> 159
   <211> 20
   <212> PRT
   <213> artificial
<220>
   <223> Peptide sequence
<400> 159
   Arg Leu Gly Phe Val Phe Thr Leu Arg Pro Pro Arg Leu Gly Phe Val
   1               5                   10                  15
   Phe Thr Leu Arg
               20
<210> 160
   <211> 20
   <212> PRT
   <213> artificial
<220>
   <223> Peptide sequence
<400> 160
   Asp Leu Gly Phe Val Phe Thr Leu Asp Pro Pro Asp Leu Gly Phe Val
   1               5                   10                  15
   Phe Thr Leu Asp
               20
<210> 161
   <211> 20
   <212> PRT
   <213> artificial
<220>
   <223> Peptide sequence
<400> 161
   Arg Leu Gly Phe Val Phe Thr Leu Arg Pro Ser Arg Leu Gly Phe Val
   1               5                   10                  15
   Phe Thr Leu Arg
               20
<210> 162
   <211> 20
   <212> PRT
   <213> artificial
<220>
   <223> Peptide sequence
<400> 162
   Arg Ala Val Gly Val Leu Ile Gly Arg Pro Pro Arg Ala Val Gly Val
   1               5                   10                  15
   Leu Ile Gly Arg
               20
<210> 163
   <211> 20
   <212> PRT
   <213> artificial
<220>
   <223> Peptide sequence
<400> 163
   Arg Ala Val Gly Val Leu Ile Gly Arg Pro Ser Arg Ala Val Gly Val
   1               5                   10                  15
   Leu Ile Gly Arg
               20
<210> 164
   <211> 6
   <212> PRT
   <213> artificial
<220>
   <223> Peptide sequence
<400> 164
   Val His Ile Tyr Tyr Leu
   1               5
<210> 165
   <211> 7
   <212> PRT
   <213> artificial
<220>
   <223> Peptide sequence
<400> 165
   Asn Leu Tyr Gly Phe Ile Ile
   1               5
<210> 166
   <211> 7
   <212> PRT
   <213> artificial
<220>
   <223> Peptide sequence
<400> 166
   Arg Gly Phe Val Tyr Phe Val
   1               5
<210> 167
   <211> 7
   <212> PRT
   <213> artificial
<220>
   <223> Peptide sequence
<400> 167
   Met Ala Leu Gln Leu Phe Ile
   1               5
<210> 168
   <211> 8
   <212> PRT
   <213> artificial
<220>
   <223> Peptide sequence
<400> 168
   Leu Ala Val Thr Trp Trp Asn Arg
   1               5
<210> 169
   <211> 9
   <212> PRT
   <213> artificial
<220>
   <223> Peptide sequence
<400> 169
   Arg Gln Ser Leu Ile Ile Ala Ala Arg
   1 5
<210> 170
   <211> 7
   <212> PRT
   <213> artificial
<220>
   <223> Peptide sequence
<400> 170
   Gly Phe Leu Ile Leu Gly Arg
   1 5
<210> 171
   <211> 7
   <212> PRT
   <213> artificial
<220>
   <223> Peptide sequence
<400> 171
   Leu Met Val Ala Tyr Met Leu
   1 5
<210> 172
   <211> 7
   <212> PRT
   <213> artificial
<220>
   <223> Peptide sequence
<400> 172
   Leu Val Trp Met Ala Cys His
   1 5
<210> 173
   <211> 7
   <212> PRT
   <213> artificial
<220>
   <223> Peptide sequence
<400> 173
   Val Ala Phe Gly Leu Val Cys
   1 5
<210> 174
   <211> 7
   <212> PRT
   <213> artificial
<220>
   <223> Peptide sequence
<400> 174
   Leu Gly Phe Val Phe Thr Leu
   1               5
<210> 175
   <211> 11
   <212> PRT
   <213> artificial
<220>
   <223> Peptide sequence
<400> 175
   Phe Leu Asp Thr Leu Val Val Leu His Arg Ala
   1               5                   10
<210> 176
   <211> 8
   <212> PRT
   <213> artificial
<220>
   <223> Peptide sequence
<400> 176
   Arg Phe Phe Ile Ala Leu Ser Arg
   1               5
<210> 177
   <211> 8
   <212> PRT
   <213> artificial
<220>
   <223> Peptide sequence
<400> 177
   Arg Ile Leu Leu Gly Leu Ile Arg
   1               5
<210> 178
   <211> 6
   <212> PRT
   <213> artificial
<220>
   <223> Peptide sequence
<400> 178
   Thr Leu Val Val Leu His
   1 5
<210> 179
   <211> 20
   <212> PRT
   <213> artificial
<220>
   <223> Peptide sequence
<400> 179
   Val Ile Ile Trp Ser Leu Gly Asn Gly Ser Gly Ser Asp Tyr Lys Asp
   1 5 10 15
   Asp Asp Asp Lys
   20
<210> 180
   <211> 20
   <212> PRT
   <213> artificial
<220>
   <223> Peptide sequence
<400> 180
   Arg Asp Arg Asn His Pro Ser Val Ile Ile Trp Ser Leu Gly Asn Glu
   1 5 10 15
   Ser Gly His Gly
   20
<210> 181
   <211> 10
   <212> PRT
   <213> artificial
<220>
   <223> Peptide sequence
<400> 181
   Asp Val Ile Ile Trp Ser Leu Gly Asn Arg
   1 5 10
<210> 182
   <211> 9
   <212> PRT
   <213> artificial
<220>
   <223> Peptide sequence
<400> 182
   Arg Trp Gln Val Leu Val Ala Ser Asp
   1 5
<210> 183
   <211> 11
   <212> PRT
   <213> artificial
<220>
   <223> Peptide sequence
<400> 183
   Gln Pro Trp Gln Val Leu Val Ala Ser Arg Gly
   1 5 10
<210> 184
   <211> 13
   <212> PRT
   <213> artificial
<220>
   <223> Peptide sequence
<400> 184
   Arg Gln Gln Val Val Phe Ser Met Ser Phe Val Gln Asp
   1 5 10
<210> 185
   <211> 13
   <212> PRT
   <213> artificial
<220>
   <223> Peptide sequence
<400> 185
   Lys Gln Gln Val Val Phe Ser Met Ser Phe Val Gln Asp
   1 5 10
<210> 186
   <211> 13
   <212> PRT
   <213> artificial
<220>
   <223> Peptide sequence
<400> 186
   Arg Gly Leu Tyr Leu Ile Tyr Ser Gln Val Leu Phe Pro
   1 5 10
<210> 187
   <211> 13
   <212> PRT
   <213> artificial
<220>
   <223> Peptide sequence
<400> 187
   Arg Gly Leu Tyr Leu Ile Tyr Ser Gln Val Leu Phe His
   1               5                   10
<210> 188
   <211> 8
   <212> PRT
   <213> artificial
<220>
   <223> Peptide sequence
<400> 188
   Gly Ser Pro Gly Ser Pro Gly Ser
   1               5
<210> 189
   <211> 9
   <212> PRT
   <213> artificial
<220>
   <223> Peptide sequence
<400> 189
   Gly Ser Pro Gly Ser Pro Gly Ser Ala
   1               5
<210> 190
   <211> 13
   <212> PRT
   <213> artificial
<220>
   <223> Peptide sequence
<400> 190
   Gly Ser Pro Ser Val Ile Ile Trp Ser Leu Gly Asn Glu
   1               5                   10
<210> 191
   <211> 19
   <212> PRT
   <213> artificial
<220>
   <223> Peptide sequence
<400> 191
   Gly Ser Pro Gly Ser Pro Gly Ser Pro Ser Val Ile Ile Trp Ser Leu
   1               5                   10                  15
   Gly Asn Glu
<210> 192
   <211> 13
   <212> PRT
   <213> artificial
<220>
   <223> Peptide sequence
<400> 192
   Gly Ser Ala Arg Val Ile Ile Trp Ser Leu Gly Asn Arg
   1               5                   10
<210> 193
   <211> 10
   <212> PRT
   <213> artificial
<220>
   <223> Peptide sequence
<400> 193
   Gly Ser Glu Leu Phe Asn Phe Leu Lys Arg
   1               5                   10
<210> 194
   <211> 16
   <212> PRT
   <213> artificial
<220>
   <223> Peptide sequence
<400> 194
   Gly Ser Pro Gly Ser Pro Gly Ser Glu Leu Phe Asn Phe Leu Lys Arg
   1               5                   10                  15
<210> 195
   <211> 10
   <212> PRT
   <213> artificial
<220>
   <223> Peptide sequence
<400> 195
   Gly Ser Ala Arg Leu Phe Asn Phe Leu Arg
   1 5 10
<210> 196
   <211> 10
   <212> PRT
   <213> artificial
<220>
   <223> Peptide sequence
<400> 196
   Gly Ser Asp Trp Val Ser Met Leu Leu Arg
   1 5 10
<210> 197
   <211> 16
   <212> PRT
   <213> artificial
<220>
   <223> Peptide sequence
<400> 197
   Gly Ser Pro Gly Ser Pro Gly Ser Asp Trp Val Ser Met Leu Leu Arg
   1 5 10 15
<210> 198
   <211> 11
   <212> PRT
   <213> artificial
<220>
   <223> Peptide sequence
<400> 198
   Gly Ser Ala Arg Trp Val Ser Met Leu Leu Arg
   1 5 10
<210> 199
   <211> 12
   <212> PRT
   <213> artificial
<220>
   <223> Peptide sequence
<400> 199
   Gly Ser Arg Pro Ile Leu Thr Ile Ile Thr Leu Glu
   1 5 10
<210> 200
   <211> 18
   <212> PRT
   <213> artificial
<220>
   <223> Peptide sequence
<400> 200
   Gly Ser Pro Gly Ser Pro Gly Ser Arg Pro Ile Leu Thr Ile Ile Thr
   1               5                   10                  15
   Leu Glu
<210> 201
   <211> 12
   <212> PRT
   <213> artificial
<220>
   <223> Peptide sequence
<400> 201
   Gly Ser Ala Arg Ile Leu Thr Ile Ile Thr Leu Arg
   1               5                   10
<210> 202
   <211> 10
   <212> PRT
   <213> artificial
<220>
   <223> Peptide sequence
<400> 202
   Gly Ser Leu Asp Thr Leu Val Val Leu His
   1               5                   10
<210> 203
   <211> 16
   <212> PRT
   <213> artificial
<220>
   <223> Peptide sequence
<400> 203
   Gly Ser Pro Gly Ser Pro Gly Ser Leu Asp Thr Leu Val Val Leu His
   1               5                   10                  15
<210> 204
   <211> 11
   <212> PRT
   <213> artificial
<220>
   <223> Peptide sequence
<400> 204
   Gly Ser Ala Arg Thr Leu Val Val Leu His Arg
   1               5                   10
<210> 205
   <211> 15
   <212> PRT
   <213> artificial
<220>
   <223> Peptide sequence
<400> 205
   Gly Ser Glu Gly Leu Phe Trp Leu Leu Val Thr Gly His Ile Pro
   1               5                   10                  15
<210> 206
   <211> 21
   <212> PRT
   <213> artificial
<220>
   <223> Peptide sequence
<400> 206
   Gly Ser Pro Gly Ser Pro Gly Ser Glu Gly Leu Phe Trp Leu Leu Val
   1               5                   10                  15
   Thr Gly His Ile Pro
               20
<210> 207
   <211> 15
   <212> PRT
   <213> artificial
<220>
   <223> Peptide sequence
<400> 207
   Gly Ser Ala Arg Leu Phe Trp Leu Leu Val Thr Gly His Ile Arg
   1               5                   10                  15
<210> 208
   <211> 11
   <212> PRT
   <213> artificial
<220>
   <223> Peptide sequence
<400> 208
   Gly Ser Asn Glu Ile Leu Ile Phe Trp Ser Lys
   1 5 10
<210> 209
   <211> 17
   <212> PRT
   <213> artificial
<220>
   <223> Peptide sequence
<400> 209
   Gly Ser Pro Gly Ser Pro Gly Ser Asn Glu Ile Leu Ile Phe Trp Ser
   1 5 10 15
   Lys
<210> 210
   <211> 11
   <212> PRT
   <213> artificial
<220>
   <223> Peptide sequence
<400> 210
   Gly Ser Ala Arg Ile Leu Ile Phe Trp Ser Arg
   1 5 10
<210> 211
   <211> 11
   <212> PRT
   <213> artificial
<220>
   <223> Peptide sequence
<400> 211
   Gly Ser Pro Gln Trp Val Leu Thr Ala Ala His
   1 5 10
<210> 212
   <211> 17
   <212> PRT
   <213> artificial
<220>
   <223> Peptide sequence
<400> 212
   Gly Ser Pro Gly Ser Pro Gly Ser Pro Gln Trp Val Leu Thr Ala Ala
   1 5 10 15
   His
<210> 213
   <211> 12
   <212> PRT
   <213> artificial
<220>
   <223> Peptide sequence
<400> 213
   Gly Ser Ala Arg Gln Trp Val Leu Thr Ala Ala Arg
   1 5 10
<210> 214
   <211> 12
   <212> PRT
   <213> artificial
<220>
   <223> Peptide sequence
<400> 214
   Gly Ser Glu Val Met Phe Leu Thr Val Gln Val Lys
   1 5 10
<210> 215
   <211> 18
   <212> PRT
   <213> artificial
<220>
   <223> Peptide sequence
<400> 215
   Gly Ser Pro Gly Ser Pro Gly Ser Glu Val Met Phe Leu Thr Val Gln
   1 5 10 15
   Val Lys
<210> 216
   <211> 13
   <212> PRT
   <213> artificial
<220>
   <223> Peptide sequence
<400> 216
   Gly Ser Ala Arg Val Met Phe Leu Thr Val Gln Val Arg
   1 5 10
<210> 217
   <211> 12
   <212> PRT
   <213> artificial
<220>
   <223> Peptide sequence
<400> 217
   Gly Ser Asp Ala Phe Phe Leu His Met Leu Met Lys
   1 5 10
<210> 218
   <211> 18
   <212> PRT
   <213> artificial
<220>
   <223> Peptide sequence
<400> 218
   Gly Ser Pro Gly Ser Pro Gly Ser Asp Ala Phe Phe Leu His Met Leu
   1 5 10 15
   Met Lys
<210> 219
   <211> 13
   <212> PRT
   <213> artificial
<220>
   <223> Peptide sequence
<400> 219
   Gly Ser Ala Arg Ala Phe Phe Leu His Met Leu Met Arg
   1 5 10
<210> 220
   <211> 19
   <212> PRT
   <213> artificial
<220>
   <223> Peptide sequence
<400> 220
   Gly Ser Pro Gly Ser Pro Gly Ser Ala Arg Val Ile Ile Trp Ser Leu
   1 5 10 15
   Gly Asn Arg
<210> 221
   <211> 25
   <212> PRT
   <213> artificial
<220>
   <223> Peptide sequence
<400> 221
   Gly Ala Arg Val Ile Ile Trp Ser Leu Gly Asn Arg Gly Ser Ala Arg
   1 5 10 15
   Val Ile Ile Trp Ser Leu Gly Asn Arg
   20 25
<210> 222
   <211> 16
   <212> PRT
   <213> artificial
<220>
   <223> Peptide sequence
<400> 222
   Gly Ser Pro Gly Ser Pro Gly Ser Ala Arg Leu Phe Asn Phe Leu Arg
   1 5 10 15
<210> 223
   <211> 19
   <212> PRT
   <213> artificial
<220>
   <223> Peptide sequence
<400> 223
   Gly Ala Arg Leu Phe Asn Phe Leu Arg Gly Ser Ala Arg Leu Phe Asn
   1 5 10 15
   Phe Leu Arg
<210> 224
   <211> 17
   <212> PRT
   <213> artificial
<220>
   <223> Peptide sequence
<400> 224
   Gly Ser Pro Gly Ser Pro Gly Ser Ala Arg Trp Val Ser Met Leu Leu
   1               5                   10                  15
   Arg
<210> 225
   <211> 20
   <212> PRT
   <213> artificial
<220>
   <223> Peptide sequence
<400> 225
   Gly Ala Arg Trp Val Ser Met Leu Leu Arg Gly Ser Arg Trp Val Ser
   1               5                   10                  15
   Met Leu Leu Arg
               20
<210> 226
   <211> 18
   <212> PRT
   <213> artificial
<220>
   <223> Peptide sequence
<400> 226
   Gly Ser Pro Gly Ser Pro Gly Ser Ala Arg Ile Leu Thr Ile Ile Thr
   1               5                   10                  15
   Leu Arg
<210> 227
   <211> 23
   <212> PRT
   <213> artificial
<220>
   <223> Peptide sequence
<400> 227
   Gly Ala Ser Arg Ile Leu Thr Ile Ile Thr Leu Arg Gly Ser Arg Ile
   1               5                   10                  15
   Leu Thr Ile Ile Thr Leu Arg
               20
<210> 228
   <211> 17
   <212> PRT
   <213> artificial
<220>
   <223> Peptide sequence
<400> 228
   Gly Ser Pro Gly Ser Pro Gly Ser Ala Arg Thr Leu Val Val Leu His
   1               5                   10                  15
   Arg
<210> 229
   <211> 19
   <212> PRT
   <213> artificial
<220>
   <223> Peptide sequence
<400> 229
   Gly Ala Thr Leu Val Val Leu His Arg Gly Ser Arg Thr Leu Val Val
   1               5                   10                  15
   Leu His Arg
<210> 230
   <211> 21
   <212> PRT
   <213> artificial
<220>
   <223> Peptide sequence
<400> 230
   Gly Ser Pro Gly Ser Pro Gly Ser Ala Arg Leu Phe Trp Leu Leu Val
   1               5                   10                  15
   Thr Gly His Ile Arg
               20
<210> 231
   <211> 28
   <212> PRT
   <213> artificial
<220>
   <223> Peptide sequence
<400> 231
   Gly Ala Arg Leu Phe Trp Leu Leu Val Thr Gly His Ile Arg Gly Ser
   1               5                   10                  15
   Arg Leu Phe Trp Leu Leu Val Thr Gly His Ile Arg
               20                  25
<210> 232
   <211> 17
   <212> PRT
   <213> artificial
<220>
   <223> Peptide sequence
<400> 232
   Gly Ser Pro Gly Ser Pro Gly Ser Ala Arg Ile Leu Ile Phe Trp Ser
   1               5                   10                  15
   Arg
<210> 233
   <211> 21
   <212> PRT
   <213> artificial
<220>
   <223> Peptide sequence
<400> 233
   Gly Ala Ser Arg Ile Leu Ile Phe Trp Ser Arg Gly Ser Arg Ile Leu
   1               5                   10                  15
   Ile Phe Trp Ser Arg
               20
<210> 234
   <211> 18
   <212> PRT
   <213> artificial
<220>
   <223> Peptide sequence
<400> 234
   Gly Ser Pro Gly Ser Pro Gly Ser Ala Arg Gln Trp Val Leu Thr Ala
   1 5 10 15
   Ala Arg
<210> 235
   <211> 22
   <212> PRT
   <213> artificial
<220>
   <223> Peptide sequence
<400> 235
   Gly Ala Arg Gln Trp Val Leu Thr Ala Ala Arg Gly Ser Arg Gln Trp
   1 5 10 15
   Val Leu Thr Ala Ala Arg
   20
<210> 236
   <211> 19
   <212> PRT
   <213> artificial
<220>
   <223> Peptide sequence
<400> 236
   Gly Ser Pro Gly Ser Pro Gly Ser Ala Arg Val Met Phe Leu Thr Val
   1 5 10 15
   Gln Val Arg
<210> 237
   <211> 24
   <212> PRT
   <213> artificial
<220>
   <223> Peptide sequence
<400> 237
   Gly Ala Arg Val Met Phe Leu Thr Val Gln Val Arg Gly Ser Arg Val
   1 5 10 15
   Met Phe Leu Thr Val Gln Val Arg
   20
<210> 238
   <211> 19
   <212> PRT
   <213> artificial
<220>
   <223> Peptide sequence
<400> 238
   Gly Ser Pro Gly Ser Pro Gly Ser Ala Arg Ala Phe Phe Leu His Met
   1               5                   10                  15
   Leu Met Arg
<210> 239
   <211> 24
   <212> PRT
   <213> artificial
<220>
   <223> Peptide sequence
<400> 239
   Gly Ala Arg Ala Phe Phe Leu His Met Leu Met Arg Gly Ser Arg Ala
   1               5                   10                  15
   Phe Phe Leu His Met Leu Met Arg
               20
<210> 240
   <211> 20
   <212> PRT
   <213> artificial
<220>
   <223> Peptide sequence
<400> 240
   Arg Leu Ala Val Ala Leu Trp Phe Arg Pro Pro Arg Leu Ala Val Ala
   1               5                   10                  15
   Leu Trp Phe Arg
               20
<210> 241
   <211> 20
   <212> PRT
   <213> artificial
<220>
   <223> Peptide sequence
<400> 241
   Asp Leu Ala Val Ala Leu Trp Phe Asp Pro Pro Asp Leu Ala Val Ala
   1 5 10 15
   Leu Trp Phe Asp
   20
<210> 242
   <211> 20
   <212> PRT
   <213> artificial
<220>
   <223> Peptide sequence
<400> 242
   Arg Ile Ala Ser Thr Val Tyr Val Arg Pro Pro Arg Ile Ala Ser Thr
   1 5 10 15
   Val Tyr Val Arg
   20
<210> 243
   <211> 20
   <212> PRT
   <213> artificial
<220>
   <223> Peptide sequence
<400> 243
   Asp Ile Ala Ser Thr Val Tyr Val Asp Pro Pro Asp Ile Ala Ser Thr
   1 5 10 15
   Val Tyr Val Asp
   20
<210> 244
   <211> 20
   <212> PRT
   <213> artificial
<220>
   <223> Peptide sequence
<400> 244
   Arg Ile Leu Thr Ile Leu Ala Asn Arg Pro Pro Arg Ile Leu Thr Ile
   1 5 10 15
   Leu Ala Asn Arg
   20
<210> 245
   <211> 20
   <212> PRT
   <213> artificial
<220>
   <223> Peptide sequence
<400> 245
   Asp Ile Leu Thr Ile Leu Ala Asn Asp Pro Pro Asp Ile Leu Thr Ile
   1               5                   10                  15
   Leu Ala Asn Asp
               20
<210> 246
   <211> 20
   <212> PRT
   <213> artificial
<220>
   <223> Peptide sequence
<400> 246
   Arg Val Ile Ile Leu Val Gly Thr Arg Pro Pro Arg Val Ile Ile Leu
   1               5                   10                  15
   Val Gly Thr Arg
               20
<210> 247
   <211> 20
   <212> PRT
   <213> artificial
<220>
   <223> Peptide sequence
<400> 247
   Asp Val Ile Ile Leu Val Gly Thr Asp Pro Pro Asp Val Ile Ile Leu
   1               5                   10                  15
   Val Gly Thr Asp
               20
<210> 248
   <211> 20
   <212> PRT
   <213> artificial
<220>
   <223> Peptide sequence
<400> 248
   Arg Met Ile Ser Tyr Ala Gly Met Arg Pro Pro Arg Met Ile Ser Tyr
   1               5                   10                  15
   Ala Gly Met Arg
               20
<210> 249
   <211> 20
   <212> PRT
   <213> artificial
<220>
   <223> Peptide sequence
<400> 249
   Asp Met Ile Ser Tyr Ala Gly Met Asp Pro Pro Asp Met Ile Ser Tyr
   1               5                   10                  15
   Ala Gly Met Asp
               20
<210> 250
   <211> 20
   <212> PRT
   <213> artificial
<220>
   <223> Peptide sequence
<400> 250
   Arg Leu Met Val Ile Val Glu Phe Arg Pro Pro Arg Leu Met Val Ile
   1               5                   10                  15
   Val Glu Phe Arg
               20
<210> 251
   <211> 20
   <212> PRT
   <213> artificial
<220>
   <223> Peptide sequence
<400> 251
   Asp Leu Met Val Ile Val Glu Phe Asp Pro Pro Asp Leu Met Val Ile
   1 5 10 15
   Val Glu Phe Asp
   20
<210> 252
   <211> 20
   <212> PRT
   <213> artificial
<220>
   <223> Peptide sequence
<400> 252
   Arg Thr Val Ser Thr Leu Val Ile Arg Pro Pro Arg Thr Val Ser Thr
   1 5 10 15
   Leu Val Ile Arg
   20
<210> 253
   <211> 20
   <212> PRT
   <213> artificial
<220>
   <223> Peptide sequence
<400> 253
   Asp Thr Val Ser Thr Leu Val Ile Asp Pro Pro Asp Thr Val Ser Thr
   1 5 10 15
   Leu Val Ile Asp
   20
<210> 254
   <211> 20
   <212> PRT
   <213> artificial
<220>
   <223> Peptide sequence
<400> 254
   Arg Leu Ile Cys Tyr Ser Phe Gln Arg Pro Pro Arg Leu Ile Cys Tyr
   1 5 10 15
   Ser Phe Gln Arg
   20
<210> 255
   <211> 20
   <212> PRT
   <213> artificial
<220>
   <223> Peptide sequence
<400> 255
   Asp Leu Ile Cys Tyr Ser Phe Gln Asp Pro Pro Asp Leu Ile Cys Tyr
   1               5                   10                  15
   Ser Phe Gln Asp
               20
<210> 256
   <211> 20
   <212> PRT
   <213> artificial
<220>
   <223> Peptide sequence
<400> 256
   Arg Val Ile Ser Phe His Val Ile Arg Pro Pro Arg Val Ile Ser Phe
   1               5                   10                  15
   His Val Ile Arg
               20
<210> 257
   <211> 20
   <212> PRT
   <213> artificial
<220>
   <223> Peptide sequence
<400> 257
   Asp Val Ile Ser Phe His Val Ile Asp Pro Pro Asp Val Ile Ser Phe
   1               5                   10                  15
   His Val Ile Asp
               20
<210> 258
   <211> 20
   <212> PRT
   <213> artificial
<220>
   <223> Peptide sequence
<400> 258
   Arg Gly Tyr Leu Ser Ile Val Met Arg Pro Pro Arg Gly Tyr Leu Ser
   1               5                   10                  15
   Ile Val Met Arg
               20
<210> 259
   <211> 20
   <212> PRT
   <213> artificial
<220>
   <223> Peptide sequence
<400> 259
   Asp Gly Tyr Leu Ser Ile Val Met Asp Pro Pro Asp Gly Tyr Leu Ser
   1               5                   10                  15
   Ile Val Met Asp
               20
<210> 260
   <211> 20
   <212> PRT
   <213> artificial
<220>
   <223> Peptide sequence
<400> 260
   Arg Leu Ala Val Ala Leu Trp Phe Arg Pro Pro Arg Ile Ala Ser Thr
   1               5                   10                  15
   Val Tyr Val Arg
               20
<210> 261
   <211> 20
   <212> PRT
   <213> artificial
<220>
   <223> Peptide sequence
<400> 261
   Asp Leu Ala Val Ala Leu Trp Phe Asp Pro Pro Asp Ile Ala Ser Thr
   1               5                   10                  15
   Val Tyr Val Asp
               20
<210> 262
   <211> 20
   <212> PRT
   <213> artificial
<220>
   <223> Peptide sequence
<400> 262
   Arg Ile Ala Ser Thr Val Tyr Val Arg Pro Pro Arg Ile Leu Thr Ile
   1               5                   10                  15
   Leu Ala Asn Arg
               20
<210> 263
   <211> 20
   <212> PRT
   <213> artificial
<220>
   <223> Peptide sequence
<400> 263
   Asp Ile Ala Ser Thr Val Tyr Val Asp Pro Pro Asp Ile Leu Thr Ile
   1               5                   10                  15
   Leu Ala Asn Asp
               20
<210> 264
   <211> 20
   <212> PRT
   <213> artificial
<220>
   <223> Peptide sequence
<400> 264
   Arg Ile Leu Thr Ile Leu Ala Asn Arg Pro Pro Arg Val Ile Ile Leu
   1               5                   10                  15
   Val Gly Thr Arg
               20
<210> 265
   <211> 20
   <212> PRT
   <213> artificial
<220>
   <223> Peptide sequence
<400> 265
   Asp Ile Leu Thr Ile Leu Ala Asn Asp Pro Pro Asp Val Ile Ile Leu
   1               5                   10                  15
   Val Gly Thr Asp
               20
<210> 266
   <211> 20
   <212> PRT
   <213> artificial
<220>
   <223> Peptide sequence
<400> 266
   Arg Val Ile Ile Leu Val Gly Thr Arg Pro Pro Arg Met Ile Ser Tyr
   1               5                   10                  15
   Ala Gly Met Arg
               20
<210> 267
   <211> 20
   <212> PRT
   <213> artificial
<220>
   <223> Peptide sequence
<400> 267
   Asp Val Ile Ile Leu Val Gly Thr Asp Pro Pro Asp Met Ile Ser Tyr
   1               5                   10                  15
   Ala Gly Met Asp
               20
<210> 268
   <211> 20
   <212> PRT
   <213> artificial
<220>
   <223> Peptide sequence
<400> 268
   Arg Met Ile Ser Tyr Ala Gly Met Arg Pro Pro Arg Leu Met Val Ile
   1               5                   10                  15
   Val Glu Phe Arg
               20
<210> 269
   <211> 20
   <212> PRT
   <213> artificial
<220>
   <223> Peptide sequence
<400> 269
   Asp Met Ile Ser Tyr Ala Gly Met Asp Pro Pro Asp Leu Met Val Ile
   1               5                   10                  15
   Val Glu Phe Asp
               20
<210> 270
   <211> 20
   <212> PRT
   <213> artificial
<220>
   <223> Peptide sequence
<400> 270
   Arg Leu Met Val Ile Val Glu Phe Arg Pro Pro Arg Thr Val Ser Thr
   1               5                   10                  15
   Leu Val Ile Arg
               20
<210> 271
   <211> 20
   <212> PRT
   <213> artificial
<220>
   <223> Peptide sequence
<400> 271
   Asp Leu Met Val Ile Val Glu Phe Asp Pro Pro Asp Thr Val Ser Thr
   1               5                   10                  15
   Leu Val Ile Asp
   20
<210> 272
   <211> 20
   <212> PRT
   <213> artificial
<220>
   <223> Peptide sequence
<400> 272
   Arg Thr Val Ser Thr Leu Val Ile Arg Pro Pro Arg Leu Ile Cys Tyr
   1 5 10 15
   Ser Phe Gln Arg
   20
<210> 273
   <211> 20
   <212> PRT
   <213> artificial
<220>
   <223> Peptide sequence
<400> 273
   Asp Thr Val Ser Thr Leu Val Ile Asp Pro Pro Asp Leu Ile Cys Tyr
   1 5 10 15
   Ser Phe Gln Asp
   20
<210> 274
   <211> 20
   <212> PRT
   <213> artificial
<220>
   <223> Peptide sequence
<400> 274
   Arg Leu Ile Cys Tyr Ser Phe Gln Arg Pro Pro Arg Val Ile Ser Phe
   1 5 10 15
   His Val Ile Arg
   20
<210> 275
   <211> 20
   <212> PRT
   <213> artificial
<220>
   <223> Peptide sequence
<400> 275
   Asp Leu Ile Cys Tyr Ser Phe Gln Asp Pro Pro Asp Val Ile Ser Phe
   1               5                   10                  15
   His Val Ile Asp
               20
<210> 276
   <211> 20
   <212> PRT
   <213> artificial
<220>
   <223> Peptide sequence
<400> 276
   Arg Val Ile Ser Phe His Val Ile Arg Pro Pro Arg Gly Tyr Leu Ser
   1               5                   10                  15
   Ile Val Met Arg
               20
<210> 277
   <211> 20
   <212> PRT
   <213> artificial
<220>
   <223> Peptide sequence
<400> 277
   Asp Val Ile Ser Phe His Val Ile Asp Pro Pro Asp Gly Tyr Leu Ser
   1               5                   10                  15
   Ile Val Met Asp
               20
<210> 278
   <211> 7
   <212> PRT
   <213> artificial
<220>
   <223> Peptide sequence
<400> 278
   Leu Ala Val Ala Leu Trp Phe
   1 5
<210> 279
   <211> 7
   <212> PRT
   <213> artificial
<220>
   <223> Peptide sequence
<400> 279
   Met Ile Ser Tyr Ala Gly Met
   1 5
<210> 280
   <211> 20
   <212> PRT
   <213> artificial
<220>
   <223> Peptide sequence
<400> 280
   Arg Ser Leu Thr Ser Thr Val Gln Arg Pro Pro Arg Ser Leu Thr Ser
   1 5 10 15
   Thr Val Gln Arg
   20
<210> 281
   <211> 20
   <212> PRT
   <213> artificial
<220>
   <223> Peptide sequence
<400> 281
   Asp Ser Leu Thr Ser Thr Val Gln Asp Pro Pro Asp Ser Leu Thr Ser
   1 5 10 15
   Thr Val Gln Asp
   20
<210> 282
   <211> 20
   <212> PRT
   <213> artificial
<220>
   <223> Peptide sequence
<400> 282
   Arg Val Trp Ser Tyr Gly Val Thr Arg Pro Pro Arg Val Trp Ser Tyr
     1             5                  10                  15
   Gly Val Thr Arg
               20
<210> 283
   <211> 20
   <212> PRT
   <213> artificial
<220>
   <223> Peptide sequence
<400> 283
   Arg Ile Thr Gly Tyr Leu Tyr Ile Arg Pro Pro Arg Ile Thr Gly Tyr
   1               5                   10                  15
   Leu Tyr Ile Arg
               20
<210> 284
   <211> 20
   <212> PRT
   <213> artificial
<220>
   <223> Peptide sequence
<400> 284
   Arg Leu Leu Gly Ile Ser Leu Thr Arg Pro Pro Arg Leu Leu Gly Ile
   1               5                   10                  15
   Ser Leu Thr Arg
               20
<210> 285
   <211> 20
   <212> PRT
   <213> artificial
<220>
   <223> Peptide sequence
<400> 285
   Arg Ser Thr Val Gln Leu Val Thr Arg Pro Pro Arg Ser Thr Val Gln
   1               5                   10                  15
   Leu Val Thr Arg
               20
<210> 286
   <211> 20
   <212> PRT
   <213> artificial
<220>
   <223> Peptide sequence
<400> 286
   Asp Ser Thr Val Gln Leu Val Thr Asp Pro Pro Asp Ser Thr Val Gln
   1               5                   10                  15
   Leu Val Thr Asp
               20
<210> 287
   <211> 20
   <212> PRT
   <213> artificial
<220>
   <223> Peptide sequence
<400> 287
   Arg Leu Gly Val Val Phe Gly Ile Arg Pro Pro Arg Leu Gly Val Val
   1               5                   10                  15
   Phe Gly Ile Arg
               20
<210> 288
   <211> 20
   <212> PRT
   <213> artificial
<220>
   <223> Peptide sequence
<400> 288
   Asp Leu Gly Val Val Phe Gly Ile Asp Pro Pro Asp Leu Gly Val Val
   1               5                   10                  15
   Phe Gly Ile Asp
               20
<210> 289
   <211> 20
   <212> PRT
   <213> artificial
<220>
   <223> Peptide sequence
<400> 289
   Arg Ser Tyr Gly Val Thr Val Trp Arg Pro Pro Arg Ser Tyr Gly Val
   1 5 10 15
   Thr Val Trp Arg
   20
<210> 290
   <211> 20
   <212> PRT
   <213> artificial
<220>
   <223> Peptide sequence
<400> 290
   Asp Ser Tyr Gly Val Thr Val Trp Asp Pro Pro Asp Ser Tyr Gly Val
   1 5 10 15
   Thr Val Trp Asp
   20
<210> 291
   <211> 20
   <212> PRT
   <213> artificial
<220>
   <223> Peptide sequence
<400> 291
   Arg Ser Ala Val Val Gly Ile Leu Arg Pro Pro Arg Ser Ala Val Val
   1 5 10 15
   Gly Ile Leu Arg
   20
<210> 292
   <211> 20
   <212> PRT
   <213> artificial
<220>
   <223> Peptide sequence
<400> 292
   Asp Ser Ala Val Val Gly Ile Leu Asp Pro Pro Asp Ser Ala Val Val
   1 5 10 15
   Gly Ile Leu Asp
   20
<210> 293
   <211> 20
   <212> PRT
   <213> artificial
<220>
   <223> Peptide sequence
<400> 293
   Arg Gly Tyr Leu Tyr Ile Ser Ala Arg Pro Pro Arg Gly Tyr Leu Tyr
   1 5 10 15
   Ile Ser Ala Arg
   20
<210> 294
   <211> 20
   <212> PRT
   <213> artificial
<220>
   <223> Peptide sequence
<400> 294
   Asp Gly Tyr Leu Tyr Ile Ser Ala Asp Pro Pro Asp Gly Tyr Leu Tyr
   1 5 10 15
   Ile Ser Ala Asp
   20
<210> 295
   <211> 20
   <212> PRT
   <213> artificial
<220>
   <223> Peptide sequence
<400> 295
   Asp Thr Gly Tyr Leu Tyr Ile Ser Asp Pro Pro Asp Thr Gly Tyr Leu
   1 5 10 15
   Tyr Ile Ser Asp
   20
<210> 296
   <211> 20
   <212> PRT
   <213> artificial
<220>
   <223> Peptide sequence
<400> 296
   Asp Ile Ile Ser Ala Val Val Gly Asp Pro Pro Asp Ile Ile Ser Ala
   1 5 10 15
   Val Val Gly Asp
   20
<210> 297
   <211> 18
   <212> PRT
   <213> artificial
<220>
   <223> Peptide sequence
<400> 297
   Arg Val Tyr Met Ile Met Val Arg Pro Pro Arg Val Tyr Met Ile Met
   1 5 10 15
   Val Arg
<210> 298
   <211> 18
   <212> PRT
   <213> artificial
<220>
   <223> Peptide sequence
<400> 298
   Asp Val Tyr Met Ile Met Val Asp Pro Pro Asp Val Tyr Met Ile Met
   1 5 10 15
   Val Asp
<210> 299
   <211> 20
   <212> PRT
   <213> artificial
<220>
   <223> Peptide sequence
<400> 299
   Arg Val Val Gly Ile Leu Leu Val Arg Pro Pro Arg Val Val Gly Ile
   1               5                   10                  15
   Leu Leu Val Arg
               20
<210> 300
   <211> 20
   <212> PRT
   <213> artificial
<220>
   <223> Peptide sequence
<400> 300
   Asp Val Val Gly Ile Leu Leu Val Asp Pro Pro Asp Val Val Gly Ile
   1               5                   10                  15
   Leu Leu Val Asp
               20
<210> 301
   <211> 20
   <212> PRT
   <213> artificial
<220>
   <223> Peptide sequence
<400> 301
   Arg Ala Val Val Gly Ile Leu Leu Arg Pro Pro Arg Ala Val Val Gly
   1               5                   10                  15
   Ile Leu Leu Arg
               20
<210> 302
   <211> 20
   <212> PRT
   <213> artificial
<220>
   <223> Peptide sequence
<400> 302
   Asp Ala Val Val Gly Ile Leu Leu Asp Pro Pro Asp Ala Val Val Gly
   1               5                   10                  15
   Ile Leu Leu Asp
               20
<210> 303
   <211> 20
   <212> PRT
   <213> artificial
<220>
   <223> Peptide sequence
<400> 303
   Arg Val Leu Gly Val Val Phe Gly Arg Pro Pro Arg Val Leu Gly Val
   1               5                   10                  15
   Val Phe Gly Arg
               20
<210> 304
   <211> 20
   <212> PRT
   <213> artificial
<220>
   <223> Peptide sequence
<400> 304
   Asp Val Leu Gly Val Val Phe Gly Asp Pro Pro Asp Val Leu Gly Val
   1               5                   10                  15
   Val Phe Gly Asp
               20
<210> 305
   <211> 20
   <212> PRT
   <213> artificial
<220>
   <223> Peptide sequence
<400> 305
   Arg Val Val Phe Gly Ile Leu Ile Arg Pro Pro Arg Val Val Phe Gly
   1               5                   10                  15
   Ile Leu Ile Arg
               20
<210> 306
   <211> 20
   <212> PRT
   <213> artificial
<220>
   <223> Peptide sequence
<400> 306
   Asp Val Val Phe Gly Ile Leu Ile Asp Pro Pro Asp Val Val Phe Gly
   1               5                   10                  15
   Ile Leu Ile Asp
               20
<210> 307
   <211> 20
   <212> PRT
   <213> artificial
<220>
   <223> Peptide sequence
<400> 307
   Arg Gly Val Val Phe Gly Ile Leu Arg Pro Pro Arg Gly Val Val Phe
   1               5                   10                  15
   Gly Ile Leu Arg
               20
<210> 308
   <211> 20
   <212> PRT
   <213> artificial
<220>
   <223> Peptide sequence
<400> 308
   Asp Gly Val Val Phe Gly Ile Leu Asp Pro Pro Asp Gly Val Val Phe
   1               5                   10                  15
   Gly Ile Leu Asp
               20
<210> 309
   <211> 7
   <212> PRT
   <213> artificial
<220>
   <223> Peptide sequence
<400> 309
   Ile His Ile Phe Val Leu Ser
   1 5
<210> 310
   <211> 7
   <212> PRT
   <213> artificial
<220>
   <223> Peptide sequence
<400> 310
   His Ile Phe Val Leu Ser Asn
   1 5
<210> 311
   <211> 7
   <212> PRT
   <213> artificial
<220>
   <223> Peptide sequence
<400> 311
   Ile Phe Val Leu Ser Asn Ile
   1 5
<210> 312
   <211> 7
   <212> PRT
   <213> artificial
<220>
   <223> Peptide sequence
<400> 312
   Phe Val Leu Ser Asn Ile Leu
   1 5
<210> 313
   <211> 5
   <212> PRT
   <213> artificial
<220>
   <223> Peptide sequence
<400> 313
   Ile Ile Val Ile Ser
   1 5
<210> 314
   <211> 5
   <212> PRT
   <213> artificial
<220>
   <223> Peptide sequence
<400> 314
   Tyr Leu Met Val Ile
   1 5
<210> 315
   <211> 7
   <212> PRT
   <213> artificial
<220>
   <223> Peptide sequence
<400> 315
   Phe Ser Thr Leu Ser Phe Ile
   1 5
<210> 316
   <211> 10
   <212> PRT
   <213> artificial
<220>
   <223> Peptide sequence
<400> 316
   Ile His Ile Phe Val Leu Ser Asn Ile Leu
   1 5 10
<210> 317
   <211> 28
   <212> PRT
   <213> artificial
<220>
   <223> Peptide sequence
<400> 317
   Arg Gly Leu Tyr Leu Ile Tyr Ser Gln Val Leu Phe Asp Pro Pro Arg
   1 5 10 15
   Gly Leu Tyr Leu Ile Tyr Ser Gln Val Leu Phe Asp
   20 25
<210> 318
   <211> 28
   <212> PRT
   <213> artificial
<220>
   <223> Peptide sequence
<400> 318
   Arg Gly Leu Tyr Leu Ile Tyr Ser Gln Val Leu Phe Arg Pro Pro Arg
   1 5 10 15
   Gly Leu Tyr Leu Ile Tyr Ser Gln Val Leu Phe Arg
   20 25
<210> 319
   <211> 28
   <212> PRT
   <213> artificial
<220>
   <223> Peptide sequence
<400> 319
   Arg Gly Leu Tyr Leu Ile Tyr Ser Gln Val Leu Phe Pro Pro Pro Arg
   1 5 10 15
   Gly Leu Tyr Leu Ile Tyr Ser Gln Val Leu Phe Pro
   20 25
<210> 320
   <211> 28
   <212> PRT
   <213> artificial
<220>
   <223> Peptide sequence
<400> 320
   Arg Gly Leu Tyr Leu Ile Tyr Ser Gln Val Leu Phe His Pro Pro Arg
   1 5 10 15
   Gly Leu Tyr Leu Ile Tyr Ser Gln Val Leu Phe His
   20 25
<210> 321
   <211> 9
   <212> PRT
   <213> artificial
<220>
   <223> Peptide sequence
<400> 321
   Gln Leu Val Glu Ile Ile Lys Val Leu
   1               5
<210> 322
   <211> 5
   <212> PRT
   <213> artificial
<220>
   <223> Peptide sequence
<400> 322
   Glu Asn Ala Val Asp
   1               5
<210> 323
   <211> 7
   <212> PRT
   <213> artificial
<220>
   <223> Peptide sequence
<400> 323
   Gly Thr Pro Thr Arg Glu Glu
   1               5
<210> 324
   <211> 15
   <212> PRT
   <213> artificial
<220>
   <223> Peptide sequence
<400> 324
   Ala Gly Ser Pro Lys Gly Ala Pro Ala Ala Lys Gly Ser Gly Ala
   1               5                   10                  15
<210> 325
   <211> 13
   <212> PRT
   <213> artificial
<220>
   <223> Peptide sequence
<400> 325
   Arg Val Val Gly Thr Gly Ser Phe Gly Ile Val Phe Lys
   1               5                   10
<210> 326
   <211> 11
   <212> PRT
   <213> artificial
<220>
   <223> Peptide sequence
<400> 326
   Arg Gln Leu Val Glu Ile Ile Lys Val Leu Arg
   1               5                   10
<210> 327
   <211> 28
   <212> PRT
   <213> artificial
<220>
   <223> Peptide sequence
<400> 327
   Gln Trp Gln Asn Ser Thr Leu Ile Val Leu Gln Asn Ser Thr Val Ile
   1               5                   10                  15
   Phe Glu Gln Asn Ser Thr Val Ile Phe Glu Gln Asn
               20                  25
<210> 328
   <211> 21
   <212> PRT
   <213> artificial
<220>
   <223> Peptide sequence
<400> 328
   Glu Asn Ala Val Asp Gln Leu Val Glu Ile Ile Lys Val Leu Gly Thr
   1               5                   10                  15
   Pro Thr Arg Glu Glu
               20
<210> 329
   <211> 6
   <212> PRT
   <213> artificial
<220>
   <223> Peptide sequence
<400> 329
   Met Ala Asp Asp Lys Glu
   1               5
<210> 330
   <211> 42
   <212> PRT
   <213> artificial
<220>
   <223> Peptide sequence
<400> 330
   Glu Asn Ala Val Asp Gln Leu Val Glu Ile Ile Lys Val Leu Gly Thr
   1               5                   10                  15
   Pro Thr Arg Glu Glu Glu Asn Ala Val Asp Gln Leu Val Glu Ile Ile
               20                  25                  30
   Lys Val Leu Gly Thr Pro Thr Arg Glu Glu
           35                  40
<210> 331
   <211> 23
   <212> DNA
   <213> artificial
<220>
   <223> Primer
<400> 331
   gtgatctcag ccgtacattt gga 23
<210> 332
   <211> 24
   <212> DNA
   <213> artificial
<220>
   <223> Primer
<400> 332
   cacgtcgaaa aactaccact tcct 24
<210> 333
   <211> 20
   <212> DNA
   <213> artificial
<220>
   <223> Primer
<400> 333
   gaatggagtg attacaagtc 20
<210> 334
   <211> 22
   <212> DNA
   <213> artificial
<220>
   <223> Primer
<400> 334
   gtgaacacat tagaagggcc tg 22
<210> 335
   <211> 21
   <212> DNA
   <213> artificial
<220>
   <223> Primer
<400> 335
   ctcatttgcc aatcctgtat c 21
<210> 336
   <211> 21
   <212> DNA
   <213> artificial
<220>
   <223> Primer
<400> 336
   gcactgagat gcgacatctt g 21
<210> 337
   <211> 22
   <212> DNA
   <213> artificial
<220>
   <223> Primer
<400> 337
   tgactcttat ccgcttgaac ag 22
<210> 338
   <211> 21
   <212> DNA
   <213> artificial
<220>
   <223> Primer
<400> 338
   tcctacgttg ctttcactga c 21
<210> 339
   <211> 22
   <212> DNA
   <213> artificial
<220>
   <223> Primer
<400> 339
   gtggttcctt cactgtcact ag 22
<210> 340
   <211> 22
   <212> DNA
   <213> artificial
<220>
   <223> Primer
<400> 340
   ttcaccaagt ctttgagtct cc 22
<210> 341
   <211> 20
   <212> DNA
   <213> artificial
<220>
   <223> Primer
<400> 341
   tgaaaggaag aggatgcagc 20
<210> 342
   <211> 22
   <212> DNA
   <213> artificial
<220>
   <223> Primer
<400> 342
   tgtatttcat cgtgagaggc tg 22
<210> 343
   <211> 20
   <212> DNA
   <213> artificial
<220>
   <223> Primer
<400> 343
   gctattctca gcggtgaagg 20
<210> 344
   <211> 21
   <212> DNA
   <213> artificial
<220>
   <223> Primer
<400> 344
   ttctcgtctt ggatggtgtt c 21
<210> 345
   <211> 20
   <212> DNA
   <213> artificial
<220>
   <223> Primer
<400> 345
   gaaacagaac cactccctcg 20
<210> 346
   <211> 21
   <212> DNA
   <213> artificial
<220>
   <223> Primer
<400> 346
   ccaatcaacc tctttgcatc g 21
<210> 347
   <211> 21
   <212> DNA
   <213> artificial
<220>
   <223> Primer
<400> 347
   aacagaacca caccgtctta c 21
<210> 348
   <211> 21
   <212> DNA
   <213> artificial
<220>
   <223> Primer
<400> 348
   accaatcaac ctcttcgcat c 21
<210> 349
   <211> 24
   <212> DNA
   <213> artificial
<220>
   <223> Primer
<400> 349
   attgcgtatt gccactctca tagg 24
<210> 350
   <211> 22
   <212> DNA
   <213> artificial
<220>
   <223> Primer
<400> 350
   tcctgacagg gataccgaat gc 22
<210> 351
   <211> 22
   <212> DNA
   <213> artificial
<220>
   <223> Primer
<400> 351
   ctggaggttt tgaggctggt at 22
<210> 352
   <211> 21
   <212> DNA
   <213> artificial
<220>
   <223> Primer
<400> 352
   ccaaggctga aagcaagaag a 21